(19) Europäisches Patentamt
European Patent Office
Office européen des brevets

(11) **EP 2 830 397 A1**

(12) **EUROPEAN PATENT APPLICATION**
published in accordance with Art. 153(4) EPC

(43) Date of publication:
**28.01.2015 Bulletin 2015/05**

(21) Application number: **13764183.3**

(22) Date of filing: **11.03.2013**

(51) Int Cl.:
***H05B 33/12*** (2006.01) ***C07D 213/06*** (2006.01)
***C07D 213/22*** (2006.01) ***C07D 213/53*** (2006.01)
***C07D 401/14*** (2006.01) ***C07D 409/14*** (2006.01)
***C07D 519/00*** (2006.01) ***H01L 51/50*** (2006.01)
***H05B 33/28*** (2006.01)

(86) International application number:
**PCT/JP2013/056616**

(87) International publication number:
**WO 2013/141057 (26.09.2013 Gazette 2013/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.03.2012 JP 2012064229**

(71) Applicant: **Konica Minolta, Inc.**
**Tokyo 100-7015 (JP)**

(72) Inventors:
- **ISHIDAI, Hiroshi**
**Hachioji-shi**
**Tokyo 1928505 (JP)**
- **HAKII, Takeshi**
**Hachioji-shi**
**Tokyo 1928505 (JP)**
- **KINOSHITA, Toshiyuki**
**Hachioji-shi**
**Tokyo 1928505 (JP)**
- **YOSHIDA, Kazuhiro**
**Hachioji-shi**
**Tokyo 1928505 (JP)**
- **TSUJIMURA, Takatoshi**
**Hachioji-shi,**
**Tokyo 1928505 (JP)**

(74) Representative: **Gille Hrabal**
**Brucknerstrasse 20**
**40593 Düsseldorf (DE)**

(54) **ORGANIC ELECTROLUMINESCENCE ELEMENT**

(57) An organic electroluminescence element includes: a first electrode and a second electrode, a plurality of light emitting units stacked one on top of another between the first electrode and the second electrode and each having a light emitting layer formed by using an organic material, and a transparent conductive layer arranged between the plurality of light emitting units. The transparent conductive layer is formed by using silver or an alloy containing silver as a main component, and is arranged adjacent to a nitrogen-containing layer formed by using a compound containing nitrogen atom.

*FIG. 2*

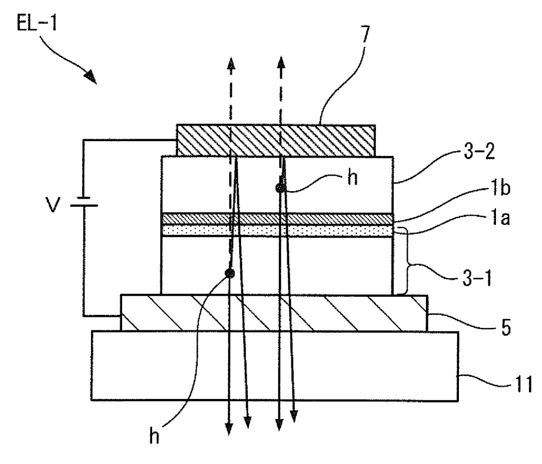


EP 2 830 397 A1

## Description

### Technical Field

The present invention relates to an organic electroluminescence element, and more particularly, relates to an organic electroluminescence element with a tandem structure in which a plurality of light emitting units, each having a light emitting layer formed by using an organic material, are stacked one on top of another.

### Background Art

In an organic electroluminescence element that uses electroluminescence (referred to as EL hereinafter) of an organic material (i.e., a so-called "organic EL element"), luminous efficiency and drive lifetime are in a relationship of trade-off. Thus, a tandem structure is proposed in which a light-emitting functional layer that includes a light emitting layer formed by using an organic material is regarded as a light emitting unit, and a plurality of such light emitting units are laminated to each other through an intermediate layer(s), to thereby ensure luminous efficiency while increasing lifetime. As the intermediate layer of such a tandem structure, various configurations are disclosed in Patent Documents 1 to 6, for example.

"Patent Document 1" discloses a configuration in which a conductive layer containing magnesium (Mg), as a main component thereof, and silver (Ag) is used in a floating state. "Patent Document 2" discloses a configuration in which a laminate obtained by laminating a layer of metal having small work function (such as Mg, Mg/Ag, As or the like) and indium tin oxide ($SnO_2$-$In_2O_3$: ITO) is used as an intermediate electrode. "Patent Document 3" discloses a configuration in which a charge-generating layer configured by an electrical insulating layer is used as an intermediate layer, and a configuration in which a hole injecting layer having an electron-accepting compound doped thereinto is formed as an intermediate layer connected to the cathode side of the charge-generating layer. "Patent Document 4" discloses an intermediate layer in which the composition ratio of an additive (an electron-releasing additive or an electron-attracting additive) in an inorganic or organic semiconductor material varies in the thickness direction. "Patent Document 5" discloses a configuration in which a three-layer laminate obtained by laminating an electron injecting layer, a metal-organic material mixed layer (for example, Ag-Alq3), and a hole injecting layer is used as an intermediate layer. "Patent Document 6" discloses a configuration in which a transparent conductive film, such as an ITO film, is used as a charge-generating layer (i.e., an intermediate layer), and a material less susceptible to be etched (such as a pyridine derivative, a benzoxazole derivative or the like) is used to form a layer closest to the charge-generating layer in an electroluminescent layer.

### Prior art documents

### Patent documents

Patent Document 1: US6337492(B1)
Patent Document 2: Japanese Patent No. 3496681
Patent Document 3: Japanese Unexamined Patent Application Publication No.2003-272860
Patent Document 4: Japanese Unexamined Patent Application Publication No.2005-135600
Patent Document 5: Japanese Unexamined Patent Application Publication No.2006-332048
Patent Document 6: Japanese Unexamined Patent Application Publication No.2005-340187

### Disclosure of the Invention

### Problems to be Solved by the Invention

As described above, with respect to the organic electroluminescence element with a tandem structure, various configurations have been proposed. However, even with the aforesaid various configurations, it is still difficult to achieve sufficient luminous efficiency and lifetime characteristic for practical use.

In view of the aforesaid problems, it is an object of the present invention to provide an organic electroluminescence element with a tandem structure capable of improving both the luminous efficiency and the lifetime characteristic.

### Means for Solving the Problems

The aforesaid object of the present invention is achieved by the following configurations

1. An organic electroluminescence element comprising: a pair of electrodes; a plurality of light emitting units stacked one on top of another between the pair of electrodes and each having a light emitting layer formed by using an organic material; and a conductive layer arranged between the plurality of light emitting units,
wherein at least one of the electrodes and the conductive layer is configured as a transparent conductive layer formed by using silver or an alloy containing silver as a main component, and is arranged adjacent to a nitrogen-containing layer formed by using a compound containing nitrogen atom.
2. The organic electroluminescence element according to configuration 1, wherein the compound is a compound whose effective action energy ΔEef between itself and silver represented by the following Formula (1) satisfies the following Formula (2):
[Mathematical Expression 1]

$$\Delta Eef = n \times \Delta E / s \quad \cdots\cdots (1)$$

n : Number of nitrogen atom(s) contained in compound and stably bonded with silver
ΔE : Interaction energy between nitrogen atom (N) and silver (Ag)
s : Surface area of compound

$$-0.5 \leqq \Delta Eef \leqq -0.10\ [\mathrm{kcal/mol \cdot \AA^2}] \quad \cdots\cdots (2)$$

3. The organic electroluminescence element according to configuration 1 or 2, wherein the compound contains a compound represented by the following General Formula (1),

[Chemical Formula 1]

General Formula(1)

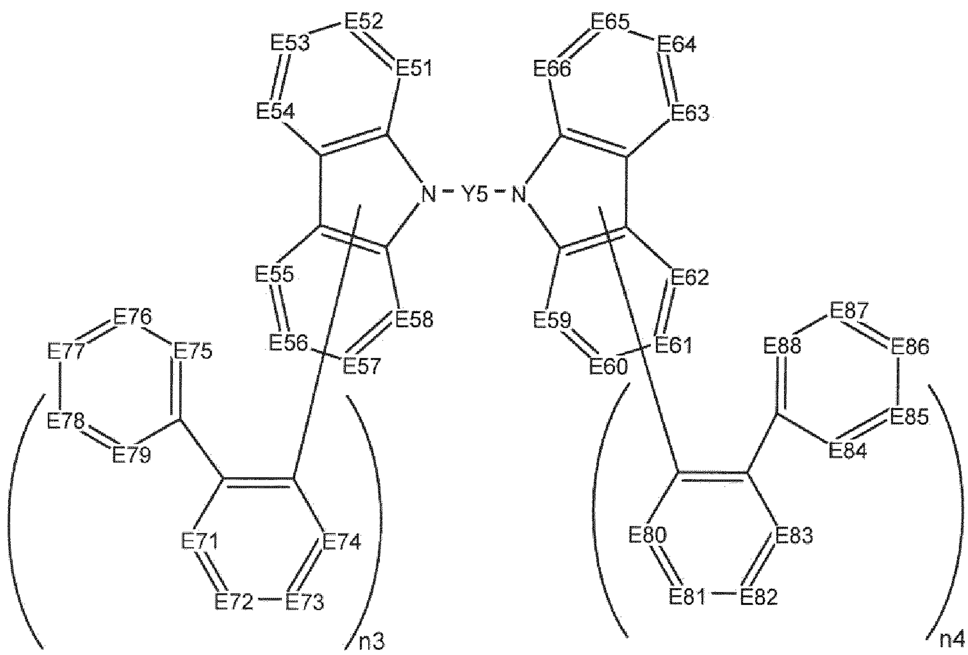

where

Y5 represents a divalent linking group which is an arylene group, a heteroarylene group or a combination of the arylene group and the heteroarylene group;
E51 to E66 and E71 to E88 each represent -C(R3)= or -N=, wherein R3 represents a hydrogen atom or a substituent, and wherein at least one of E71 to E79 and at least one of E80 to E88 each represent -N=; and

n3 and n4 each represent an integer of 0 to 4, wherein the sum of n3 and n4 is an integer of 2 or more.

4. The organic electroluminescence element according to configuration 1 or 2, wherein the compound contains a compound represented by the following General Formula (2),

[Chemical Formula 2]

General Formula (2)

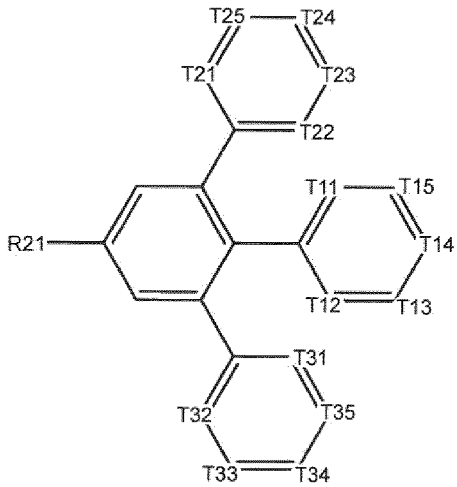

where

R21 represents a substituent;
T11, T12, T21 to T25, and T31 to T35 each represent - C(R22)= or -N=; and
T13 to T15 each represent -C(R22)=,
wherein R22 represents a hydrogen atom (H) or a substituent, at least one of T11 and T12 represents -N=, at least one of T21 to T25 represents -N=, and at least one of T31 to T35 represents -N=.

5. The organic electroluminescence element according to any one of configurations 1 to 4, wherein the organic electroluminescence element is driven by applying a voltage to the pair of electrodes only.
6. The organic electroluminescence element according to any one of configurations 1 to 4, wherein the organic electroluminescence element is driven by applying voltages to the pair of electrodes and the conductive layer.

The organic electroluminescence element of the present invention described above has a configuration in which a transparent conductive layer formed by using silver or an alloy containing silver as a main component is used as a conductive layer arranged between the electrodes and/or between the light emitting units, and a nitrogen-containing layer formed by using a compound containing nitrogen atom is arranged adjacent to the transparent conductive layer. Thus, when forming the transparent conductive layer adjacent to the nitrogen-containing layer, the silver atom which constituting the transparent conductive layer will interact with the nitrogen-atom-containing compound constituting the nitrogen-containing layer, so that diffusion distance of silver atom on the surface of the nitrogen-containing layer is reduced, and therefore aggregation of silver is inhibited. Therefore, a silver thin-film will be formed in a manner in which the silver thin-film grows in a single-layer growth mode (Frank-van der Merwe: FW mode); while in contrast, the silver thin-film is generally formed in a manner in which the silver thin-film grows in a nuclear growth mode (Volumer-Weber: VW mode) and thereby the silver thin-film tends to be isolated into an island shape. Thus, it is possible to obtain a transparent conductive layer which has thin yet uniform film-thickness. As a result, the transparent conductive layer, as a thinner film whose light transmissibility is maintained and whose electrical conductivity is ensured, can be used as a conductive layer arranged between the electrodes and/or between the light emitting units.

## Advantages of the Invention

As described above, according to the present invention, since the transparent conductive layer, as a thinner film whose light transmissibility is maintained and whose electrical conductivity is ensured, can be used as a conductive layer arranged between the electrodes and/or between the light emitting units, it becomes possible to provide an organic

electroluminescence element with a tandem structure capable of improving both the luminous efficiency and the lifetime characteristic.

## Brief Description of Drawings

FIG. 1 is a view schematically showing a cross-sectional configuration of a transparent conductive layer used in an organic electroluminescence element according to the present invention;
FIG. 2 is a cross-sectional view schematically showing a configuration of an organic electroluminescence element according to a first embodiment;
FIG. 3 is a schematic cross-sectional view for explaining Modification 1 of the first embodiment;
FIG. 4 is a schematic cross-sectional view for explaining Modification 2 of the first embodiment;
FIG. 5 is a cross-sectional view schematically showing a configuration of an organic electroluminescence element according to a second embodiment;
FIG. 6 is a cross-sectional view schematically showing a configuration of an organic electroluminescence element according to a third embodiment;
FIG. 7 is a cross-sectional view schematically showing a configuration of an organic electroluminescence element according to a fourth embodiment;
FIG. 8 is a cross-sectional view schematically showing a configuration of an organic electroluminescence element according to a fifth embodiment;
FIG. 9 is a cross-sectional view schematically showing a configuration of an organic electroluminescence element according to a sixth embodiment;
FIG. 10 is a graph showing a relationship between effective action energy $\Delta Eef$ and sheet resistance of each transparent electrode produced in Example 1;
FIG. 11 is a cross-sectional view schematically showing a configuration of an organic electroluminescence element produced in Example 2;
FIG. 12 is a cross-sectional view schematically showing a configuration of a comparison of Example 2;
FIG. 13 is a cross-sectional view schematically showing a configuration of an organic electroluminescence element produced in Example 3;
FIG. 14 is a cross-sectional view schematically showing a configuration of a comparison of Example 3;
FIG. 15 is a cross-sectional view schematically showing a configuration of an organic electroluminescence element produced in Example 4;
FIG. 16 is a cross-sectional view schematically showing a configuration of a comparison of Example 4;
FIG. 17 is a cross-sectional view schematically showing a configuration of an organic electroluminescence element produced in Example 5; and
FIG. 18 is a cross-sectional view schematically showing a configuration of an organic electroluminescence element produced in Example 6.

## Modes for Carrying Out the Invention

An embodiment about an organic electroluminescence element with a tandem structure of the present invention will be described in the following order with reference to the attached drawings.

1. Configuration of transparent conductive layer used in organic electroluminescence element
2. First embodiment (an example in which a transparent conductive layer is arranged between two light emitting units)
3. Modification 1 of first embodiment
4. Modification 2 of first embodiment
5. Second embodiment (an example in which a conductive layer between two light emitting units and a second electrode are each a transparent conductive layer)
6. Third embodiment (an example in which a conductive layer between two light emitting units and a first electrode are each a transparent conductive layer)
7. Fourth embodiment (an example in which a conductive layer between two light emitting units and two electrodes are each a transparent conductive layer)
8. Fifth embodiment (an example in which transparent conductive layers are arranged between three or more light emitting units)
9. Sixth embodiment (an example in which a transparent conductive layer is arranged between two adjacent light emitting units of three or more light emitting units)

10. Applications of organic electroluminescence element
11. Illumination device 1
12. Illumination device 2

<<1. Configuration of Transparent Conductive Layer Used in Organic Electroluminescence Element>>

FIG. 1 is a view schematically showing a cross-sectional configuration of a transparent conductive layer used in an organic electroluminescence element with a tandem structure. As shown in FIG. 1, a transparent conductive layer 1b is characterized that it is arranged adjacent to a nitrogen-containing layer 1a. The transparent conductive layer 1b is formed by using silver (Ag) or an alloy having silver as a main component. Further, the nitrogen-containing layer 1a is characterized that it is layer formed by using a compound containing nitrogen atom, particularly formed by using a compound having a specific relationship of effective action energy ΔEef (which is to be described later) with silver, which is the main material constituting the transparent conductive layer 1b.

<Nitrogen-containing Layer 1a>

The nitrogen-containing layer 1a is a layer formed by using a compound having a specific relationship with silver (Ag), which is the main material constituting the transparent conductive layer 1b, selected from the compounds containing nitrogen atom. Here, the effective action energy ΔEef shown in the following Formula (1) is defined as interaction energy between the compound and silver. A compound whose effective action energy ΔEef satisfies the following Formula (2) and has a specific relationship is used to form the nitrogen-containing layer 1a.
[Mathematical Expression 2]

$$\Delta Eef = n \times \Delta E / s \quad \cdots\cdots (1)$$

n : Number of nitrogen atom(s) contained in compound and stably bonded with silver
ΔE : Interaction energy between nitrogen atom (N) and silver (Ag)
s : Surface area of compound

$$-0.5 \leqq \Delta Eef \leqq -0.10 \ [\text{kcal/mol} \cdot \text{Å}^2] \quad \cdots\cdots (2)$$

The "number [n] of nitrogen atom(s) contained in the compound and stably bonded with silver" is a number obtained by selecting and counting, from the nitrogen atom(s) contained in the compound, only the nitrogen atom(s) stably bonded with silver, as specific nitrogen atom(s). The nitrogen atoms from which the specific nitrogen atom(s) is (are) to be selected include all nitrogen atoms contained in the compound, instead of being limited to the nitrogen atom(s) which constitute the heterocycle. The selection of the specific nitrogen atom(s) from all nitrogen atoms contained in the compound is performed in the following manner either with a bond distance [r(Ag•N)] between silver and nitrogen atom in the compound calculated by, for example, a molecular orbital calculation method as an index, or with an angle between nitrogen atom and silver with respect to the ring containing nitrogen atom in the compound (i.e., a dihedral angle [D]) as an index. Incidentally, the molecular orbital calculation is performed by using Gaussian 03 (Gaussian, Inc., Wallingford, CT, 2003).

First, in the case where the selection of the specific nitrogen atom(s) is performed with the bond distance [r(Ag•N)] as an index, considering the steric structure of each compound, the distance at which nitrogen atom(s) in the compound is stably bonded with silver is set as a "stable bond distance". Further, the bond distance [r(Ag•N)] for each nitrogen atom contained in the compound is calculated by using the molecular orbital calculation method. The nitrogen atom(s) whose calculated bond distance [r(Ag•N)] is close to the "stable bond distance" is selected as the specific nitrogen atom(s). Such selection of the nitrogen atom(s) is applicable to a compound having many nitrogen atoms which constitute the heterocycle, and a compound having many nitrogen atoms which do not constitute the heterocycle.

While in the case where the selection of the specific nitrogen atom(s) is performed with the dihedral angle [D] as an index, the molecular orbital calculation method is used to calculate the dihedral angle [D]. Further, the nitrogen atom(s) whose calculated dihedral angle [D] satisfies "D<10 degrees" is selected as the specific nitrogen atom(s). Such selection of the nitrogen atom(s) is applicable to a compound having many nitrogen atoms which constitute the heterocycle.

The interaction energy [ΔE] between silver (Ag) and nitrogen (N) contained in the compound is an interaction energy between nitrogen selected in the above manner and silver, and is possible to be calculated by the molecular

orbital calculation method.

Further, the surface area [s] is calculated with respect to the aforesaid optimized structure, by using a Tencube/WM (manufactured by Tencube Co., Ltd).

It is preferred that the effective action energy ΔEef defined above falls in a range satisfying the following Formula (3).

[Mathematical Expression 3]

$$\Delta Eef \leqq -0.20\ [\mathrm{kcal/mol \cdot Å^2}] \quad \cdots\cdots (3)$$

Examples of the compound containing nitrogen atom constituting the nitrogen-containing layer 1a include a compound having a heterocycle with a nitrogen atom (N) as a hetero atom. Examples of the heterocycle with a nitrogen atom (N) as a hetero atom include aziridine, azirine, azetidine, azete, azolidine, azole, piperidine, pyridine, azepane, azepine, imidazole, pyrazole, oxazole, thiazole, imidazoline, pyrazine, pyridazine, pyrimidine, morpholine, thiazine, indole, isoindole, benzimidazole, purine, quinoline, isoquinoline, quinoxaline, cinnoline, pteridine, acridine, carbazole, benzo-C-cinnoline, porphyrin, chlorine, choline, and the like. Among these heterocycles, heterocycle compounds containing one or two nitrogen atom(s) are exemplified.

For example, a compound represented by the following General Formula (1) or a compound represented by General Formula (2) shown later is favorably used as the compound having a heterocycle with a nitrogen atom as a hetero atom. In the present invention, the nitrogen-containing layer 1a arranged adjacent to the transparent conductive layer 1b is formed by using a compound represented by General Formula (1) or (2) and selected from the compounds satisfying Formula (1) or (2).

[Chemical Formula 3]

General Formula(1)

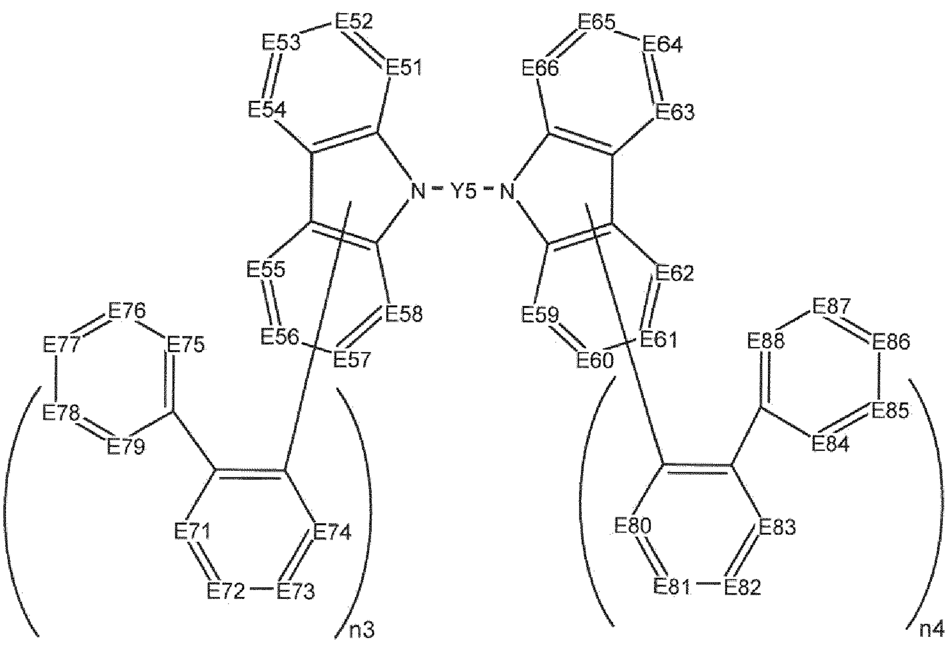

In General Formula (1), Y5 represents a divalent linking group which is an arylene group, a heteroarylene group or a combination of the arylene group and the heteroarylene group; E51 to E66 and E71 to E88 each represent -C(R3)= or -N=, wherein R3 represents a hydrogen atom or a substituent, and wherein at least one of E71 to E79 and at least one of E80 to E88 each represent -N=; and n3 and n4 each represent an integer of 0 to 4, wherein the sum of n3 and n4 is 2 or more.

Examples of the arylene group represented by Y5 in General Formula (1) include an o-phenylene group, a p-phenylene group, a naphthalenediyl group, an anthracenediyl group, a naphthacenediyl group, a pyrenediyl group, a naphthylnaphthalenediyl group, a biphenyldiyl group (for example, a [1,1'-biphenyl]-4,4'-diyl group, a 3,3'-biphenyldiyl

group, and a 3,6-biphenyldiyl group), a terphenyldiyl group, a quaterphenyldiyl group, a quinquephenyldiyl group, a sexiphenyldiyl group, a septiphenyldiyl group, an octiphenyldiyl group, a nobiphenyldiyl group and a deciphenyldiyl group and the like.

Examples of the heteroarylene group represented by Y5 in General Formula (1) include a divalent group derived from the group consisting of a carbazole ring, a carboline ring, a diazacarbazole ring (also referred to as a monoazacarboline ring, indicating a ring structure formed in such a manner that one of the carbon atoms constituting the carboline ring is replaced with a nitrogen atom), a triazole ring, a pyrrole ring, a pyridine ring, a pyrazine ring, a quinoxaline ring, a thiophene ring, an oxadiazole ring, a dibenzofuran ring, a dibenzothiophene ring and an indole ring.

It is preferred that the divalent linking group (which is an arylene group, a heteroarylene group or a combination thereof represented by Y5) contains, among the heteroarylene groups, a group derived from a condensed aromatic heterocycle formed by condensing three or more rings, and further, it is preferred that the group derived from the condensed aromatic heterocycle formed by condensing three or more rings is a group derived from a dibenzofuran ring or a group derived from a dibenzothiophene ring.

Examples of the substituent represented by R3 of -C(R3)= represented by each of E51 to E66 and E71 to E88 in General Formula (1) include: an alkyl group (for example, a methyl group, an ethyl group, a propyl group, an isopropyl group, a tert-butyl group, a pentyl group, a hexyl group, an octyl group, a dodecyl group, a tridecyl group, a tetradecyl group, a pentadecyl group and the like); a cycloalkyl group (for example, a cyclopentyl group, a cyclohexyl group and the like); an alkenyl group (for example, a vinyl group, an allyl group and the like); an alkynyl group (for example, an ethynyl group, a propargyl group and the like); an aromatic hydrocarbon group (also referred to as an aromatic carbon ring group, an aryl group or the like, and examples of the aromatic hydrocarbon group include a phenyl group, a p-chlorophenyl group, a mesityl group, a tolyl group, a xylyl group, a naphthyl group, an anthryl group, an azulenyl group, an acenaphthenyl group, a fluorenyl group, a phenanthryl group, an indenyl group, a pyrenyl group, a biphenyryl group and the like); an aromatic heterocyclic group (for example, a furyl group, a thienyl group, a pyridyl group, a pyridazinyl group, a pyrimidinyl group, a pyrazinyl group, a triazinyl group, an imidazolyl group, a pyrazolyl group, a thiazolyl group, a quinazolinyl group, a carbazolyl group, a carbolinyl group, a diazacarbazolyl group (which is formed by substituting any one of carbon atoms constituting a carboline ring of the aforesaid carbolinyl group with a nitrogen atom), a phtharazinyl group and the like); a heterocyclic group (for example, a pyrrolidyl group, an imidazolidyl group, a morpholyl group, an oxazolidyl group and the like); an alkoxy group (for example, a methoxy group, an ethoxy group, a propyloxy group, a pentyloxy group, an hexyloxy group, an octyloxy group, a dodecyloxy group and the like); a cycloalkoxy group (for example, a cyclopentyloxy group, a cyclohexyloxy group and the like); an aryloxy group (for example, a phenoxy group, a naphthyloxy group and the like); an alkylthio group (for example, a methylthio group, an ethylthio group, a propylthio group, a pentylthio group, a hexylthio group, an octylthio group, a dodecylthio group and the like); a cycloalkylthio group (for example, a cyclopentylthio group, a cyclohexylthio group and the like); an arylthio group (for example, a phenylthio group, a naphthylthio group and the like); an alkoxycarbonyl group (for example, a methyloxycarbonyl group, an ethyloxycarbonyl group, a butyloxycarbonyl group, an octyloxycarbonyl group, a dodecyloxycarbonyl group and the like); an aryloxycarbonyl group (for example, a phenyloxycarbonyl group, a naphthyloxycarbonyl group and the like); a sulfamoyl group (for example, an aminosulfonyl group, a methylaminosulfonyl group, a dimethylaminosulfonyl group, a butylaminosulfonyl group, a hexylaminosulfonyl group, a cyclohexylaminosulfonyl group, an octylaminosulfonyl group, a dodecylaminosulfonyl group, a phenylaminosulfonyl group, a naphthylaminosulfonyl group, a 2-pyridylaminosulfonyl group and the like); an acyl group (for example, an acetyl group, an ethylcarbonyl group, a propylcarbonyl group, a pentylcarbonyl group, a cyclohexylcarbonyl group, an octylcarbonyl group, a 2-ethylhexylcarbonyl group, a dodecylcarbonyl group, a phenylcarbonyl group, a naphthylcarbonyl group, a pyridylcarbonyl group and the like); an acyloxy group (for example, an acetyloxy group, an ethylcarbonyloxy group, a butylcarbonyloxy group, an octylcarbonyloxy group, a dodecylcarbonyloxy group, a phenylcarbonyloxy group and the like); an amido group (for example, a methylcarbonylamino group, an ethylcarbonylamino group, a dimethylcarbonylamino group, a propylcarbonylamino group, a pentylcarbonylamino group, a cyclohexylcarbonylamino group, a 2-ethylhexylcarbonylamino group, an octylcarbonylamino group, a dodecylcarbonylamino group, a phenylcarbonylamino group, a naphthylcarbonylamino group and the like); a carbamoyl group (for example, an aminocarbonyl group, a methylaminocarbonyl group, a dimethylaminocarbonyl group, a propylaminocarbonyl group, a pentylaminocarbonyl group, a cyclohexylaminocarbonyl group, an octylaminocarbonyl group, a 2-ethylhexylaminocarbonyl group, a dodecylaminocarbonyl group, a phenylaminocarbonyl group, a naphthylaminocarbonyl group, a 2-pyridylaminocarbonyl group and the like); an ureido group (for example, a methylureido group, an ethylureido group, a pentylureido group, a cyclohexylureido group, an octylureido group, a dodecylureido group, a phenylureido group, a naphthylureido group, a 2-pyridylaminoureido group and the like); a sulfinyl group (for example, a methylsulfinyl group, an ethylsulfinyl group, a butylsulfinyl group, a cyclohexylsulfinyl group, a 2-ethylhexylsulfinyl group, a dodecylsulfinyl group, a phenylsulfinyl group, a naphthylsulfinyl group, a 2-pyridylsulfinyl group and the like); an alkylsulfonyl group (for example, a methylsulfonyl group, an ethylsulfonyl group, a butylsulfonyl group, a cyclohexylsulfonyl group, a 2-ethylhexylsulfonyl group, a dodecylsulfonyl group and the like); an arylsulfonyl group or a heteroarylsulfonyl group (for example, a phenylsulfonyl group, a naphthylsulfonyl group, a 2-pyridylsulfonyl group and the like); an amino group (for

example, an amino group, an ethylamino group, a dimethylamino group, a butylamino group, a cyclopentylamino group, a 2-ethylhexylamino, a dodecylamino group, an anilino group, a naphthylamino group, a 2-pyridylamino group, a piperidyl group (also referred to as a piperidinyl group), a 2,2,6,6-tetramethyl piperidinyl group and the like); a halogen atom (for example, a fluorine atom, a chlorine atom, a bromine atom and the like); a fluorohydrocarbon group (for example, a fluoromethyl group, a trifluoromethyl group, a pentafluoroethyl group, a pentafluorophenyl group and the like); a cyano group; a nitro group; a hydroxyl group; a mercapto group; a silyl group (for example, a trimethylsilyl group, a triisopropylsilyl group, a triphenylsilyl group, a phenyldiethylsilyl group and the like); a phosphate group (for example, dihexylphosphoryl group and the like); a phosphite group (for example, diphenylphosphinyl group and the like); a phosphono group, and the like.

Part of these substituents may be further substituted by the above-mentioned substituent. Further, a plurality of these substituents may be bonded to each other to form a ring.

In General Formula (1), it is preferable that six or more of E51 to E58 and six or more of E59 to E66 are each represented by -C(R3)=.

It is preferable that, in General Formula (1), at least one of E75 to E79 and at least one of E84 to E88 each represent -N=.

It is further preferable that, in General Formula (1), any one of E75 to E79 and any one of E84 to E88 each represent -N=.

Further, it is preferable that, in General Formula (1), E71 to E74 and E80 to E83 are each represented by -C(R3)=.

Further, it is preferred that, in the compound represented by General Formula (1), E53 is represented by -C(R3)=, wherein R3 represents a liking site; and it is further preferred that E61 is also represented by -C(R3)=, wherein R3 represents a liking site.

Furthermore, it is preferred that E75 and E84 are each represented by -N=, and E71 to E74 and E80 to E83 are each represented by -C(R3)=.

As another example of the compound constituting the nitrogen-containing layer 1a, a compound represented by the following General Formula (2) is used.

[Chemical Formula 4]

General Formula(2)

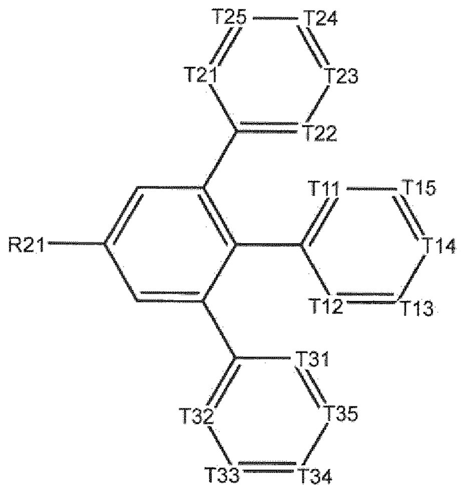

In General Formula (2), R21 represents a substituent. Further, in General Formula (2), T11, T12, T21 to T25, and T31 to T35 each represent -C(R22)= or -N=, and T13 to T15 each represent -C(R22)=; wherein R22 represents a hydrogen atom (H) or a substituent. At least one of T11 and T12 represents nitrogen atom (-N=), at least one of T21 to T25 represents nitrogen atom (-N=), and at least one of T31 to T35 represents nitrogen atom (-N=).

In General Formula (2), examples of the substituent represented by R21 and R22 include the same ones as described in the examples of R3 in General Formula (1). Part of these substituents may be further substituted by the above-mentioned substituent.

In the case where the aforesaid nitrogen-containing layer 1a is formed on the substrate 11, the nitrogen-containing layer 1a may be formed by a method based on wet process (such as a coating method, an ink-jet method, a dipping method or the like), a method based on dry process (such as a deposition method (e.g., a resistance heating method, an EB method or the like), a sputtering method, a CVD method or the like) or the like. Among these methods, the deposition method is preferably used.

[Concrete Examples of Compound]

Concrete examples (1 to 134) of the compound constituting the nitrogen-containing layer 1a are shown below; however, the present invention is not limited thereto. Note that, the concrete examples also include examples of compounds which are not included in General Formula (1) and General Formula (2). Further, in the present invention, the nitrogen-containing layer 1a arranged adjacent to the transparent conductive layer 1b is formed by using a compound satisfying Formula (1) or Formula (2) and selected from the compounds (1 to 134) exemplified below.

[Chemical Formula 5]

1

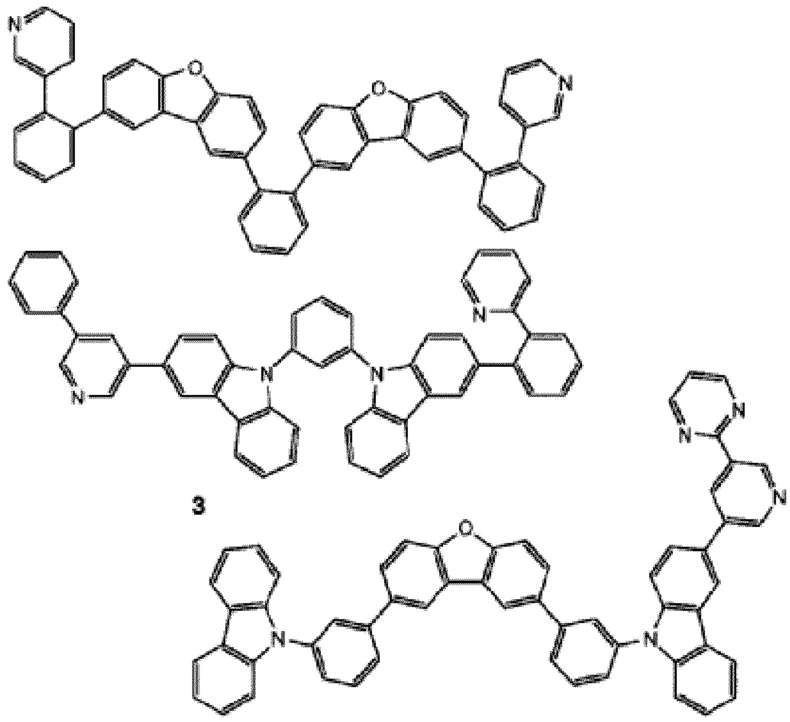

2

3

4

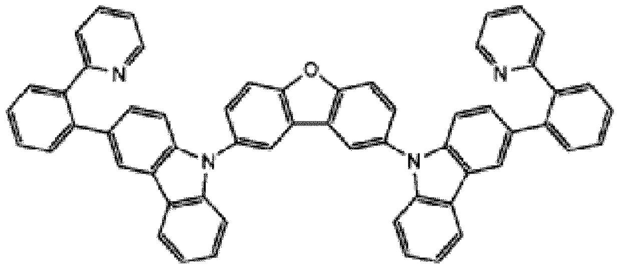

5

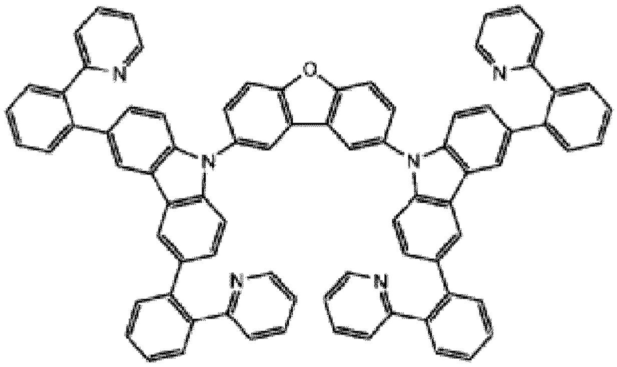

[Chemical Formula 6]

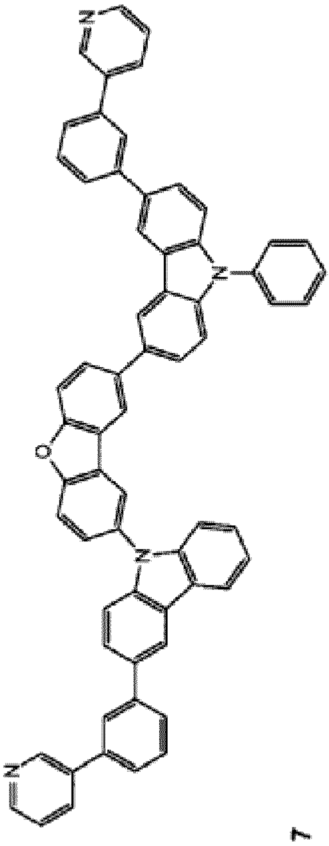

6

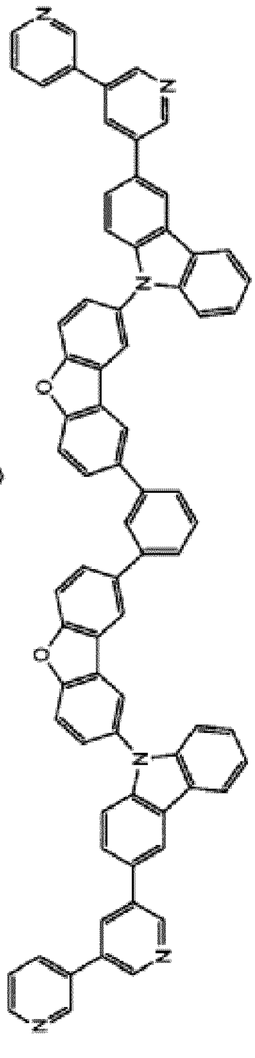

7

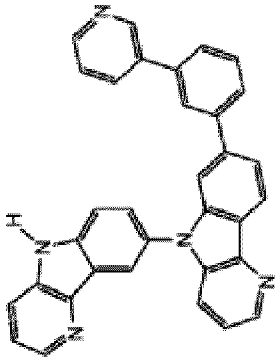

8

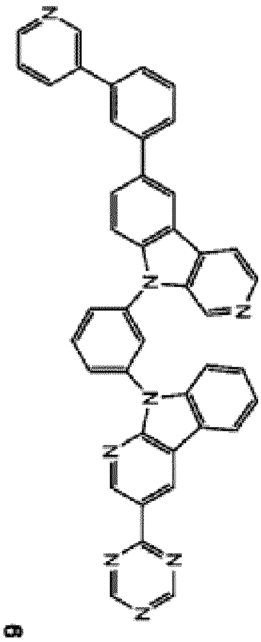

9

[Chemical Formula 7]

10

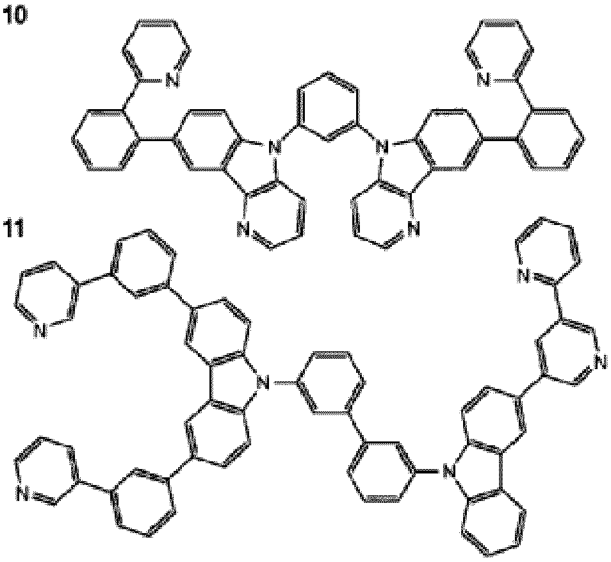

11

12

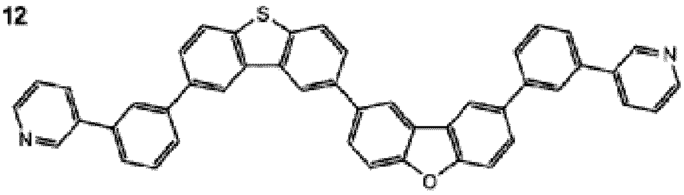

13

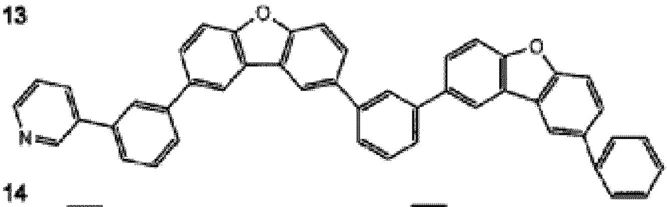

14

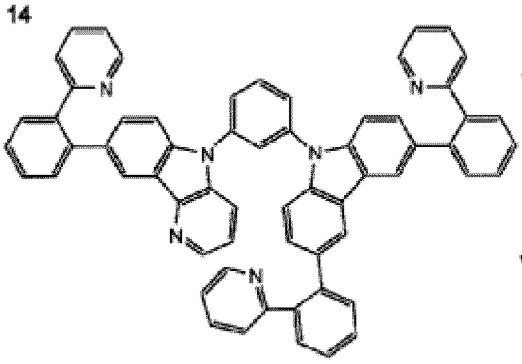

15

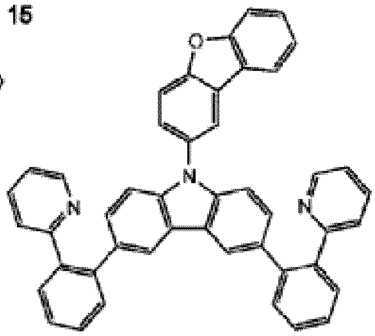

[Chemical Formula 8]

16

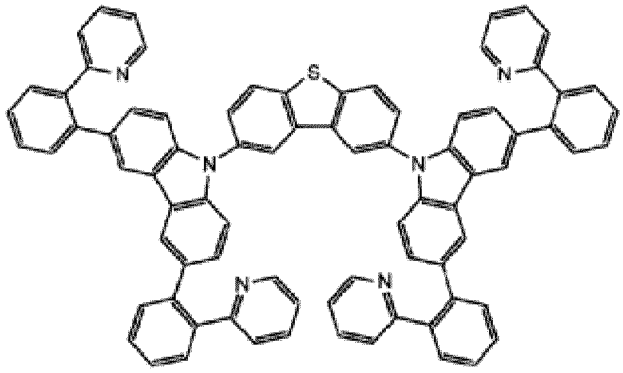

17

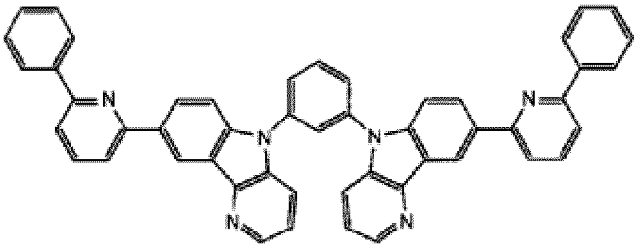

18

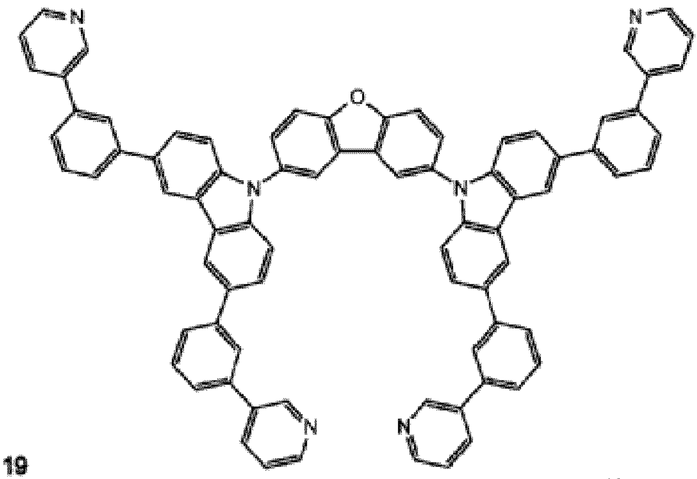

19

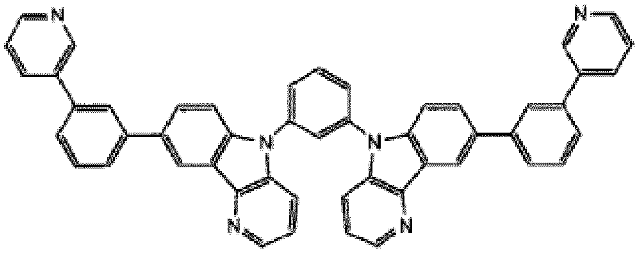

[Chemical Formula 9]

20

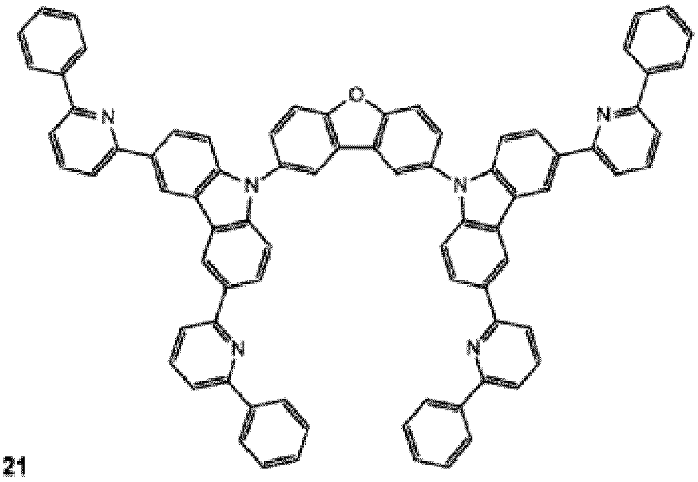

21

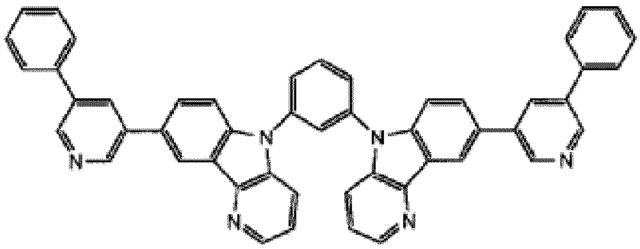

22

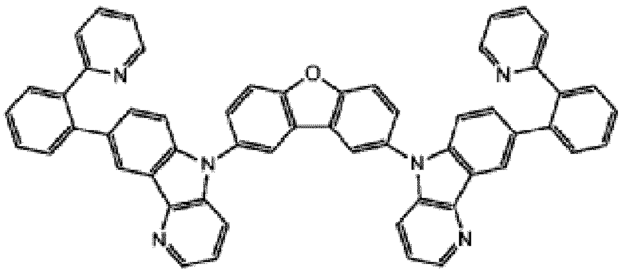

23

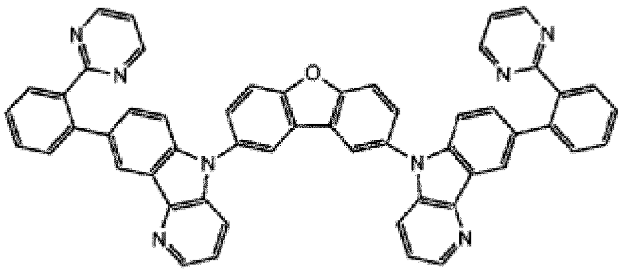

[Chemical Formula 10]

24

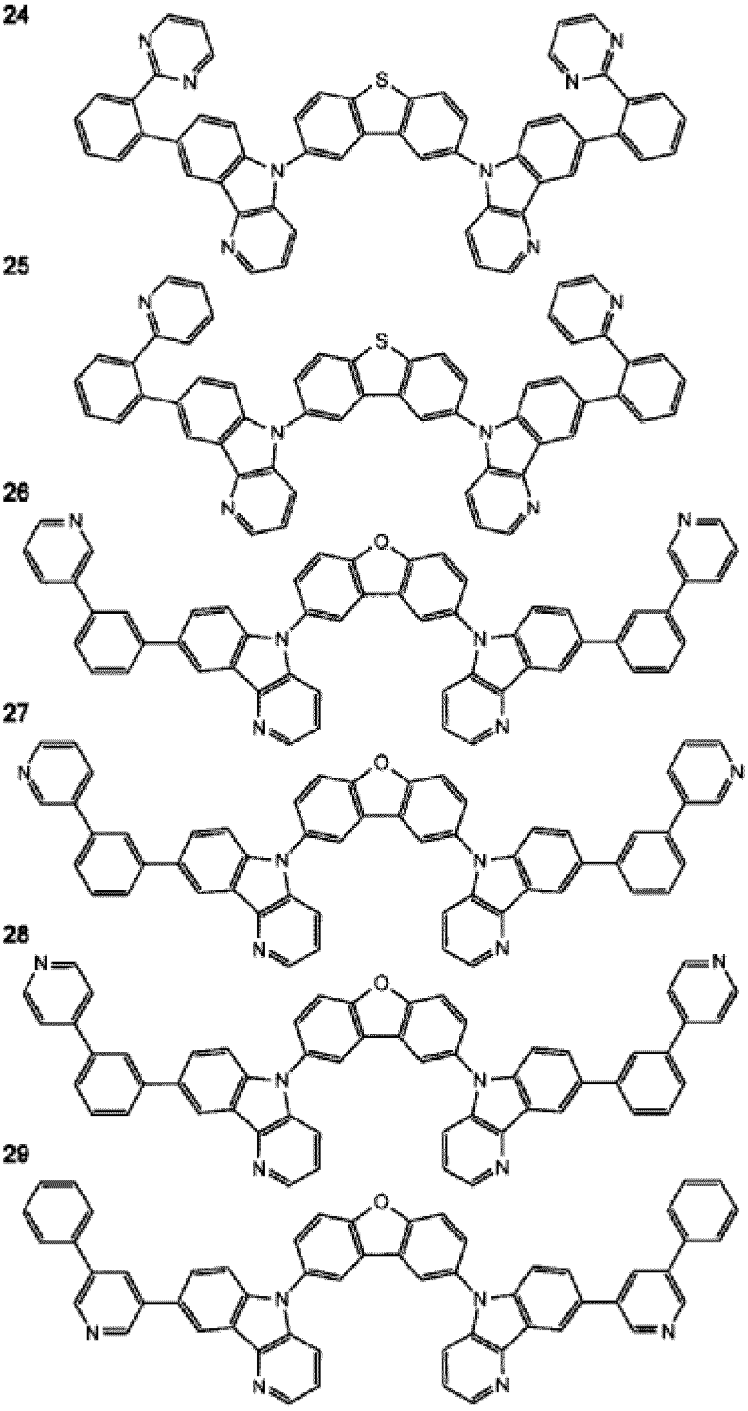

25

26

27

28

29

[Chemical Formula 11]

30

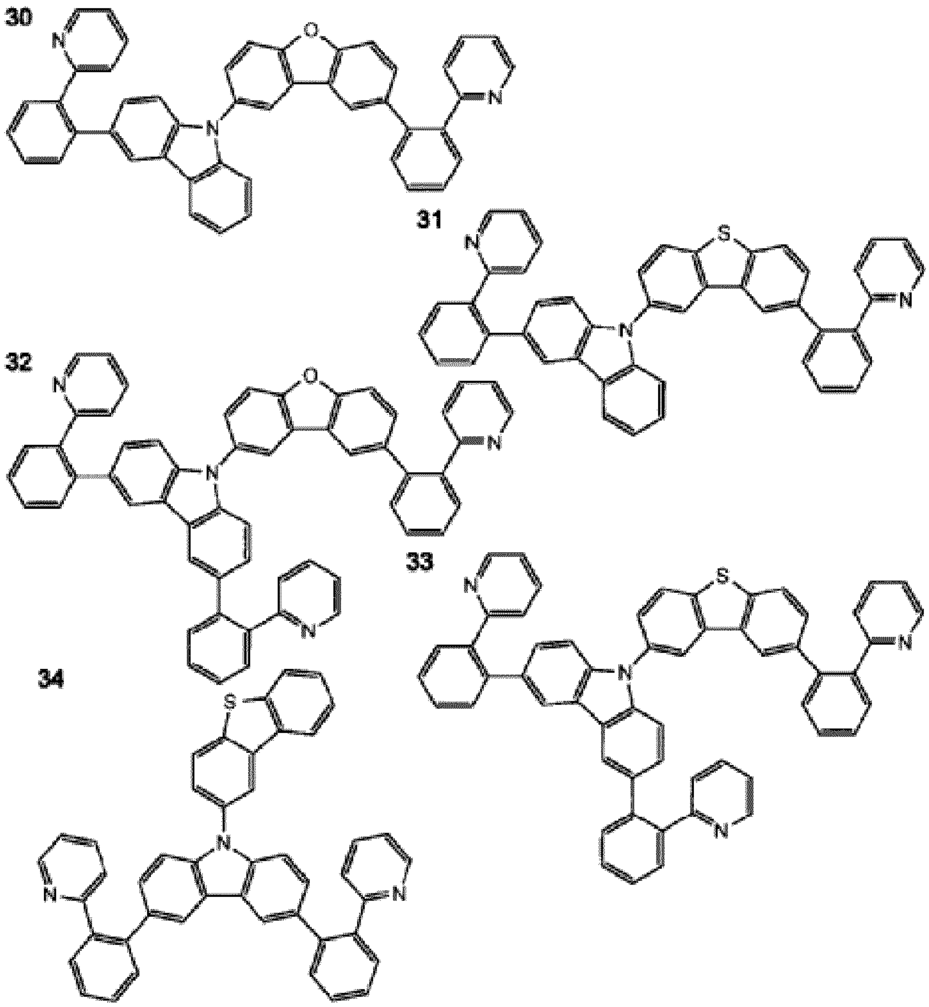

31

32

33

34

35

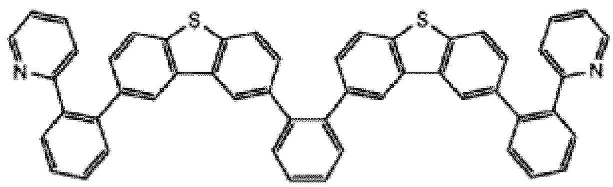

36

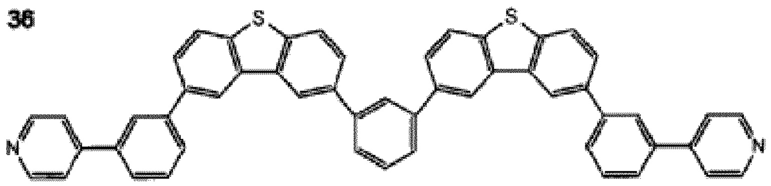

[Chemical Formula 12]

37

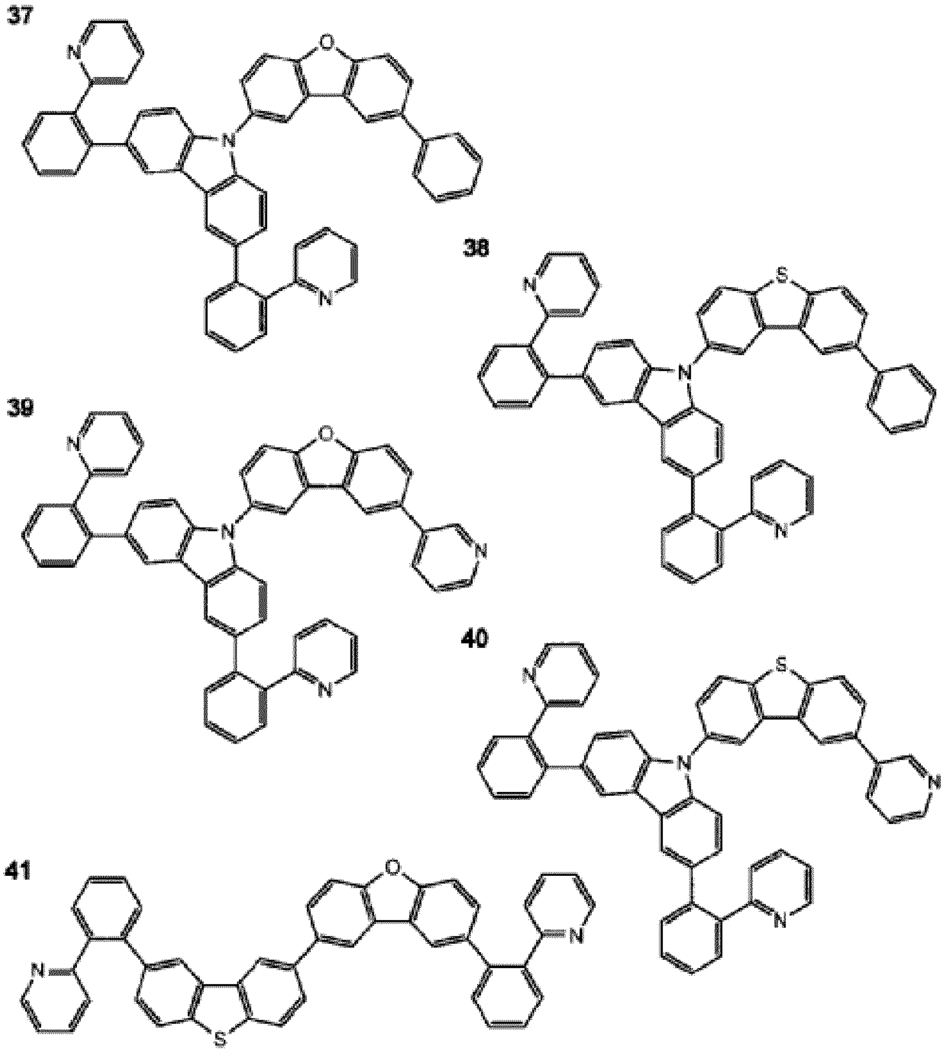

38

39

40

41

42

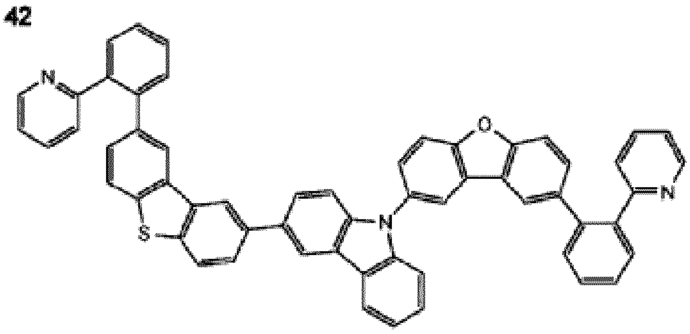

[Chemical Formula 13]

43

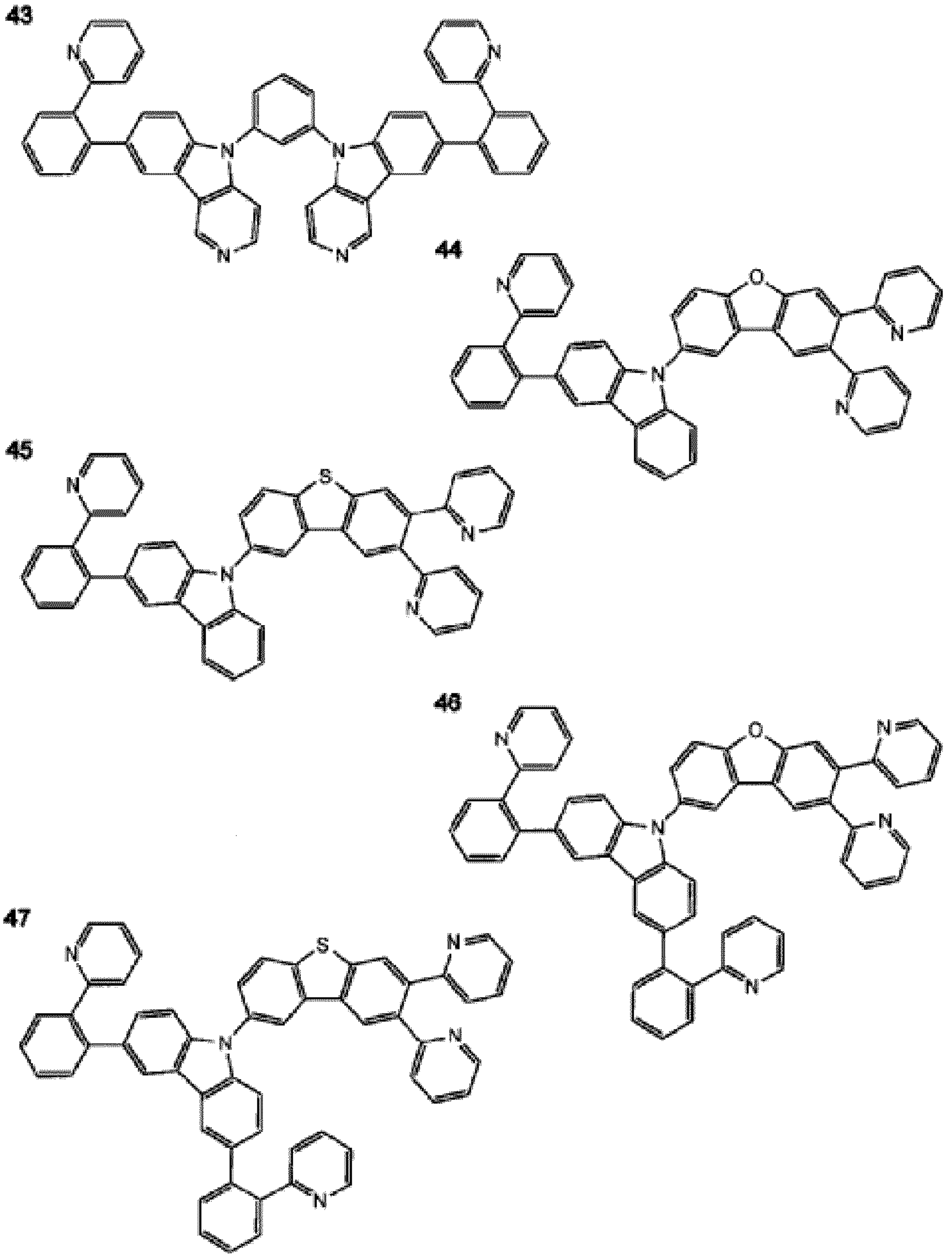

44

45

46

47

[Chemical Formula 14]

48

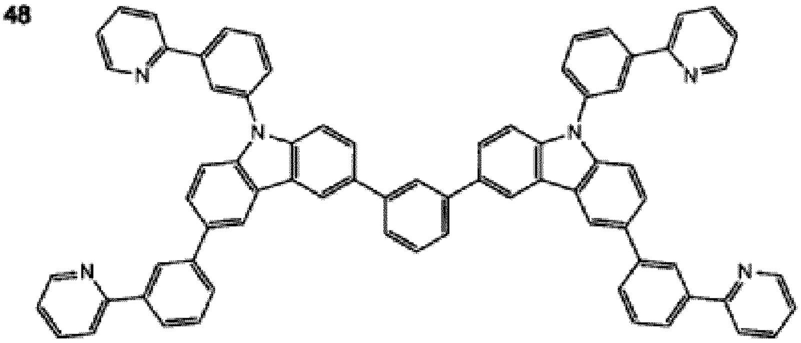

49

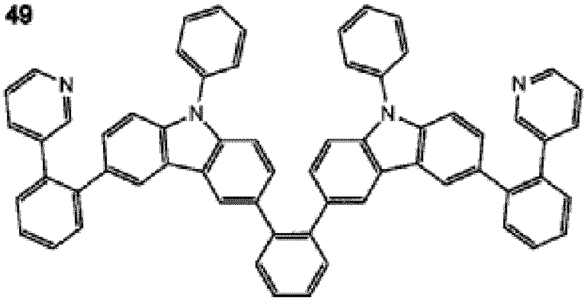

50

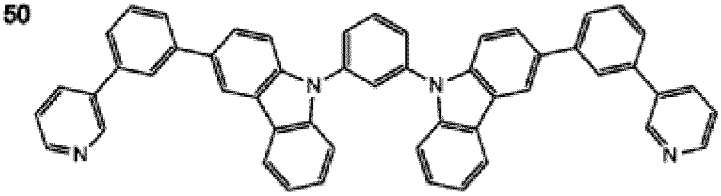

51

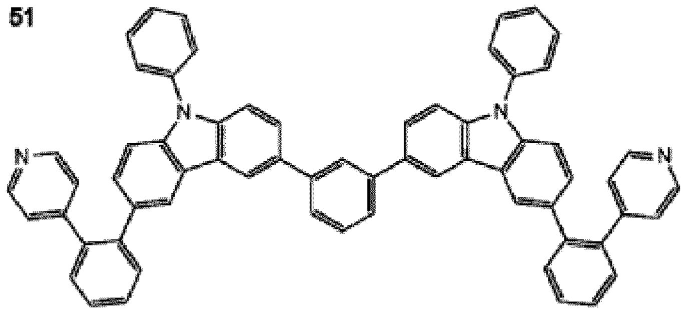

52

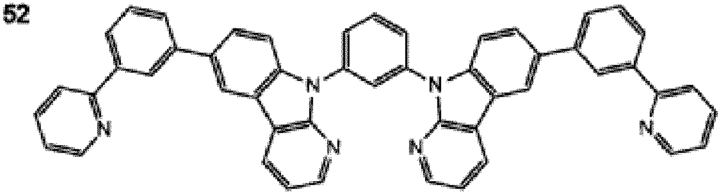

[Chemical Formula 15]

53

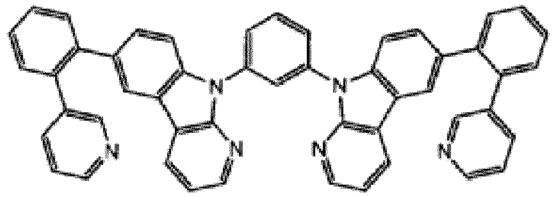

54

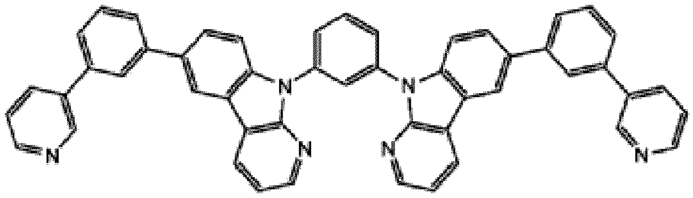

55

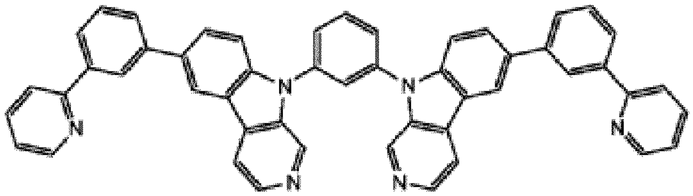

56

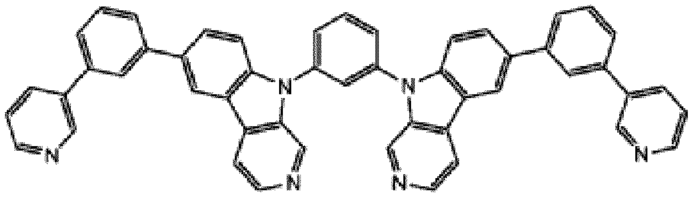

57

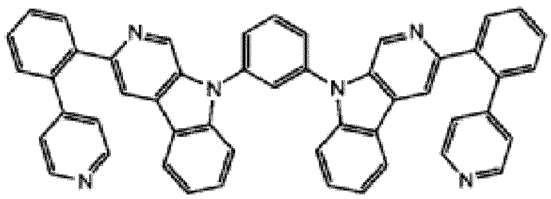

58

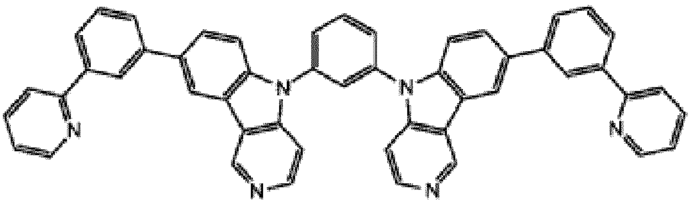

[Chemical Formula 16]

59

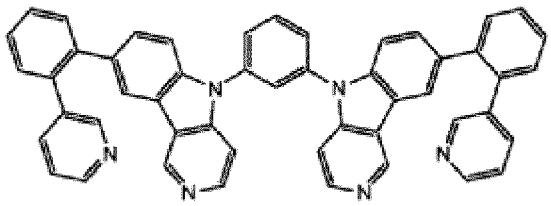

60

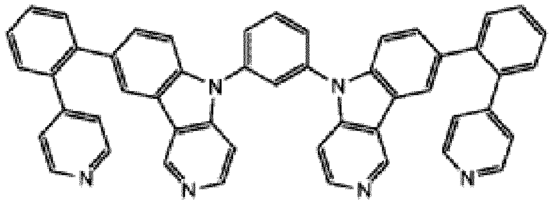

61

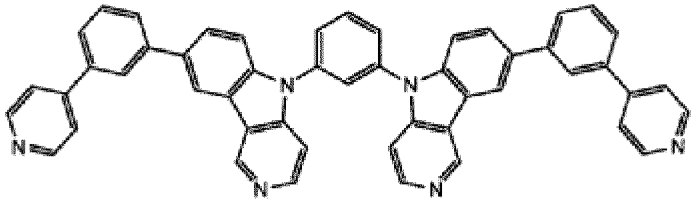

62

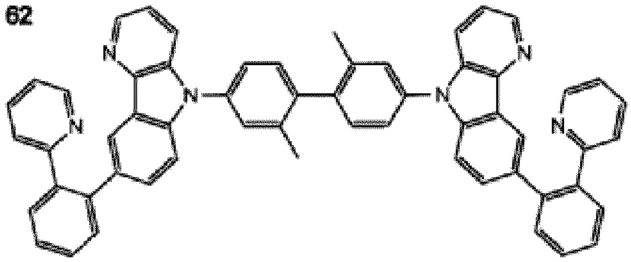

63

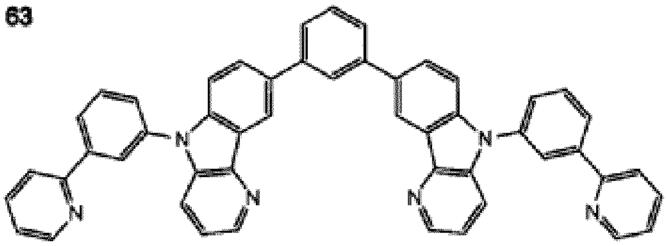

64

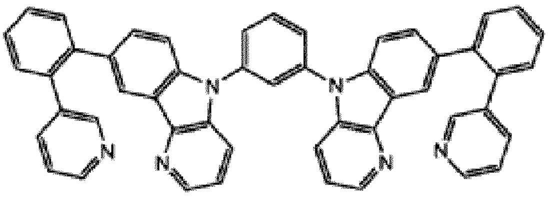

[Chemical Formula 17]

65

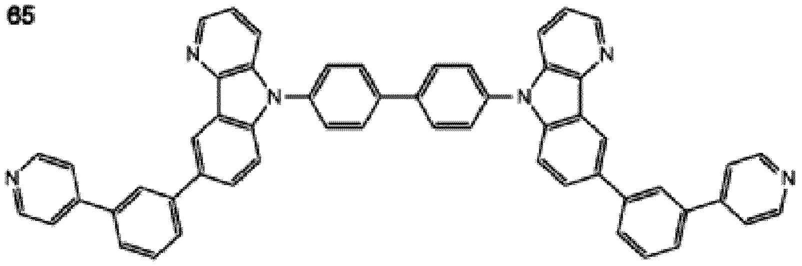

66

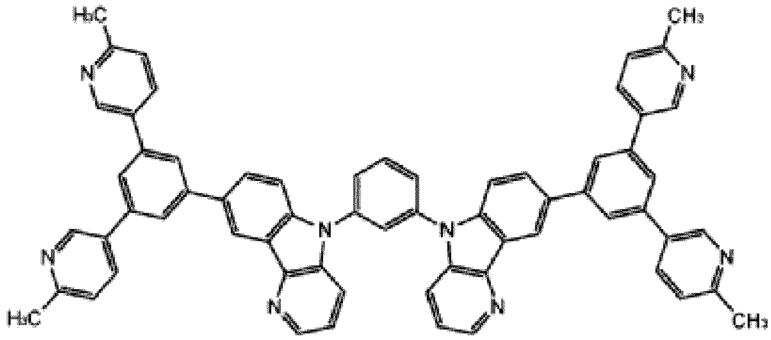

67

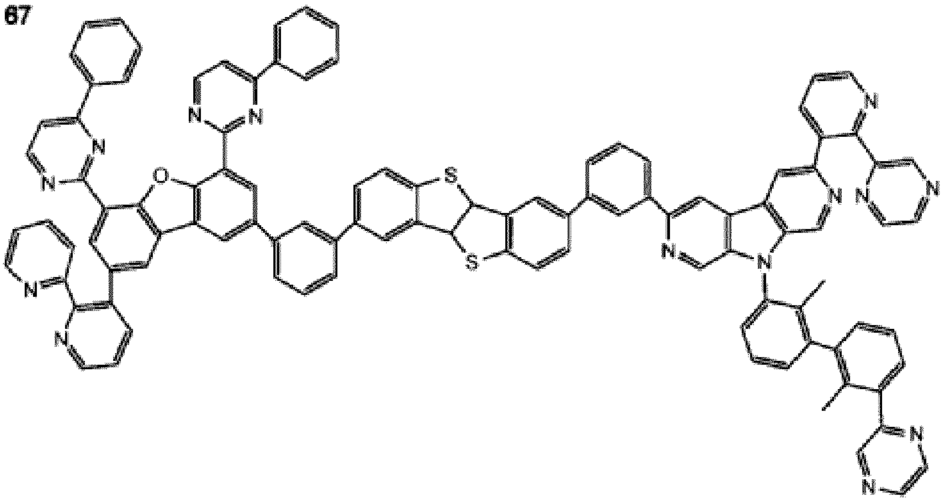

[Chemical Formula 18]

68

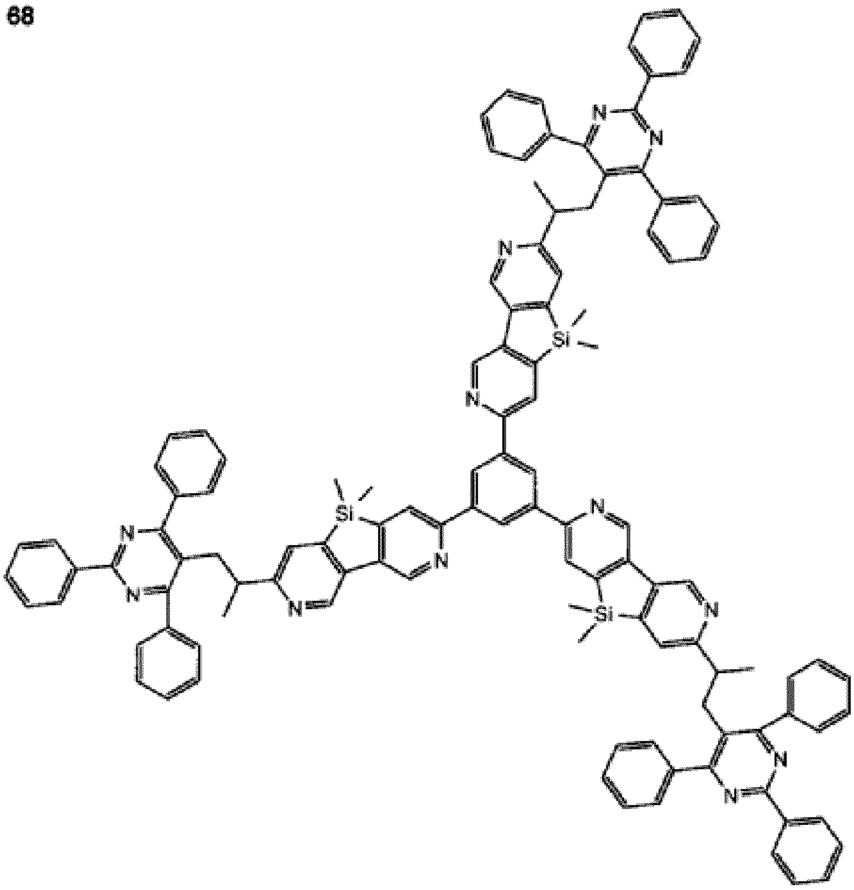

69

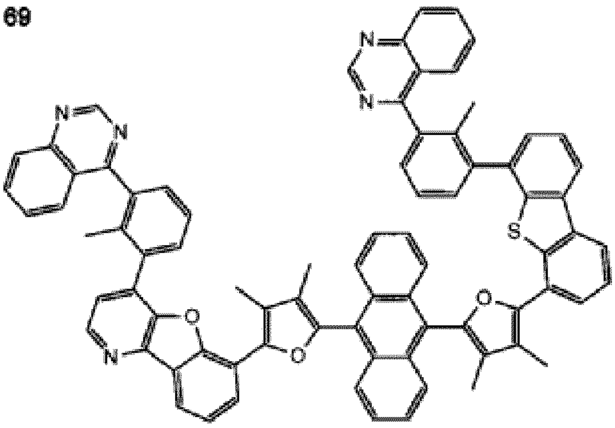

[Chemical Formula 19]

70

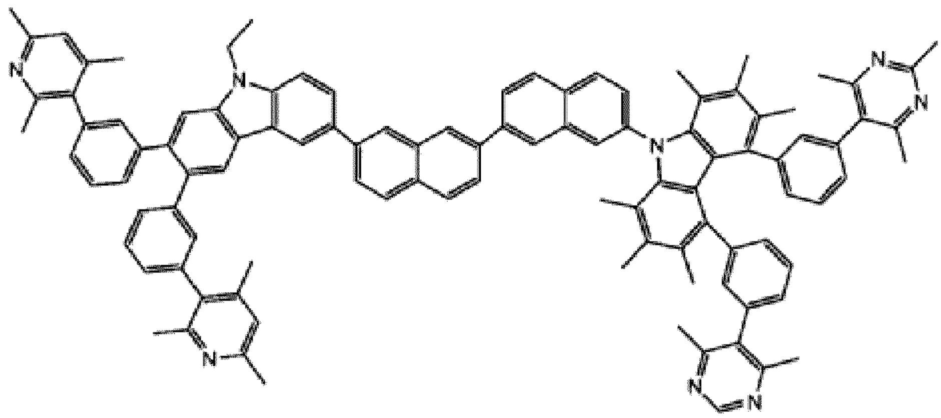

71

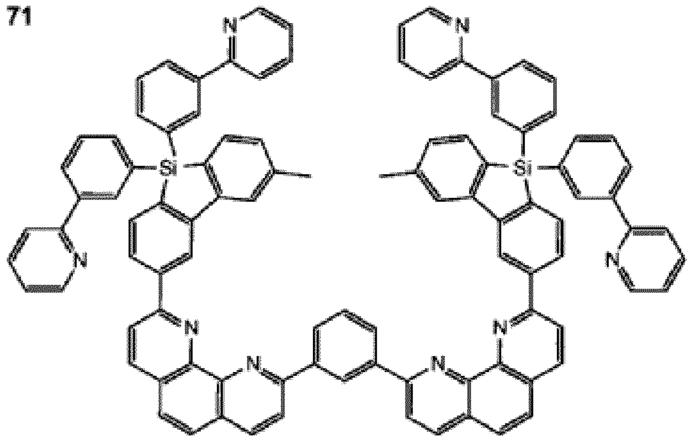

72

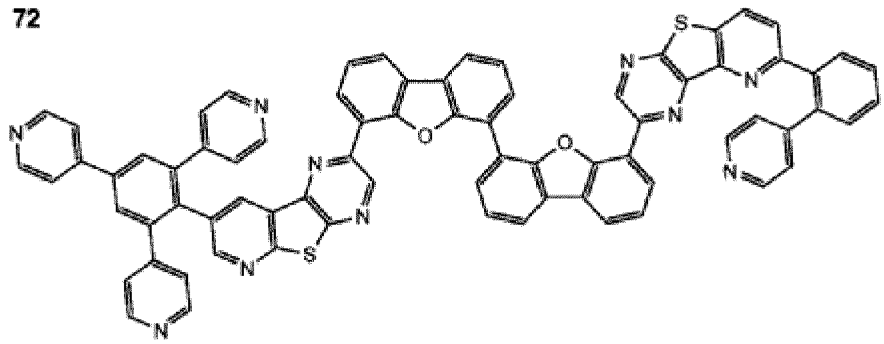

[Chemical Formula 20]

73

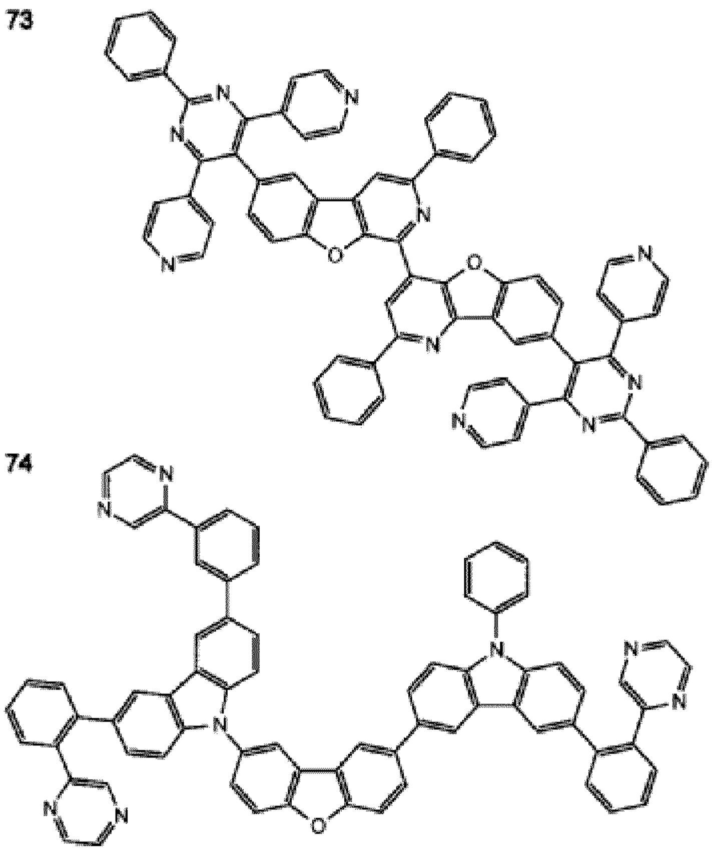

74

75

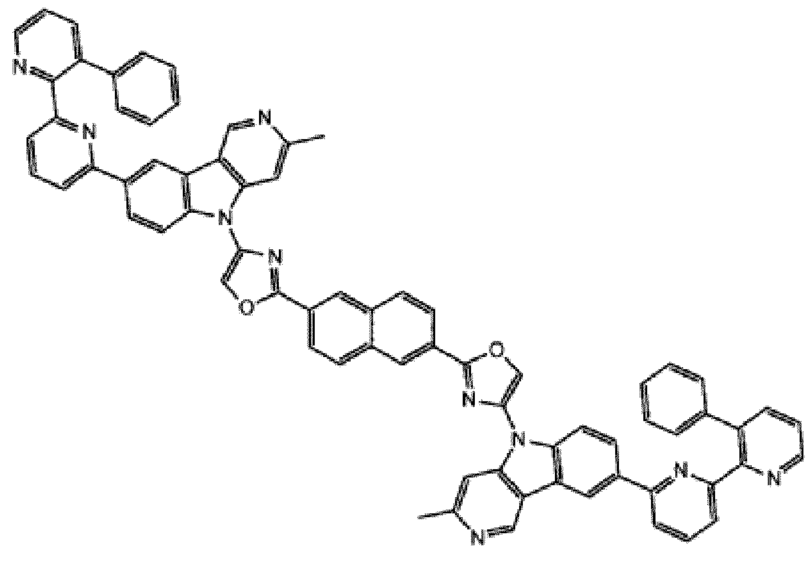

[Chemical Formula 21]

76

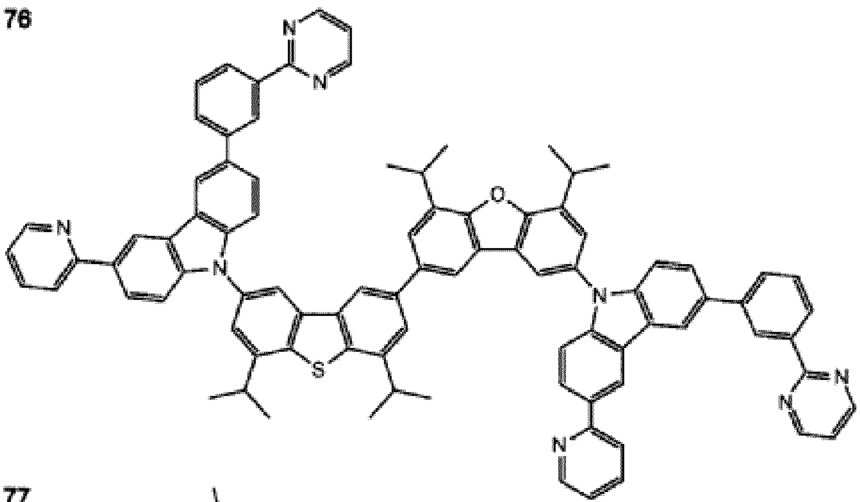

77

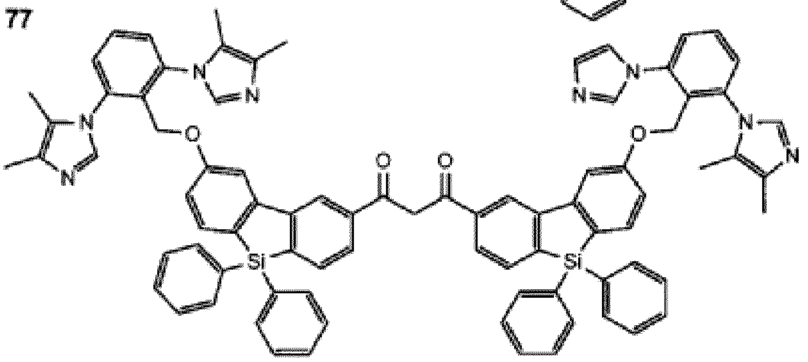

78

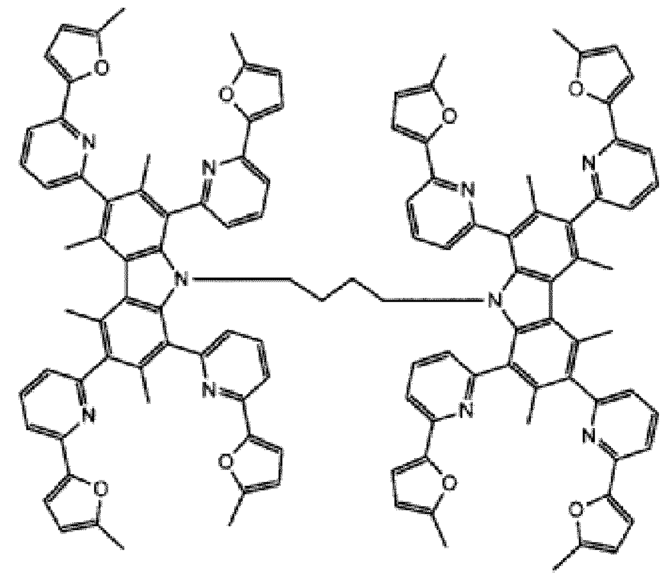

[Chemical Formula 22]

79

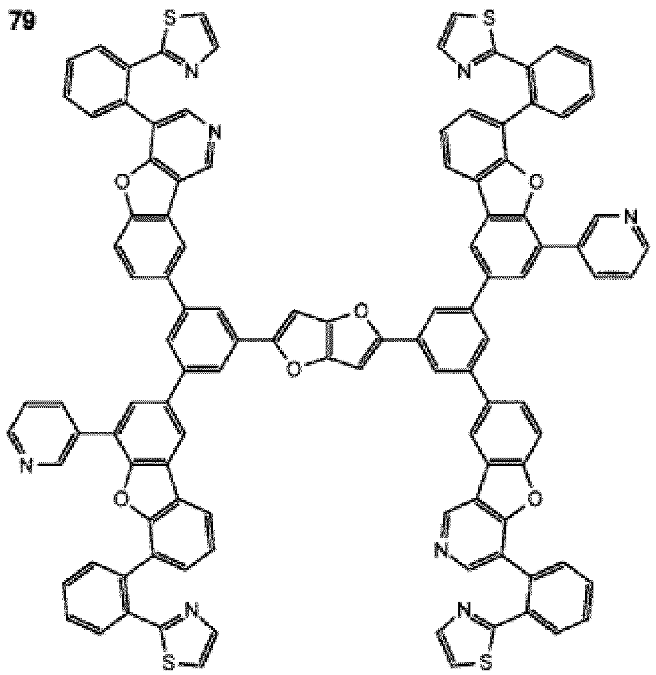

80

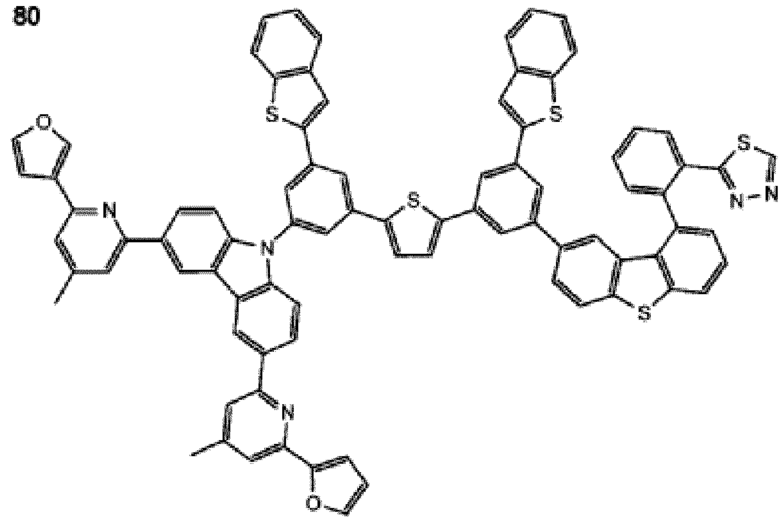

[Chemical Formula 23]

81

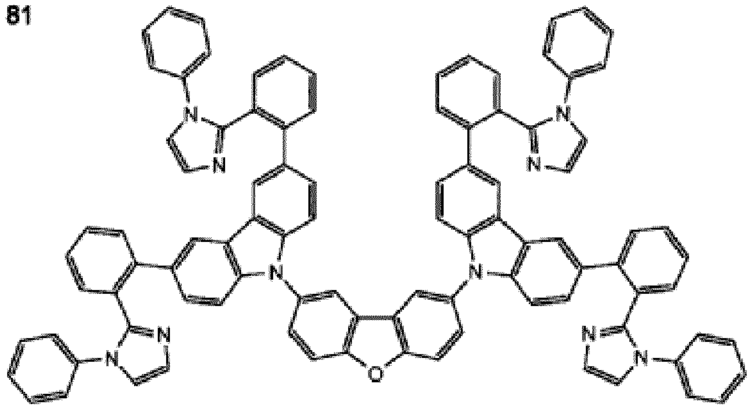

82

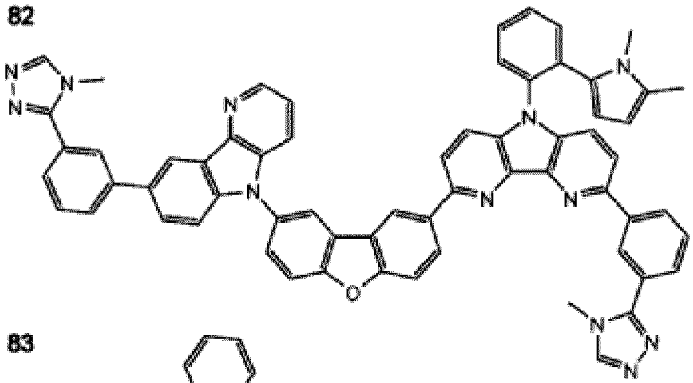

83

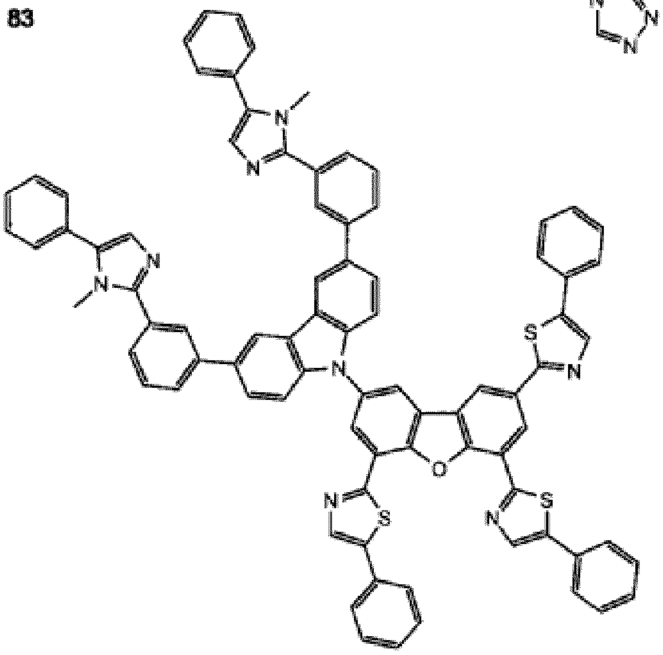

[Chemical Formula 24]

84

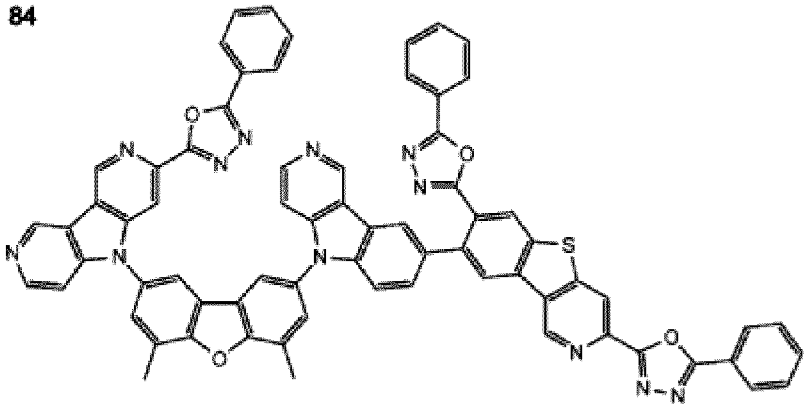

85

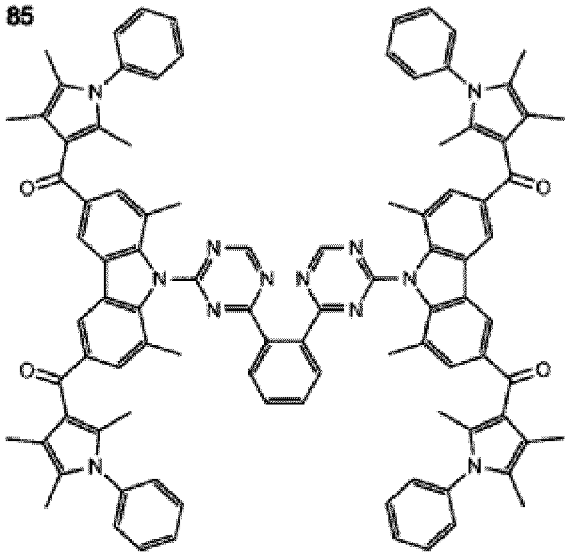

[Chemical Formula 25]

86

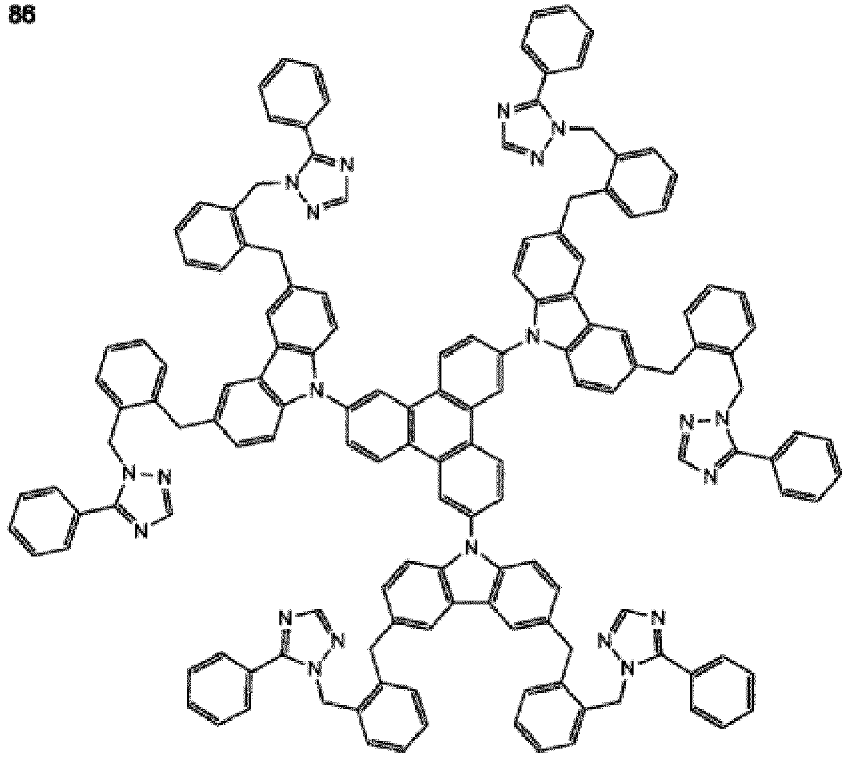

87

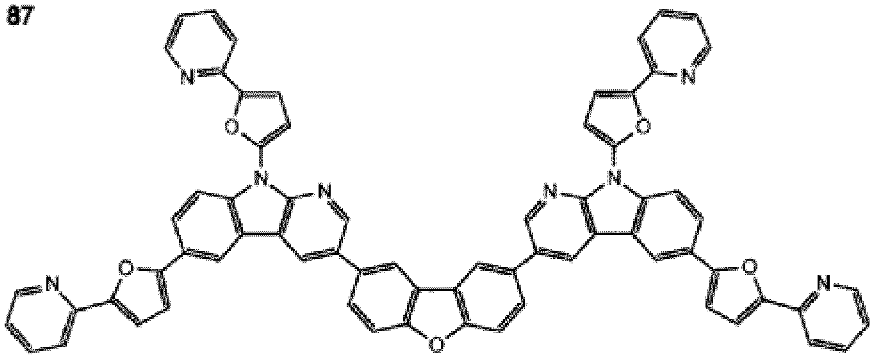

88

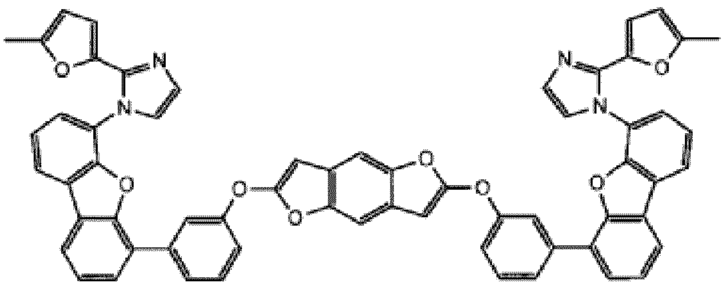

[Chemical Formula 26]

89

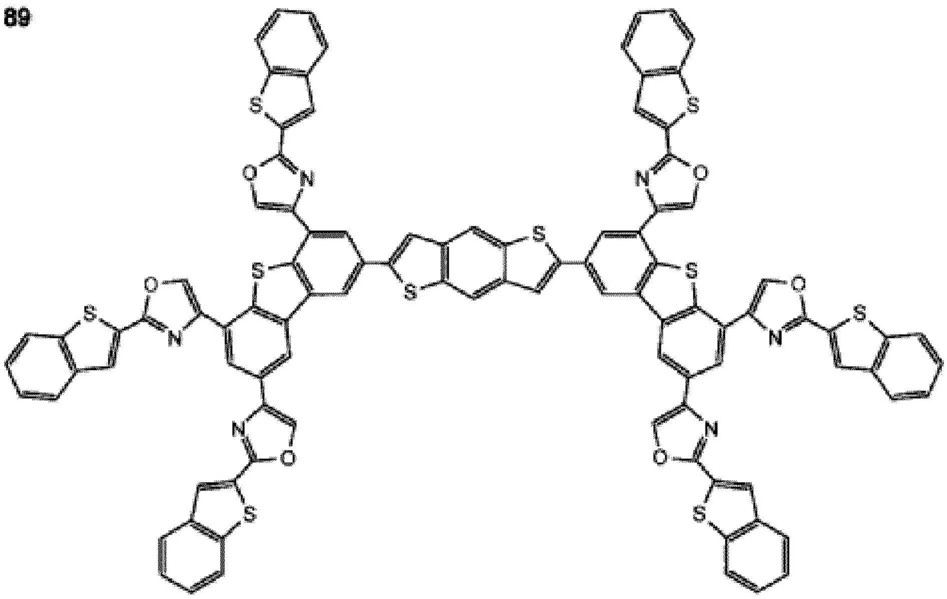

90

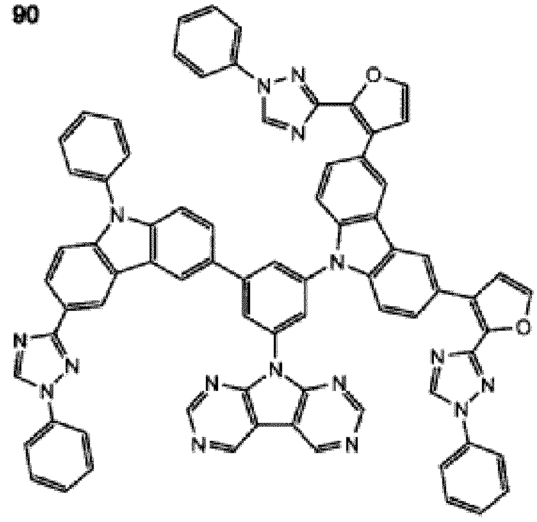

91

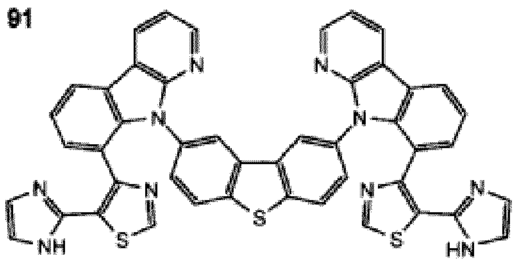

[Chemical Formula 27]

92

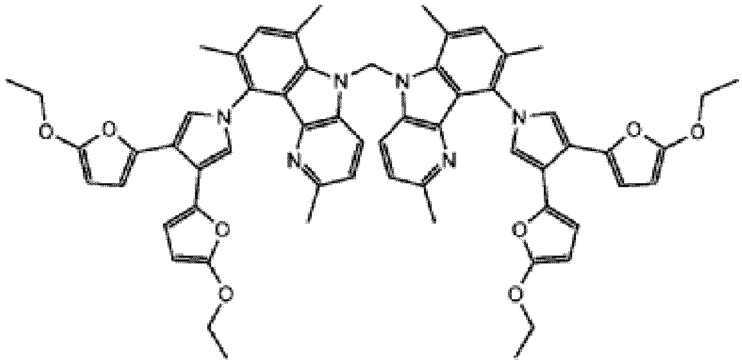

93

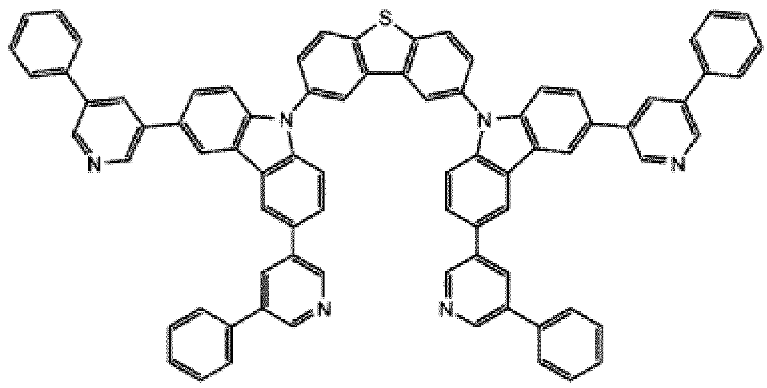

94

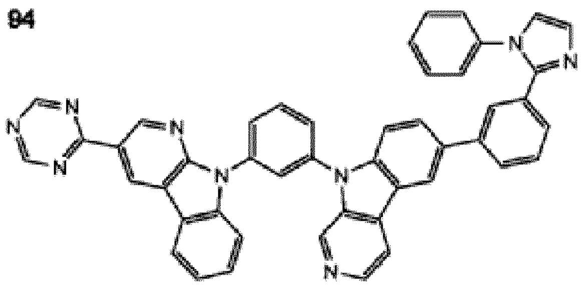

95

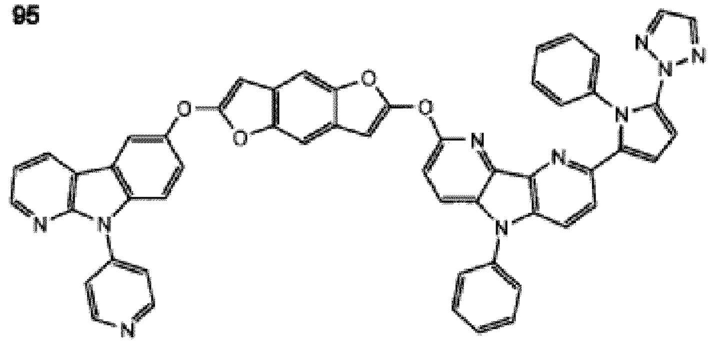

[Chemical Formula 28]

96

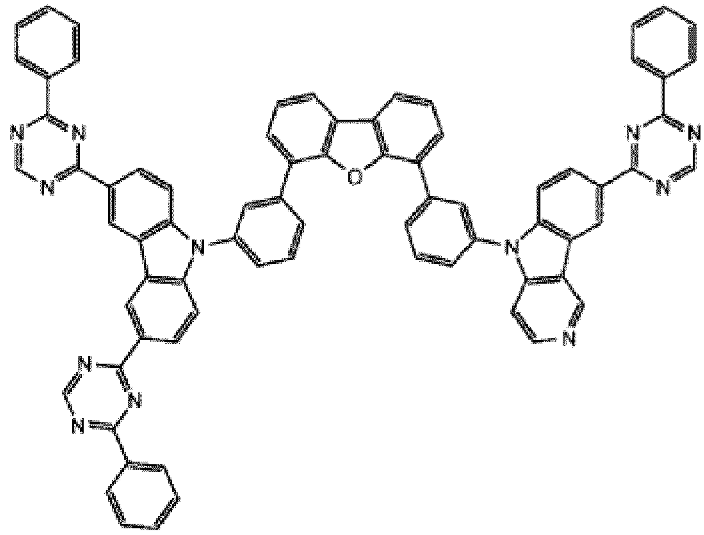

97

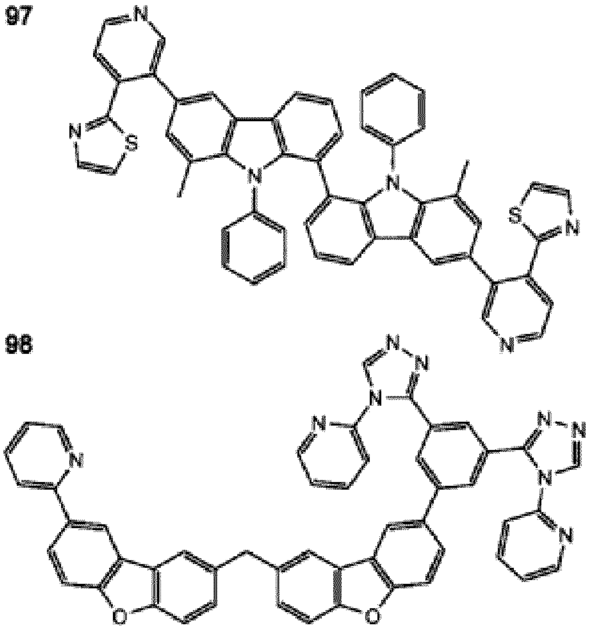

98

99

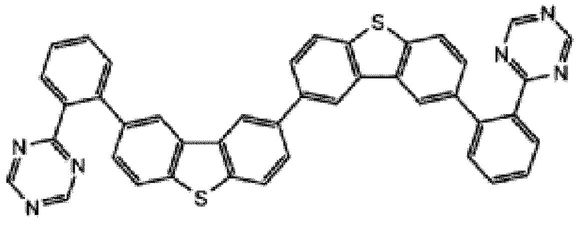

[Chemical Formula 29]

100

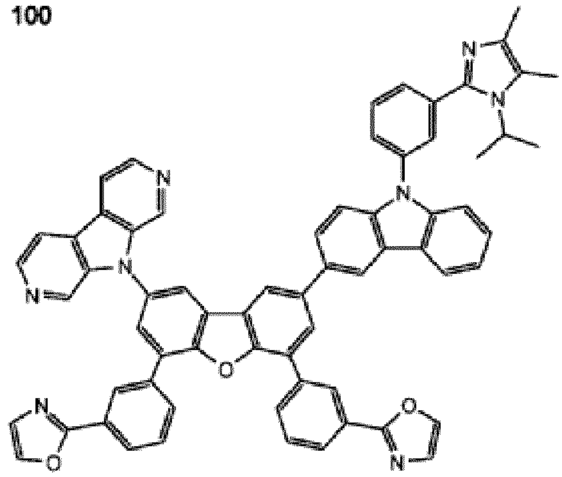

101

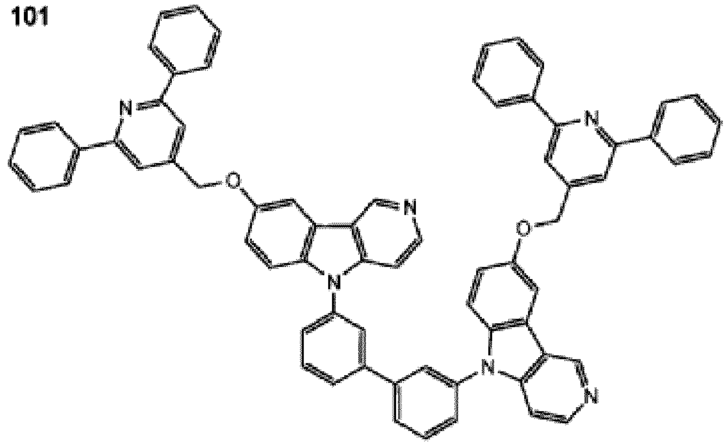

102

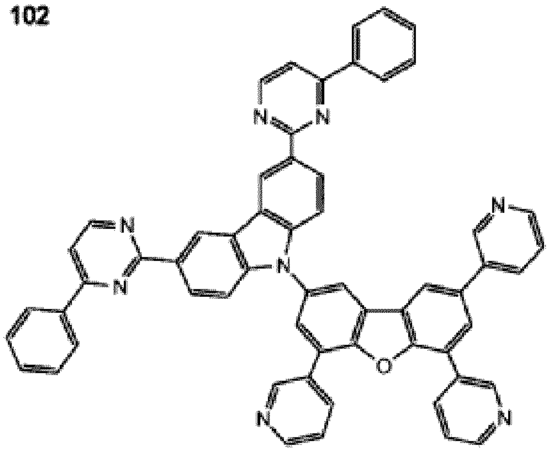

[Chemical Formula 30]

103

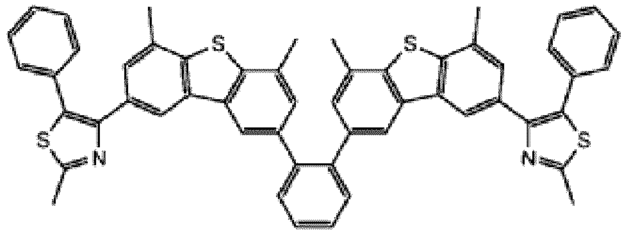

104

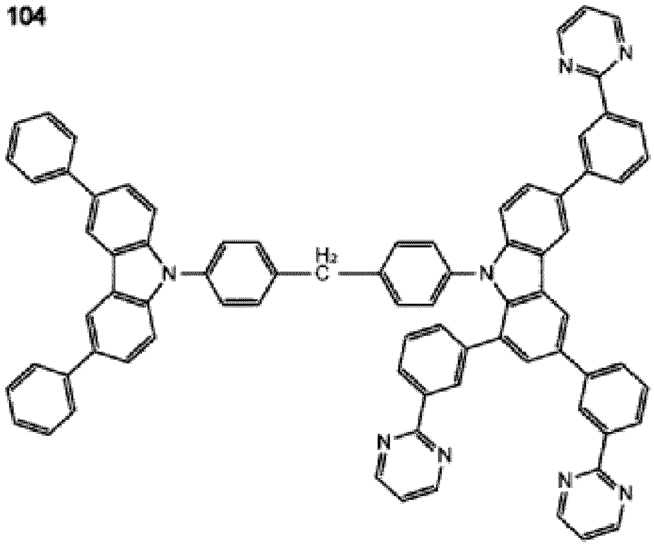

105

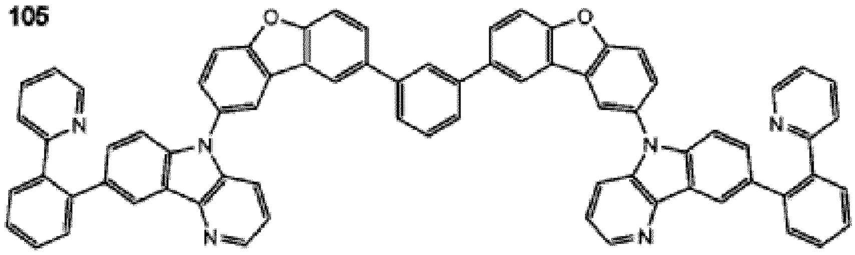

106

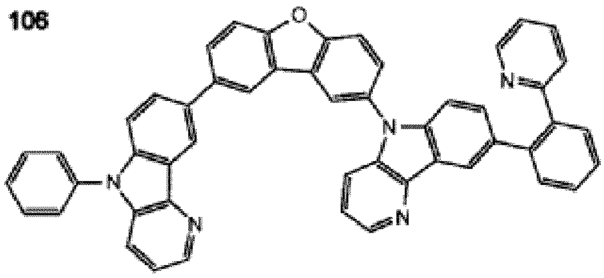

[Chemical Formula 31]

107

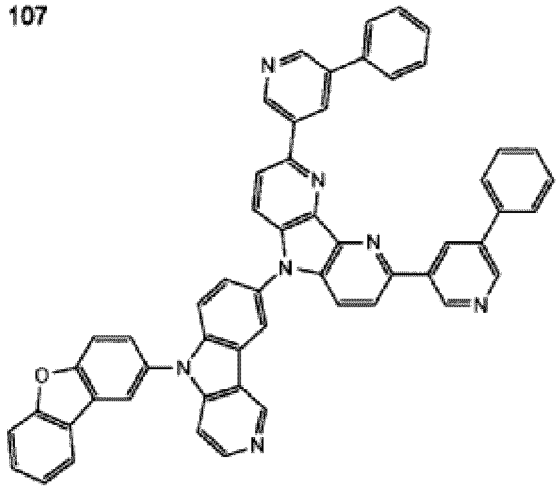

108

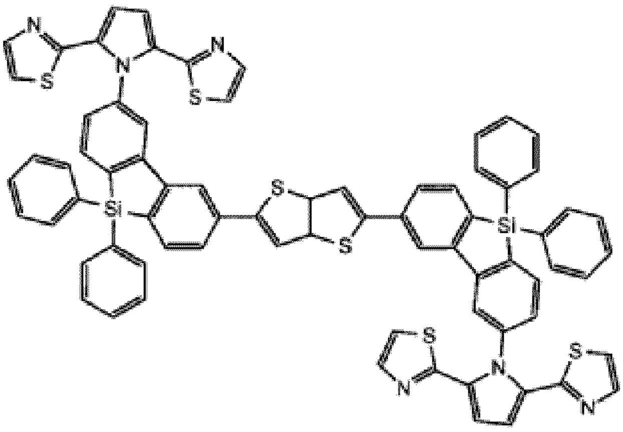

109

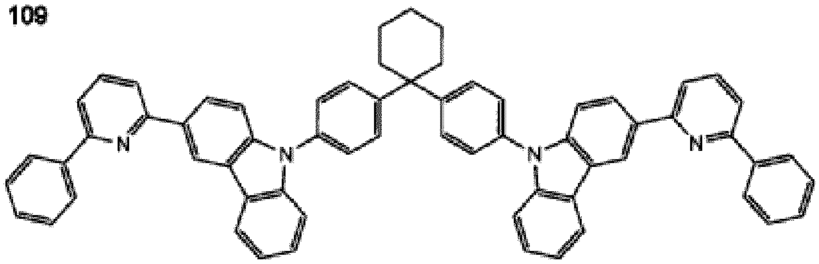

[Chemical Formula 32]

110

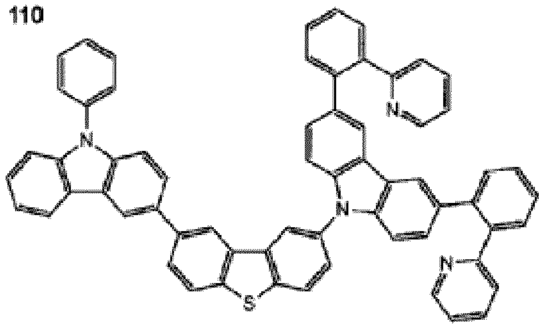

111

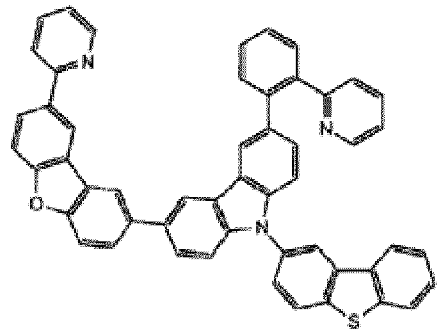

112

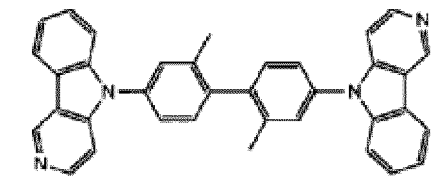

113

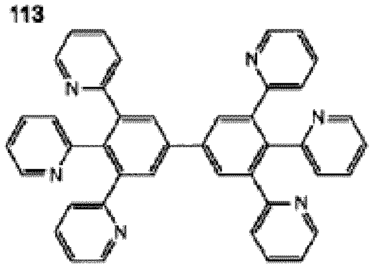

114

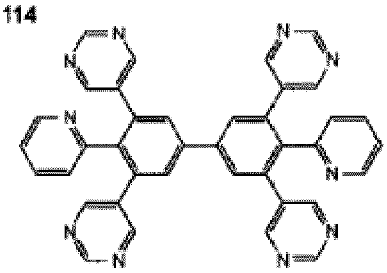

[Chemical Formula 33]

115

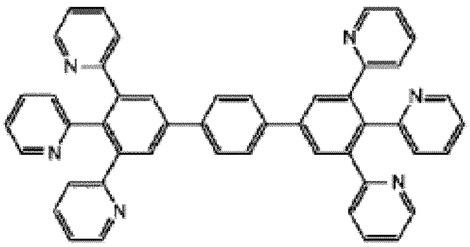

116

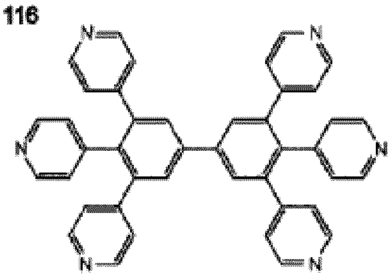

117

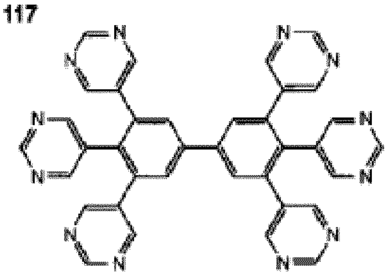

118

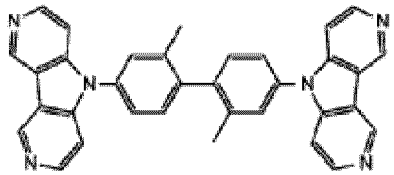

119

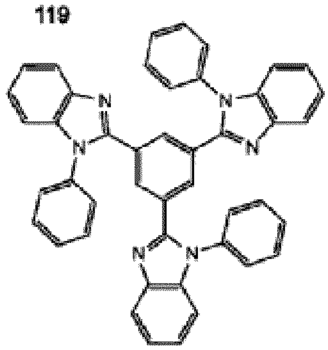

120

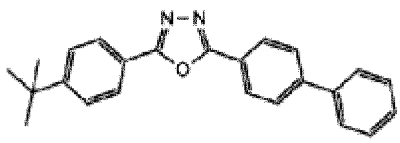

121

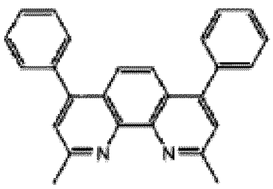

122

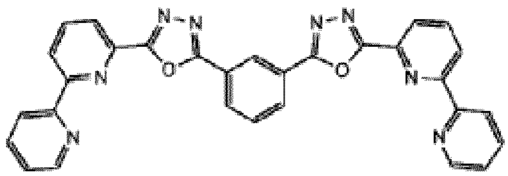

[Chemical Formula 34]

123

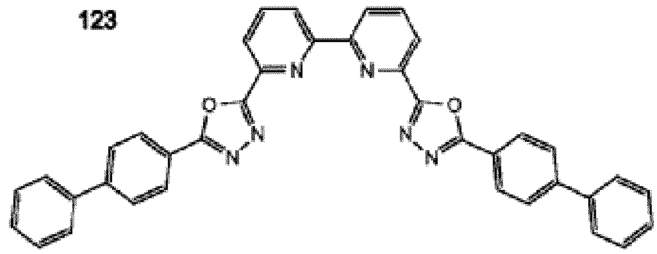

124

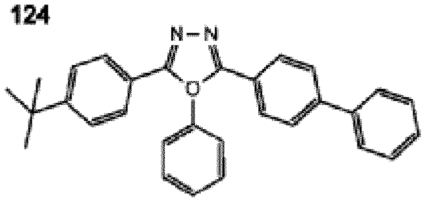

125

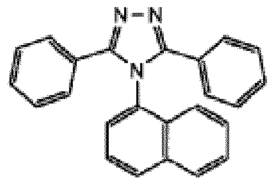

126

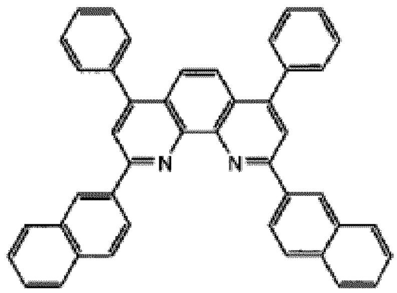

127

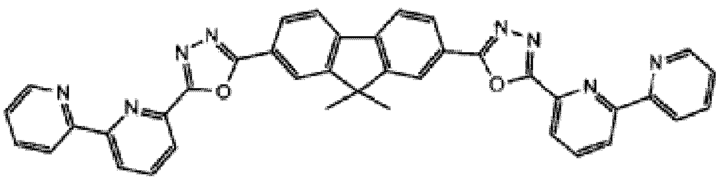

128

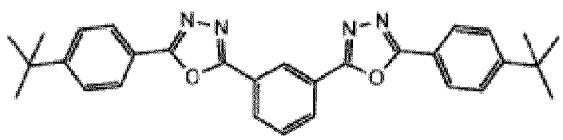

[Chemical Formula 35]

129

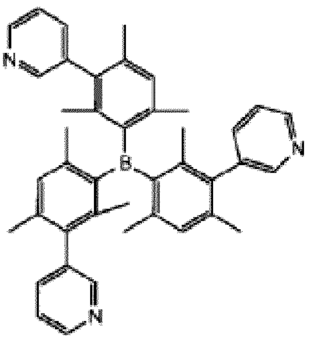

130

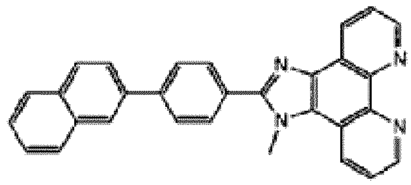

131

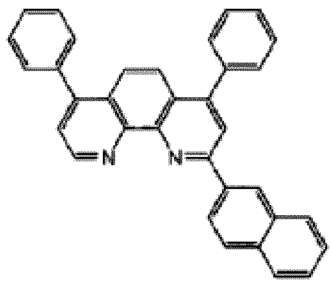

132

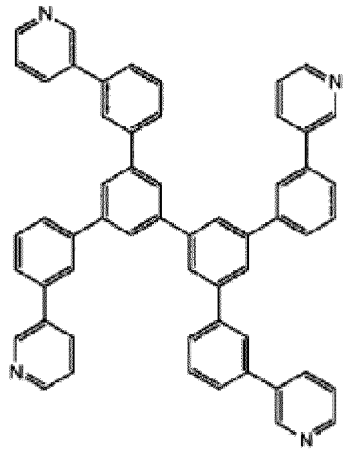

133

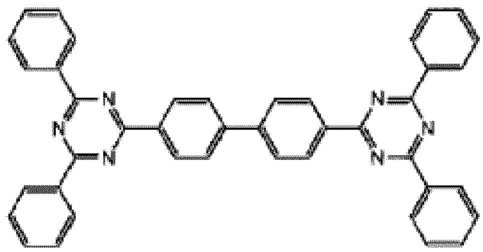

134

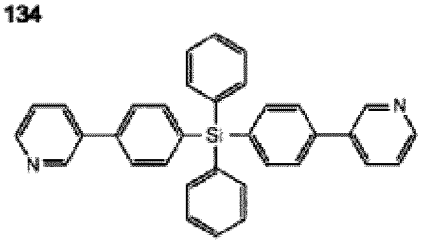

[Example of Synthesizing Compound]

As an example of synthesizing a typical compound, a concrete example of synthesizing Compound 5 is described below; however, the present invention is not limited thereto.

[Chemical Formula 36]

Synthesis of compound 5

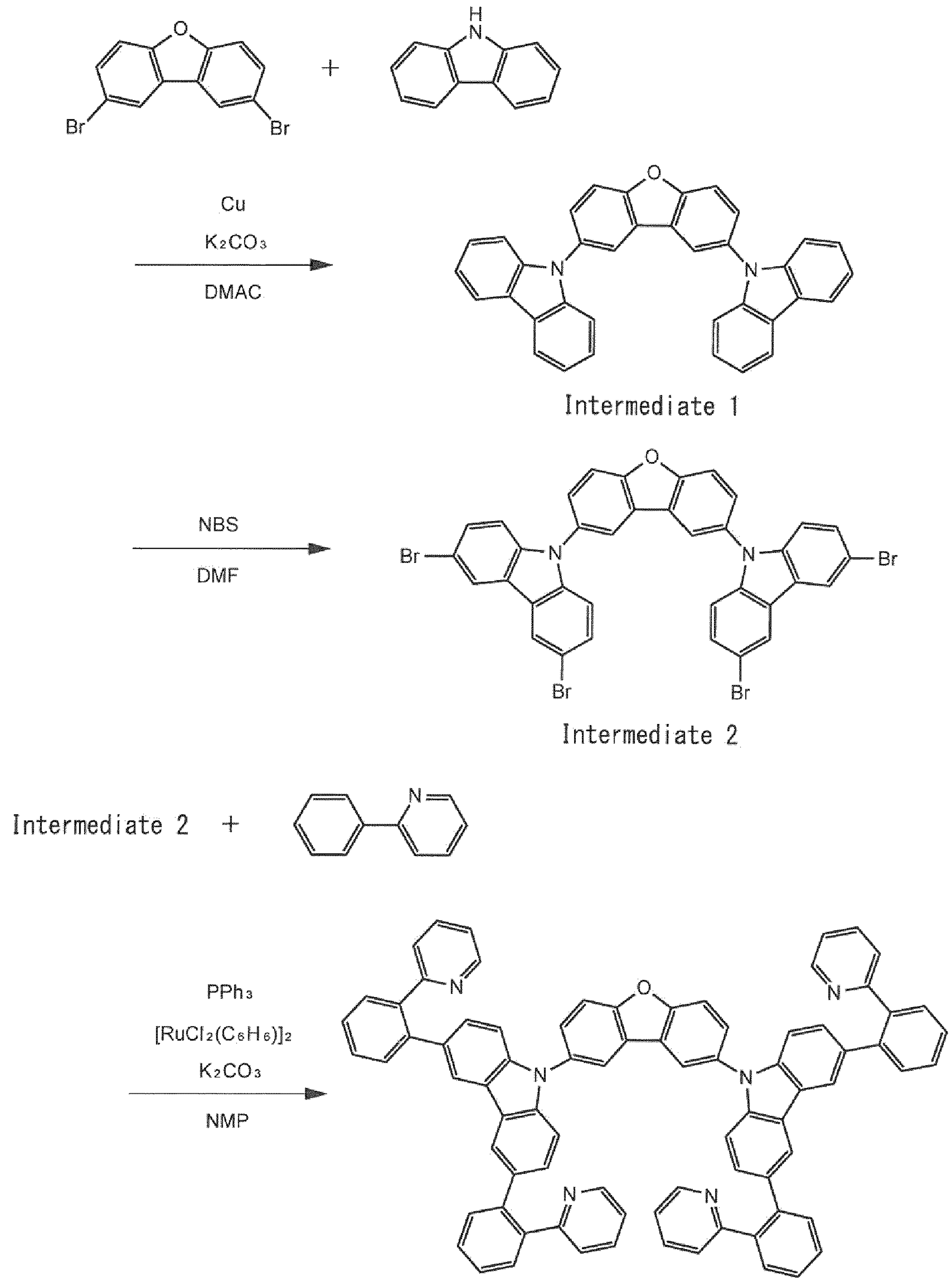


Compound 5

Process 1: (Synthesis of Intermediate 1)

Under nitrogen atmosphere, 2,8-dibromodibenzofuran (1.0 mol), carbazole (2.0 mol), copper powder (3.0 mol)

and potassium carbonate (1.5 mol) were mixed in 300 ml of DMAc (dimethylacetamide) and then stirred for 24 hours at 130°C. After the reaction liquid obtained in the above-described manner was cooled to room temperature, 1 liter of toluene was added to the reaction liquid, the resultant substance was washed three times with distilled water, the solvent was distilled off from the washed substance under a reduced pressure, and the residue was purified with silica gel flash chromatography (n-heptane : toluene = 4 : 1 to 3 : 1) to obtain an intermediate 1 at a yield of 85%.

Process 2: (Synthesis of Intermediate 2)

At room temperature under atmospheric pressure, the intermediate 1 (0.5 mol) was dissolved into 100 ml of DMF (dimethylformamide), and 2.0 mol of NBS (N-bromosuccinimide) was added, and then the resultant liquid was stirred for one night at room temperature. The obtained precipitate was filtered and washed with methanol to obtain an intermediate 2 at a yield of 92%.

Process 3: (Synthesis of Compound 5)

Under nitrogen atmosphere, the intermediate 2 (0.25 mol), 2-phenylpyridine (1.0 mol), ruthenium complex $[(\eta_6\text{-}C_6H_6)RuCl_2]_2$ (0.05 mol), triphenylphosphine (0.2 mol), and potassium carbonate (12 mol) were mixed in 3 liters of NMP (N-methyl-2-pyrrolidone), and then the mixture was stirred for one night at 140°C.

After the reaction liquid was cooled to room temperature, 5 liters of dichloromethane was added to the reaction liquid, and then the reaction liquid was filtered. Next, the solvent was distilled off from the filtrate under a reduced-pressure atmosphere (800 Pa, 80°C), and the residue was purified with silica gel flash chromatography ($CH_2Cl_2$:$Et_3N$=20:1 to 10:1).

After the solvent had been distilled off from the purified substance under the reduced-pressure atmosphere, the residue was dissolved again into dichloromethane and washed three times with water. The substance obtained by washing was dried with anhydrous magnesium sulfate, and the solvent was distilled off from the dried substance under the reduced-pressure atmosphere to thereby obtain Compound 5 at a yield of 68%.

<Transparent Conductive Layer 1b>

The transparent conductive layer 1b is a layer formed adjacent to the nitrogen-containing layer 1a by using silver or an alloy having silver as a main component.

In the case where the transparent conductive layer 1b is formed by using silver (Ag), the Ag content is 3 atm% or higher. While in the case where the transparent conductive layer 1b is formed by using an alloy containing silver (Ag) as a main component, the silver (Ag) content is 3 atm% or higher. Examples of the metal possible to be used with silver include aluminum (Al), indium (In), tin (Sn), copper (Cu), palladium (Pd), gold (Au), platinum (Pt), magnesium (Mg) and the like.

Alternatively, in the transparent conductive layer 1b described above, the layer of silver or an alloy having silver as a main component may include a plurality of layers, according to necessity, laminated one on another.

Further, it is preferred that the film-thickness of the transparent conductive layer 1b is in a range between 4 nm and 12 nm. By setting the film-thickness to 12 nm or less, absorption component or reflection component of the layer is reduced, and therefore the light transmittance of the transparent conductive layer can be maintained, which is desirable. While by setting the film-thickness to 4 nm or more, the electrical conductivity of the layer can be maintained.

The transparent conductive layer 1b may be formed by a method based on wet process, a method based on dry process or the like, wherein examples of the method based on wet process include a coating method, an ink-jet method, a dipping method and the like, and the method based on dry process include a deposition method (e.g., a resistance heating method, an EB method or the like), a sputtering method, a CVD method and the like). Among these methods, the deposition method is preferably used. The transparent conductive layer 1b is characterized that, by being formed on the nitrogen-containing layer 1a, the transparent conductive layer 1b has sufficient electrical conductivity without performing high-temperature annealing or the like after film-formation; however, high-temperature annealing or the like may also be performed after film-formation according to necessity.

<Effect of Transparent Conductive Layer>

The transparent conductive layer 1b having the aforesaid configuration is formed adjacent to the nitrogen-containing layer 1a, which is formed by using a compound containing nitrogen atom, and is formed by using silver or an alloy containing silver as a main component. Thus, when forming the transparent conductive layer 1b adjacent to the nitrogen-containing layer 1a, the silver atom (which constitutes the transparent conductive layer 1b) will interact with the compound containing nitrogen atom (which constitutes the nitrogen-containing layer 1a), so that diffusion distance of

silver atom on the surface of the nitrogen-containing layer 1a is reduced, and therefore aggregation of silver is inhibited. Therefore, the silver thin-film will be formed in a manner in which the silver thin-film grows in a single-layer growth mode (Frank-van der Merwe: FW mode); while in contrast, the silver thin-film is generally formed in a manner in which the silver thin-film grows in a nuclear growth mode (Volumer-Weber: VW mode) and thereby the silver thin-film tends to be isolated into an island shape. Thus, it is possible to obtain the transparent conductive layer 1b which has thin yet uniform film-thickness.

Particularly, the effective action energy ΔEef shown in the aforesaid Formula (1) is defined as the interaction energy between the compound constituting the nitrogen-containing layer 1a and silver constituting the transparent conductive layer 1b, and a compound which satisfies a condition of "$-0.5 \leq \Delta Eef \leq -0.10$" is used to constitute the nitrogen-containing layer 1a. Thus, it becomes possible to form the nitrogen-containing layer 1a by using a compound with which the effect of "inhibiting aggregation of silver" can be reliably achieved. This is also confirmed by facts, which will be described in detail in the below-mentioned examples, that a transparent conductive layer 1b having ultrathin thickness as well as having sheet resistance measurable by a 4-probe method or the like is formed on such a nitrogen-containing layer 1a.

As a result, it is possible to reliably obtain a transparent conductive layer 1b adjacent to such a nitrogen-containing layer 1a, wherein the light transmissibility of the transparent conductive layer 1b is ensured due to the thin film-thickness while the electrical conductivity of the transparent conductive layer 1b is ensured due to the uniform film-thickness, so that it becomes possible to improve both the electrical conductivity and the light transmissibility of the transparent conductive layer 1b that uses silver.

Since indium (In), which is a kind of rare metal, is not used as the main component, the cost of the transparent electrode 1 is low; and further, since chemically unstable material, such as ZnO or the like, is not used, the transparent electrode 1 is excellent in long-term reliability.

Incidentally, the transparent conductive layer 1b having the aforesaid configuration is not limited to be used in the organic electroluminescence element to be described below, but may also be used in various types of electronic devices. Examples of the electronic devices include organic electroluminescence elements, LED (Light Emitting Diode), liquid crystal elements, solar cells, touch panels and the like; in these electronic devices, the transparent conductive layer 1b arranged adjacent to the nitrogen-containing layer 1a can be used as an electrode layer having light transmissibility.

<<2. First Embodiment>>

(An example in which a transparent conductive layer is arranged between two light emitting units)

FIG. 2 is a view showing a cross-sectional configuration of a first embodiment of an organic electroluminescence element with a tandem structure that uses the aforesaid transparent conductive layer 1b. The configuration of an organic electroluminescence element EL-1 will be described below with reference to FIG. 2.

<Configuration of Organic Electroluminescence Element EL-1>

The organic electroluminescence element EL-1 shown in FIG. 2 is arranged on a substrate 11, and is formed by laminating a first electrode 5, a first light emitting unit 3-1, a nitrogen-containing layer 1a, a transparent conductive layer 1b, a second light emitting unit 3-2 and a second electrode 7, in this order, from the side of the substrate 11. It is characterized that, in the organic electroluminescence element EL-1, the aforesaid laminate formed by the nitrogen-containing layer 1a and the transparent conductive layer 1b is sandwiched between the two light emitting units 3-1 and 3-2.

The details of each layer constituting the organic electroluminescence element EL-1 will be described below in the order of: the substrate 11, the first electrode 5 and second electrode 7, the nitrogen-containing layer 1a and transparent conductive layer 1b, the light emitting units 3-1 and 3-1, other components, and method for producing the organic electroluminescence element.

<Substrate 11>

Examples of the substrate 11, on which the organic electroluminescence element is arranged, include but not limited to glass, plastic and the like. Further, the substrate 11 may be either transparent or non-transparent. In the case where the organic electroluminescence element EL-1 is a bottom emission type organic electroluminescence element in which the light is extracted from the side of the substrate 11, the substrate 11 is preferably transparent. Examples of the material favorably to be used as the transparent substrate 11 11 include a glass, quartz, and a transparent resin film.

Examples of the glass include silica glass, soda-lime-silica glass, lead glass, borosilicate glass, alkali-free glass, and the like. From the viewpoint of durability, smoothness, and adhesiveness with the nitrogen-containing layer 1a, the

surface of the aforesaid glass materials is subjected to a physical treatment such as polishing, and/or formed with an inorganic material coat, an organic material coat or a hybrid coat according to necessity, wherein the hybrid coat is obtained by combining the inorganic material coat and the organic material coat.

Examples of the material for the resin film include polyesters (such as polyethylene terephthalate (PET) and polyethylene naphthalate (PEN)), polyethylene, polypropylene, cellophane, cellulose esters and their derivatives (such as cellulose diacetate, cellulose triacetate, cellulose acetate butylate, cellulose acetate propionate (CAP), cellulose acetate phthalate (TAC), and cellulose nitrate), polyvinylidene chloride, polyvinylalcohol, polyethylenevinylalcohol, syndiotactic polystyrene, polycarbonate, norbornane resin, polymethylpentene, polyetherketone, polyimide, polyether sulfone (PES), polyphenylene sulfide, polysulfones, polyether imide, polyetherketone imide, polyamide, fluorine resin, nylon, polymethyl methacrylate, acryl or polyarylates, and cyclo-olefin resins (such as ARTON (trade name, manufactured by JSR Corp.) and APEL (trade name, manufactured by Mitsui Chemicals Inc.)).

The surface of the resin film may be formed with an inorganic material coat, an organic material coat or a hybrid coat, wherein the hybrid coat is obtained by combining the inorganic material coat and the organic material coat. It is preferred that the aforesaid coats and hybrid coat are each a barrier film having a water vapor permeability of 0.01 g/($m^2$•24h) or less (at temperature of 25$\pm$0.5°C and relative humidity of (90$\pm$2)%RH) measured by a method in conformity with JIS K 7129-1992. It is further preferred that the aforesaid coats and hybrid coat are each a high barrier film having an oxygen permeability of $1\times10^{-3}$ ml/($m^2$•24h•atm) or less and a water vapor permeability of $1\times10^{-5}$ g/($m^2$•24h) or less measured by a method in conformity with JIS K 7126-1987.

Any material capable of preventing penetration of substances that cause the element to degrade, such as moisture, oxygen and the like, may be used to form the aforesaid barrier film, and examples of such material include silicon oxide, silicon dioxide, silicon nitride and the like. Further, in order to reduce the fragility of the barrier film, it is more preferred that the barrier film has a laminated structure composed of the aforesaid inorganic layer and organic material layer (i.e., organic layer). There is no particular limitation on the order of laminating the inorganic layer and organic layer; however, it is preferred that the both layers are alternately laminated multiple times.

There is no particular limitation on the method of forming the barrier film. For example, the barrier film may be formed by a vacuum deposition method, a sputtering method, a reactive sputtering method, a molecular beam epitaxy method, a cluster ion beam method, an ion plating method, a plasma polymerization method, an atmospheric pressure plasma polymerization method, a plasma CVD method, a laser CVD method, a thermal CVD method, a coating method or the like; and it is particularly preferred that the barrier film is formed by an atmospheric pressure plasma polymerization method described in Japanese Unexamined Patent Application Publication No. 2004-68143.

On the other hand, in the case where the substrate 11 is non-transparent, a metal plate (such as an aluminum plate, a stainless steel plate or the like), a film, a non-transparent resin substrate, a ceramic substrate or the like may be used as the substrate 11.

<First Electrode 5 and Second Electrode 7>

Either one of the first electrode 5 and second electrode 7 functions as an anode for supplying holes to the light emitting units 3-1 and 3-2, and the other functions as a cathode for supplying electrons to the light emitting units 3-1 and 3-2. Here, as an example, the first electrode 5 arranged on the side of the substrate 11 is the anode, and the second electrode 7 arranged on the opposite side is the cathode.

Further, at least one or both of the first electrode 5 and second electrode 7 is an electrode layer functioning as a transparent electrode. For example, in the case where the first electrode 5 arranged on the side of the substrate 11 is configured as the transparent electrode, the luminescence light h generated by the light emitting units 3-1, 3-2 is extracted from the side of the substrate 11, so that the organic electroluminescence element EL-1 is a bottom emission type organic electroluminescence element. While in the case where the second electrode 7 is configured as the transparent electrode, the luminescence light h generated by the light emitting units 3-1, 3-2 is extracted from the side opposite to the side of the substrate 11, so that the organic electroluminescence element EL-1 is a top emission type organic electroluminescence element. In such a case, the other electrode may be an electrode film functioning as a reflective electrode. In contrast, in the case where the first electrode 5 and the second electrode 7 are both configured as the transparent electrode, the organic electroluminescence element EL-1 is a dual emission type organic electroluminescence element. Described here is an example in which the first electrode 5 is configured as the transparent electrode.

Further, the first electrode 5 and the second electrode 7 are both formed by using a metal, an alloy, an organic or inorganic conductive compound, or a mixture of these materials. To be specific, the first electrode 5 and the second electrode 7 are both formed by using gold, aluminum, silver, magnesium, lithium, a magnesium/copper mixture, a magnesium/silver mixture, a magnesium/aluminum mixture, a magnesium/indium mixture, indium, a lithium/aluminum mixture, a rare earth metal, an oxide semiconductor (such as ITO, ZnO, TiO2, SnO2 and the like) or the like. From these materials, a material suitable as a cathode material or an anode material is selected to configure the first electrode 5 and the second electrode 7.

Further, from these materials, a material with high light transmissibility is used to configure the electrode arranged on the light extraction side, which is either one of the first electrode 5 and the second electrode 7 (here, the first electrode is such electrode). Examples of such material include an oxide semiconductor (such as ITO, ZnO, TiO2, SnO2 and the like) and the like.

The first electrode 5 and the second electrode 7 may have a laminated structure according to necessity. In such a configuration, only a part where the light emitting units 3-1 and 3-2 are sandwiched by the first electrode 5 and second electrode 7 is a light-emitting region of the organic electroluminescence element EL-1.

The first electrode 5 and second electrode 7 described above can be produced by forming a thin-film with the aforesaid conductive material by a method such as deposition, sputtering or the like. At this time, the sheet resistance of the first electrode 5 and second electrode 7 is preferably several hundred Ω/□ or less; and the film-thickness of the first electrode 5 and second electrode 7 is generally within a range from 5 nm to 5 μm, preferably within a range from 5 nm to 200 nm. A deposition method is used to form the second electrode on the top of the light emitting unit 3-2.

<Nitrogen-containing Layer 1a and Transparent Conductive Layer 1b>

The configuration of the nitrogen-containing layer 1a and the configuration of the transparent conductive layer 1b have been described before; and here the nitrogen-containing layer 1a and the transparent conductive layer 1b are arranged, in this order, from the side of the substrate 11, for example. Here, in the case where the first electrode 5 is the anode and the second electrode 7 is the cathode, the transparent conductive layer 1b functions as a cathode with respect to the light emitting unit 3-1 arranged on the side of the first electrode (anode) 5, and functions as an anode with respect to the light emitting unit 3-2 arranged on the side of the second electrode (cathode) 7.

The nitrogen-containing layer 1a sandwiched between the light emitting unit 3-1 and the transparent conductive layer 1b may also be regarded as a layer that forms a portion of the light emitting unit 3-1. In such a case, since the transparent conductive layer 1b functions as a cathode with respect to the light emitting unit 3-1, it is preferred that the nitrogen-containing layer 1a sandwiched between the light emitting unit 3-1 and the transparent conductive layer 1b is formed by using a material having electron transport performance or electron injection performance selected from the materials which satisfy the aforesaid Formula (1) or Formula (2). Further, such a nitrogen-containing layer 1a may also be formed by using a material satisfying Formula (1) or Formula (2) and selected from the below-mentioned materials exemplified as electron transporting materials.

In the case where the nitrogen-containing layer 1a is used as a layer having electron transport performance or electron injection performance as described above, an alkali metal, an alkali metal salt, an alkali earth metal, an alkaline earth metal salt or the like may be mixed into the nitrogen-containing layer 1a, so that the nitrogen-containing layer 1a contains such material. To be specific, examples of these materials include metals such as lithium, potassium, sodium, cesium, magnesium, calcium, and strontium, and salts of these metals. Examples of preferred method of mixing and adding these materials include co-deposition with a nitrogen-containing compound. With such a configuration, the moving speed of the electrons injected from the cathode toward the light emitting layer can be improved, and the luminous efficiency can be improved.

Incidentally, in the case where the first electrode 5 is the cathode and the second electrode 7 is the anode, the transparent conductive layer 1b functions as an anode with respect to the light emitting unit 3-1 arranged on the side of the first electrode (cathode) 5. Thus, it is preferred that the nitrogen-containing layer 1a sandwiched between the transparent conductive layer 1b and the light emitting unit 3-1 is formed by using a material having hole transport performance or hole injection performance.

<Light Emitting Units 3-1, 3-2>

The entire layer-structure of each of the light emitting units 3-1 and 3-2 is not particularly limited, but may individually have a generic layer-structure. As an example, the layer-structure is formed by laminating [a hole injecting layer / a hole transporting layer / a light emitting layer / an electron transporting layer / an electron injecting layer], in this order, from the side of the anode; among these layers, the light emitting layer formed by using at least an organic material is indispensable. The hole injecting layer and the hole transporting layer may also be formed as a hole transporting/injecting layer. The electron transporting layer and the electron injecting layer may also be formed as an electron transporting/injecting layer. Further, among the layers constituting each of the light emitting units 3-1 and 3-2, the electron injecting layer, for example, may also be formed of an inorganic material.

In addition to the aforesaid layers, the light emitting units 3-1 and 3-2 may have other layer (s), such as a hole blocking layer, an electron blocking layer and/or the like, laminated on required place(s) according to necessity. Further, the light emitting layer may also be formed as a light emitting layer unit which includes a plurality of emitting layers each emit light of different wavelength range, wherein the plurality of emitting layers are laminated one on another with a non-luminescent intermediate layer interposed between each two layers. The intermediate layer may function as a hole

blocking layer or an electron blocking layer.

Further, the light emitting units 3-1, 3-2 may either be configured so that they each obtain luminescence light h of the same color, or be configured so that they respectively obtain luminescence light h of different colors.

The details of each of the layers constituting the light emitting units 3-1 and 3-2 will be described below in the following order: the light emitting layer, the injecting layer, the hole transporting layer, the electron transporting layer and the blocking layer.

[Light Emitting Layer]

The light emitting layer used in the present invention contains a phosphorescent compound, for example, as a light emitting material.

The light emitting layer is a layer where electrons injected from the electrodes or the electron transporting layer and holes injected from the hole transporting layer are recombined to emit light, and light emitting portion may be either the inside of the light emitting layer or an interface between the light emitting layer and its adjacent layer.

The structure of the light emitting layer is not particularly limited as long as the light emitting material contained therein satisfies light emitting requirements. Further, the light emitting layer 3c may also include a plurality layers having the same emission spectrum and/or emission maximum wavelength. In such a case, it is preferable that a non-luminescent intermediate layer (not shown) is provided between each two adjacent light emitting layers.

The total thickness of the light emitting layers is preferably within a range from 1 nm to 100 nm and, and more preferably within a range from 1 nm to 30 nm in view of obtaining a lower driving voltage. Incidentally, the total thickness of the light emitting layers is, if the non-luminescent intermediate layer is provided between each two light emitting layers, the total thickness including the thickness of the intermediate layer(s).

In the case where the light emitting layer has a configuration in which a plurality of layers are laminated one on another, the film-thickness of each emitting layer is preferably to be adjusted to a range from 1 nm to 50 nm, and further preferably to be adjusted to a range from 1 nm to 20 nm. In the case where the plurality of laminated light emitting layers respectively correspond to luminescent colors of blue, green and red, the relationship between the film-thickness of the light emitting layer of blue, the film-thickness of the light emitting layer of green and the film-thickness of the light emitting layer of red is not particularly limited.

The aforesaid light emitting layer can be formed by forming a thin film of a light emitting material or a host compound (which are to be described later) using a known thin film forming method such as a vacuum deposition method, a spin coating method, a casting method, an LB method, an ink-jet method or the like.

Each light emitting layer may be formed of a plurality of materials in mixture; or a phosphorescent material and a fluorescent material (also referred to as "fluorescent dopant" or "fluorescent compound") may be used in mixture in the same light emitting layer.

It is preferred that the light emitting layer contains a host compound (also referred to as light-emitting host or the like) and a light emitting material (also referred to as light-emitting dopant compound), and light is emitted by the light emitting material.

(Host Compound)

It is preferred that the host compound contained in the light emitting layer is a compound whose phosphorescence quantum yield preferably is, when emitting phosphorescence at the room temperature (25°C), less than 0.1, and further preferably is less than 0.01. Further, it is preferred that the volume ratio of the host compound of the compounds contained in the light emitting layer is 50% or more.

One type of known host compound may be used as the host compound, or a plurality of types of known host compounds may be used as the host compound. By using a plurality of types of known host compounds, it is possible to adjust the transfer of electrical charges, and it is possible to improve the efficiency of the organic electroluminescence element EL-1. Further, by using a plurality of types of below-mentioned light emitting materials, it is possible to mix different colors of emission light, and thereby it is possible to obtain any luminescent color.

A known low-molecular compound, a high-molecular compound having a repeating unit, or a low-molecular compound having a polymerizable group such as a vinyl group or an epoxy group (a deposition polymerizable emission host) may be used as the host compound.

It is preferred that the known host compound is a compound which has hole transporting capability and electron transporting capability, which prevents increase in emission wavelength, and which has high Tg (glass transition temperature). The glass transition temperature (Tg) herein is a value obtained by using DSC (Differential Scanning Colorimetry) in conformity with JIS-K-7121.

Concrete examples (H1 to H79) of the host compound possible to be used in the present invention are shown below; however, the present invention is not limited thereto. Incidentally, in host compounds H68 to H79, x and y represent

ratio of a random copolymer. Such ratio can be set to x : y = 1 : 10, for example.

[Chemical Formula 37]

H1

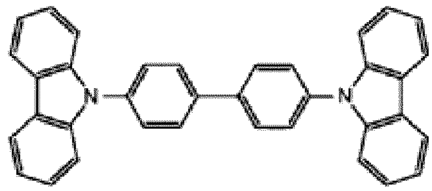

H2

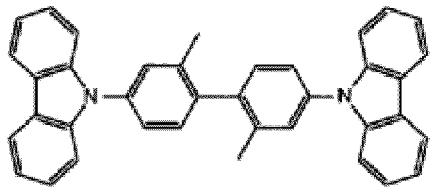

H3

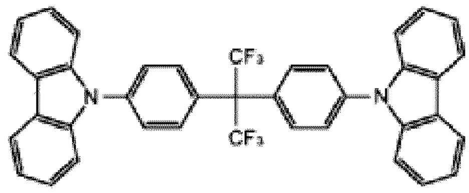

H4

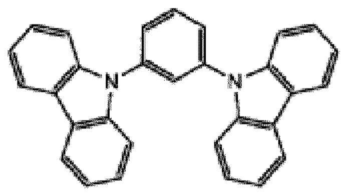

H5

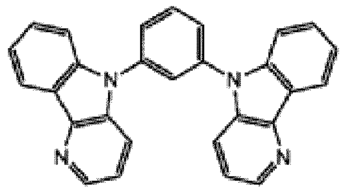

H6

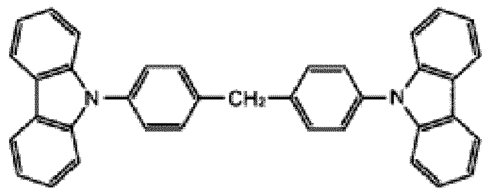

H7

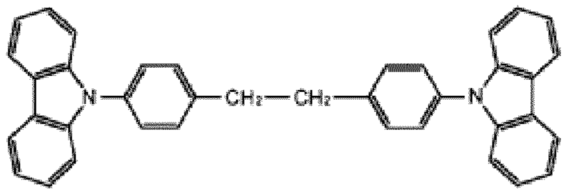

H8

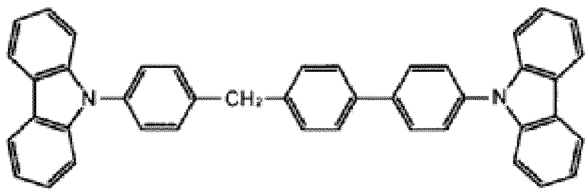

H9

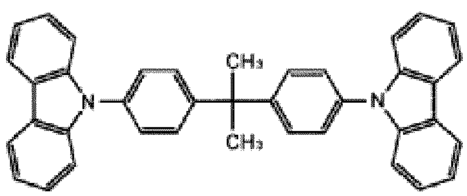

[Chemical Formula 38]

H10

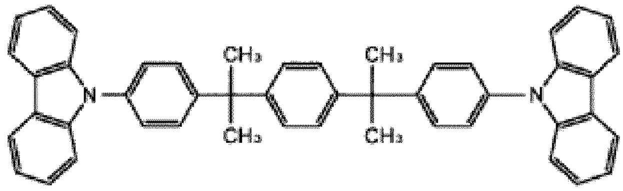

H11

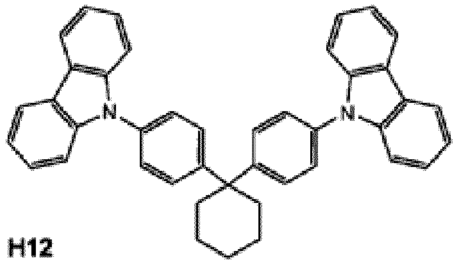

H12

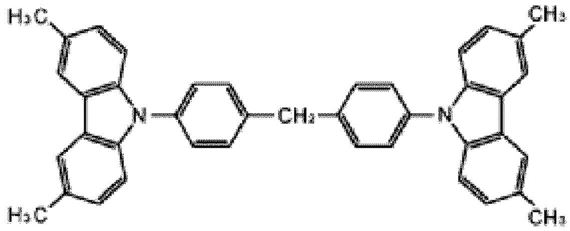

H13

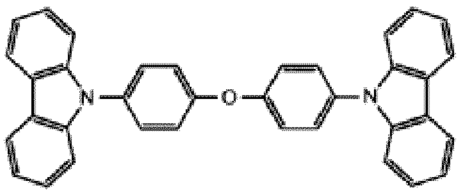

H14

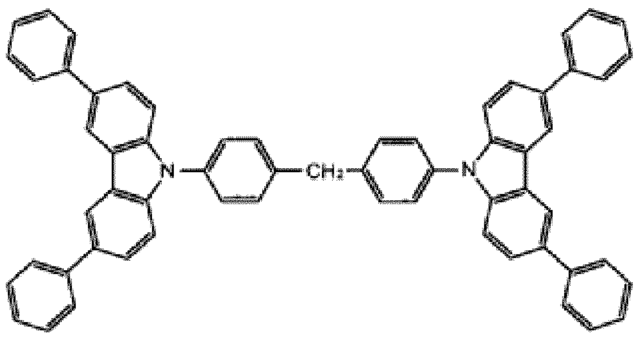

[Chemical Formula 39]

H15

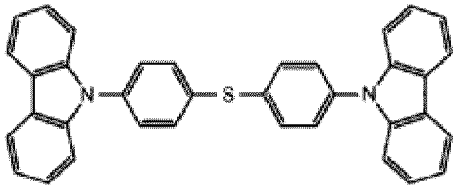

H16

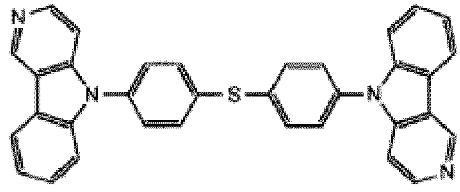

H17

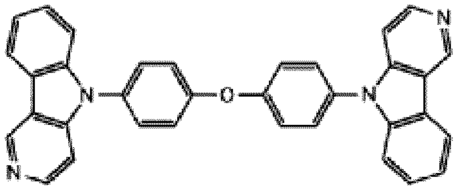

H18

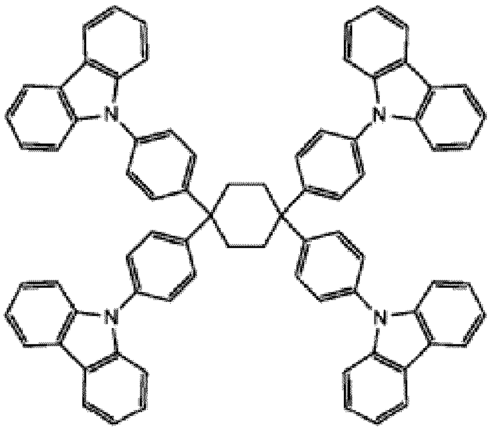

H19

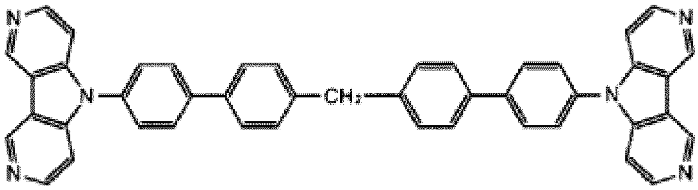

H20

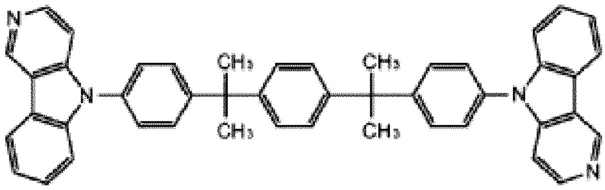

H21

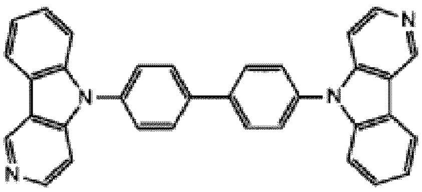

H22

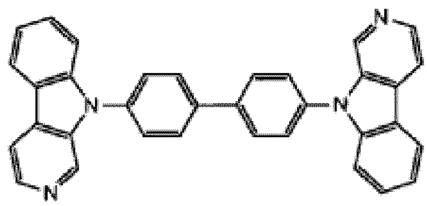

[Chemical Formula 40]

H23

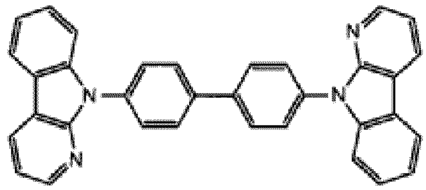

H24

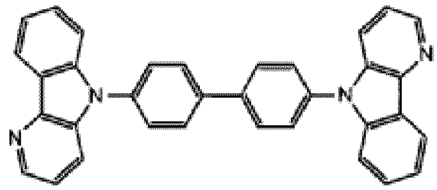

H25

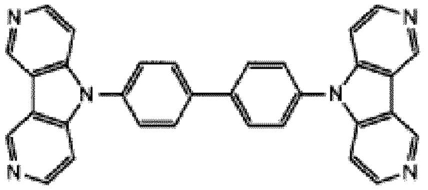

H26

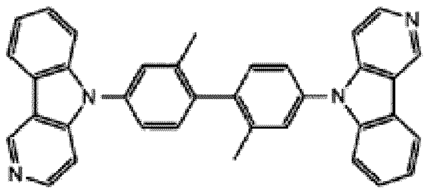

H27

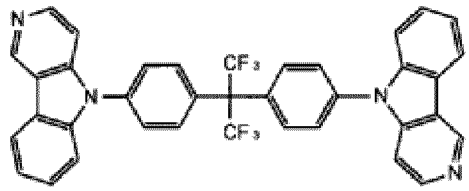

H28

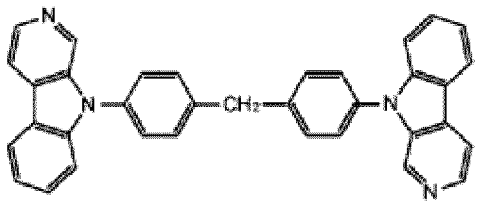

H29

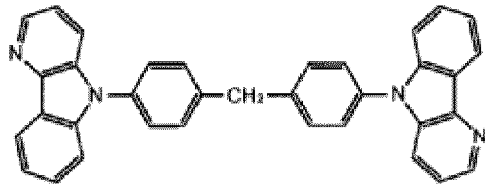

H30

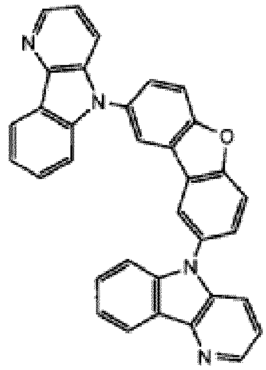

[Chemical Formula 41]

H31

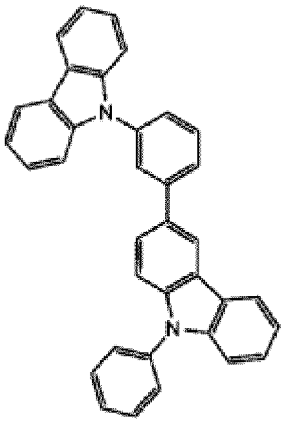

H32

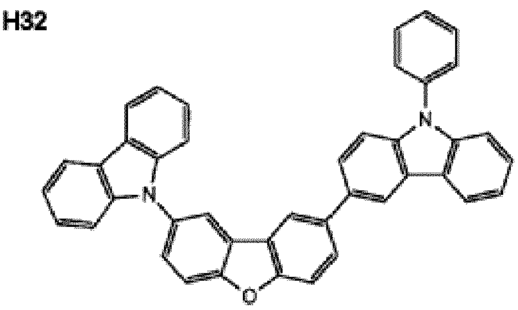

H33

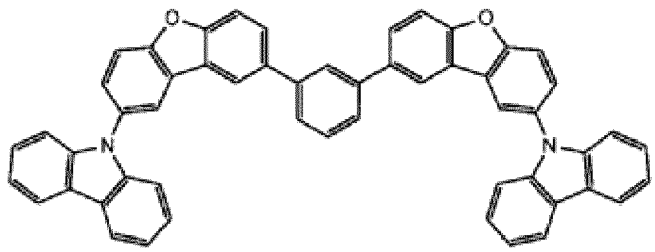

[Chemical Formula 42]

H34

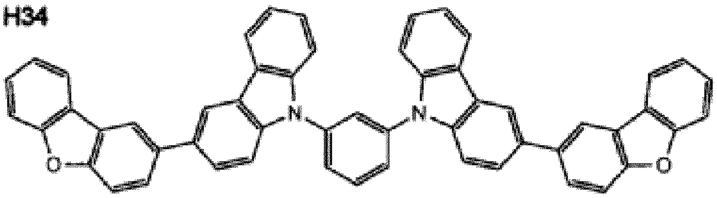

H35

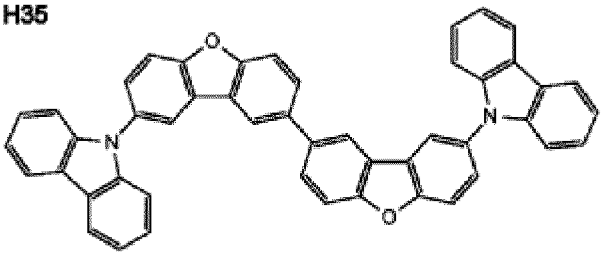

H36

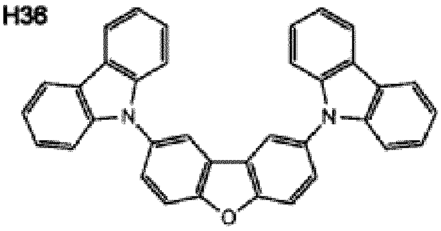

H37

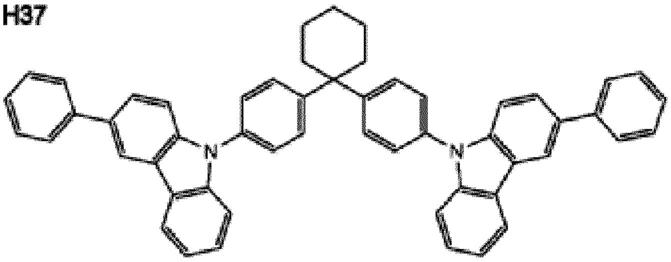

H38

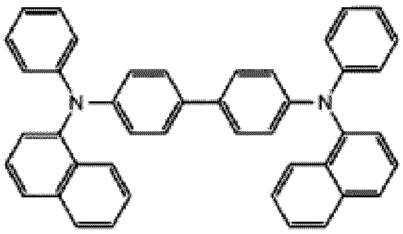

H39

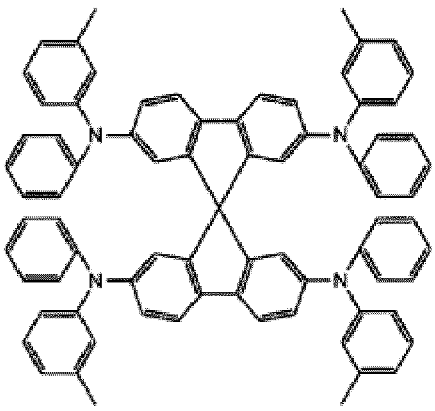

[Chemical Formula 43]

H40

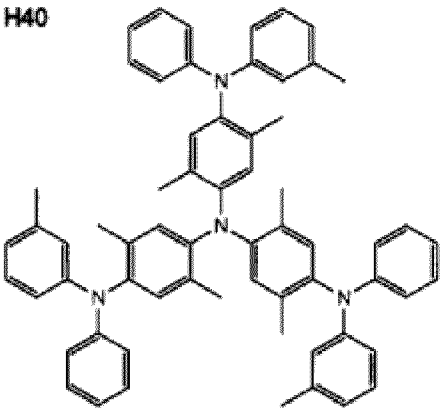

H41

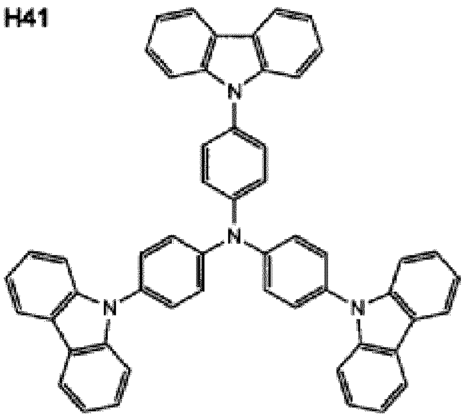

H42

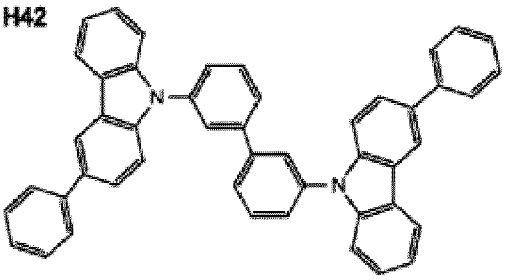

H43

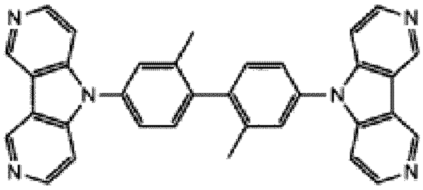

H44

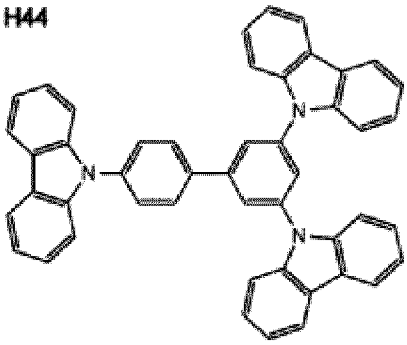

H45

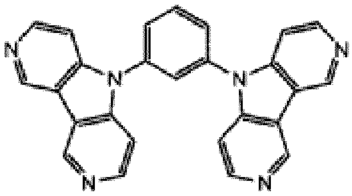

H46

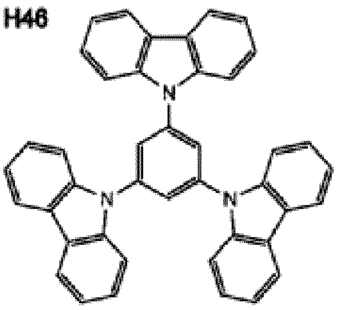

H47

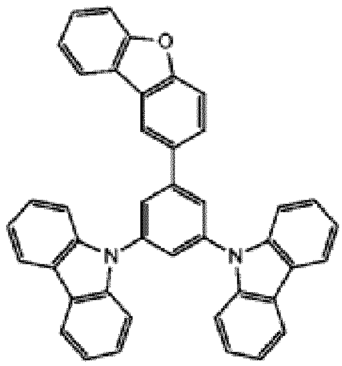

[Chemical Formula 44]

H48

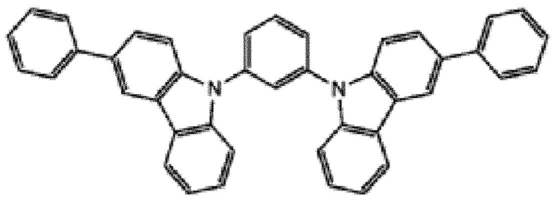

H49

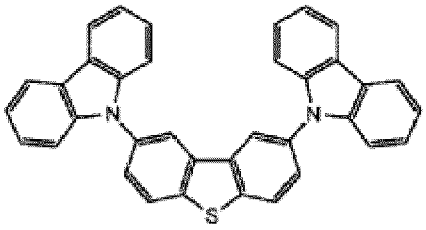

H50

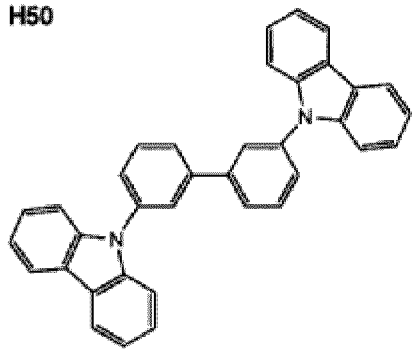

H51

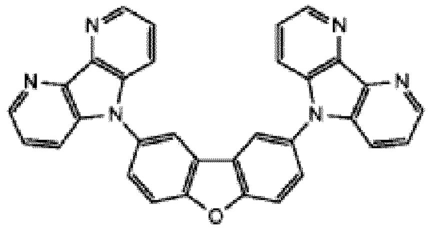

H52

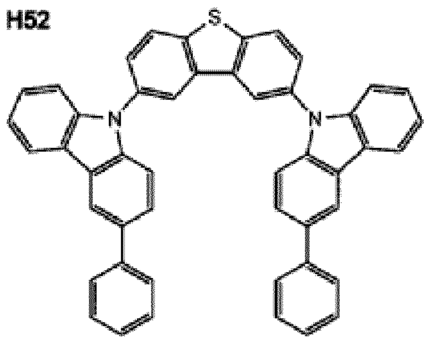

H53

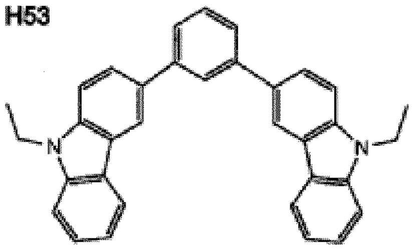

H54

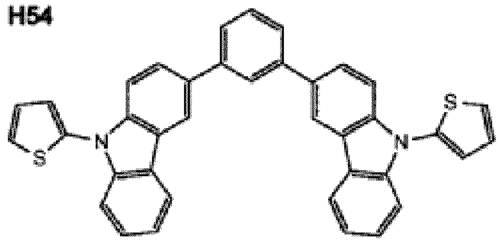

H55

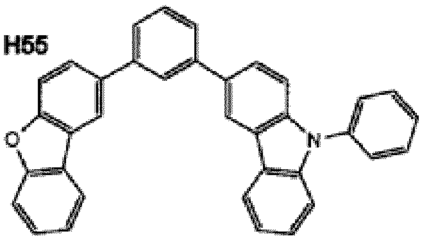

[Chemical Formula 45]

H56

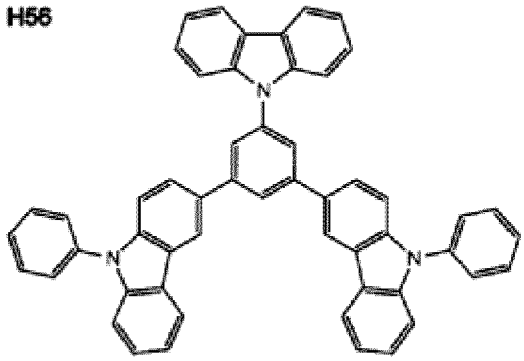

H57

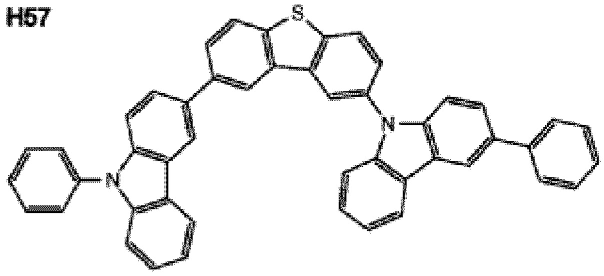

H58

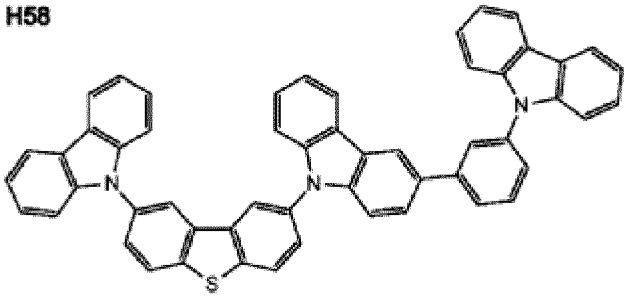

H59

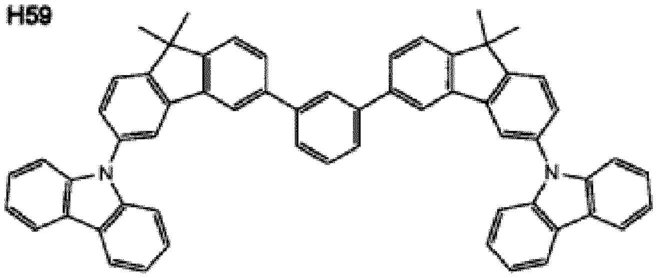

[Chemical Formula 46]

H60

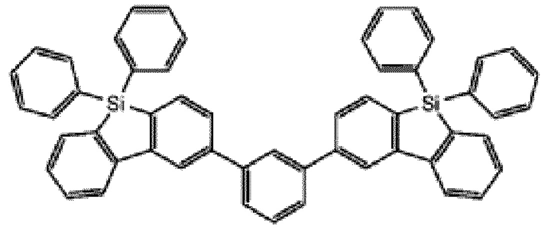

H61

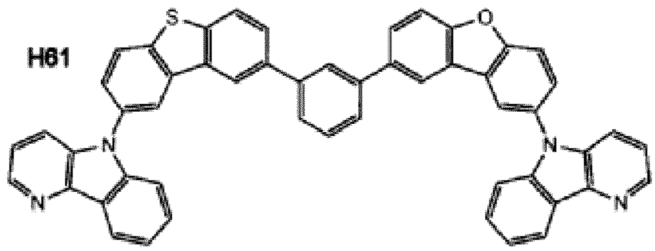

H62

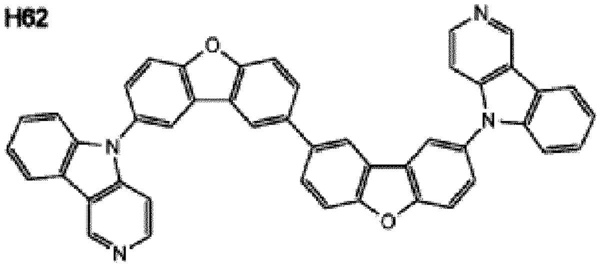

H63

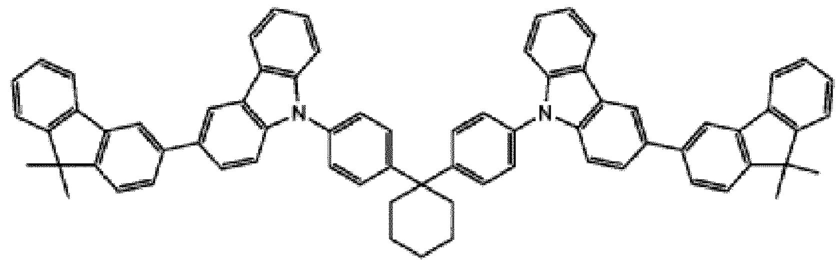

H64

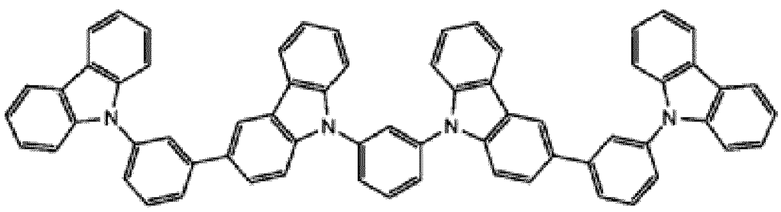

[Chemical Formula 47]

H65

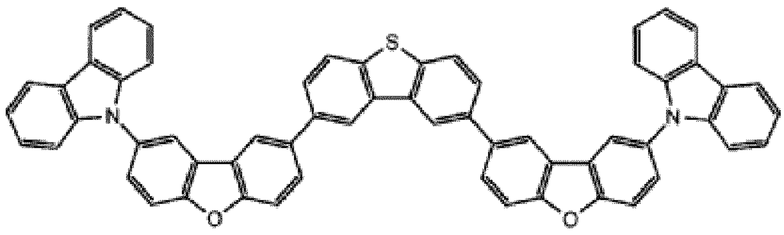

H66

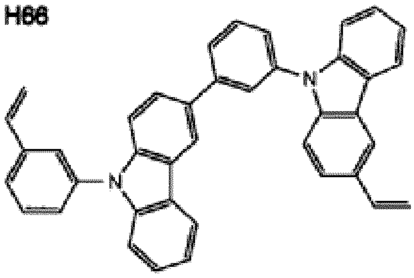

H67

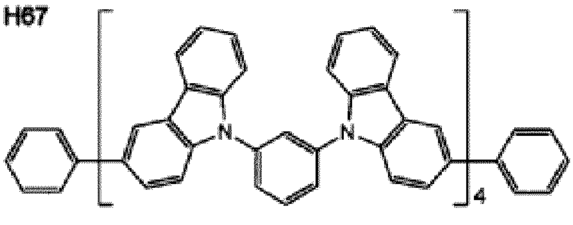

[Chemical Formula 48]

H68

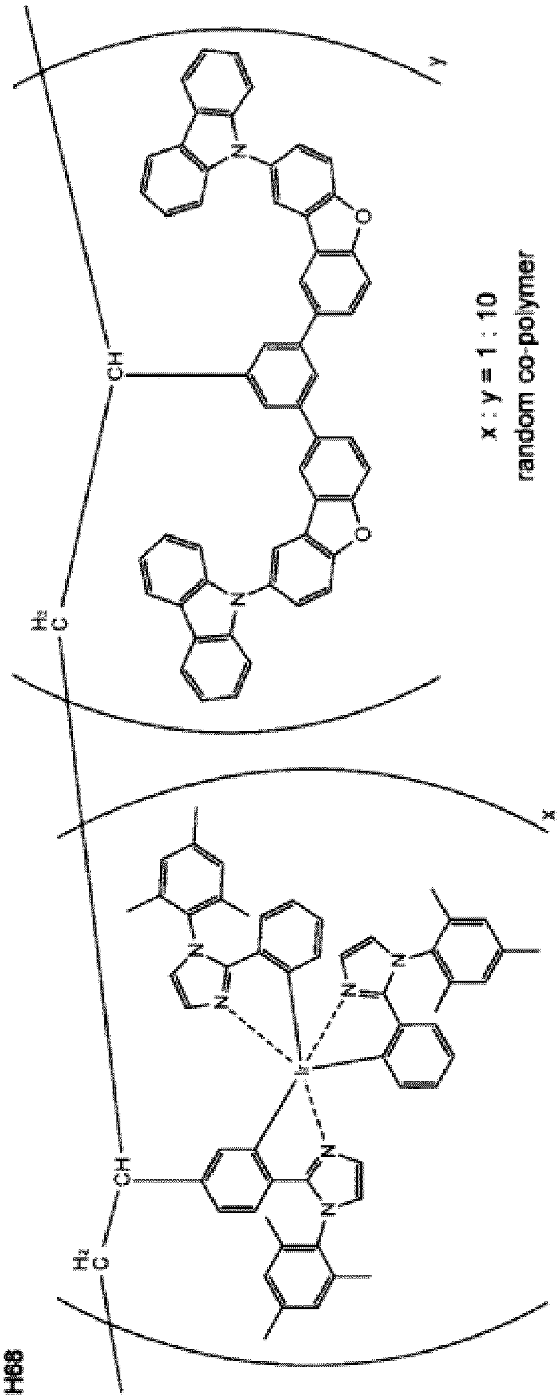

x : y = 1 : 10
random co-polymer

[Chemical Formula 49]

H69

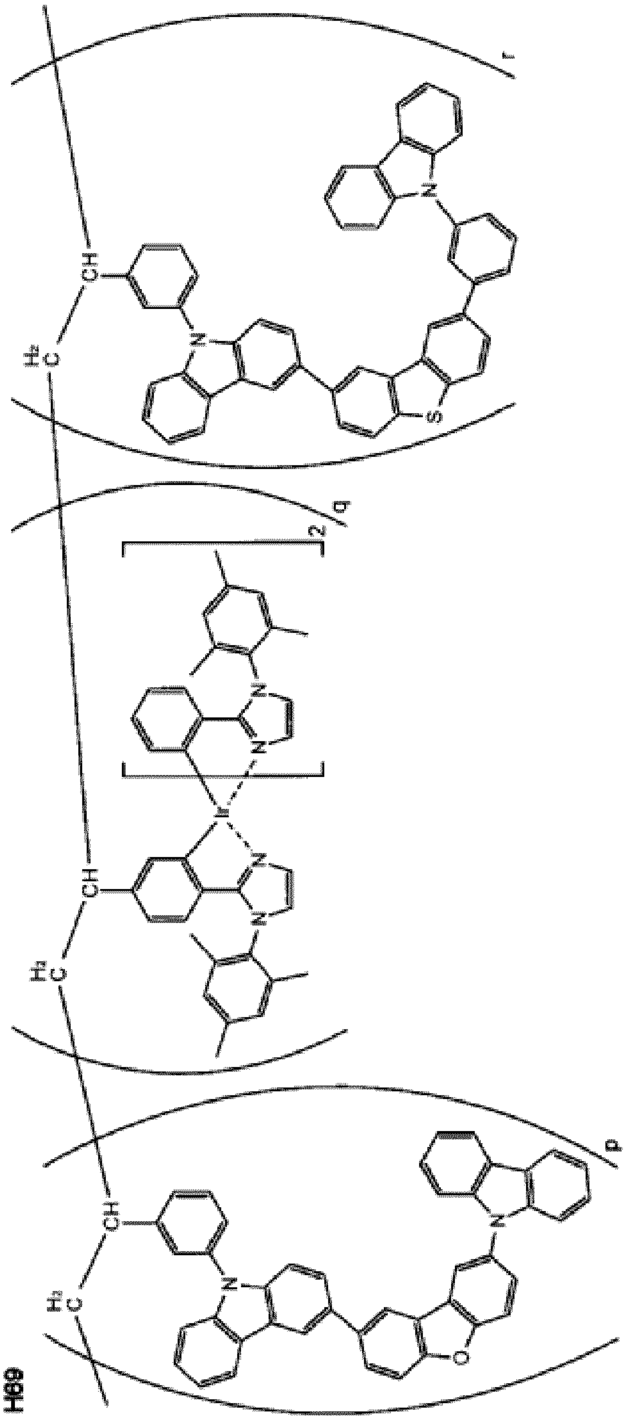

[Chemical Formula 50]

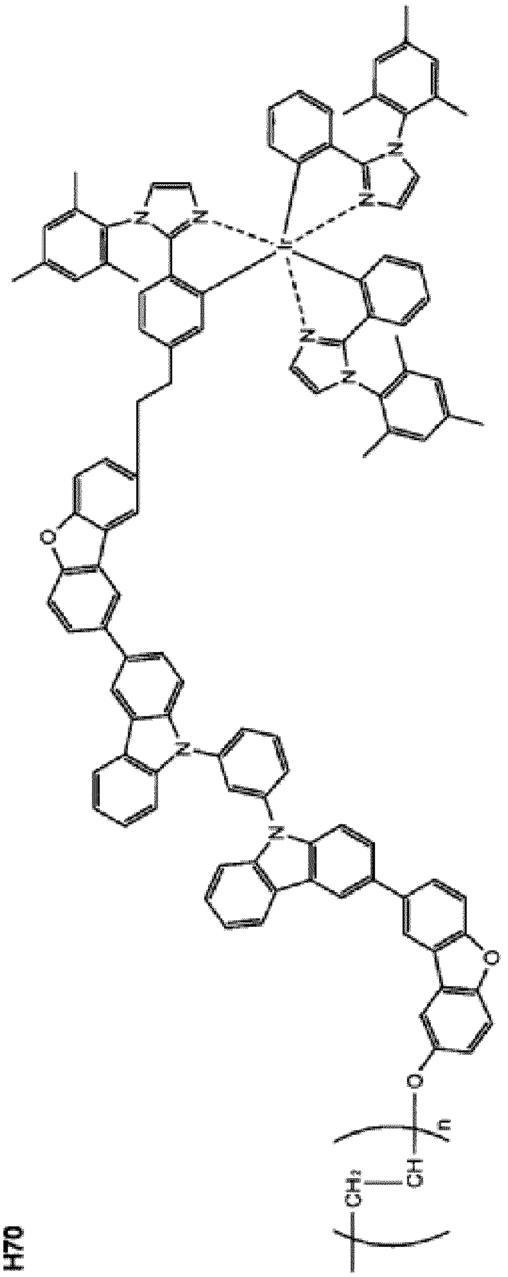

H70

[Chemical Formula 51]

H71

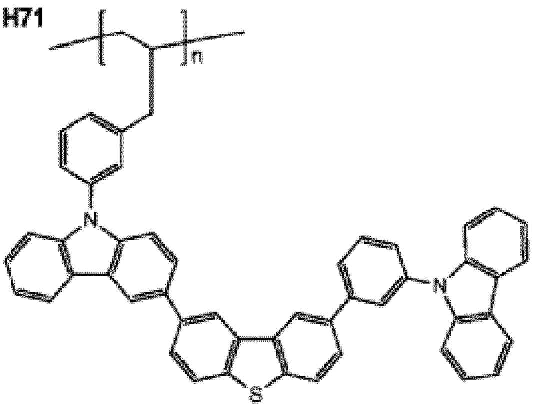

H72

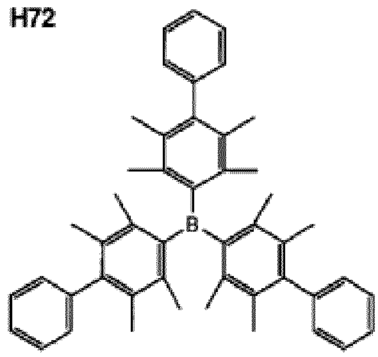

H73

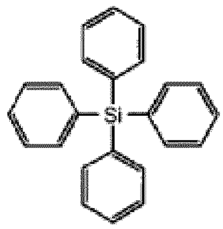

H74

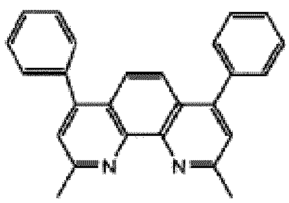

H75

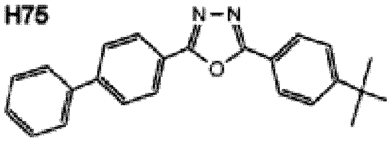

H76

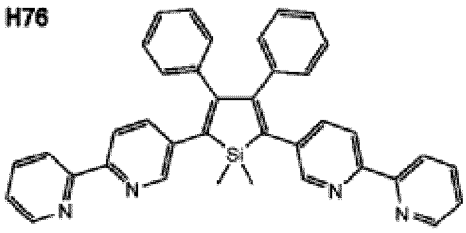

H77

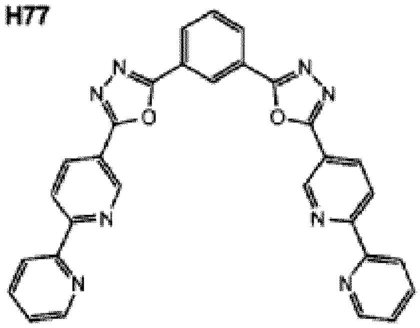

H78

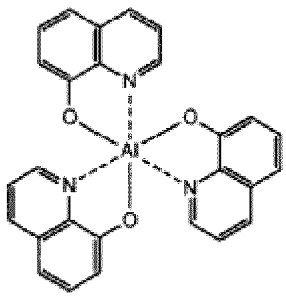

H79

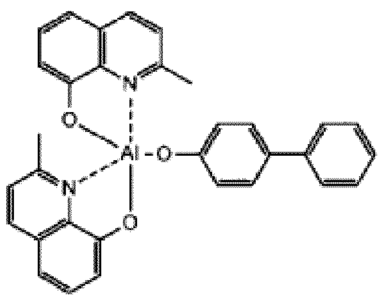

Concrete examples of the known host compound possible to be used are described in the following documents, for example: Japanese Unexamined Patent Application Publication Nos. 2001-257076, 2002-308855, 2001-313179, 2002-319491, 2001-357977, 2002-334786, 2002-8860, 2002-334787, 2002-15871, 2002-334788, 2002-43056, 2002-334789, 2002-75645, 2002-338579, 2002-105445, 2002-343568, 2002-141173, 2002-352957, 2002-203683, 2002-363227, 2002-231453, 2003-3165, 2002-234888, 2003-27048, 2002-255934, 2002-260861, 2002-280183, 2002-299060, 2002-302516, 2002-305083, 2002-305084, 2002-308837 and the like.

(Light Emitting Material)

Examples of the light emitting material possible to be used in the present invention include a phosphorescent compound (also referred to as "phosphorescent material").

A phosphorescent compound is a compound in which light emission from an excited triplet state is observed; to be specific, a phosphorescent compound is a compound which emits phosphorescence at room temperature (25°C)

and which exhibits a phosphorescence quantum yield 0.01 or more at 25°C; however, preferable phosphorescence quantum yield is 0.1 or more.

The phosphorescence quantum yield can be measured by a method described on page 398 of Spectroscopy II of Lecture of Experimental Chemistry, vol. 7, 4th edition) (1992, published by Maruzen Co., Ltd.). The phosphorescence quantum yield in a solution can be measured by using various solvents. In the present invention, in the case where a phosphorescent compound is used, it is only necessary to achieve the aforesaid phosphorescence quantum yield (0.01 or more) with any one arbitrary solvent.

Examples of light-emitting principle of the phosphorescent compound include the following two: one is an energy transfer type, in which carriers recombine on a host compound that transports the carriers, so as to generate an excited state of the host compound, and the energy is transferred to a phosphorescent compound to thereby emit light from the phosphorescent compound; the other is a carrier trap type, wherein a phosphorescent compound serves as a carrier trap, and carriers recombine on the phosphorescent compound to thereby emit light from the phosphorescent compound. In either case, the energy of the phosphorescent compound in excited state is required to be lower than that of the host compound.

The phosphorescent compound can be suitably selected from the known phosphorescent compounds used for the light emitting layer of a generic organic electroluminescence element; the phosphorescent compound is preferably a complex compound containing a metal of groups 8 to 10 in the periodic table of elements, and further preferably an iridium compound, an osmium compound, a platinum compound (a platinum complex compound) or a rare-earth complex, and most preferably an iridium compound.

In the present invention, at least one light emitting layer may contain two or more types of phosphorescent compounds, and the ratio of concentration of the phosphorescent compounds may vary in a direction of the thickness of the light emitting layer.

It is preferred that the content of the phosphorescent compounds is equal to or higher than 0.1 vol.% but less than 30 vol.% of the total amount of the light emitting layer.

(Compound Represented by General Formula (3))

It is preferred that the compound (i.e., the phosphorescent compound) contained in the light emitting layer is a compound represented by the following General Formula (3).

It is preferred that the phosphorescent compound (also referred to as "phosphorescent metal complex") represented by General Formula (3) is contained in the light emitting layer of the organic electroluminescence element EL-1 as a light-emitting dopant; however, the phosphorescent compound may also be contained in other layer(s) of the light-emitting functional layer than the light emitting layer.

[Chemical Formula 52]

General Formula(3)

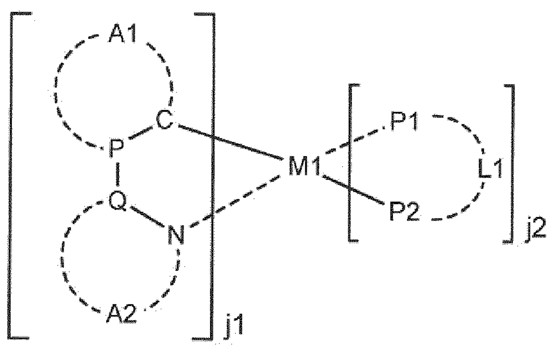

In General Formula (3), P and Q each represent a carbon atom or a nitrogen atom; A1 represents an atom group which forms an aromatic hydrocarbon ring or an aromatic heterocycle with P-C; A2 represents an atom group which forms an aromatic heterocycle with Q-N; P1-L1-P2 represents a bidentate ligand, wherein P1 and P2 each independently represent a carbon atom, a nitrogen atom or an oxygen atom, and L1 represents an atom group which forms the bidentate ligand with P1 and P2; j1 represents an integer of 1 to 3, and j2 represents an integer of 0 to 2, wherein

the sum of j1 and j2 is 2 or 3; and M1 represents a transition metal element of groups 8 to 10 in the periodic table of elements.

In General Formula (3), P and Q each represent a carbon atom or a nitrogen atom.

Examples of the aromatic hydrocarbon ring which is formed by A1 with P-C in General Formula (3) include a benzene ring, a biphenyl ring, a naphthalene ring, an azulene ring, an anthracene ring, a phenanthrene ring, a pyrene ring, a chrysene ring, a naphthacene ring, a triphenylene ring, an o-terphenyl ring, an m-terphenyl ring, a p-terphenyl ring, an acenaphthene ring, a coronene ring, a fluorene ring, a fluoranthrene ring, a naphthacene ring, a pentacene ring, a perylene ring, a pentaphene ring, a picene ring, a pyrene ring, a pyranthrene ring, an anthranthrene ring and the like.

Each of these rings may have a substituent represented by R3 of -C(R3)= represented by each of E51 to E66 and E71 to E88 in General Formula (1).

Examples of an aromatic heterocycle which is formed by A1 with P-C in General Formula (3) include a furan ring, a thiophene ring, an oxazole ring, a pyrrole ring, a pyridine ring, a pyridazine ring, a pyrimidine ring, a pyrazine ring, a triazine ring, a benzimidazole ring, an oxadiazole ring, a triazole ring, an imidazole ring, a pyrazole ring, a triazole ring, an indole ring, a benzimidazole ring, a benzothiazole ring, a benzoxazole ring, a quinoxaline ring, a quinazoline ring, a phthalazine ring, a carbazole ring and an azacarbazole ring.

Here, the azacarbazole ring indicates a ring formed by substituting at least one of carbon atoms of a benzene ring constituting a carbazole ring with a nitrogen atom.

Each of these rings may have a substituent represented by R3 of -C(R3)= represented by each of E51 to E66 and E71 to E88 in General Formula (1).

Examples of the aromatic heterocycle which is formed by A2 with Q-N in General Formula (3) include an oxazole ring, an oxadiazole ring, an oxatriazole ring, an isoxazole ring, a tetrazole ring, a thiadiazole ring, a thiatriazole ring, an isothiazole ring, a pyrrole ring, a pyridine ring, a pyridazine ring, a pyrimidine ring, a pyrazine ring, a triazine ring, an imidazole ring, a pyrazole ring, a triazole ring and the like.

Each of these rings may have a substituent represented by R3 of -C(R3)= represented by each of E51 to E66 and E71 to E88 in General Formula (1).

In General Formula (3), P1-L1-P2 represents a bidentate ligand, P1 and P2 each independently represent a carbon atom, a nitrogen atom or an oxygen atom, and L1 represents an atom group which forms the bidentate ligand with P1 and P2;

Examples of the bidentate ligand represented by P1-L1-P2 include phenylpyridine, phenylpyrazole, phenylimidazole, phenyltriazole, phenyltetrazole, pyrazabole, acetylacetone, picolinic acid and the like.

In General Formula (3), j1 represents an integer of 1 to 3, j2 represents an integer of 0 to 2, and the sum of j1 and j2 is 2 or 3, wherein it is preferred that j2 is 0.

In General Formula (3), M1 represents a transition metal element (also simply referred to as transition metal) of groups 8 to 10 in the periodic table of elements, wherein it is preferred that transition metal element is iridium.

(Compound Represented by General Formula (4))

Among the compounds represented by General Formula (3), a compound represented by the following General Formula (4) is further preferable.

[Chemical Formula 53]

General Formula(4)

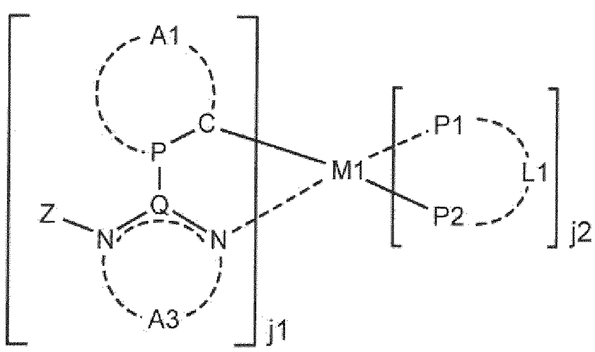

In General Formula (4), Z represents a hydrocarbon ring group or a heterocyclic group; P and Q each represent a carbon atom or a nitrogen atom; A1 represents an atom group which forms an aromatic hydrocarbon ring or an aromatic

heterocycle with P-C; A3 represents -C(R01)=C(R02)-, -N=C(R02)-, - C(R01)=N- or -N=N-, wherein R01 and R02 each represent a hydrogen atom or a substituent; P1-L1-P2 represents a bidentate ligand, wherein P1 and P2 each independently represent a carbon atom, a nitrogen atom or an oxygen atom, and L1 represents an atom group which forms the bidentate ligand with P1 and P2; j1 represents an integer of 1 to 3, and j2 represents an integer of 0 to 2, wherein the sum of j1 and j2 is 2 or 3; and M1 represents a transition metal element of groups 8 to 10 in the periodic table of elements.

Examples of the hydrocarbon ring group represented by Z in General Formula (4) include a non-aromatic hydrocarbon ring group and an aromatic hydrocarbon ring group, wherein examples of the non-aromatic hydrocarbon ring group include a cyclopropyl group, a cyclopentyl group, a cyclohexyl group and the like. These groups may each be unsubstituted or may each have a substituent described later.

Examples of the aromatic hydrocarbon ring group (also referred to as aromatic hydrocarbon group, aryl group or the like) include a phenyl group, a p-chlorophenyl group, a mesityl group, a tolyl group, a xylyl group, a naphthyl group, an anthryl group, an azulenyl group, an acenaphthenyl group, a fluorenyl group, a phenanthryl group, an indenyl group, a pyrenyl group, a biphenyl group and the like.

Each of these groups may be unsubstituted, or may have a substituent represented by R3 of -C(R3)= represented by each of E51 to E66 and E71 to E88 in General Formula (1).

Examples of a heterocyclic group represented by Z in General Formula (4) include a non-aromatic heterocyclic group and an aromatic heterocyclic group; wherein examples of the non-aromatic heterocyclic group include an epoxy ring, an aziridine ring, a thiirane ring, an oxetane ring, an azetidine ring, a thietane ring, a tetrahydrofuran ring, a dioxorane ring, a pyrrolidine ring, a pyrazolidine ring, an imidazolidine ring, an oxazolidine ring, a tetrahydrothiophene ring, a sulforane ring, a thiazolidine ring, an ε-caprolactone ring, an ε-caprolactam ring, a piperidine ring, a hexahydropyridazine ring, a hexahydropyrimidine ring, a piperazine ring, a morpholine ring, a tetrahydropyrane ring, a 1,3-dioxane ring, a 1,4-dioxane ring, a trioxane ring, a tetrahydrothiopyrane ring, a thiomorpholine ring, a thiomorpholine-1,1-dioxide ring, a pyranose ring and a diazabicyclo[2,2,2]-octane ring.

Each of these groups may be unsubstituted, or may have a substituent represented by R3 of -C(R3)= represented by each of E51 to E66 and E71 to E88 in General Formula (1).

Examples of the aromatic heterocyclic group include a pyridyl group, a pyrimidinyl group, a furyl group, a pyrrolyl group, an imidazolyl group, a benzimidazolyl group, a pyrrazolyl group, a pyradinyl group, a triazolyl group (for example, a 1,2,4-triazole-1-yl group, a 1,2,3-triazole-1-yl group and the like), an oxazolyl group, a benzoxazolyl group, a triazolyl group, an isooxazolyl group, an isothiazolyl group, a furazanyl group, a thienyl group, a quinolyl group, a benzofuryl group, a dibenzofuryl group, a benzothienyl group, a dibenzothienyl group, an indolyl group, a carbazolyl group, a carbolinyl group, a diazacarbazolyl group (indicating a ring formed by substituting one of carbon atoms constituting a carboline ring of a carbolinyl group with a nitrogen atom), a quinoxalinyl group, a pyridazinyl group, a triazinyl group, a quinazolinyl group, a phthalazinyl group and the like.

Each of these groups may be unsubstituted, or may have a substituent represented by R3 of -C(R3)= represented by each of E51 to E66 and E71 to E88 in General Formula (1).

The group represented by Z is preferably an aromatic hydrocarbon ring group or an aromatic heterocyclic group.

Examples of the aromatic hydrocarbon ring which is formed by A1 with P-C in General Formula (4) include a benzene ring, a biphenyl ring, a naphthalene ring, an azulene ring, an anthracene ring, a phenanthrene ring, a pyrene ring, a chrysene ring, a naphthacene ring, a triphenylene ring, an o-terphenyl ring, an m-terphenyl ring, a p-terphenyl ring, an acenaphthene ring, a coronene ring, a fluorene ring, a fluoranthrene ring, a naphthacene ring, a pentacene ring, a perylene ring, a pentaphene ring, a picene ring, a pyrene ring, a pyranthrene ring, an anthranthrene ring and the like.

Each of these rings may have a substituent represented by R3 of -C(R3)= represented by each of E51 to E66 and E71 to E88 in General Formula (1).

Examples of an aromatic heterocycle which is formed by A1 with P-C in General Formula (4) include a furan ring, a thiophene ring, an oxazole ring, a pyrrole ring, a pyridine ring, a pyridazine ring, a pyrimidine ring, a pyrazine ring, a triazine ring, a benzimidazole ring, an oxadiazole ring, a triazole ring, an imidazole ring, a pyrazole ring, a triazole ring, an indole ring, a benzimidazole ring, a benzothiazole ring, a benzoxazole ring, a quinoxaline ring, a quinazoline ring, a phthalazine ring, a carbazole ring, a carboline ring and an azacarbazole ring.

Here, the azacarbazole ring indicates a ring formed by substituting at least one of carbon atoms of a benzene ring constituting a carbazole ring with a nitrogen atom.

Each of these rings may have a substituent represented by R3 of -C(R3)= represented by each of E51 to E66 and E71 to E88 in General Formula (1).

The substituent represented by each of R01 and R02 in - C(R01)=C(R02)-, -N=C(R02)- and -C(R01)=N- represented by A3 in General Formula (4) is synonymous with the substituent represented by R3 of -C(R3)= represented by each of E51 to E66 and E71 to E88 in General Formula (1).

Examples of the bidentate ligand represented by P1-L1-P2 in General Formula (4) include phenylpyridine, phenylpyrazole, phenylimidazole, phenyltriazole, phenyltetrazole, pyrazabole, acetylacetone, picolinic acid and the like.

Further, j1 represents an integer of 1 to 3, j2 represents an integer of 0 to 2, and the sum of j1 and j2 is 2 or 3,

wherein it is preferred that j2 is 0.

The transition metal element (also simply referred to as transition metal) of groups 8 to 10 in the periodic table of elements represented by M1 in General Formula (4) is synonymous with the transition metal element of groups 8 to 10 in the periodic table of elements represented by M1 in General Formula (3).

(Compound Represented by General Formula (5))

A compound represented by the following General Formula (5) is one of preferable examples of the compounds represented by General Formula (4).

[Chemical Formula 54]

General Formula (5)

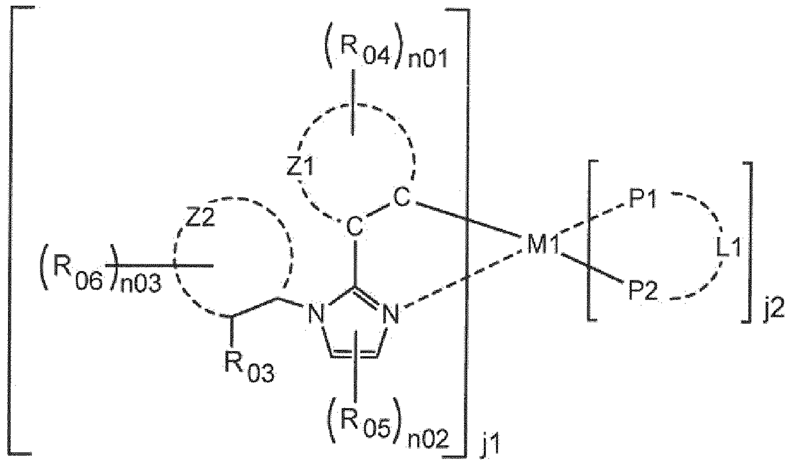

In General Formula (5), $R_{03}$ represents a substituent, $R_{04}$ represents a hydrogen atom or a substituent, and a plurality of $R_{04}$ may be bonded to each other to form a ring; n01 represents an integer of 1 to 4; $R_{05}$ represents a hydrogen atom or a substituent, and a plurality of $R_{05}$ may be bonded to each other to form a ring; n02 represents an integer of 1 to 2; $R_{06}$ represents a hydrogen atom or a substituent, and a plurality of $R_{06}$ may be bonded to each other to form a ring; n03 represents an integer of 1 to 4; Z1 represents an atom group necessary to form, along with C-C, a 6-membered aromatic hydrocarbon ring, or a 5-membered or 6-membered aromatic heterocycle; Z2 represents an atom group necessary to form a hydrocarbon ring group or a heterocyclic group; P1-L1-P2 represents a bidentate ligand, wherein P1 and P2 each independently represent a carbon atom, a nitrogen atom or an oxygen atom, and L1 represents an atom group which forms the bidentate ligand with P1 and P2; j1 represents an integer of 1 to 3, and j2 represents an integer of 0 to 2, wherein the sum of j1 and j2 is 2 or 3; and M1 represents a transition metal element of groups 8 to 10 in the periodic table of elements. $R_{03}$ and $R_{06}$ may be bonded to each other to form a ring, $R_{04}$ and $R_{06}$ may be bonded to each other to form a ring, and $R_{05}$ and $R_{06}$ may be bonded to each other to form a ring.

The substituents respectively represented by $R_{03}$, $R_{04}$, $R_{05}$, and $R_{06}$ in General Formula (5) are each synonymous with the substituent represented by R3 of -C(R3)= represented by each of E51 to E66 and E71 to E88 in General Formula (1).

Examples of the 5-membered aromatic hydrocarbon ring which is formed by Z1 with C-C in General Formula (5) include a benzene ring and the like.

Each of these rings may have a substituent represented by R3 of -C(R3)= represented by each of E51 to E66 and E71 to E88 in General Formula (1).

Examples of the 5-membered or 6-membered aromatic heterocycle which is formed by Z1 with C-C in General Formula (5) include an oxazole ring, an oxadiazole ring, an oxatriazole ring, an isoxazole ring, a tetrazole ring, a thiadiazole ring, a thiatriazole ring, an isothiazole ring, a thiophene ring, furan ring, a pyrrole ring, a pyridine ring, a pyridazine ring, a pyrimidine ring, a pyrazine ring, a triazine ring, an imidazole ring, a pyrazole ring and a triazole ring.

Each of these rings may have a substituent represented by R3 of -C(R3)= represented by each of E51 to E66 and E71 to E88 in General Formula (1).

Examples of the hydrocarbon ring group represented by Z2 in General Formula (5) include a non-aromatic

hydrocarbon ring group and an aromatic hydrocarbon ring group, wherein examples of the non-aromatic hydrocarbon ring group include a cyclopropyl group, a cyclopentyl group, a cyclohexyl group and the like. These groups may each be unsubstituted or may each have a substituent described later.

Examples of the aromatic hydrocarbon ring group (also referred to as aromatic hydrocarbon group, aryl group or the like) include a phenyl group, a p-chlorophenyl group, a mesityl group, a tolyl group, a xylyl group, a naphthyl group, an anthryl group, an azulenyl group, an acenaphthenyl group, a fluorenyl group, a phenanthryl group, an indenyl group, a pyrenyl group, a biphenyl group and the like. Each of these groups may be unsubstituted, or may have a substituent represented by R3 of -C(R3)= represented by each of E51 to E66 and E71 to E88 in General Formula (1).

Examples of a heterocyclic group represented by Z2 in General Formula (5) include a non-aromatic heterocyclic group and an aromatic heterocyclic group; wherein examples of the non-aromatic heterocyclic group include an epoxy ring, an aziridine ring, a thiirane ring, an oxetane ring, an azetidine ring, a thietane ring, a tetrahydrofuran ring, a dioxorane ring, a pyrrolidine ring, a pyrazolidine ring, an imidazolidine ring, an oxazolidine ring, a tetrahydrothiophene ring, a sulforane ring, a thiazolidine ring, an ε-caprolactone ring, an ε-caprolactam ring, a piperidine ring, a hexahydropyridazine ring, a hexahydropyrimidine ring, a piperazine ring, a morpholine ring, a tetrahydropyrane ring, a 1,3-dioxane ring, a 1,4-dioxane ring, a trioxane ring, a tetrahydrothiopyrane ring, a thiomorpholine ring, a thiomorpholine-1,1-dioxide ring, a pyranose ring and a diazabicyclo[2,2,2]-octane ring. Each of these groups may be unsubstituted, or may have a substituent represented by R3 of -C(R3)= represented by each of E51 to E66 and E71 to E88 in General Formula (1).

Examples of the aromatic heterocyclic group include a pyridyl group, a pyrimidinyl group, a furyl group, a pyrrolyl group, an imidazolyl group, a benzimidazolyl group, a pyrrazolyl group, a pyradinyl group, a triazolyl group (for example, a 1,2,4-triazole-1-yl group, a 1,2,3-triazole-1-yl group and the like), an oxazolyl group, a benzoxazolyl group, a triazolyl group, an isooxazolyl group, an isothiazolyl group, a furazanyl group, a thienyl group, a quinolyl group, a benzofuryl group, a dibenzofuryl group, a benzothienyl group, a dibenzothienyl group, an indolyl group, a carbazolyl group, a carbolinyl group, a diazacarbazolyl group (indicating a ring formed by substituting one of carbon atoms constituting a carboline ring of a carbolinyl group with a nitrogen atom), a quinoxalinyl group, a pyridazinyl group, a triazinyl group, a quinazolinyl group, a phthalazinyl group and the like.

Each of these rings may be unsubstituted, or may have a substituent represented by R3 of -C(R3)= represented by each of E51 to E66 and E71 to E88 in General Formula (1).

It is preferred that, in General Formula (5), the group formed by Z1 and Z2 is a benzene ring.

The bidentate ligand represented by P1-L1-P2 in General Formula (5) is synonymous with the bidentate ligand represented by P1-L1-P2 in General Formula (3).

The transition metal element of groups 8 to 10 in the periodic table of elements represented by M1 in General Formula (5) is synonymous with the transition metal element of groups 8 to 10 in the periodic table of elements represented by M1 in General Formula (3).

The phosphorescent compound may be suitably selected from the known phosphorescent compounds used for the light emitting layer of the organic electroluminescence element EL-1.

The phosphorescent compound of the present invention is preferably a complex compound containing a metal of groups 8 to 10 in the periodic table of elements, further preferably an iridium compound, an osmium compound, a platinum compound (a platinum complex compound) or a rare-earth complex, and most preferably an iridium compound.

Concrete examples (Pt-1 to Pt-3, A-1, Ir-1 to Ir-50) of the phosphorescent compound of the present invention are shown below; however, the present invention is not limited thereto. Note that, in these compounds, m and n each represent number of replication.

[Chemical Formula 55]

Pt-1

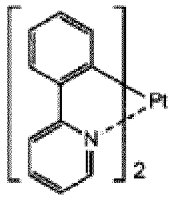

Pt-2

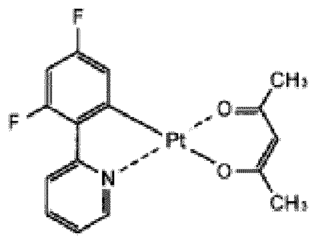

Pt-3

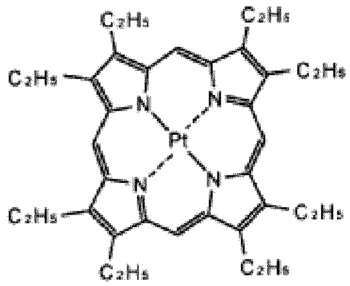

A-1

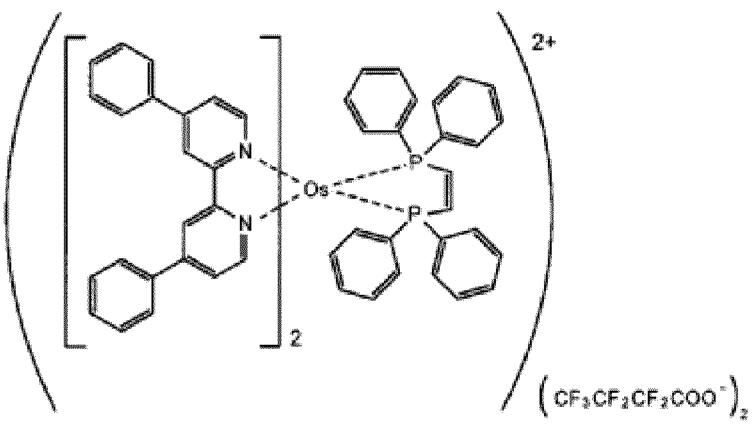

[Chemical Formula 56]

Ir-1

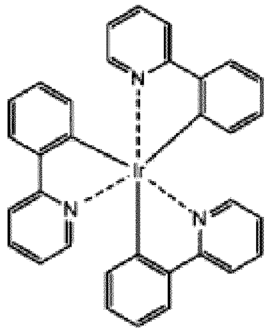

Ir-2

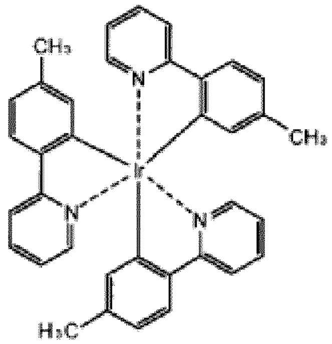

Ir-3

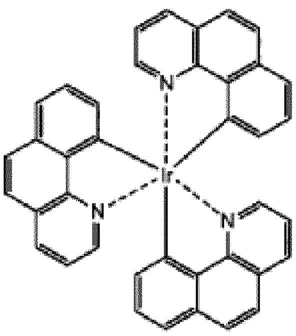

Ir-4

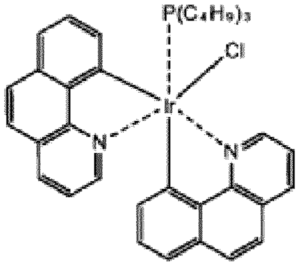

Ir-5

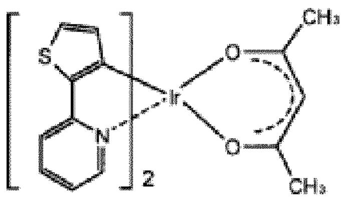

Ir-6

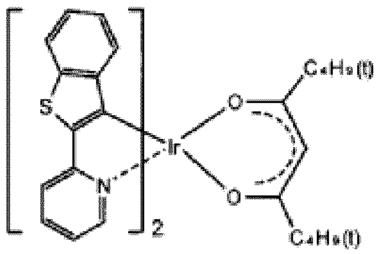

[Chemical Formula 57]

Ir-7

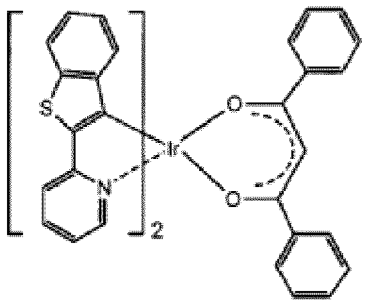

Ir-8

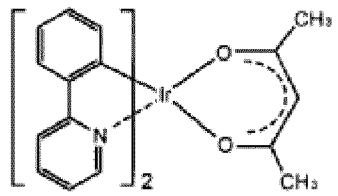

Ir-9

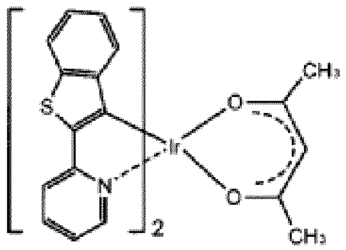

Ir-10

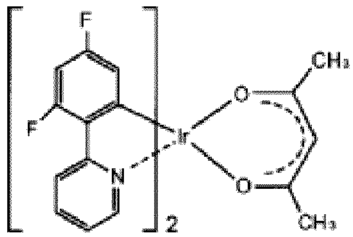

Ir-11

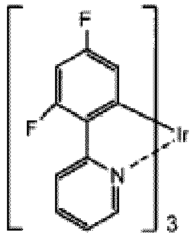

Ir-12

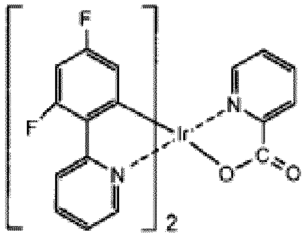

Ir-13

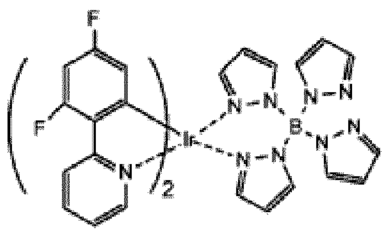

Ir-14

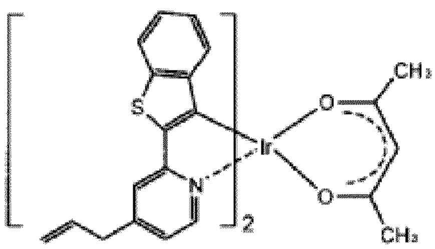

[Chemical Formula 58]

Ir-15

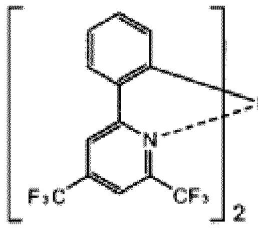

Ir-16

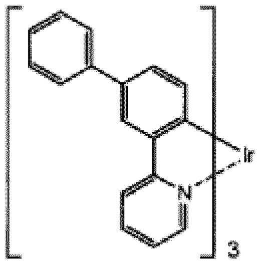

Ir-17

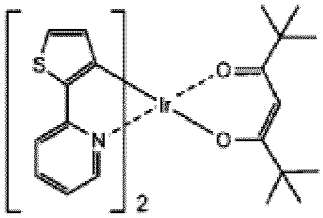

Ir-18

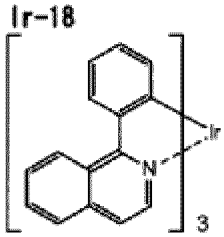

Ir-19

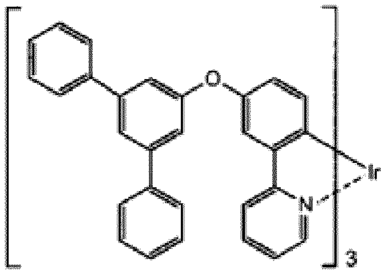

Ir-20

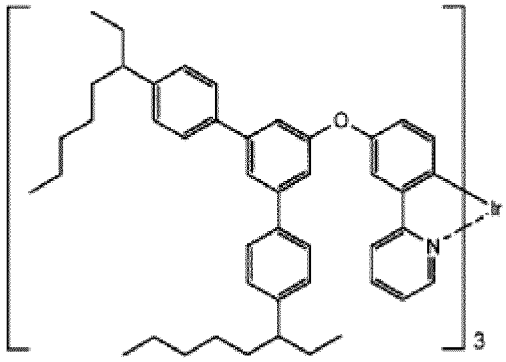

Ir-21

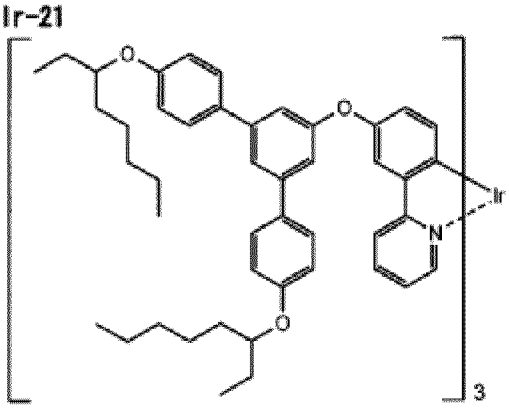

[Chemical Formula 59]

Ir-22

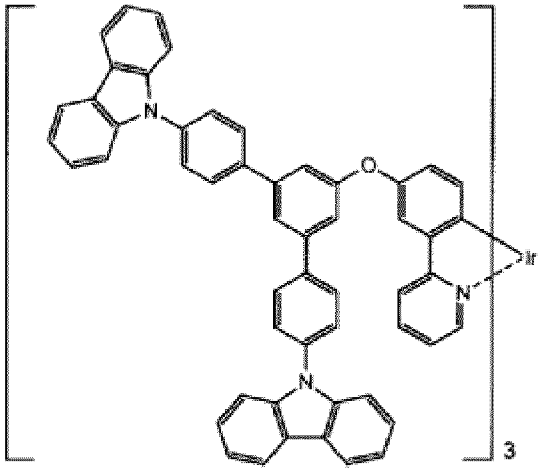

Ir-23

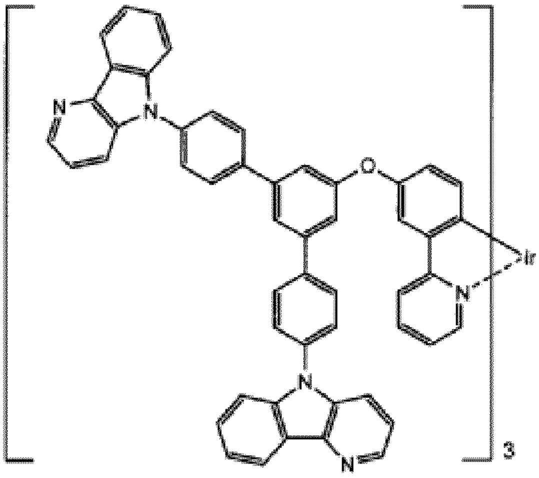

Ir-24

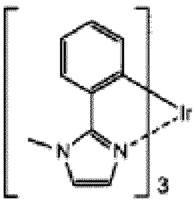

Ir-25

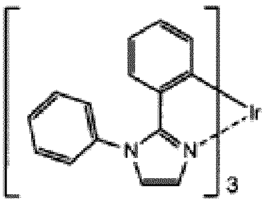

Ir-26

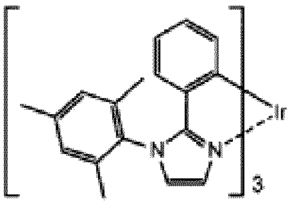

[Chemical Formula 60]

Ir-27

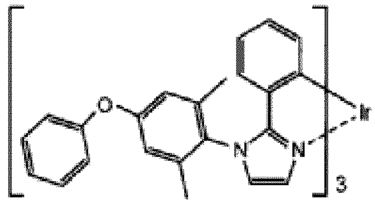

Ir-28

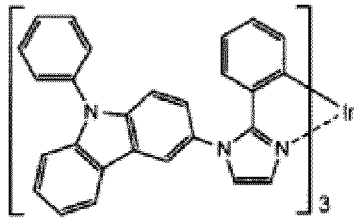

Ir-29

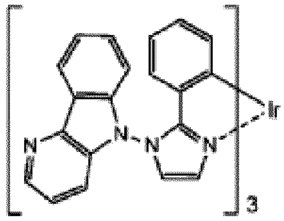

Ir-30

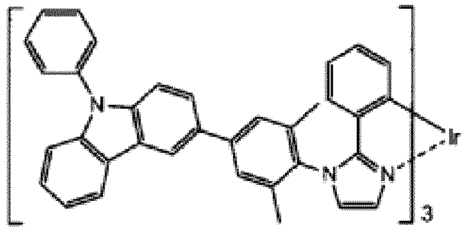

Ir-31

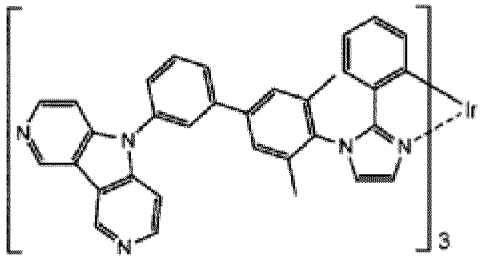

Ir-32

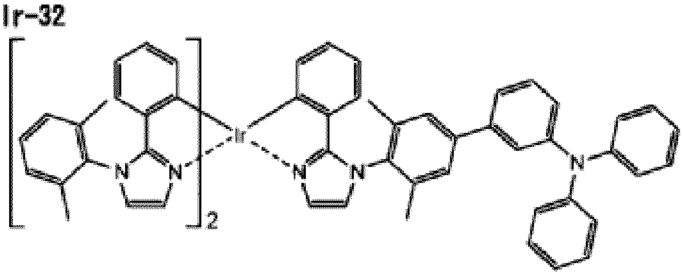

Ir-33

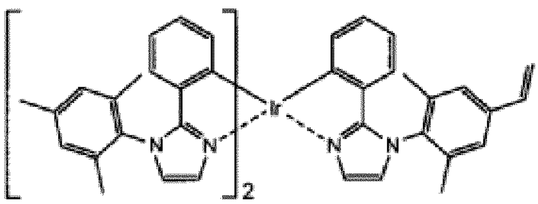

[Chemical Formula 61]

Ir-34

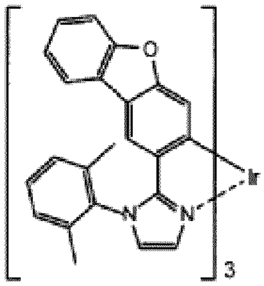

Ir-35

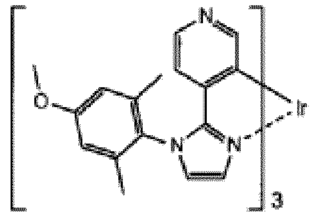

Ir-36

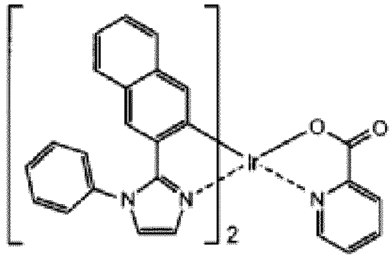

Ir-37

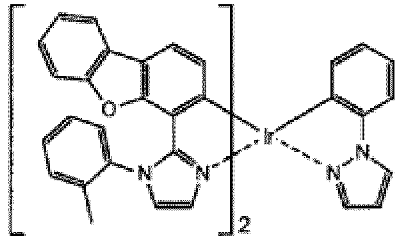

Ir-38

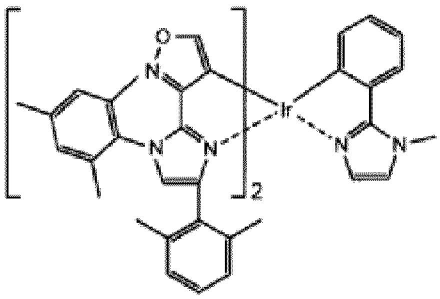

Ir-39

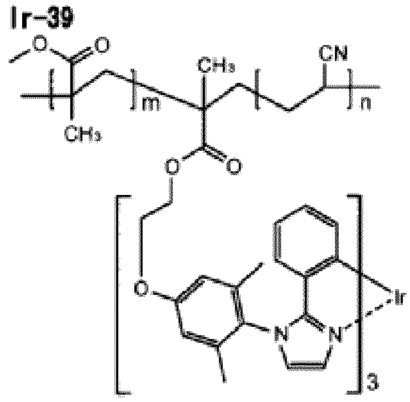

[Chemical Formula 62]

Ir-40

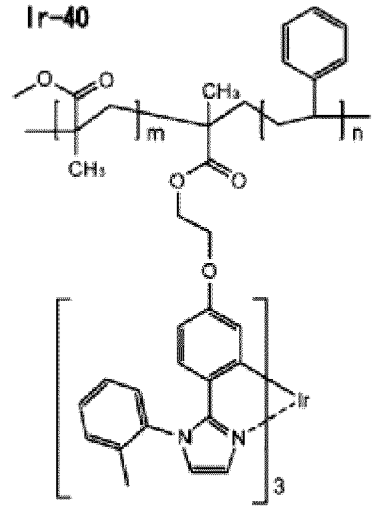

Ir-41

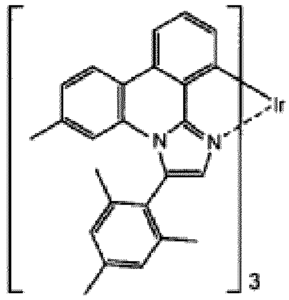

Ir-42

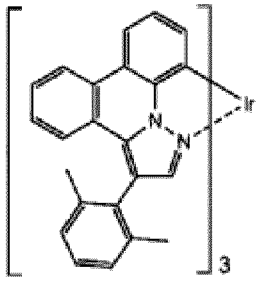

Ir-43

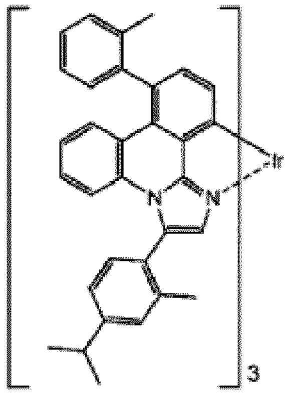

Ir-44

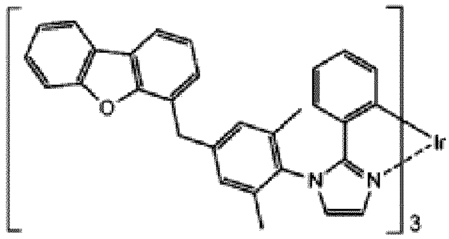

Ir-45

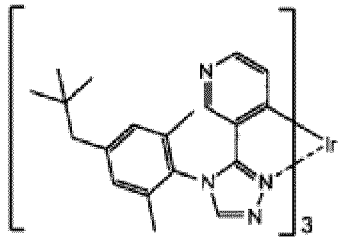

[Chemical Formula 63]

Ir-46

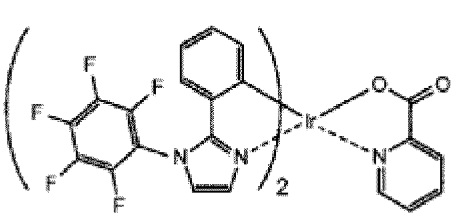

Ir-47

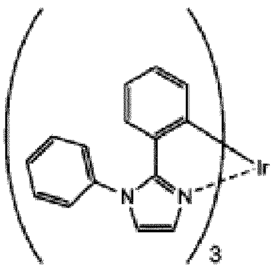

Ir-48

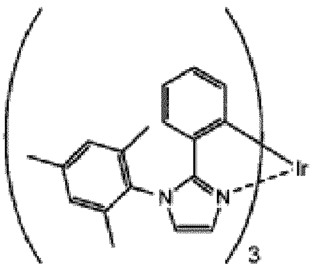

Ir-49

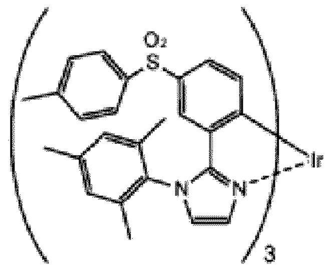

Ir-50

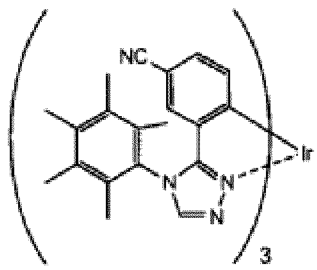

The aforesaid phosphorescent compounds (also referred to as phosphorescent metal complexes or the like) can be synthesized by employing methods described in documents such as Organic Letters, vol. 3, No. 16, pp. 2579-2581 (2001); Inorganic Chemistry, vol. 30, No. 8, pp. 1685-1687 (1991); J. Am. Chem. Soc., vol. 123, pp. 4304 (2001); Inorganic Chemistry, vol. 40, No. 7, pp. 1704-1711 (2001); Inorganic Chemistry, vol. 41, No. 12, pp. 3055-3066 (2002); New Journal of Chemistry, vol. 26, pp. 1171 (2002); and European Journal of Organic Chemistry, vol. 4, pp. 695-709 (2004); and reference documents described in these documents.

(Fluorescent Material)

Examples of the fluorescent material include a coumarin dye, a pyran dye, a cyanine dye, a chloconium dye, a squarylium dye, an oxobenzanthracene dye, a fluorescein dye, a rhodamine dye, a pyrylium dye, a perylene dye, a stilbene dye, a polythiophene dye, a rare earth complex based phosphor and the like.

[Injecting Layer: Hole Injecting Layer and Electron Injecting Layer]

An injecting layer is a layer arranged between an electrode and a light emitting layer in order to decrease driving voltage and improve brightness of the emitted light; the details of the injecting layer are described in "Electrode Material" (pp. 123-166, Part 2, Chapter 2 of "Organic EL Element and Front of Industrialization thereof" Nov. 30, 1998, published by N. T. S Co., Ltd.), and examples of the injecting layer include a hole injecting layer and an electron injecting layer.

The injecting layer can be provided according to necessity. If the injecting layer is a hole injecting layer, it can be arranged between the anode and the light emitting layer or the hole transporting layer; while if the injecting layer is an electron injecting layer, it can be arranged between the cathode and the light emitting layer or the electron transporting

layer.

The details of the hole injecting layer is also described in documents such as Japanese Unexamined Patent Application Publication Nos. 9-45479, 9-260062 and 8-288069, and concrete examples of the hole injecting layer include a layer of a phthalocyanine represented by copper phthalocyanine, a layer of an oxide represented by vanadium oxide, a layer of an amorphous carbon and a layer of a polymer employing conductive polymer such as polyaniline (emeraldine), polythiophene and the like.

The details of the electron injecting layer is also described in documents such as Japanese Unexamined Patent Application Publication Nos. 6-325871, 9-17574 and 10-74586, and concrete examples of the electron injecting layer include a layer of a metal represented by strontium, aluminum or the like; a layer of an alkali metal halide represented by potassium fluoride; a layer of an alkali earth metal compound represented by magnesium fluoride; and a layer of an oxide represented by molybdenum oxide. It is preferred that the electron injecting layer of the present invention is a very thin film; specifically, it is preferred that the film-thickness of the electron injecting layer is within a range from 1 nm to 10 $\mu$m depending on the material thereof.

[Hole Transporting Layer]

A hole transporting layer is made of a hole transporting material having a function of transporting holes; and, in a broad sense, a hole injecting layer and an electron blocking layer are included in the hole transporting layer. One hole transporting layer or a plurality of hole transporting layers can be provided.

The hole transporting material is a material either having a capability of injecting or transporting holes, or having a barrier property against electrons; the hole transporting material may either be an organic material or an inorganic material. Examples of the hole transporting material include a triazole derivative, an oxadiazole derivative, an imidazole derivative, a polyarylalkane derivative, a pyrazoline derivative, a pyrazolone derivative, a phenylenediamine derivative, an arylamine derivative, an amino-substituted chalcone derivative, an oxazole derivative, a styrylanthracene derivative, a fluorenone derivative, a hydrazone derivative, a stilbene derivative, a silazane derivative, an aniline copolymer, a conductive oligomer such as a thiophene oligomer, and the like.

Although the aforesaid compounds can be used as the hole transporting material, it is preferred that a porphyrin compound, an aromatic tertiary amine compound or a styrylamine compound is used as hole transporting material, and wherein it is particularly preferred that an aromatic tertiary amine compound is used as the hole transporting material.

Typical examples of the aromatic tertiary amine compound and the styrylamine compound include: N,N,N',N'-tetraphenyl-4,4'-diaminophenyl; N,N'-diphenyl-N,N'-bis(3-methylphenyl)-[1,1'-biphenyl]-4,4'-diamine (TDP); 2,2-bis(4-di-p-tolylaminophenyl)propane; 1,1-bis(4-di-p-tolylaminophenyl)cyclohexane; N,N,N',N'-tetra-p-tolyl-4,4'-diaminobiphenyl; 1,1-bis(4-di-p-tolylaminophenyl)-4-phenylcyclohexane; bis(4-dimethylamino-2-methyl)phenylmethane; bis(4-di-p-tolylaminophenyl)phenylmethane; N,N'-diphenyl-N,N'-di(4-methoxyphenyl)-4,4'-diaminobiphenyl; N,N,N',N'-tetraphenyl-4,4'-diaminodiphenylether; 4,4'-bis(diphenylamino)quadriphenyl; N,N,N-tri(p-tolyl)amine; 4-(di-p-tolylamino)-4'-[4-(di-p-tolylamino)styryl]stilbene; 4-N,N-diphenylamino-(2-diphenylvinyl)benzene; 3-methoxy-4'-N,N-diphenylaminostilbene; N-phenylcarbazole; those having two condensed aromatic rings in a molecule described in U.S. Pat. No. 5,061,569, such as 4,4'-bis[N-(1-naphthyl)-N-phenylamino]biphenyl (NDP); and 4,4',4"-tris[N-(3-methylphenyl)-N-phenylamino]triphenylamine (MTDATA) in which three triphenylamine units are bonded in a star burst form described in Japanese Patent Application Laid-Open Publication No. 4-308688.

Further, a polymer material in which any of these materials is introduced into a polymer chain or a polymer material in which a polymer main chain is constituted by any of these materials may also be used. Further, inorganic compounds such as a p-type Si and a p-type SiC may also be used as the hole injecting material and the hole transporting material.

Further, it is also possible to use a so-called p-type hole transporting material described Japanese Unexamined Patent Application Publication No. 11-251067 and Applied Physics Letters 80 (2002), pp. 139 by J. Huang et. al. In the present invention, it is preferable to use these materials in order to produce a light-emitting element having high efficiency.

The hole transporting layer can be formed by forming a thin film of the aforesaid hole transporting material by employing a known method such as a vacuum deposition method, a spin coating method, a casting method, a printing method (which includes an ink-jet method), a LB method or the like. The film-thickness of the hole transporting layer is not particularly limited; however, the film-thickness of the hole transporting layer is typically within a range about from 5 nm to 5 $\mu$m, preferably within a range from 5 nm to 200 nm. The hole transporting layer may have a single layer structure formed of one type of the aforesaid materials, or formed of two or more types of the aforesaid materials.

Further, it is also possible to dope impurities into the material of the hole transporting layer to improve p-property. Examples of doping impurities into the material of the hole transporting layer include those described in documents such as Japanese Unexamined Patent Application Publication Nos. 4-297076, 2000-196140 and 2001-102175 and J. Appl. Phys., 95, 5773 (2004).

It is preferred to dope impurities into the material of the hole transporting layer, because improved p-property

makes it possible to produce an element which consumes lower electric power.

[Electron Transporting Layer]

The electron transporting layer is formed of a material having a function to transport electrons; in a broad sense, an electron injecting layer and a hole blocking layer (not shown) are included in the electron transporting layer. The electron transporting layer may have either a single-layer structure or a laminated structure composed of a plurality of layers.

In either an electron transporting layer having a single-layer structure or an electron transporting layer having a laminated structure, an electron transporting material (which also functions as a hole blocking material) constituting a layer-portion adjacent to the light emitting layer may be a material having a function of transferring electrons injected from the cathode to the light emitting layer. Such material can be selected from known compounds. Examples of the known compounds include a nitro-substituted fluorene derivative, a diphenylquinone derivative, a thiopyrandioxide derivative, carbodiimide, a fluorenylidenemethane derivative, anthraquinonedimethane, an anthrone derivative, an oxadiazole derivative and the like. Further, in the aforesaid oxadiazole derivative, a thiadiazole derivative formed by substituting an oxygen atom of an oxadiazole ring with a sulfur atom and a quinoxaline derivative having a quinoxaline ring which is known as an electron withdrawing group may also be used as the material of the electron transporting layer. Further, a polymer material in which any of these materials is introduced into a polymer chain or a polymer material in which a polymer main chain is constituted by any of these materials may also be used.

Further, metal complexes of an 8-quinolinol derivative such as tris(8-quinolinol)aluminum ($Alq_3$), tris(5,7-dichloro-8-quinolinol)aluminum, tris(5,7-dibromo-8-quinolinol)aluminum, tris(2-methyl-8-quinolinol)aluminum, tris(5-methyl-8-quinolinol)aluminum, bis(8-quinolinol)zinc (Znq) and the like, as well as metal complexes formed by substituting the central metal of the aforesaid metal complexes with In, Mg, Cu, Ca, Sn, Ga or Pb may also be used as the material of the electron transporting layer.

Further, metal-free or metal phthalocyanine and those formed by substituting the terminal of metal-free or metal phthalocyanine with an alkyl group, a sulfonic acid group or the like may be preferably used as the material of the electron transporting layer. Further, the distyrylpyrazine derivative mentioned as an example of the material for the light emitting layer may also be used as the material of the electron transporting layer; and, similar to the cases of the hole injecting layer and the hole transporting layer, inorganic semiconductors such as an n type-Si and an n type-SiC may also be used as the material of the electron transporting layer.

The electron transport layer can be formed by forming the aforesaid material into a thin film by a known method such as a vacuum deposition method, a spin coating method, a casting method, a printing method (which includes an ink-jet method), an LB method or the like. The film-thickness of the electron transporting layer is not particularly limited; however, the film-thickness of the electron transporting layer is typically within a range about from 5 nm to 5 $\mu$m, preferably within a range from 5 nm to 200 nm. The electron transporting layer may have a single layer structure formed of one type of the aforesaid materials, or formed of two or more types of the aforesaid materials.

Further, it is also possible to dope impurities into the electron transporting layer to improve n-property. Examples of doping impurities into the electron transporting layer include those described in documents such as Japanese Unexamined Patent Application Publication Nos. 4-297076, 10-270172, 2000-196140 and 2001-102175 and J. Appl. Phys., 95, 5773 (2004). Further, it is preferred that the electron transporting layer contains potassium, a potassium compound and/or the like. For example, potassium fluoride or the like can be used as the potassium compound. If the n-property of the electron transporting layer is improved in the aforesaid manner, it is possible to produce an element which consumes lower electric power.

Further, a compound represented by the following General Formula (6) can be preferably used as the material of the electron transporting layer (the electron transporting compound).

(Ar1)n1 - Y1 General Formula (6)

In General Formula (6), n1 represents an integer of 1 or greater; Y1 represents a substituent if n1 is equal to 1, or a bond or an n1-valent linking group if n1 is equal to or greater than 2; Ar1 represents a group represented by below-mentioned General Formula (A), and if n1 is equal to or greater than 2, a plurality of pieces of Ar1 may either be the same, or be different from each other. However, the compound represented by General Formula (6) has, in the molecule, at least two condensed aromatic heterocycles each formed by condensing three or more rings.

The substituent represented by Y1 in General Formula (6) is synonymous with the substituent represented by R3 of-C(R3)= represented by each of E51 to E66 and E71 to E88 in General Formula (3) which represents a compound constituting the aforesaid nitrogen-containing layer 1a.

Concrete examples of the n1-valent linking group represented by Y1 in General Formula (6) include a divalent linking group, a trivalent linking group, a tetravalent linking group and the like.

Examples of the divalent linking group represented by Y1 in General Formula (6) include: an alkylene group (for example, an ethylene group, a trimethylene group, a tetramethylene group, a propylene group, an ethylethylene group, a pentamethylene group, a hexamethylene group, a 2,2,4-trimethylhexamethylene group, a heptamethylene group, an octamethylene group, nonamethylene group, a decamethylene group, an undecamethylene group, a dodecamethylene group, a cyclohexylene group (for example, a 1,6-cyclohexanediyl group and the like), a cyclopenthylene group (for example, a 1,5-cyclopentanediyl group and the like) and the like); an alkenylene group (for example, a vinylene group, a propenylene group, a butenylene group, a pentenylene group, a 1-methylvinylene group, a 1-methylpropenylene group, a 2-methylpropenylene group, a 1-methylpentenylene group, a 3-methylpentenylene group, a 1-ethylvinylene group, a 1-ethylpropenylene group, a 1-ethylbutenylene group, a 3-ethylbutenylene group and the like); an alkynylene group (for example, an ethynylene group, a 1-propynylene group, a 1-butynylene group, a 1-pentynylene group, a 1-hexynylene group, a 2-butynylene group, a 2-pentynylene group, a 1-methylethynylene group, a 3-methyl-1-propynylene group, a 3-methyl-1-butynylene group and the like); an arylene group (for example, an o-phenylene group, a p-phenylene group, a naphthalenediyl group, an anthracenediyl group, a naphthacenediyl group, a pyrenediyl group, a naphthylnaphthalenediyl group, a biphenyldiyl group (for example, a [1,1'-biphenyl]-4,4'-diyl group, a 3,3'-biphenyldiyl group and, 3,6-biphenyldiyl group and the like), a terphenyldiyl group, a quaterphenyldiyl group, a quinquephenyldiyl group, a sexiphenyldiyl group, a septiphenyldiyl group, an octiphenyldiyl group, a nobiphenyldiyl group, a deciphenyldiyl group and the like); a heteroarylene group (for example, a divalent group derived from a group consisting of a carbazole group, a carboline ring, a diazacarbazole ring (also referred to as a monoazacarboline group, indicating a ring formed by substituting one of carbon atoms constituting a carboline ring with a nitrogen atom), a triazole ring, a pyrrole ring, a pyridine ring, a pyrazine ring, a quinoxaline ring, a thiophene ring, an oxadiazole ring, a dibenzofuran ring, a dibenzothiophene ring, an indole ring and the like), a chalcogen atom such as oxygen, sulfur or the like, a group derived from a condensed aromatic heterocycle formed by condensing three or more, and the like (herein, it is preferred that the condensed aromatic heterocycle formed by condensing three or more rings is a condensed aromatic heterocycle which contains a hetero atom selected from N, O and S as an element constituting a condensed ring; concrete examples of such condensed aromatic heterocycle include an acridine ring, a benzoquinoline ring, a carbazole ring, a phenazine ring, a phenanthridine ring, a phenanthroline ring, a carboline ring, a cycladine ring, a quindoline ring, a thebenidine ring, a quinindoline ring, a triphenodithiazine ring, a triphenodioxazine ring, a phenanthrazine ring, an anthrazine ring, a perimizine ring, a diazacarbazole ring (indicating a ring formed by substituting one of carbon atoms constituting a carboline ring with a nitrogen atom), a phenanthroline ring, a dibenzofuran ring, a dibenzothiophene ring, a naphthofuran ring, a naphthothiophene ring, a benzodifuran ring, a benzodithiophene ring, a naphthodifuran ring, a naphthodithiophene ring, an anthrafuran ring, an anthradifuran ring, an anthrathiophene ring, an anthradithiophene ring, a thianthrene ring, a phenoxathiin ring, a thiophanthrene ring (naphthothiophene ring) and the like).

Examples of the trivalent linking group represented by Y1 in General Formula (6) include an ethanetriyl group, a propanetriyl group, a butanetriyl group, a pentanetriyl group, a hexanetriyl group, a heptanetriyl group, an octanetriyl group, a nonanetriyl group, a decanetriyl group, an undecanetriyl group, a dodecanetriyl group, a cyclohexanetriyl group, a cyclopentanetriyl group, a benzenetriyl group, a naphthalenetriyl group, a pyridinetriyl group, a carbazoletriyl group and the like.

The tetravalent linking group represented by Y1 in General Formula (6) is a group having a linking group added to any one of the above-mentioned trivalent linking groups. Examples of the tetravalent linking group include a propandiylidene group, a 1,3-propandiyl-2-ylidene group, a butanediylidene group, a pentanediylidene group, a hexanediylidene group, a heptanediylidene group, an octanediylidene group, a nonanediylidene group, a decanediylidene group, an undecanediylidene group, a dodecanediylidene group, a cyclohexanediylidene group, a cyclopentanediylidene group, a benzenetetrayl group, a naphthalenetetrayl group, a pyridinetetrayl group, a carbazoletetrayl group and the like.

Incidentally, the aforesaid divalent, trivalent and tetravalent linking groups may each have a substituent represented by R3 of -C(R3)= represented by each of E51 to E66 and E71 to E88 in General Formula (1).

In the compound represented by General Formula (6), it is preferred that Y1 represent a group derived from a condensed aromatic heterocycle formed by condensing three or more rings, and it is preferred that the condensed aromatic heterocycle formed by condensing three or more rings is a dibenzofuran ring or a dibenzothiophene ring. Further, it is preferred that n1 is 2 or more.

Further, the compound represented by General Formula (6) has, in the molecule, at least two condensed aromatic heterocycles each formed by condensing three or more rings.

When Y1 represents an n1-valent linking group, Y1 is preferably non-conjugated in order to keep the triplet excitation energy of the compound represented by General Formula (6) high, and is preferably constituted of aromatic rings (an aromatic hydrocarbon ring + an aromatic heterocycle) in order to improve Tg (also referred to as glass transition point or glass transition temperature).

Here, the "non-conjugated" indicates that a linking group cannot be expressed with alternation of single and

double bonds, or that a conjugation of aromatic rings which constitute a linking group is sterically broken.

(Group Represented by General Formula (A))

Ar1 in General Formula (6) is a group represented by the following General Formula (A).

[Chemical Formula 64]

General Formula(A)

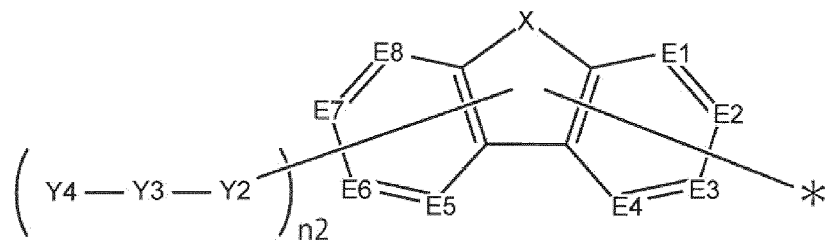

In General Formula (A), X represents -N(R)-, -O-, -S- or -Si (R)(R')-, and E1 to E8 each represent -C(R1)= or -N=, wherein R, R' and R1 each represent a hydrogen atom, a substituent or a linking site with Y1; * represents a linking site with Y1; Y2 merely represents a bond or a divalent linking group; Y3 and Y4 each represent a group derived from a 5-membered or 6-membered aromatic ring, wherein at least one of Y3 and Y4 represents a group derived from an aromatic heterocycle containing a nitrogen atom as a ring constituent atom; and n2 represents an integer of 1 to 4.

Here, the substituents represented by R, R' or R1 both in -N(R)- or -Si (R)(R')- represented by X and in -C(R1)= represented by each of E1 to E8 in General Formula (A) are each synonymous with the substituent represented by R3 of - C(R3)= represented by each of E51 to E66 and E71 to E88 in General Formula (1).

Further, the divalent linking group represented by Y2 in General Formula (A) is synonymous with the divalent linking group represented by Y1 in General Formula (6).

Examples of the 5-membered or 6-membered aromatic ring used to form a group derived from a 5-membered or 6-membered aromatic ring represented by each of Y3 and Y4 in General Formula (A) include a benzene ring, an oxazole ring, a thiophene ring, a furan ring, a pyrrole ring, a pyridine ring, a pyridazine ring, a pyrimidine ring, a pyrazine ring, a diazine ring, a triazine ring, an imidazole ring, an isoxazole ring, a pyrazole ring, a triazole ring and the like.

At least one of the groups derived from 5-membered or 6-membered aromatic rings respectively represented by Y3 and Y4 is a group derived from an aromatic heterocycle containing a nitrogen atom as a ring constituent atom. Examples of the aromatic heterocycle containing a nitrogen atom as a ring constituent atom include an oxazole ring, a pyrrole ring, a pyridine ring, a pyridazine ring, a pyrimidine ring, a pyrazine ring, the diazine ring, a triazine ring, an imidazole ring, an isoxazole ring, a pyrazole ring, a triazole ring and the like.

(Preferred Group Represented by Y3)

In General Formula (A), the group represented by Y3 is preferably a group derived from the aforesaid 6-membered aromatic ring, and further preferably a group derived from a benzene ring.

(Preferred Group Represented by Y4)

In General Formula (A), the group represented by Y4 is preferably a group derived from the aforesaid 6-membered aromatic ring, and further preferably a group derived from the aromatic heterocycle containing a nitrogen atom as a ring constituent atom, and particularly preferably a group derived from a pyridine ring.

(Preferred Group Represented by General Formula (A))

Examples of preferred group represented by General Formula (A) include a group represented by one of the following General Formulas (A-1), (A-2), (A-3) and (A-4).

[Chemical Formula 65]

General Formula (A-1)

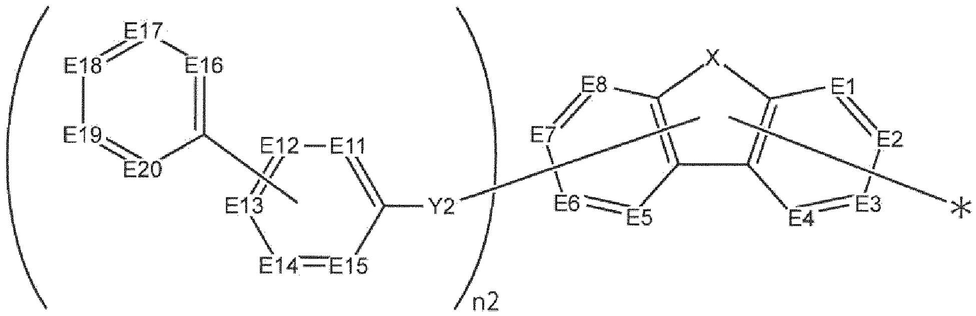

In General Formula (A-1), X represents -N(R)-, -O-, -S- or -Si (R)(R')-, and E1 to E8 each represent -C(R1)= or -N=, wherein R, R' and R1 each represent a hydrogen atom, a substituent or a linking site with Y1; Y2 merely represents a bond or a divalent linking group; E11 to E20 each represent - C(R2)= or -N=, wherein at least one of E11 to E20 represents - N=, wherein R2 represents a hydrogen atom, a substituent or a linking site, and wherein at least one of E11 and E12 represents -C(R2)=, where R2 represents a linking site; n2 represents an integer of 1 to 4; and * represents a linking site with Y1 in General Formula (6).

[Chemical Formula 66]

General Formula (A-2)

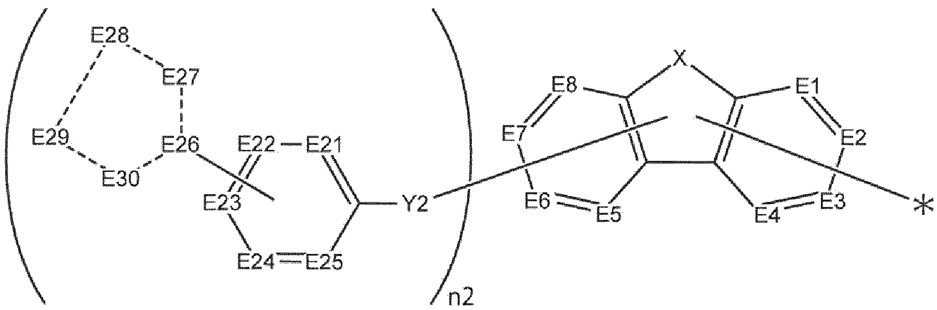

In General Formula (A-2), X represents -N(R)-, -O-, -S- or -Si (R)(R')-, and E1 to E8 each represent -C(R1)= or -N=, wherein R, R' and R1 each represent a hydrogen atom, a substituent or a linking site with Y1; Y2 merely represents a bond or a divalent linking group; E21 to E25 each represent - C(R2)= or -N=, and E26 to E30 each represent -C(R2)=, -N=, -O-, -S- or -Si(R3)(R4)-, wherein at least one of E21 to E30 represents -N=, wherein R2 represents a hydrogen atom, a substituent or a linking site, and R3 and R4 each represent a hydrogen atom or a substituent, and wherein at least one of E21 and E22 represents -C(R2)=, where R2 represents a linking site; n2 represents an integer of 1 to 4; and * represents a linking site with Y1 in General Formula (6).

[Chemical Formula 67]

General Formula(A-3)

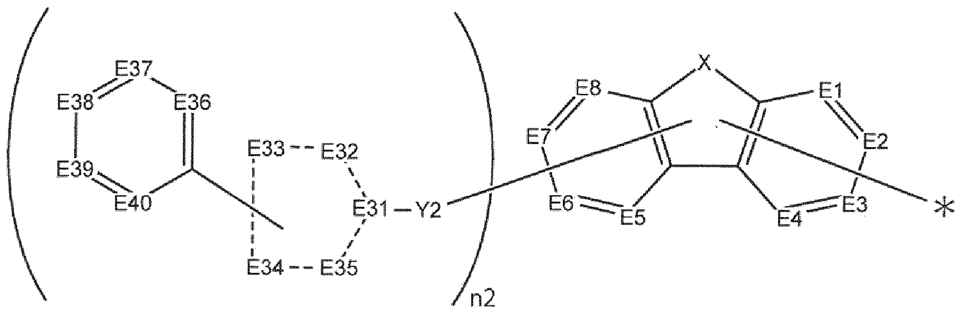

In General Formula (A-3), X represents -N(R)-, -O-, -S- or -Si (R)(R')-, and E1 to E8 each represent -C(R1)= or -N=, wherein R, R' and R1 each represent a hydrogen atom, a substituent or a linking site with Y1; Y2 merely represents a bond or a divalent linking group; E31 to E35 each represent - C(R2)=, -N=, -O-, -S- or -Si(R3)(R4)-, and E36 to E40 each represent -C(R2)= or -N=, wherein at least one of E31 to E40 represents -N=, wherein R2 represents a hydrogen atom, a substituent or a linking site, and R3 and R4 each represent a hydrogen atom or a substituent, and wherein at least one of E32 and E33 represents -C(R2)=, where R2 represents a linking site; n2 represents an integer of 1 to 4; and * represents a linking site with Y1 in General Formula (6).

[Chemical Formula 68]

General Formula(A-4)

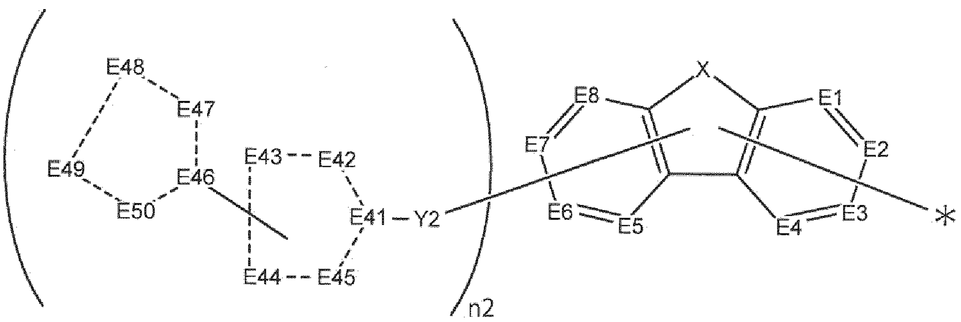

In General Formula (A-4), X represents -N(R)-, -O-, -S- or -Si (R)(R')-, and E1 to E8 each represent -C(R1)= or -N=, wherein R, R' and R1 each represent a hydrogen atom, a substituent or a linking site with Y1; Y2 merely represents a bond or a divalent linking group; E41 to E50 each represent - C(R2)=, -N=, -O-, -S- or -Si(R3)(R4)-, wherein at least one of E41 to E50 represents -N=, wherein R2 represents a hydrogen atom, a substituent or a linking site, and R3 and R4 each represent a hydrogen atom or a substituent, and wherein at least one of E42 and E43 represents -C(R2)=, where R2 represents a linking site; n2 represents an integer of 1 to 4; and * represents a linking site with Y1 in General Formula (6).

The group represented by any one of General Formulas (A-1) to (A-4) is described below.

The substituents represented by R, R' or R1 both in - N (R) - or -Si(R)(R')- represented by X and in -C(R1)= represented by each of E1 to E8 of the group represented by any one of General Formulas (A-1) to (A-4) are each synonymous with the substituent represented by R3 of -C(R3)= represented by each of E51 to E66 and E71 to E88 in General Formula (1).

The divalent linking group represented by Y2 of the group represented by any one of General Formulas (A-1) to (A-4) is synonymous with the divalent linking group represented by Y1 in General Formula (6).

The substituent represented by R2 of -C(R2)= represented by each of E11 to E20 in General Formula (A-1), each of E21 to E30 in General Formula (A-2), each of E31 to E40 in General Formula (A-3) or each of E41 to E50 in

General Formula (A-4) is synonymous with the substituent represented by R3 of - C(R3)= represented by each of E51 to E66 and E71 to E88 in General Formula (1).

Further preferred compound represented by General Formula (6) will be described below.

(Compound Represented by General Formula (7))

In the present invention, among the compounds represented by General Formula (6), a compound represented by General Formula (7) is preferable. General Formula (7) includes General Formula (1) shown as the compound constituting the nitrogen-containing layer 1a. The compound represented by General Formula (7) will be described below.

[Chemical Formula 69]

General Formula(7)

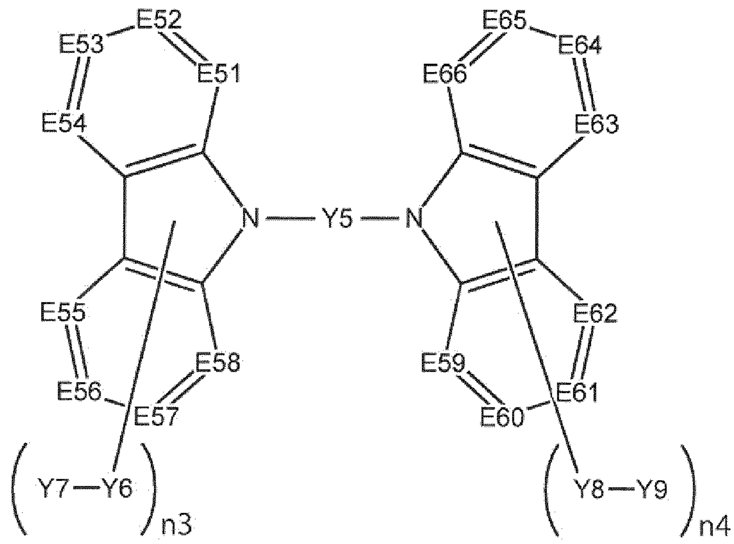

In General Formula (7), Y5 represents a divalent linking group which is an arylene group, a heteroarylene group or a combination of the arylene group and the heteroarylene group; E51 to E66 each represent -C(R3)= or -N=, wherein R3 represents a hydrogen atom or a substituent; Y6 to Y9 each represent a group derived from an aromatic hydrocarbon ring or a group derived from an aromatic heterocycle, wherein at least one of Y6 and Y7 and at least one of Y8 and Y9 each represent a group derived from an aromatic heterocycle containing a nitrogen atom; and n3 and n4 each represent an integer of 0 to 4, wherein the sum of n3 and n4 is 2 or more.

Y5 in General Formula (7) is synonymous with Y5 in General Formula (1).

E51 to E66 in General Formula (7) is synonymous with E51 to E66 in General Formula (1).

In General Formula (7), it is preferable that as groups represented by E51 to E66, six or more of E51 to E58 and six or more of E59 to E66 are each represented by -C(R3)=.

Examples of aromatic hydrocarbon ring used to form the group derived from an aromatic hydrocarbon ring represented by each of Y6 to Y9 in General Formula (7) include a benzene ring, a biphenyl ring, a naphthalene ring, an azulene ring, an anthracene ring, a phenanthrene ring, a pyrene ring, a chrysene ring, a naphthacene ring, a triphenylene ring, an o-terphenyl ring, an m-terphenyl ring, a p-terphenyl ring, an acenaphthene ring, a coronene ring, a fluorene ring, a fluoranthrene ring, a naphthacene ring, a pentacene ring, a perylene ring, a pentaphene ring, a picene ring, a pyrene ring, a pyranthrene ring, an anthranthrene ring and the like.

Further, the aforesaid aromatic hydrocarbon ring may have a substituent represented by R3 of -C(R3)= represented by each of E51 to E66 in General Formula (1).

Examples of aromatic heterocycle used to form the group derived from an aromatic heterocycle represented by each of Y6 to Y9 in General Formula (7) include a furan ring, a thiophene ring, an oxazole ring, a pyrrole ring, a pyridine ring, a pyridazine ring, a pyrimidine ring, a pyrazine ring, a triazine ring, a benzimidazole ring, an oxadiazole ring, a triazole ring, an imidazole ring, a pyrazole ring, a triazole ring, an indole ring, an indazole ring, a benzimidazole ring, a benzothiazole ring, a benzoxazole ring, a quinoxaline ring, a quinazoline ring, a cinnoline ring, a quinoline ring,

an isoquinoline ring, a phthalazine ring naphthylidine ring, a carbazole ring, a carboline ring, a diazacarbazole ring (which is a ring formed by further substituting one of carbon atoms constituting a carboline ring with a nitrogen atom) and the like.

Further, the aforesaid aromatic hydrocarbon ring may have a substituent represented by R3 of -C(R3)= represented by each of E51 to E66 in General Formula (1).

Examples of aromatic heterocycle containing an N atom used to form a group derived from an aromatic heterocycle containing an N atom represented by each of at least one of Y6 and Y7 and at least one of Y8 and Y9 in General Formula (7) include an oxazole ring, a pyrrole ring, a pyridine ring, a pyridazine ring, a pyrimidine ring, a pyrazine ring, a triazine ring, a benzimidazole ring, an oxadiazole ring, a triazole ring, an imidazole ring, a pyrazole ring, a triazole ring, an indole ring, an indazole ring, a benzimidazole ring, a benzothiazole ring, a benzoxazole ring, a quinoxaline ring, a quinazoline ring, a cinnoline ring, a quinoline ring, an isoquinoline ring, a phthalazine ring, a naphthylidine ring, a carbazole ring, a carboline ring, a diazacarbazole ring (which is a ring formed by further substituting one of carbon atoms constituting a carboline ring with a nitrogen atom) and the like.

In General Formula (7), it is preferred that the groups represented by Y7 and Y9 are each a group derived from a pyridine ring.

In General Formula (7), it is preferred that the groups represented by Y6 and Y8 are each a group derived from a benzene ring.

The compound represented by General Formula (1), as the compound constituting the nitrogen-containing layer 1a, is exemplified as a further preferable example of the compound represented by General Formula (7).

The compounds (1 to 118) exemplified above are shown as concrete examples of the compound represented by General Formula (6), (7), or General Formula (1).

[Blocking Layer: Hole Blocking Layer and Electron Blocking Layer]

As described above, a blocking layer is a layer provided according to necessity in addition to the basic constituent layers of the thin-film of the organic compound. Examples of the blocking layer include a hole blocking layer described in documents such as Japanese Unexamined Patent Application Publication Nos. 11-204258 and 11-204359 and pp. 273 of "Organic EL Element and Front of Industrialization thereof" (Nov. 30, 1998, published by N. T. S Co., Ltd.)".

The hole blocking layer has, in a broad sense, the function of an electron transporting layer. The hole blocking layer is formed of a hole blocking material having a function of transporting electrons with very little capability of transporting holes; the hole blocking layer transports electrons while blocking holes, so that probability of recombination of electrons and holes can be increased. Further, the configuration of an electron transporting layer (which is to be described later) can be used as the hole blocking layer according to necessity. It is preferred that the hole blocking layer is arranged adjacent to the light emitting layer.

On the other hand, the electron blocking layer has, in a broad sense, the function of a hole transporting layer. The electron blocking layer is made of a material having a function of transporting holes with very little capability of transporting electrons; the electron blocking layer transports holes while blocking electrons, so that probability of recombination of electrons and holes can be increased. Further, the configuration of the hole transporting layer (which is to be described later) can be used as the electron blocking layer according to necessity. The film-thickness of the blocking layer of the present invention is preferably within a range from 3 to 100 nm, and further preferably within a range from 5 to 30 nm.

<Other Components>

In the organic electroluminescence element EL-1 described above, in order to reduce the resistance of the transparent electrode arranged on the light extraction side (for example, the first electrode 5 in the present example), an auxiliary electrode may be provided abutting the first electrode 5. Further, in order to prevent the deterioration of the light emitting units 3-1 and 3-2 composed of an organic material and the like, the organic electroluminescence element EL-1 is sealed by a below-mentioned sealing material on the substrate 11. Incidentally, a below-mentioned protective film or protective plate may be provided, so that the organic electroluminescence element EL-1 and the sealing material are sandwiched between the substrate 11 and the protective film or protective plate.

[Auxiliary Electrode]

The auxiliary electrode is provided for reducing the resistance of the electrode having light transmissibility (for example, the first electrode 5 in the present example), and is arranged in contact with the first electrode 5. It is preferred that a metal having low resistance, such as aurum, platinum, argent, copper, aluminum or the like, is used to form the auxiliary electrode. Since each of these metals has low light transmissibility, the auxiliary electrode is formed into a pattern in a range where the luminescence light h is not prevented from being extracted from a light extracting face.

Examples of the method for forming such an auxiliary electrode include a deposition method, a sputtering method, a printing method, an ink-jet method, an aerosol jet method and the like. In view of aperture ratio of light extraction, it is preferred that the line width of the auxiliary electrode is 50 $\mu$m or less; while in view of electrical conductivity, it is preferred that the film-thickness of the auxiliary electrode is 1 $\mu$ or more.

[Sealing Material]

The sealing material is provided to cover the organic electroluminescence element EL-1; the sealing material may either be a plate-like (film-like) sealing member to be fixed to the side of the substrate 11 by an adhesive, or be a sealing film. However, the terminal portions of both the first electrode 5 and the second electrode 7 are exposed from the sealing material in a state where the first electrode 5 and the second electrode 7 are insulated from each other by the light emitting units 3-1 and 3-2 on the substrate 11.

Concrete examples of the plate-like (film-like) sealing material include a glass substrate, a polymer substrate, a metallic material substrate and the like; and each of these substrate materials may also be formed into a further thinner film to be used as the sealing material.

Examples of the glass substrate include a soda-lime glass substrate, a barium/strontium-containing glass substrate, a lead glass substrate, an aluminosilicate glass substrate, a borosilicate glass substrate, a barium borosilicate glass substrate, a quartz substrate, and the like. Examples of the polymer substrate include a polycarbonate substrate, an acryl resin substrate, a polyethylene terephthalate substrate, a polyether sulfide substrate, a polysulfone substrate and the like. Examples of the metallic material substrate include substrates each formed of one or more kinds of metals selected from the group consisting of stainless steel, iron, copper, aluminum, magnesium, nickel, zinc, chromium, titanium, molybdenum, silicon, germanium, tantalum, and alloys thereof.

Among these substances, it is preferred that a thin-film-like polymer substrate or metallic material substrate is used as the sealing material in order to reduce the thickness of the element. However, in the case where the organic electroluminescence element EL-1 is configured as an organic electroluminescence element in which the light is also extracted from the side of the second electrode 7, which is arranged on the side opposite to the substrate 11, a glass substrate or polymer substrate having light transmissibility is used as the sealing material.

Further, it is preferred that the film-like polymer substrate has an oxygen permeability of $1\times10^{-3}$ml/(m$^2$•24h•atm) or less measured by a method in conformity with JIS K 7126-1987 and a water vapor permeability of 1•$10^{-3}$ g/(m$^2$•24h) or less (at temperature of 25$\pm$0.5°C and relative humidity of (90$\pm$2)%RH) measured by a method in conformity with JIS K 7129-1992.

Further, the aforesaid substrate material may also be formed into a concave plate so as to be used as the sealing material. In such a case, the aforesaid substrate member is subjected to a sand blasting process, a chemical etching process and/or the like, and formed into a concave shape.

The adhesive for fixing the plate-like sealing material to the side of the substrate 11 is used as a sealing agent for sealing the organic electroluminescence element EL-1 sandwiched between the sealing material and the substrate 11. Concrete examples of the adhesive include a photo-curable or thermo-curable adhesive agent containing a reactive vinyl group such as an acrylic acid oligomer or a methacrylic acid oligomer, and a moisture curable adhesive agent such as 2-cyanoacrylate.

Concrete examples of the adhesive also include an epoxy based thermally and chemically (two liquid type) curable adhesive agents; a hot-melt type polyamide, polyester or polyolefin adhesive agents; and a cationic curable type UV-curable epoxy adhesive.

Incidentally, since there is a possibility that the organic material constituting the organic electroluminescence element EL-1 might be degraded by heat treatment, it is preferred that the adhesive is an adhesive possible to be cured in a temperature from room temperature to 80°C. Further, a drying agent may be dispersed in the adhesive.

The adhesive may be coated onto the adhesion portion between the sealing material and the substrate 11 either by a commercially available dispenser, or by printing such as screen printing.

In the case where a gap is formed between the sealing material, the substrate 11 and the adhesive, it is preferred that an inert gas (such as nitrogen, argon or the like) or an inert liquid (such as fluorinated hydrocarbon, silicone oil or the like) is injected, in the form of gas or liquid phase, into the gap. Alternatively, the gap may also be in a vacuum state, or may have a hygroscopic compound enclosed therein.

Examples of the hygroscopic compound include a metal oxide (such as sodium oxide, potassium oxide, calcium oxide, barium oxide, magnesium oxide and aluminum oxide), a sulfate (such as sodium sulfate, calcium sulfate, magnesium sulfate and cobalt sulfate), a metal halide (such as calcium chloride, magnesium chloride, cesium fluoride, tantalum fluoride, cerium bromide, magnesium bromide, barium iodide and magnesium iodide), a perchloric acid (such as barium perchlorate and magnesium perchlorate) and the like; wherein if the sulfate, the metal halide or the perchlorate is used, anhydrides thereof will be preferable.

On the other hand, in the case where a sealing film is used as the sealing material, the sealing film is formed

on the substrate 11 in a state where the light emitting units 3-1 and 3-2 of the organic electroluminescence element EL-1 is completely covered, and the terminal portions of the first electrode 5 and second electrode 7 of the organic electroluminescence element EL-1 are exposed to the outside.

Such a sealing film is formed of an inorganic material or an organic material. Particularly, such a sealing film is formed of a material capable of inhibiting penetration of substances, such as moisture, oxygen and the like, which cause the light emitting units 3-1 and 3-2 of the organic electroluminescence element EL-1 to degrade. Examples of such material include silicon oxide, silicon dioxide, silicon nitride and the like. Further, in order to reduce the fragility of the sealing film, the sealing film may have a laminated structure composed of the film(s) formed of one of the aforesaid inorganic materials and the film(s) formed of an organic material.

There is no particular limitation on the method of forming the aforesaid films. For example, the aforesaid films may be formed by a vacuum deposition method, a sputtering method, a reactive sputtering method, a molecular beam epitaxy method, a cluster ion beam method, an ion plating method, a plasma polymerization method, an atmospheric pressure plasma polymerization method, a plasma CVD method, a laser CVD method, a thermal CVD method, a coating method or the like.

Incidentally, the aforesaid sealing material may further be provided with electrodes, so that the terminal portions of the light emitting units 3-1 and 3-2 of the organic electroluminescence element EL-1 are brought into conduction with the electrodes.

[Protective Film, Protective Plate]

The protective film or protective plate is provided for mechanically protecting the organic electroluminescence element EL-1; it is preferred that such protective film or protective plate is provided particularly in the case where the sealing material is a sealing film, because in such a case mechanical protection to the organic electroluminescence element EL-1 is not sufficient.

A glass plate, a polymer plate, a polymer film (which is thinner than the polymer plate), a metal plate, a metal film (which is thinner than the metal plate), a polymer material film or a metal material film may be used as the aforesaid protective film or protective plate. Among these materials, the polymer film is preferably used in order to reduce the weight and thickness.

<Method for Producing Organic Electroluminescence Element EL-1>

First, the first electrode 5 having light transmissibility is formed as an anode on the substrate 11 by a suitable film-forming method such as a deposition method, a sputtering method or the like. Further, before and after the formation of the first electrode 5, the auxiliary electrode is formed into a pattern if necessary.

Next, the hole injecting layer, the hole transporting layer, the light emitting layer, the electron transporting layer and the electron injecting layer are formed, in this order, on the first electrode 5 to thereby form the light emitting unit 3-1. Examples of the method for forming each of these layers include a spin coating method, a casting method, an ink-jet method, a deposition method, a printing method and the like; however, a vacuum deposition method or a spin coating method is particularly preferable because by such method, it is easy to form a uniform film and unlikely to cause pinholes. Further, these layers may each be formed by a different film-forming method. When the deposition method is used to form each of these layers, the deposition conditions vary depending on the type of the compound used; generally, it is preferred that the boat heating temperature is selected in a range from 50°C to 450°C, the vacuum degree is selected in a range from $10^{-6}$ Pa to $10^{-2}$ Pa, the deposition rate is selected in a range from 0.01 nm/sec to 50 nm/sec, the substrate temperature is selected in a range from -50°C to 300°C, and the film-thickness is selected in a range from 0.1 μm to 5 μm.

Then, the nitrogen-containing layer 1a consisting of a compound containing nitrogen atom is formed on the light emitting unit 3-1 so that the film-thickness of the nitrogen-containing layer 1a is 1 μm or less, preferably between 10 nm and 100 nm. Then, the transparent conductive layer 1b composed of silver (or an alloy having silver as a main component) is formed on the nitrogen-containing layer 1a so that the film-thickness of the transparent conductive layer 1b is in a range of 4 nm to 12 nm. Examples of the method for forming the nitrogen-containing layer 1a and the transparent conductive layer 1b include a spin coating method, a casting method, an ink-jet method, a deposition method, a printing method and the like; however, a vacuum deposition method is particularly preferable, because by such method, it is easy to form a uniform film and unlikely to cause pinholes.

Thereafter, the hole injecting layer, the hole transporting layer, the light emitting layer, the electron transporting layer and the electron injecting layer are formed, in this order, on the transparent conductive layer 1b to thereby form the light emitting unit 3-2. Each layer was formed in the same manner as that of the light emitting unit 3-1.

Thereafter, the second electrode 7 is formed as a cathode on the light emitting unit 3-2 by a suitable film-forming method such as a deposition method, a sputtering method or the like.

By performing the above processes, the organic electroluminescence element EL-1 is obtained. Thereafter,

the sealing material (not shown) is arranged to cover at least the light emitting units 3-1 and 3-2 in a state where the terminal portions of both the first electrode 5 and the second electrode 7 of the organic electroluminescence element EL-1 are exposed to the outside. At this time, the adhesive is used to bond the sealing material to the side of the substrate 11, so that the organic electroluminescence element EL-1 is sealed between the sealing material and the substrate 11.

By performing the above process, a desired organic electroluminescence element EL-1 is obtained on the substrate 11. It is preferred that, when producing the organic electroluminescence element EL-1, the layers from the light emitting unit 3-1 to the second electrode 7 are continuously formed with one vacuuming operation; however, it is also possible to take out the substrate 11 from the vacuum atmosphere during production to perform different film-forming method. In such a case, necessary considerations (such as performing the production process in dry inert gas atmosphere) should be taken into account.

When driving the organic electroluminescence element EL-1 obtained in the aforesaid manner, if a DC voltage is applied, light emission can be observed if a voltage of about 2 V to 40 V is applied, wherein the electrode "+" is connected to the first electrode 5 (which is the anode) and the electrode "-" is connected to the second electrode 7 (which is the cathode). Also, an AC voltage may be applied to the first electrode 5 and the second electrode 7, wherein the AC voltage may have any waveform.

<Advantages of Organic Electroluminescence Element EL-1>

The organic electroluminescence element EL-1 has a configuration in which the aforesaid transparent conductive layer 1b having electrical conductivity and light transmissibility is sandwiched between two light emitting units 3-1, 3-2. Thus, it is possible to improve the luminous efficiency by sufficiently injecting charges from the transparent conductive layer 1b into the light emitting units 3-1, 3-2 to ensure luminous efficiency while restraining absorption, in the transparent conductive layer 1b, of the luminescence light h generated by the light emitting units 3-1, 3-2. Further, due to this feature, it is also possible to increase the light-emitting lifetime by reducing the driving voltage necessary for obtaining a given brightness.

«3. Modification 1 of First Embodiment»

FIG. 3 is a schematic cross-sectional view for explaining Modification 1 of the organic electroluminescence element EL-1 of the first embodiment. As shown in FIG. 3, an organic electroluminescence element EL-1' is configured so that, in addition to the first electrode 5 and second electrode 7, the transparent conductive layer 1b also has a driving voltage applied thereto; and the laminated structure of the organic electroluminescence element EL-1' is identical to that of the first embodiment.

In such a case, when driving the organic electroluminescence element EL-1, in the case where a DC voltage is applied wherein a driving voltage V1 is applied between the first electrode 5 and the transparent conductive layer 1b and a driving voltage V2 is applied between the transparent conductive layer 1b and the second electrode 7, a voltage of about 2 V to 40 V is applied with the electrode "+" connected to the first electrode 5 (which is the anode) and with the electrode "-" connected to the second electrode 7 (which is the cathode), and further, an intermediate voltage between the anode and the cathode is applied to the transparent conductive layer 1b.

In such an organic electroluminescence element EL-1' of Modification 1 having such a configuration, the ratio of the light emitted by the light emitting unit 3-1 to the light emitted by the light emitting unit 3-2 can be arbitrarily changed by adjusting the intermediate voltage applied to the transparent conductive layer 1b. In the case where the light emitting units 3-1 and 3-2 are configured so that they each obtain luminescence light h of different color, it is possible to control color luminescence light by controlling the ratio of the light emitted by the light emitting unit 3-1 to the light emitted by the light emitting unit 3-2.

«4. Modification 2 of First Embodiment»

FIG. 4 is a schematic cross-sectional view for explaining Modification 2 of the organic electroluminescence element EL-1 of the first embodiment. As shown in FIG. 4, an organic electroluminescence element EL-1" has a configuration in which a driving voltage is applied so that the first electrode 5 and the second electrode 7 have the same polarity, and the transparent conductive layer 1b has the opposite polarity, and in which one of the two light emitting units 3-1 and 3-2 is inversely laminated. Other configurations of the organic electroluminescence element EL-1" are identical to those of the first embodiment.

Here, as an example, the first electrode 5 and the second electrode 7 are used as anodes. In such a case, the transparent conductive layer 1b is used as a cathode. Thus, a light emitting unit 3-2' arranged on the side of the second electrode 7 has an inversely laminated configuration in which [an electron injecting layer / an electron transporting layer / a light emitting layer / a hole transporting layer / a hole injecting layer] are laminated in this order from the side of the

substrate 11. In such a configuration, the nitrogen-containing layer 1a and the transparent conductive layer 1b are also formed by laminating a nitrogen-containing layer 1a and a transparent conductive layer 1b, in this order, from the side of the substrate 11.

The organic electroluminescence element EL-1" is driven in the same manner as the first embodiment. In other words, in the case where a DC voltage is applied wherein a driving voltage V1 is applied between the first electrode 5 and the transparent conductive layer 1b and a driving voltage V2 is applied between the transparent conductive layer 1b and the second electrode 7, light emission can be observed if a voltage of about 2 V to 40 V is applied with the electrode "+" connected to the first electrode 5 and the second electrode 7 (which both are the anodes) and with the electrode "-" connected to the transparent conductive layer 1b (which is the cathode). Also, an AC voltage may be applied to the first electrode 5, the second electrode 7, and the transparent conductive layer 1b, wherein the waveform of the AC voltage is not particularly limited. Further, in the case where the layers constituting the light emitting unit 3-2’ and the layers constituting the light emitting unit 3-1 are inversely laminated, since the first electrode 5 and the second electrode 7 are the cathodes, the first electrode 5 and the second electrode 7 are regarded as the electrode "-"; and since the transparent conductive layer 1b is the anode, the transparent conductive layer 1b is regarded as the electrode "+".

Compared to the organic electroluminescence element of the first embodiment described with reference to FIG. 2, in the organic electroluminescence element EL-1" of Modification 2 having the aforesaid configuration, since current density necessary for obtaining a given brightness can be reduced, it becomes possible to improve light-emitting lifetime although a higher driving voltage is needed.

«5. Second Embodiment»

(An example in which the conductive layer between the two light emitting units and the second electrode are each a transparent conductive layer)

FIG. 5 is a view showing a cross-sectional configuration of a second embodiment of the organic electroluminescence element with a tandem structure that uses the aforesaid transparent conductive layer 1b.The characteristic configuration of an organic electroluminescence element EL-2 according to a second embodiment will be described below with reference to FIG. 5. Note that, in the following paragraphs, components identical to those of the first embodiment will not be repeatedly described in detail.

The organic electroluminescence element EL-2 shown in FIG. 5 differs from the organic electroluminescence element of the first embodiment described with reference to FIG. 2 in that a transparent conductive layer 1b-2 is used as the second electrode 7 provided on the side opposite to the substrate 11. Further, the transparent conductive layer 1b-2 is arranged adjacent to a nitrogen-containing layer 1a-2. Other configurations of the organic electroluminescence element EL-2 are identical to those of the first embodiment.

In other words, similar to the first embodiment, in the organic electroluminescence element EL-2, a nitrogen-containing layer 1a-1 and a transparent conductive layer 1b-1 are arranged between the two light emitting units 3-1, 3-2, and further, the nitrogen-containing layer 1a-2 and the transparent conductive layer 1b-2 are arranged, in this order, on the light emitting unit 3-2. The nitrogen-containing layer 1a-1 and the transparent conductive layer 1b-1 arranged between the two light emitting units 3-1, 3-2, and the nitrogen-containing layer 1a-2 and the transparent conductive layer 1b-2 arranged on the light emitting units 3-2 are identical to the nitrogen-containing layer and the transparent conductive layer described in the first embodiment.

Thus, the nitrogen-containing layer 1a-2 arranged adjacent to the light emitting unit 3-2 may also be regarded as a layer that forms a portion of the light emitting unit 3-2. For this reason, if the transparent conductive layer 1b-2 used as the second electrode 7 is a cathode, it is preferred that the nitrogen-containing layer 1a-2 arranged adjacent to the transparent conductive layer 1b-2 has electron injection performance or electron transport performance. In contrast, if the transparent conductive layer 1b-2 used as the second electrode 7 is an anode, it is preferred that the nitrogen-containing layer 1a-2 arranged adjacent to the transparent conductive layer 1b-2 has hole injection performance or hole transport performance.

The organic electroluminescence element EL-2, in which the second electrode 7 is configured by the transparent conductive layer 1b-2, may either be a dual emission type organic electroluminescence element in which the luminescence light h is also extracted from the side opposite to the substrate 11, or be a top emission type organic electroluminescence element if the first electrode 5 is an electrode having no light transmissibility, such as a reflective material, for example.

Similar to the method for producing the organic electroluminescence element of the first embodiment, the organic electroluminescence element EL-2 having the aforesaid configuration may be produced by forming each of the layers into a pattern sequentially from the layer on the side of the substrate 11.

<Advantages of Organic Electroluminescence Element EL-2>

The organic electroluminescence element EL-2 described above has a configuration in which the transparent conductive layer 1b-1 having electrical conductivity and light transmissibility is sandwiched between two light emitting units 3-1, 3-2, and the transparent conductive layer 1b-2 having electrical conductivity and light transmissibility is used as the second electrode 7. Thus, it is possible to improve light extraction efficiency by sufficiently injecting charges from the two transparent conductive layers 1b-1, 1b-2 into the two light emitting units 3-1, 3-2 to ensure luminous efficiency while restraining absorption, in the transparent conductive layers 1b-1, 1b-2, of the luminescence light h generated by the light emitting units 3-1, 3-2. Further, due to such a configuration, it is also possible to increase the light-emitting lifetime by reducing current density necessary for obtaining a given brightness.

<Combination with Modification 1>

The configuration of the second embodiment may be combined with the configuration of Modification 1 of the first embodiment. In such a case, a driving voltage V1 is applied between the first electrode 5 and the transparent conductive layer 1b-1, and further, a driving voltage V2 is applied between the transparent conductive layer 1b-1 and the second electrode 7 (the transparent conductive layer 1b-2). With such a configuration, the same advantages as those of Modification 1 of the first embodiment can be achieved.

<Combination with Modification 2>

Further, the configuration of the second embodiment may be combined with the configuration of Modification 2 of the first embodiment. In such a case, the layers constituting the light emitting unit 3-1 and the layers constituting the light emitting unit 3-2 are laminated in opposite order to each other. In such a configuration, the nitrogen-containing layer 1a-1 and the transparent conductive layer 1b-1 are arranged, in this order, between the two light emitting units 3-1, 3-2 from the side of the substrate 11, and the nitrogen-containing layer 1a-2 and the transparent conductive layer 1b-2 are arranged, in this order, on the light emitting unit 3-2 from the side of the substrate 11.
Further, in such a case, similar to Modification 2 of the first embodiment, a driving voltage V1 and a driving voltage V2 are applied to the first electrode 5, the transparent conductive layer 1b-1, and the second electrode 7 (the transparent conductive layer 1b-2).
With the configuration obtained by combining the second embodiment with Modification 2, the same advantages as those of Modification 2 of the first embodiment can also be achieved.

<<6. Third Embodiment>>

(An example in which the conductive layer between the two light emitting units and the first electrode are each a transparent conductive layer)

FIG. 6 is a view showing a cross-sectional configuration of a third embodiment of an organic electroluminescence element with a tandem structure that uses the aforesaid transparent conductive layer 1b. The characteristic configuration of an organic electroluminescence element EL-3 according to the third embodiment will be described below with reference to FIG. 6. Note that, in the following paragraphs, components identical to those of the first embodiment will not be repeatedly described in detail.
The organic electroluminescence element EL-3 shown in FIG. 6 differs from the organic electroluminescence element of the first embodiment described with reference to FIG. 2 in that a transparent conductive layer 1b-1 is used as the first electrode 5 provided on the side of the substrate 11. Further, the transparent conductive layer 1b-1 is arranged adjacent to a nitrogen-containing layer 1a-1. Other configurations of the organic electroluminescence element EL-3 are identical to those of the first embodiment.
In other words, in the organic electroluminescence element EL-3, the nitrogen-containing layer 1a-1 and the transparent conductive layer 1b-1 are arranged, in this order, in contact with the substrate 11, and further, similar to the first embodiment, a nitrogen-containing layer 1a-2 and a transparent conductive layer 1b-2 are arranged between two light emitting units 3-1, 3-2. The nitrogen-containing layer 1a-1 and the transparent conductive layer 1b-1 arranged adjacent to the substrate 11, and thenitrogen-containing layer 1a-2 and the transparent conductive layer 1b-2 arranged between the two light emitting units 3-1, 3-2 are identical to the nitrogen-containing layer and the transparent conductive layer described in the first embodiment.
Similar to the method for producing the organic electroluminescence element of the first embodiment, the organic electroluminescence element EL-3 may be produced by forming each of the layers into a pattern sequentially from the layer on the side of the substrate 11.

<Advantages of Organic Electroluminescence Element EL-3>

The organic electroluminescence element EL-3 described above has a configuration in which the transparent conductive layer 1b-2 having electrical conductivity and light transmissibility is sandwiched between the two light emitting units 3-1, 3-2, and the transparent conductive layer 1b-1 having electrical conductivity and light transmissibility is used as the first electrode 5. Thus, it is possible to improve light extraction efficiency by sufficiently injecting charges from the two transparent conductive layers 1b-1, 1b-2 into the two light emitting units 3-1, 3-2 to ensure luminous efficiency while restraining absorption, in the transparent conductive layer 1b, of the luminescence light h generated by the light emitting units 3-1, 3-2. Further, due to such a configuration, it is also possible to increase the light-emitting lifetime by reducing current density necessary for obtaining a given brightness.

<Combination with Modification 1>

The configuration of the third embodiment may also be combined with the configuration of Modification 1 of the first embodiment. In such a case, a driving voltage V1 is applied between the first electrode 5 (which is constituted by the transparent conductive layer 1b-1) and the transparent conductive layer 1b-2, and further, a driving voltage V2 is applied between the transparent conductive layer 1b-2 and the second electrode 7. With such a configuration, the same advantages as those of Modification 1 of the first embodiment can be achieved.

<Combination with Modification 2>

Further, the configuration of the third embodiment may be combined with the configuration of Modification 2 of the first embodiment. In such a case, the layers constituting the light emitting unit 3-1 and the layers constituting the light emitting unit 3-2 may be laminated in opposite order to each other. In such a configuration, the nitrogen-containing layer 1a-1 and the transparent conductive layer 1b-1 are arranged, in this order, on the substrate 11 from the side of the substrate 11, and the nitrogen-containing layer 1a-2 and the transparent conductive layer 1b-2 are arranged, in this order, between the two light emitting units 3-1, 3-2 from the side of the substrate 11.

In such a case, similar to Modification 2 of the first embodiment, the driving voltages V1, V2 are applied to the first electrode 5 (the transparent conductive layer 1b-1), the transparent conductive layer 1b-2, and the second electrode 7.

With the configuration obtained by combining the second embodiment with Modification 2, the same advantages as those of Modification 2 of the first embodiment can be achieved.

<<7. Fourth Embodiment>>

(An example in which the conductive layer between the two light emitting units and the two electrodes are each a transparent conductive layer)

FIG. 7 is a view showing a cross-sectional configuration of a fourth embodiment of the organic electroluminescence element with a tandem structure that uses the aforesaid transparent conductive layer 1b. The characteristic configuration of an organic electroluminescence element EL-4 according to the fourth embodiment will be described below with reference to FIG. 7. Note that, in the following paragraphs, components identical to those of the first embodiment will not be repeatedly described in detail.

The organic electroluminescence element EL-4 shown in FIG. 7 differs from the organic electroluminescence element of the first embodiment described with reference to FIG. 2 in that a transparent conductive layer 1b-1 and a transparent conductive layer 1b-3 are used as the first electrode 5 and the second electrode 7. Further, the transparent conductive layer 1b-1 and the transparent conductive layer 1b-3 are arranged adjacent to a nitrogen-containing layer 1a-1 and a nitrogen-containing layer 1a-3 respectively. Other configurations of the organic electroluminescence element EL-4 are identical to those of the first embodiment.

In other words, similar to the first embodiment, in the organic electroluminescence element EL-4, a nitrogen-containing layer 1a-2 and a transparent conductive layer 1b-2 are arranged between two light emitting units 3-1, 3-2. Further, the nitrogen-containing layer 1a-1 and the transparent conductive layer 1b-1 are arranged, in this order, between the substrate 11 and the light emitting unit 3-1 from the side of the substrate 11. Further, the nitrogen-containing layer 1a-3 and the transparent conductive layer 1b-3 are arranged, in this order, on the light emitting unit 3-2 from the side of the substrate 11. The nitrogen-containing layers 1a-1 to 1a-3 and the transparent conductive layers 1b-1 to 1b-3 each have the configuration described before.

Thus, the nitrogen-containing layer 1a-3 arranged adjacent to the light emitting unit 3-2 may also be regarded as a layer that forms a portion of the light emitting unit 3-2. Thus, if the transparent conductive layer 1b-3 used as the second electrode 7 is a cathode, it is preferred that the nitrogen-containing layer 1a-3 arranged adjacent to the transparent

conductive layer 1b-3 has electron injection performance or electron transport performance. In contrast, if the transparent conductive layer 1b-3 used as the second electrode 7 is an anode, it is preferred that the nitrogen-containing layer 1a-3 arranged adjacent to the transparent conductive layer 1b-3 has hole injection performance or hole transport performance.

The organic electroluminescence element EL-4, in which the first electrode 5 and the second electrode 7 are each configured by the transparent conductive layer 1b, is a dual emission type organic electroluminescence element in which the luminescence light h is also extracted from the side opposite to the substrate 11.

Similar to the method for producing the organic electroluminescence element of the first embodiment, the organic electroluminescence element EL-4 may be produced by forming each of the layers into a pattern sequentially from the layer on the side of the substrate 11.

<Advantages of Organic Electroluminescence Element EL-4>

The organic electroluminescence element EL-4 described above has a configuration in which the transparent conductive layer 1b-2 having electrical conductivity and light transmissibility is sandwiched between two light emitting units 3-1, 3-2, and the transparent conductive layer 1b-1 and transparent conductive layer 1b-3 having electrical conductivity and light transmissibility are used as the first electrode 5 and the second electrode 7 respectively. Thus, it is possible to improve light extraction efficiency by sufficiently injecting charges from the three transparent conductive layers 1b-1 to 1b-3 into the light emitting units 3-1, 3-2 to ensure luminous efficiency while restraining absorption, in the transparent conductive layers 1b-1 to 1b-3, of the luminescence light h generated by the light emitting units 3-1, 3-2. Further, due to this feature, it is also possible to increase the light-emitting lifetime by reducing current density necessary for obtaining a given brightness.

<Combination with Modification 1>

Further, the configuration of the fourth embodiment may also be combined with the configuration of Modification 1 of the first embodiment. In such a case, a driving voltage V1 is applied between the first electrode 5 (the transparent conductive layer 1b-1) and the transparent conductive layer 1b-2, and further, a driving voltage V2 is applied between the transparent conductive layer 1b-2 and the second electrode 7 (the transparent conductive layer 1b-3). With such a configuration, the same advantages as those of Modification 1 of the first embodiment can be achieved.

<Combination with Modification 2>

Further, the configuration of the fourth embodiment may be combined with the configuration of Modification 2 of the first embodiment. In such a case, the layers constituting the light emitting unit 3-1 and the layers constituting the light emitting unit 3-2 may be laminated in opposite order to each other. In such a configuration, the nitrogen-containing layers 1a-1 to 1a-3 are arranged adjacent to the substrate 11 side of the transparent conductive layers 1b-1 to 1b-3.

In such a case, similar to Modification 2 of the first embodiment, driving voltages V1, V2 are applied to the first electrode 5 (the transparent conductive layer 1b-1), the transparent conductive layer 1b-2, and the second electrode 7 (the transparent conductive layer 1b-3).

With the configuration obtained by combining the fourth embodiment with Modification 2, the same advantages as those of Modification 2 of the first embodiment can be achieved.

«8. Fifth Embodiment»

(An example in which a plurality of transparent conductive layers are arranged between three or more light emitting units)

FIG. 8 is a view showing a cross-sectional configuration of a fifth embodiment of the organic electroluminescence element with a tandem structure that uses the aforesaid transparent conductive layer 1b. The characteristic configuration of an organic electroluminescence element EL-5 according to the fifth embodiment will be described below with reference to FIG. 8. Note that, in the following paragraphs, components identical to those of the first embodiment will not be repeatedly described in detail.

The organic electroluminescence element EL-5 shown in FIG. 8 differs from the organic electroluminescence element of the first embodiment described with reference to FIG. 2 in that three or more (in this example, the number is three) light emitting units 3-1, 3-2, 3-3 are laminated, and nitrogen-containing layer 1a-1, 1a-2 and transparent conductive layer 1b-1, 1b-2 are arranged between each two of the light emitting units 3-1, 3-2, 3-3. Other configurations of the organic electroluminescence element EL-5 are identical to those of the organic electroluminescence element of the first embodiment.

In other words, similar to the first embodiment, the entire layer-structure of each of the light emitting units 3-1, 3-2, 3-2 is not particularly limited, but the light emitting units 3-1, 3-2, 3-2 may individually have a generic layer-structure. Further, the light emitting units 3-1, 3-2, 3-3 may either be configured so that they each emit luminescence light h of the same color, or be configured so that they respectively emit luminescence light h of different colors. In such a case, the light emitting units 3-1, 3-2, 3-3 may respectively emit luminescence light of red (R), luminescence light of green (G) and luminescence light of blue (B), so that the organic electroluminescence element emits white light.

The nitrogen-containing layers 1a-1, 1a-2 and the transparent conductive layers 1b-1, 1b-2 have the configuration described before; and here the nitrogen-containing layers 1a-1, 1a-2 and the transparent conductive layers 1b-1, 1b-2 are arranged, in this order, from the side of the substrate 11, for example.

Similar to the method for producing the organic electroluminescence element of the first embodiment, the organic electroluminescence element EL-5 may be produced by forming each of the layers into a pattern sequentially from the layer on the side of the substrate 11.

<Advantages of Organic Electroluminescence Element EL-5>

The organic electroluminescence element EL-5 has a configuration in which the transparent conductive layer 1b-1 and the transparent conductive layer 1b-2 having electrical conductivity and light transmissibility are sandwiched between the two light emitting unit 3-1 and the two light emitting unit 3-2 and between the two light emitting unit 3-2 and the two light emitting unit 3-3. Thus, similar to the first embodiment, it is possible to improve light extraction efficiency by sufficiently injecting charges from the transparent conductive layers 1b-1, 1b-2 into the light emitting units 3-1, 3-2, 3-3 to ensure luminous efficiency while restraining absorption, in the transparent conductive layers 1b-1, 1b-2, of the luminescence light h generated by the light emitting units 3-1, 3-2, 3-3. Further, due to this feather, it is also possible to increase the light-emitting lifetime by reducing the driving voltage necessary for obtaining a given brightness.

Incidentally, although the fifth embodiment is described based on a configuration in which three light emitting units 3-1, 3-2, 3-3 are laminated, the present invention also includes a configuration in which more than three light emitting units are laminated. In such a case, the organic electroluminescence element also has a configuration in which the transparent conductive layers 1b-1, 1b-2 and the nitrogen-containing layer 1a-1, 1a-2 adjacent to the transparent conductive layers 1b-1, 1b-2 are sandwiched between the light emitting units. The nitrogen-containing layer 1a-1, 1a-2 are arranged adjacent to the substrate 11 side of the transparent conductive layers 1b-1, 1b-2.

<Combination with Modification 1>

Further, the configuration of the fifth embodiment may also be combined with the configuration of Modification 1 of the first embodiment. In such a case, driving voltages are also applied to the transparent conductive layers 1b-1, 1b-2, in addition to the first electrode 5 and the second electrode 7. At this time, it is possible to select a configuration for applying driving voltages between the first electrode 5, at least one of the transparent conductive layers 1b-1, 1b-2n, and the second electrode 7, between which each of light emitting units 3-1, 3-2, 3-3 is sandwiched. The driving voltage applied to the transparent conductive layers 1b-1, 1b-2 is an intermediate potential between the voltage applied to the anode (for example, the first electrode 5) and the voltage applied to the cathode (for example, the second electrode 7), and the potentials are set so that they are different from high value to low value from the side of the anode.

In such a configuration obtained by combining the fifth embodiment with Modification 1, the percentage of the light emitted by the light emitting units 3-1, 3-2, 3-3 can be arbitrarily changed by adjusting the intermediate potentials applied to the transparent conductive layers 1b-1, 1b-2. In the case where the organic electroluminescence element EL-5 is configured so that the light emitting units 3-1, 3-2, 3-3 respectively obtain luminescence light h of R(red), G(green) and B(blue), it is possible to control full-color light emission by controlling the percentage of the light emitted by the light emitting units 3-1, 3-2, 3-3.

<Combination with Modification 2>

Further, the configuration of the fifth embodiment may be combined with the configuration of Modification 2 of the first embodiment. In such a case, the layers constituting each light emitting unit may not be laminated in the same order for all light emitting units 3-1, 3-2, 3-3, but several of the light emitting units 3-1, 3-2, 3-3 may be inversely laminated. In such a case, the nitrogen-containing layers 1a-1, 1a-2 and the transparent conductive layers 1b-1, 1b-2 between each two of the light emitting units 3-1, 3-2, 3-3 are also arranged in the order of the nitrogen-containing layers 1a-1, 1a-2 and the transparent conductive layers 1b-1, 1b-2 from the side of the substrate 11.

Further, in such a case, the driving voltage applied between the first electrode 5 and the second electrode 7, between which the light emitting units 3-1, 3-2, 3-3 are sandwiched, and the driving voltage applied between the two transparent conductive layer 1b-1, 1b-2 are adjusted so that the polarity on the hole injection side of each of the light

emitting units 3-1, 3-2, 3-3 becomes the electrode "+", and the polarity on the electron injection side becomes the electrode "-".

With the configuration obtained by combining the fifth embodiment with Modification 2, the same advantages as those of Modification 2 of the first embodiment can also be achieved.

The configuration obtained by combining the fifth embodiment with Modification 1 and the configuration obtained by combining the fifth embodiment with Modification 2 may each be combined with the second embodiment and the third embodiment to obtain a configuration in which at least one of the first electrode 5 and second electrode 7 is formed by a transparent conductive layer, and a nitrogen-containing layer is arranged adjacent to the substrate 11 side of the transparent conductive layer.

«9. Sixth Embodiment»

(An example in which a transparent conductive layer is arranged between two adjacent light emitting units of three or more light emitting units)

FIG. 9 is a view showing a cross-sectional configuration of a sixth embodiment of the organic electroluminescence element with a tandem structure that uses the aforesaid transparent conductive layer 1b. The characteristic configuration of an organic electroluminescence element EL-6 according to the sixth embodiment will be described below with reference to FIG. 9. Note that, in the following paragraphs, components identical to those of the other embodiments will not be repeatedly described in detail.

The organic electroluminescence element EL-6 shown in FIG. 9 is obtained by replacing one of the laminates, which are each configured by the nitrogen-containing layer 1a and the transparent conductive layer 1b, of the organic electroluminescence element of the fifth embodiment described above with reference to FIG. 8 with an insulating charge-generating layer 21. In other words, the organic electroluminescence element EL-6 has a configuration in which three or more (in this example, the number is three) light emitting units 3-1, 3-2, 3-3 are laminated, and a laminate configured by the nitrogen-containing layer 1a and the transparent conductive layer 1b or an insulating charge-generating layer 21 is sandwiched between two adjacent light emitting units of the three light emitting units 3-1, 3-2, 3-3. Here, as an example, the laminate configured by the nitrogen-containing layer 1a and the transparent conductive layer 1b is sandwiched between the two light emitting units 3-1 and 3-2 on the side of the substrate 11, and the insulating charge-generating layer 21 is sandwiched between the two light emitting units 3-2 and 3-3; however, the position of the laminate configured by the nitrogen-containing layer 1a and the transparent conductive layer 1b and the position of the charge-generating layer 21 may also be inverse to each other.

Similar to the first embodiment, the entire layer-structure of the light emitting units 3-1, 3-2, 3-3 is not particularly limited, but each of the light emitting units 3-1, 3-2, 3-3 may individually have a generic layer-structure. Further, the light emitting units 3-1, 3-2, 3-3 may either be configured so that they each obtain luminescence light h of the same color, or be configured so that they respectively obtain luminescence light h of different colors. In such a case, the light emitting units 3-1, 3-2, 3-3 may obtain luminescence light of red (R), green (G) and blue (B) respectively, so that white light is obtained.

The configuration of the nitrogen-containing layer 1a and configuration of the transparent conductive layer 1b have been described before; and here the nitrogen-containing layer 1a and the transparent conductive layer 1b are arranged, in this order, from the side of the substrate 11, for example.

Similar to the method for producing the organic electroluminescence element of the first embodiment, the organic electroluminescence element EL-6 may be produced by forming each of the layers into a pattern sequentially from the layer on the side of the substrate 11.

<Charge-generating Layer>

Examples of the insulating charge-generating layer 21 include a layer where a charge-transfer complex consisting of radical cations and radical anions is formed by causing an oxidation-reduction reaction between two materials. In such a case, in the charge-transfer complex, radical cation state (holes) and radical anion state (electrons) move toward the cathode and the anode respectively when a voltage is applied, and thereby the holes will be injected into the light emitting unit in contact with the cathode side of the charge-generating layer, and the electrons will be injected into the light emitting unit in contact with the anode side of the charge generation layer. Examples of the insulating charge-generating layer 21 include a mixed layer or laminated layer formed of two materials which are an organic compound having electron-donating property (for example, an arylamine compound) and a material (a metal oxide, such as vanadium pentoxide, a metal halide or the like) that causes an oxidation-reduction reaction with the organic compound to form a charge-transfer complex (see Japanese Unexamined Patent Application Publication No. 2003-272860).

Similar to the method for producing the organic electroluminescence element of the first embodiment, the

organic electroluminescence element EL-6 having the aforesaid configuration may be produced by forming each of the layers into a pattern sequentially from the layer on the side of the substrate 11. The charge-generating layer 21 can be formed by any film-forming method; however, it is preferred that a deposition method is used to formed the charge-generating layer 21.

<Advantages of Organic Electroluminescence Element EL-6>

The organic electroluminescence element EL-6 also has a configuration in which the aforesaid transparent conductive layer 1b having electrical conductivity and light transmissibility is sandwiched between two light emitting units 3-1, 3-2. Thus, similar to the first embodiment, it is possible to improve light extraction efficiency by sufficiently injecting charges from the transparent conductive layer 1b into the light emitting units 3-1, 3-2, 3-3 to ensure luminous efficiency while restraining absorption, in the transparent conductive layer 1b, of the luminescence light h generated by the light emitting units 3-1, 3-2, 3-3. Further, due to this feature, it is possible to increase the light-emitting lifetime by reducing current density necessary for obtaining a given brightness.

Incidentally, although the sixth embodiment has a configuration in which three layers of light emitting units 3-1, 3-2, 3-3 are laminated, the present invention include a configuration in which more than three layers of light emitting units are laminated. In such a case, the organic electroluminescence element also has a configuration in which the transparent conductive layer 1b with the nitrogen-containing layer 1a adjacent thereto is sandwiched in at least one interspace between two adjacent light emitting units of the plurality of light emitting units, and the charge-generating layer(s) 21 is sandwiched in other interspace(s) of the plurality of organic electroluminescence elements. The nitrogen-containing layer 1a is arranged adjacent to the substrate 11 side of the transparent conductive layer 1b.

<Combination with Modification 1>

Further, the configuration of the sixth embodiment may also be combined with the configuration of Modification 1 of the first embodiment. In such a case, a driving voltage V1 is applied between the first electrode 5 and the transparent conductive layer 1b, and further, a driving voltage V2 is applied between the transparent conductive layer 1b and the second electrode 7. With such a configuration, the same advantages as those of Modification 1 of the first embodiment can be achieved.

<Combination with Modification 2>

Further, the configuration of the sixth embodiment may also be combined with the configuration of Modification 2 of the first embodiment. In such a case, the light emitting unit 3-1 and the light emitting units 3-2, 3-3, which are arranged with the transparent conductive layer 1b sandwiched therebetween, may be laminated in opposite order to each other. In such a configuration, the nitrogen-containing layer 1a and the transparent conductive layer 1b arranged between the light emitting unit 3-1 and the light emitting units 3-2, 3-3 are configured by laminating the nitrogen-containing layer 1a and the transparent conductive layer 1b, in this order, from the side of the substrate 11.

Further, in such a case, the driving voltages applied to the first electrode 5, the second electrode 7, and the transparent conductive layer 1b are adjusted so that the polarity on the hole injection side of each of the light emitting units 3-1, 3-2, 3-3 becomes the electrode "+", and the polarity on the electron injection side becomes the electrode "-".

With the configuration obtained by combining the sixth embodiment with Modification 2, the same advantages as those of Modification 2 of the first embodiment can also be achieved.

The configuration obtained by combining the sixth embodiment with Modification 1 and the configuration obtained by combining the sixth embodiment with Modification 2 may each be combined with the second embodiment and the third embodiment to obtain a configuration in which at least one of the first electrode 5 and second electrode 7 is formed by a transparent conductive layer 1b, and a nitrogen-containing layer 1a is arranged adjacent to the substrate 11 side of the transparent conductive layer 1b.

«10. Applications of Organic Electroluminescence Element»

As mentioned above, since the organic electroluminescence elements having the aforesaid configurations are each a planar light-emitting body, they can be used as various kinds of luminescent light sources. Examples of the various kinds of luminescent light sources include, but not limited to, an illumination device (such as a home lighting fixture, a car lighting fixture or the like), a backlight for timepiece or liquid crystal, an illumination for billboard, a light source for traffic light, a light source for optical storage medium, a light source for electrophotographic copier, a light source for optical communication processor and a light source for optical sensor; particularly, the light emitting source can be effectively used as a backlight for a liquid crystal display device combined with a color filter, and as a light source

for illumination.

Further, the organic electroluminescence element according to the present invention may either be used as a kind of lamp such as an illuminating source, an exposing source or the like, or be used as a projection device where an image is projected, a display device (a display) where a still image or dynamic image is directly viewed, or the like. In such a case, as the size of the illumination devices and displays becomes large in recent years, the area of the light-emitting face may be increased by a method of so-called "tiling", in which a plurality of light-emitting panels each having an organic electroluminescence element are planarly connected with each other.

In the case where the organic electroluminescence element is used as a display device for replaying dynamic image, the driving method may either be a simple matrix driving method (i.e., passive matrix driving method) or an active matrix driving method. Further, it is possible to produce a color or full color display device by using two or more organic electroluminescence elements of the present invention each having different luminescent color.

As an example of the applications, an illumination device will be described below, and thereafter an illumination device whose light-emitting face is made large by tiling will be described.

«11. Illumination Device 1»

The illumination device of the present invention has an aforesaid organic electroluminescence element.

The organic electroluminescence element used in the illumination device according to the present invention may also have a design in which each of the organic electroluminescence elements having the aforesaid configurations is provided with a resonator structure. Examples of the intended use of the organic electroluminescence element configured as the resonator structure include, but not limited to, a light source for an optical storage medium, a light source for an electrophotographic copier, a light source for an optical communication processor, a light source for an optical sensor, and the like. Further, the illumination device may also be used for the aforesaid purpose by laser-oscillating.

Incidentally, the material used in the organic electroluminescence element of the present invention may be used for an organic electroluminescence element which emits while light (also referred to as a "white organic electroluminescence element"). For example, it is possible to cause a plurality of luminescent colors to be simultaneously emitted by a plurality of light emitting materials, so that white luminescent color can be obtained by mixing the plurality of luminescent colors. The combination of the plurality of luminescent colors may be a combination including three light emission maximum wavelengths of three primary colors of blue, green and red, or a combination including two light emission maximum wavelengths using the complementary color relationship such as blue and yellow, bluish-green and orange, or the like.

Further, the combination of light emitting materials for obtaining a plurality of luminescent colors may be a combination of a plurality of materials which emit a plurality of phosphorescent lights or fluorescent lights, or a combination of a light emitting material which emits phosphorescent light or fluorescent light and a dye material which emits light with the light emitted from the light emitting material as exciting light; however, in a white organic electroluminescence element, the combination of light emitting materials for obtaining a plurality of luminescent colors may also be a combinations of a plurality of light-emitting dopants.

In such a white organic electroluminescence element, the organic electroluminescence element itself emits white light, in contrast to a configuration in which a plurality of organic electroluminescence elements each emitting different color are parallelly arranged in array to obtain white-light emission. Thus, almost all layers constituting the element can be formed without mask, and it is possible to form an electrode film, for example, on one surface by a deposition method, a casting method, a spin coating method, an ink-jet method, a printing method or the like, so that productivity can be improved.

The light emitting material used in the light emitting layer of such a white organic electroluminescence element is not particularly limited; for example, if the light emitting material is used for the back light of a liquid crystal display element, arbitrary light emitting materials may be selected from the metal complexes of the present invention or known light emitting materials and combined to obtain white light in a manner in which the light is matched to the wavelength range corresponding to CF (color filter) characteristics.

By using the white organic electroluminescence element described above, it is possible to produce an illumination device which substantially emits white light.

<<12. Illumination Device 2>>

The organic electroluminescence element according to the present invention can be used as an illumination device whose light-emitting face is made large by using a plurality of the organic electroluminescence elements. In such a case, the area of the light-emitting face is increased by arranging (i.e., so-called "tiling") a plurality of light-emitting panels on a supporting substrate, wherein each light-emitting panel is formed by arranging an organic electroluminescence element on a transparent substrate. The supporting substrate may also serves as a sealing material; the light-emitting panels are tiled in a state where the organic electroluminescence elements are sandwiched between the supporting substrate and the transparent substrates of the light-emitting panels. An adhesive may be filled between the supporting substrate and the transparent substrates, and thereby the organic electroluminescence elements are sealed. Incidentally, the terminal of the first electrode and the terminal of the second electrode are exposed from the periphery of the light-emitting panel. In the case where a driving voltage is applied to a conductive layer (for example, the transparent conductive layer 1b), the terminal of the conductive layer is also exposed.

In the illumination device with such a configuration, the center of each light-emitting panel is a light-emitting region, and the region between adjacent light-emitting panels is a light non-emitting region. Thus, a light extraction member for increasing the amount of the light extracted from the light non-emitting region may be arranged in the light non-emitting region of the light extracting face. A light-collecting sheet, a light-diffusing sheet or the like can be used as the light extraction member.

Example 1

«Production of Transparent Conductive Layer»

As described below, transparent conductive layers of Samples No. 101 to 112 were produced so that the area of the conductive region of each transparent conductive layer became 5 cm × 5cm.

In Sample 101, a transparent conductive layer with a single-layer structure was produced, wherein the transparent conductive layer was composed of silver and had a film-thickness of 5 nm. In each of Samples 102 to 112, a nitrogen-containing layer composed of each different compound was formed, and a transparent conductive layer composed of silver and having a film-thickness of 5 nm was formed on the top of the nitrogen-containing layer.

In the production of the transparent conductive layer of Sample 102, instead of the nitrogen-containing layer (which is a ground layer), a layer composed of anthracene (Compound No. 01) containing no nitrogen was formed.

On the other hand, in the production of the respective transparent electrodes of Samples 103 to 112, the nitrogen-containing Compounds No. 02 to No. 11 shown below were used as the compounds constituting the nitrogen-containing layers. In these compounds, Compound No. 08 is Compound 10 included in General Formula (1), and Compound No. 11 is a compound included in General Formula (2).

[Chemical Formula 70]

No.02

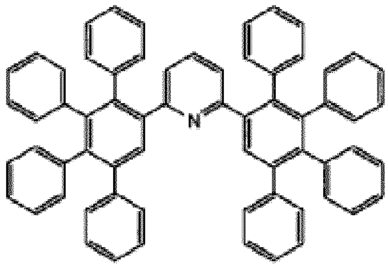

No.03

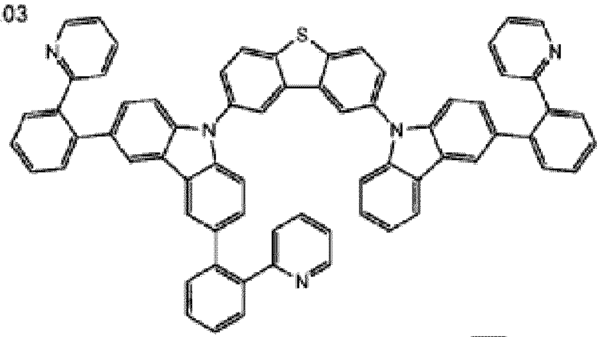

No.04

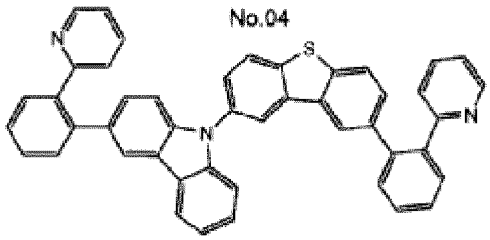

No.05

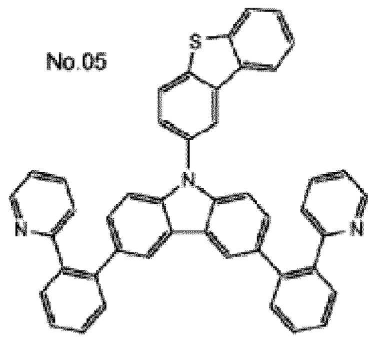

No.06

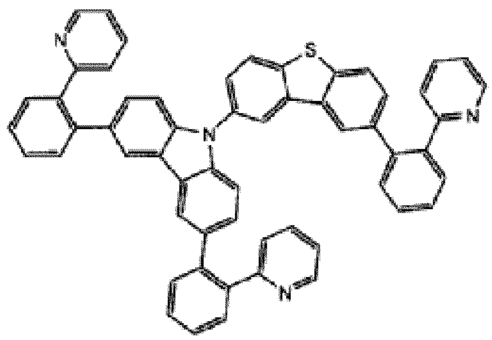

[Chemical Formula 71]

No.07

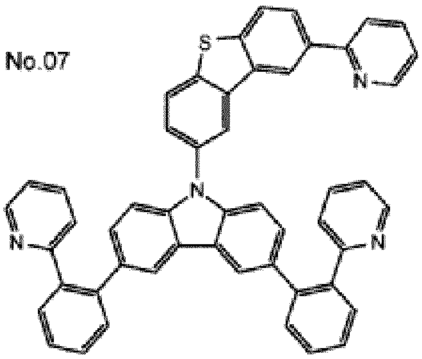

No.08

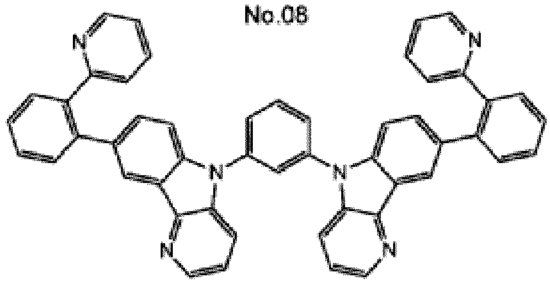

No.09

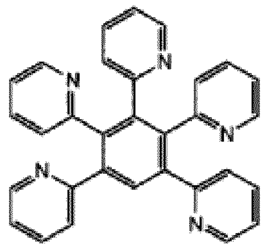

No.10

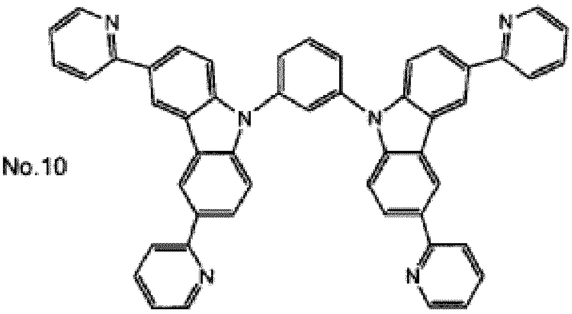

No.11

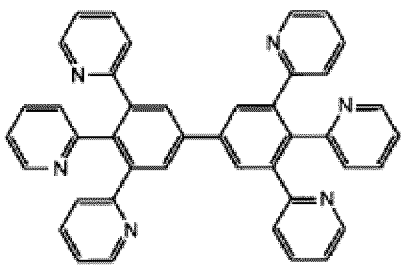

The number [n] of nitrogen atom(s) contained in the compound and stably bonded with silver, the interaction energy [ΔE] between silver (Ag) and nitrogen (N) contained in the compound, the surface area [s] of the compound, and the effective action energy [ΔEef] calculated based on the number [n], the interaction energy [ΔE] and the surface area [s] are shown in the following Table 1 for each of Compounds No. 02 to No. 11 used in Samples 103 to 112. The dihedral angle [D] between nitrogen atom and silver with respect to the ring containing nitrogen atom in the compound, and the [ΔE] were calculated by using Gaussian 03 (Gaussian, Inc., Wallingford, CT, 2003), wherein the dihedral angle [D] was used to obtain the number [n]. Incidentally, in each of Compounds No. 02 to No. 11 used in Samples 103 to 112, the nitrogen atom(s) whose dihedral angle [D] satisfies "D<10 degrees" was counted as the number [n].

[Table 1]

| Example 1 | | | | | | |
|---|---|---|---|---|---|---|
| Sample | Nitrogen-containing layer (25nm) | | | | | Transparent conductive layer (Ag:5nm) |
| | Compound | n | ⊿E [kcal/mol] | s [Å$^2$] | ⊿Eef [kcal/mol·Å$^2$] | Sheet resistance [Ω/sq.] |
| 101 | - | - | - | - | - | Not measurable |
| 102 | No. 01 (anthracene) | - | - | - | - | Not measurable |
| 103 | No. 02 | 1 | -51.6 | 902.7 | -0.057161848 | Not measurable |
| 104 | No. 03 | 3 | -36.418 | 981 | -0.111370031 | 963 |
| 105 | No. 04 | 2 | -48.933 | 666 | -0.146945946 | 915750 |
| 106 | No. 05 | 2 | -49.419 | 666 | -0.148405405 | 39120 |
| 107 | No. 06 | 3 | -43.768 | 821 | -0.15993179 | 769 |
| 108 | No. 07 | 3 | -45.834 | 739 | -0.186064953 | 3337 |
| 109 | No. 08 | 4 | -41.989 | 729 | -0.230392318 | 278 |
| 110 | No. 09 | 2 | -56.976 | 479 | -0.237895616 | 122 |
| 111 | No. 10 | 4 | -45.01 | 723 | -0.249017981 | 792 |
| 112 | No. 11 | 4 | -57.302 | 634 | -0.361526814 | 52 |

In Compound No. 02, the effective energy [ΔEef], which shows the relationship between nitrogen atom (N) contained in the compound and silver (Ag) constituting the transparent conductive layer, satisfies the condition of ΔEef>-0.1. In contrast, in each of Compounds No. 03 to No. 11, the effective energy [ΔEef] satisfies the condition of ΔEef≤-0.1.

<Process for Producing Transparent Conductive Layer of Sample 101>

First, a base material made of transparent alkali-free glass was fixed to a base material holder of a commercially available vacuum deposition device, and then the base material holder was mounted on a vacuum chamber of the vacuum deposition device. Further, silver (Ag) was placed in a tungsten resistance heating boat, and then the tungsten resistance heating boat was mounted on the vacuum chamber. Next, the pressure of the vacuum chamber was reduced to $4 \times 10^{-4}$ Pa, and then the resistance heating boat was electrically heated at a deposition rate of 0.1 nm/sec to 0.2 nm/sec so as to form, on the base material, a transparent conductive layer composed of silver and having a film-thickness of 5 nm.

<Process for Producing Transparent Conductive Layers of Samples 102 to 112>

A base material made of transparent alkali-free glass was fixed to a base material holder of a commercially available vacuum deposition device. In the production of each transparent conductive layer, each of Compounds No. 01 to No. 11 was placed in a tantalum resistance heating boat. The base material holder and the heating boat were mounted on a first vacuum chamber of the vacuum deposition device. Further, silver (Ag) was placed in a tungsten resistance heating boat, and the tungsten resistance heating boat was mounted on a second vacuum chamber.

In such state, the pressure of the first vacuum chamber was reduced to $4 \times 10^{-4}$ Pa, and then the heating boat having each of the compounds placed therein was electrically heated to form a nitrogen-containing layer composed of each of the compounds on the base material at a deposition rate of 0.1 nm/sec to 0.2 nm/sec, wherein the film-thickness of the nitrogen-containing layer was 25 nm.

Next, the base material, on which the layers up to the nitrogen-containing layer had been formed, was transferred to the second vacuum chamber while maintaining the vacuum state. After the pressure of the second vacuum chamber was reduced to $-4 \times 10^{-4}$ Pa, the heating boat having silver placed therein was electrically heated. Thus, a transparent conductive layer composed of silver and having a film-thickness of 5 nm was formed at a deposition rate of 0.1 nm/sec to 0.2 nm/sec, and thereby a transparent conductive layer of each of Samples 102 to 112 was obtained in a state where the transparent conductive layer was adjacent to the nitrogen-containing layer.

<Evaluation of Each Sample of Example 1>

Sheet resistance of each transparent conductive layer of Samples 101 to 112 produced above was measured. The measurement of the sheet resistance was performed by a 4-probe constant current applying method using a resistivity meter (MCP-T610, manufactured by Mitsubishi Chemical Corporation). The results of the measurement are also shown in Table 1.

<Evaluation Results of Example 1>

As is clear from Table 1, each transparent conductive layer of Samples 104 to 112, which is adjacent to the nitrogen-containing layer formed by using each of Compounds No. 03 to No. 11 whose effective action energy $\Delta Eef$ satisfies a condition of $\Delta Eef \leq -0.1$, has a very small thickness of 5 nm yet has a measurable sheet resistance; so that it is confirmed that the silver grows in a single-layer growth mode (Frank-van der Merwe: FW mode) and thereby is formed into a substantially uniform ultrathin film. In contrast, the transparent conductive layer of Sample 101 (which has a single-layer structure with no nitrogen-containing layer), the transparent conductive layer of Sample 102 (which is adjacent to a ground layer formed by using Compound No. 1 containing no nitrogen), and the transparent conductive layer of Sample 103 (which is adjacent to the nitrogen-containing layer formed by using Compound No. 02 whose effective action energy $\Delta Eef$ satisfies a condition of $\Delta Eef > -0.1$) each have unmeasurable sheet resistance.

FIG. 10 shows a relationship between the effective action energy $\Delta Eef$ of each of Compounds No. 03 to No. 11, which constitutes the nitrogen-containing layer, and the sheet resistance measured for each transparent conductive layer formed adjacent to the nitrogen-containing layer. It is clear from FIG. 10 that, if the effective action energy $\Delta Eef$ falls in a confirmed range of $-0.5 \leq \Delta Eef \leq -0.1$, the smaller the value of $\Delta Eef$ is, the lower the sheet resistance of the transparent conductive layer tends to become. It is further preferred that, if the effective action energy $\Delta Eef$ falls in a range of $-0.5 \leq \Delta Eef \leq -0.2$, the sheet resistance is maintained at 1000 [Ω/sq.] or lower.

Based on the above facts, it has been confirmed that, by selecting a compound to form the nitrogen-containing layer with the effective action energy $\Delta Eef$ as an index, it is possible to obtain a transparent conductive layer which is a thin-film (in order to have light transmissibility) yet has lower resistance.

Example 2

«Production of Two-layer Tandem: Bottom Emission Type Organic Electroluminescence Element»

As shown in FIG. 11, a two-layer tandem: bottom emission type organic electroluminescence element was produced as each of Samples 201 to 210. Further, as shown in FIG. 12, a single-layer structure bottom emission type organic electroluminescence element was produced as each of Samples 211 and 212 for comparison. Incidentally, the configuration of main components of each Samples 201 to 212 is shown in Table 2.

<Production of Samples 201 to 208>

As shown in FIG. 11, first, ITO was formed into a patterned film on a substrate 11, which was made of transparent glass and had a size of 50 mm x 50 mm and a thickness of 0.7 mm, by a sputtering method in a condition that the thickness of the patterned film was 180 nm, so that a planar first electrode 5 composed of ITO was formed, wherein the first electrode 5 included an extracting electrode portion. The first electrode 5 was formed as an anode. Next, the substrate 11 having the first electrode 5 (which is formed of ITO) formed thereon was subjected to ultrasonic cleaning with isopropyl alcohol, then dried with dry nitrogen gas, and then subjected to UV ozone cleaning for 5 minutes.

Thereafter, the substrate 11, on which the first electrode 5 had been formed, was fixed to a substrate holder of a commercially available vacuum deposition device, and a deposition mask was set to face a surface of the substrate 11 where the first electrode 5 had been formed, and then the substrate holder was mounted on a first vacuum chamber of the vacuum deposition device. Further, respective materials for forming the light emitting units 3-1, 3-2 and the nitrogen-containing layer 1a were filled into respective heating boats provided in the vacuum deposition device, and the heating boats were mounted on the first vacuum chamber, wherein the amounts of the respective materials filled into respective heating boats were optimized to form the respective layers. Incidentally, the heating boats were each produced by using a resistance heating material made of tungsten.

Next, the pressure of the first vacuum chamber of the vacuum deposition device was reduced to $4 \times 10^{-4}$ Pa, and the heating boats having respective materials placed therein were electrically heated sequentially, and thereby the respective layers were formed as follows.

First, a heating boat having a compound represented by the following structural formula HI-1, as a hole injecting material, placed therein was electrically heated, and further, a heating boat having a compound represented by the

following structural formula HI-2 placed therein was electrically heated, so that a hole injecting layer was formed on the first electrode 5. At this time, the currents of the two heating boats were adjusted so that the deposition rate of the compound HI-1 and the deposition rate of the compound HI-2 became the same value of 0.1 nm/sec to 0.2 nm/sec, and the film-thickness became 15 nm.

[Chemical Formula 72]

HI-1

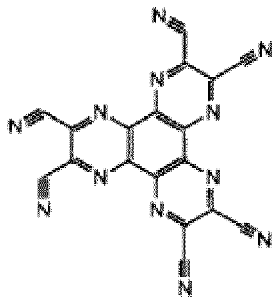

HI-2

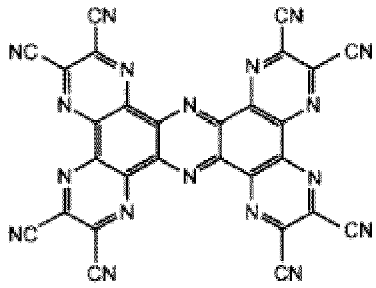

Next, a heating boat having below-mentioned α-NPD, as a hole transporting material, placed therein was electrically heated to form a hole transporting layer on the hole injecting layer. At this time, the deposition rate was 0.1 nm/sec to 0.2 nm/sec, and the film-thickness was 20 nm.

[Chemical Formula 73]

α-NPD

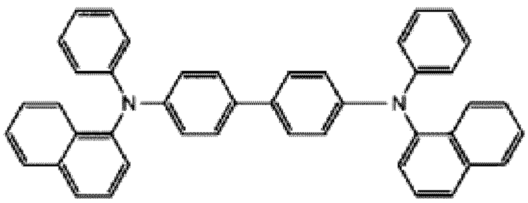

Next, a heating boat having a host material H4 represented by a structural formula shown before placed therein, a heating boat having a phosphorescent compound Ir-48 (blue) represented by the structural formula shown before placed therein, a heating boat having a phosphorescent compound Ir-1 (green) represented by a structural formula shown before placed therein, and a heating boat having a phosphorescent compound Ir-9 (red) represented by a structural formula shown before placed therein were each independently electrically heated to form an light emitting layer composed of the host material and the phosphorescent compounds of each color on the hole transporting layer. At this time, the currents of the aforesaid heating boats were adjusted so that the deposition rates of the host material H4 and the phosphorescent compounds of each color become H4 : Ir-48 : Ir-1 : Ir-9 = 88 : 7 : 4 : 1 (by volume). The film-thickness was 30 nm.

Next, a heating boat having BAlq, as a hole blocking material, represented by the following formula placed therein was electrically heated to form a hole blocking layer composed of BAlq on the light emitting layer. At this time, the deposition rate was 0.1 nm/sec to 0.2 nm/sec, and the film-thickness was 10 nm.

[Chemical Formula 74]

BAlq

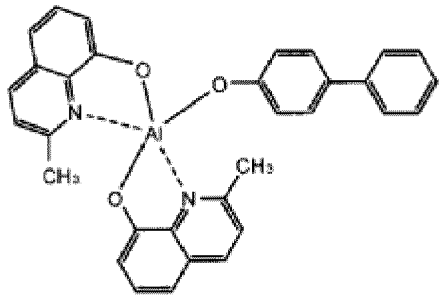

Thereafter, in the production of each of Samples 201 to 208, a heating boat having one of Compounds A to G represented by the below formulas placed therein and a heating boat having potassium fluoride placed therein were each independently electrically heated to form, on the light emitting layer, a nitrogen-containing layer 1a composed of potassium fluoride and one of Compounds A to G. At this time, the currents of the two heating boats were adjusted so that (deposition rate of the compound) : (deposition rate of potassium fluoride) = 75 : 25. The film-thickness was 30 nm. The nitrogen-containing layer 1a functions as an electron transporting/injecting layer which serves both as an electron injecting layer and as an electron transporting layer, and the layers from the hole injecting layer to the nitrogen-containing layer 1a configures the light emitting unit 3-1. By performing the above processes, a light emitting unit 3-1 that emits white light is obtained, which has a laminated structure formed by layers from the hole injecting layer to the nitrogen-containing layer 1a. Incidentally, Compound D is Compound No. 08 of Example 1, and is Compound 10 included in the compounds represented by General Formula (1); and Compound F is Compound No. 11 of Example 1, and is Compound 113 included in the compounds represented by General Formula (2).

[Chemical Formula 75]

Compound A

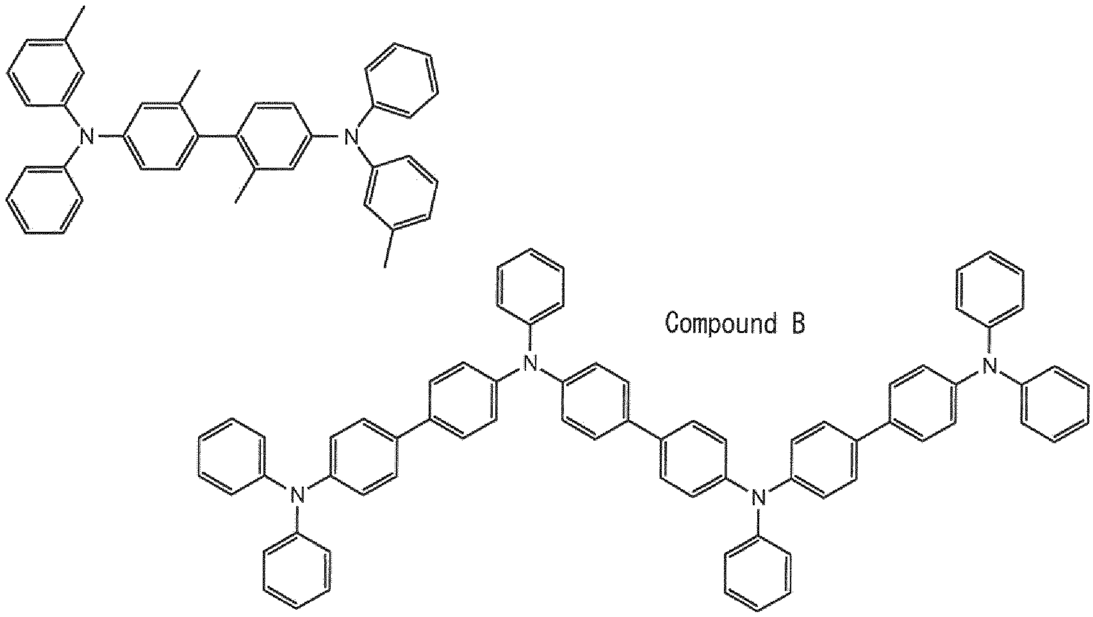

Compound B

Compound C

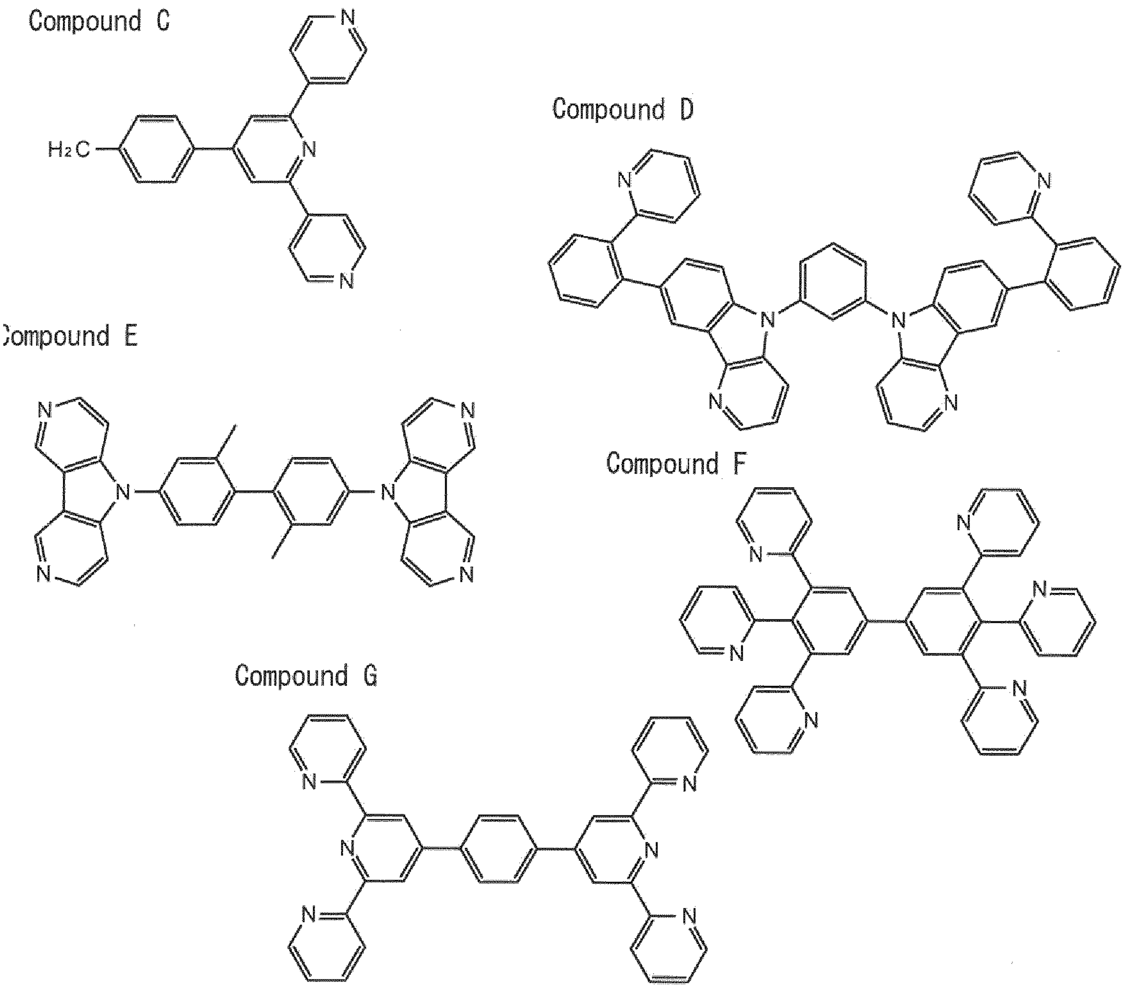

Compound D

Compound E

Compound F

Compound G

Next, the substrate 11 having the layers up to the nitrogen-containing layer 1a formed thereon was transferred to the second vacuum chamber of the vacuum deposition device. After the pressure of the second vacuum chamber

was reduced to $-4 \times 10^{-4}$ Pa, the heating boat having silver placed therein and mounted on the second vacuum chamber was electrically heated. By performing the above process, a transparent conductive layer 1b composed of silver (Ag) was formed at a deposition rate of 0.3 nm/sec. At this time, in Sample 201, the film-thickness of the transparent conductive layer 1b was 14 nm; and in each of Samples 202 to 208, the film-thickness of the transparent conductive layer 1b was 8.4 nm.

Thereafter, the second layer of light emitting unit 3-2 was formed on the transparent conductive layer 1b by repeating the same processes as those for forming the light emitting unit 3-1. At this time, the aforesaid nitrogen-containing layer 1a was formed as an electron transporting/injecting layer constituting the uppermost layer of the light emitting unit 3-2.

Next, the substrate 11 having the light emitting unit 3-2 formed thereon was transferred to a third vacuum chamber of the vacuum deposition device. After the pressure of the third vacuum chamber was reduced to $-4 \times 10^{-4}$ Pa, a heating boat having aluminum placed therein and mounted on the third vacuum chamber was electrically heated, and thereby the second electrode 7 composed of aluminum and having a film-thickness of 120 nm was formed at a deposition rate of 0.2 nm/sec. The first electrode 7 was used as a cathode. By performing the above processes, a two-layer tandem bottom emission type organic electroluminescence element was formed on the substrate 11.

Thereafter, the organic electroluminescence element was covered by a transparent sealing material formed of a glass substrate having a thickness of 300 μm, and, in a state where the organic electroluminescence element was enclosed by the transparent sealing material, the gap between the transparent sealing material and the substrate was filled with an adhesive (a sealing material). An epoxy-based light curable adhesive (Lux track LC0629B, manufactured by Toa Gosei Co. Ltd.) was used as the adhesive. Ultraviolet light was irradiated from the side of the glass substrate (a transparent sealing material 17) onto the adhesive filled into the gap between the transparent sealing material and the substrate to thereby cure the adhesive so as to seal the organic electroluminescence element.

Incidentally, in the formation of the organic electroluminescence element, a deposition mask was used to form each of the layers; so that in the substrate 13 which had a size of (5 cm × 5 cm), the central area (4.5 cm × 4.5 cm) was formed as a light-emitting region A, and a light non-emitting region having a width of 0.25 cm was formed surrounding the light-emitting region A. Further, the first electrode 5 (the anode) and the second electrode 7 (the cathode) were formed into a shape such that the terminal portions of the first electrode 5 and the second electrode 7 were drawn out to the edge of the substrate 11 in a state where the first electrode 5 and the second electrode 7 were insulated from each other by the light emitting units 3-1, 3-2.

In such a manner, the organic electroluminescence element was arranged on the transparent substrate 11, and the organic electroluminescence element was sealed by the transparent sealing material and the adhesive, so that the light-emitting panel of each of samples was obtained. In each light-emitting panel, the luminescence light h generated in the light emitting units 3-1, 3-2 was extracted from the side of the substrate 11.

<Production of Sample 209>

The organic electroluminescence element of Sample 209 was produced in the same manner as that of each of Samples 201 to 208 except that the transparent conductive layer 1b was formed by using magnesium (Mg) and silver (Ag) by a co-deposition method, instead of being formed by using silver (Ag). At this time, the transparent conductive layer 1b was formed so that Mg : Ag = 90 : 10 (atom%). The film-thickness of the transparent conductive layer 1b was 8.4 nm. Incidentally, the nitrogen-containing layer 1a was formed by using Compound C.

<Production of Sample 210>

The organic electroluminescence element of Sample 210 was produced in the same manner as that of each of Samples 201 to 208 except that the transparent conductive layer 1b is formed using ITO by a sputtering method, instead of being formed by using silver (Ag). At this time, in order to prevent damage occurring to the light emitting unit 3-1, the film-forming rate is controlled to 0.01 nm/sec. By performing the aforesaid processes, a transparent conductive layer 1b having a film-thickness of 120 nm and composed of ITO was formed. Incidentally, the nitrogen-containing layer 1a was formed by using Compound C.

<Production of Samples 211 and 212>

As shown in FIG. 12, single-layer structure bottom emission type organic electroluminescence elements were produced as comparisons. At this time, similar to the process for forming the organic electroluminescence element of each of Samples 201 to 208, a light emitting unit 3-1 having a nitrogen-containing layer 1a arranged as the uppermost layer thereof was formed. Next, the substrate 11 having the light emitting unit 3 formed thereon was transferred to a third vacuum chamber of the vacuum deposition device. After the pressure of the third vacuum chamber was reduced to $-4 \times 10^{-4}$ Pa, a heating boat having aluminum placed therein and mounted on the third vacuum chamber was electrically

heated. A second electrode 7 composed of aluminum and having a film-thickness of 120 nm was formed on the nitrogen-containing layer 1a at a deposition rate of 0.2 nm/sec, and thereby an organic electroluminescence element was produced. Incidentally, in Sample 211, Compound A was used to form the nitrogen-containing layer 1a, and in Sample 212, Compound E was used to form the nitrogen-containing layer 1a.

<Evaluation of Each Sample of Example 2, Part 1>

The luminescent chromaticity of the organic electroluminescence element (the light-emitting panel) produced in each of Samples 201 to 212 was evaluated. As a result, it is confirmed that, for all of the Samples 201 to 212, when 2-degree viewing angle front brightness is 1000 cd/$m^2$, the chromaticity in a CIE color system falls in a range of $X=0.45\pm0.02$ and $Y=0.41\pm0.02$, which shows the luminescent color is white. Incidentally, the brightness was measured using a spectroradiometer CS-1000 (manufactured by Konica Minolta Sensing Inc.).

<Evaluation of Each Sample of Example 2, Part 2>

The driving voltage of the organic electroluminescence element (the light-emitting panel) produced in each of Samples 201 to 212 was measured. In the measurement of the driving voltage, the voltage at the time when the front brightness on the side of the substrate 11 of each organic electroluminescence element was equal to 1000 cd/$m^2$ was measured as the driving voltage by using the aforesaid spectroradiometer. The smaller the value of the obtained driving voltage is, the more preferable the result is. The results of the measurement are also shown in the following Table 2.

<Evaluation of Each Sample of Example 2, Part 3>

Further, brightness unevenness of the organic electroluminescence element (the light-emitting panel) produced in each of Samples 201 to 212 was evaluated. In the evaluation of the brightness unevenness, an electric current of 2.5 mA/$cm^2$ was applied to each organic electroluminescence element, and the brightness at the center of the light-emitting face (i.e., center brightness) on the side of the substrate 11 and the brightness at an end portion near the electric feeding point (i.e., end portion brightness) on the side of the transparent electrode 1 were measured. The brightness was measured using the aforesaid spectroradiometer. The measured center brightness with respect to the end portion brightness was calculated as the brightness unevenness. Thus, the more the value of the brightness unevenness is close to 1, the more the result of the measurement is preferable. The results of the measurement are also shown in the following Table 2.

<Evaluation of Each Sample of Example 2, Part 4>

Further, external quantum efficiency (EQE) of the organic electroluminescence element (the light-emitting panel) produced in each of Samples 201 to 212 was evaluated. Here, the brightness and emission spectrum at the time when each organic electroluminescence element is emitting light are measured using spectroradiometer CS-1000 (manufactured by Konica Minolta Sensing Inc.), and the external quantum efficiency is calculated by using a brightness conversion method based on the measured values of the brightness and emission spectrum. Here, the relative value of the external quantum efficiency of each organic electroluminescence element is calculated in the case where the value of external quantum efficiency of the organic electroluminescence element of Sample 211 is regarded as 100, and the results are also shown in the following Table 2.

<Evaluation of Each Sample of Example 2, Part 5>

Further, the brightness half-life, as a lifetime characteristic, of the organic electroluminescence element (the light-emitting panel) produced in each of Samples 201 to 212 was measured. In the measurement of the brightness half-life, the electric current at the time when the front brightness on the side of the substrate 11 of each organic electroluminescence element was equal to 1000 cd/$m^2$ was obtained. The obtained electric current was maintained at that value, the temporal change of the brightness was measured by using the aforesaid spectroradiometer, and the time required for the brightness to become 50% with respect to the initial brightness was regarded as the brightness half-life of each organic electroluminescence element. Here, the relative lifetime of each organic electroluminescence element is calculated in the case where the brightness half-life of the organic electroluminescence element of Sample 211 is regarded as 100, and the results are also shown in the following Table 2.

[Table 2]

Example 2 <<Two-layer Tandem: Bottom Emission>>

| Sample | Configuration of element | First electrode 5 (anode) | Nitrogen-containing layer 1a (light emitting unit) | | Transparent conductive layer 1b | | Second electrode 7 (cathode) | Result of Evaluation | | | | Other | Remark |
|---|---|---|---|---|---|---|---|---|---|---|---|---|---|
| | | Material/film-thickness (nm) | Compound | ⊿Eef | Material/production method | Film-thickness (nm) | Material/film-thickness (nm) | Deriving voltage (V) | Brightness unevenness | Relative value of EQE | Relative value of lifetime | | |
| 201 | Fig11 | ITO/180 | Compound A | -0.01 | Ag/deposition | 14 | Al/120 | 8.4 | 0.86 | 131 | 153 | | Present invention |
| 202 | | | | | | 8.4 | | 8.4 | 0.67 | 171 | 176 | | Present invention |
| 203 | | | Compound B | -0.03 | | | | 8.9 | 0. 69 | 167 | 171 | | Present invention |
| 204 | | | Compound C | -0.08 | | | | 7.8 | 0.70 | 188 | 201 | | Present invention |
| 205 | | | Compound D | -0.23 | | | | 7.1 | 0.92 | 245 | 235 | | Present invention |
| 206 | | | Compound E | -0.33 | | | | 7.2 | 0.94 | 232 | 220 | | Present invention |
| 207 | | | Compound F | -0.34 | | | | 7.0 | 0.94 | 242 | 237 | | Present invention |
| 208 | | | Compound G | -0.41 | | | | 7.9 | 0.89 | 189 | 196 | | Present invention |

(continued)

Example 2 <<Two-layer Tandem: Bottom Emission>>

| Sample | Configuration of element | First electrode 5 (anode) | Nitrogen-containing layer 1a (light emitting unit) | | Transparent conductive layer 1b | | Second electrode 7 (cathode) | Result of Evaluation | | | | Other | Remark |
|---|---|---|---|---|---|---|---|---|---|---|---|---|---|
| | | Material/film-thickness (nm) | Compound | $\Delta$Eef | Material/production method | Film-thickness (nm) | Material/film-thickness (nm) | Deriving voltage (V) | Brightness unevenness | Relative value of EQE | Relative value of lifetime | | |
| 209 | Fig11 | ITO/180 | Compound C | -0.08 | MgAg(10%)/co-deposition | 8.4 | Al/120 | 7.9 | 0.54 | 174 | 82 | Short lifetime | comparison |
| 210 | | | | | ITO/sputtering | 120 | | 8.5 | 0.62 | 148 | 17 | leakcurrentoccur Luminescence disappearsuddenly | comparison |
| 211 | Fig12 | ITO/180 | Compound A | -0.01 | - | | | 5.3 | 0.68 | 100 | 100 | | comparison |
| 212 | | | Compound E | -0.33 | - | - | | 4.1 | 0.95 | 120 | 124 | | comparison |

<Evaluation Results of Each Sample of Example 2>

As is clear from Table 2, it has been confirmed that, compared to the organic electroluminescence element of each of Samples 211 and 212 (in which the light emitting unit 3 has a single-layer structure), the organic electroluminescence element of each of Samples 201 to 208 (in which the transparent conductive layer 1b having silver (Ag) as a main component is arranged, adjacent to the nitrogen-containing layer 1a, between the laminated light emitting units 3-1 and 3-2) is improved in both the external quantum efficiency (EQE) and the lifetime.
Particularly, it has been confirmed that, compared to the organic electroluminescence element of each of Samples 211 and 212 (in which the light emitting unit 3 has a single-layer structure), the organic electroluminescence element of each of Samples 205 to 208 (in which a compound whose effective action energy $\Delta Eef$ satisfies the condition of $\Delta Eef \leq -0.1$ is used as the compound containing nitrogen atom used in the nitrogen-containing layer 1a) is improved by two times or more in both the external quantum efficiency (EQE) and the lifetime.
In contrast, the organic electroluminescence element of each of Samples 209 and 210 (in which a transparent conductive layer 1b not having silver (Ag) as a main component is arranged between the laminated light emitting units 3-1 and 3-2) has large brightness unevenness and short lifetime, although having high external quantum efficiency (EQE).
Particularly, it has been confirmed that in Sample 210 in which ITO is used as the transparent conductive layer 1b, when the organic electroluminescence element is driven by a voltage of $\pm 2.5$V, the rectification ratio is high, and leak current occurs, and further, a phenomenon that light emission disappears suddenly has been observed.
Based on the above results, it is confirmed that, with the organic electroluminescence element with a tandem structure using a transparent conductive layer of the configuration of the present invention, it is possible to reduce the brightness unevenness while improving both the luminous efficiency and the lifetime characteristic.

Example 3

<<Production of Three-layer Tandem: Dual Emission Type Organic Electroluminescence Element>>

As shown in FIG. 13, a three-layer tandem: dual emission type organic electroluminescence element was produced as each of Samples 301 to 313. Further, as shown in FIG. 14, a single-layer structure dual emission type organic electroluminescence element was produced as each of Samples 314 and 315 for comparison. Incidentally, the configuration of main components of each of Samples 301 to 315 is shown in Table 3.

<Production of Samples 301 to 308>

First, similar to each of Samples 201 to 208 of Example 2, a first electrode 5 composed of ITO and having a film-thickness of 180 nm was formed as an anode, and then the first layer of light emitting unit 3-1 was formed on the top of the first electrode 5, wherein the light emitting unit 3-1 was formed in the same manner as that of each of Samples 201 to 208 except that a compound represented by the structural formula HI-2 shown before is used as the hole injecting material.
Next, the first layer of transparent conductive layer 1b-1 composed of silver (Ag) was formed in the same manner as that for forming the transparent conductive layer of each of the Samples 201 to 208 of Example 2. At this time, in each of Samples 301 to 306 and 308, the film-thickness of the transparent conductive layer 1b-1 was 8.4 nm; and in Sample 307, the film-thickness of the transparent conductive layer 1b was 5.5 nm.
Thereafter, in the production of each of Samples 301 to 308, the aforesaid processes from the process of forming the first layer of light emitting unit 3-1 to the process of forming the first layer of transparent conductive layer 1b-1 were repeatedly performed twice. By performing the aforesaid processes, the second layer of light emitting unit 3-2 (including the nitrogen-containing layer 1a-2), the second layer of transparent conductive layer 1b-2, the third layer of light emitting unit 3-3 (including the nitrogen-containing layer 1a-3), and the third layer of transparent conductive layer 1b-3 were formed. The third layer of transparent conductive layer 1b-3 was formed as the second electrode 7 (as a cathode). However, in Sample 307, the film-thickness of the third layer of transparent conductive layer 1b-3 (the second electrode 7) was 8.4 nm, which was the same film-thickness of that as each of Samples 301 to 306 and 308.
By performing the above processes, a three-layer tandem dual emission type organic electroluminescence element was formed on the substrate 11. Thereafter, the organic electroluminescence element was sealed by performing the same process as that of Example 2.

<Production of Samples 309 to 311>

The organic electroluminescence element of each of Samples 309 to 311 was produced in the same manner as that of each of Samples 301 to 308 except that all the three layers of transparent conductive layers 1b-1 to 1b-3 were

each formed by using magnesium (Mg) and silver (Ag) by a co-deposition method, instead of being formed by using silver (Ag). At this time, the transparent conductive layers 1b-1 to 1b-3 were formed so that Mg : Ag = 45 : 55 (atom%) for Sample 309, Mg : Ag = 55 : 45 (atom%) for Sample 310, and Mg : Ag = 90 : 10 (atom%) for Sample 311. The film-thicknesses of three layers were each 8.4 nm. Incidentally, the nitrogen-containing layers 1a-1 to 1a-3 were formed by using Compound E.

<Production of Samples 312 and 313>

The organic electroluminescence element of each of Samples 312 and 313 was produced in the same manner as that of each of Samples 301 to 308 except that all the three layers of transparent conductive layers 1b-1 to 1b-3 were each formed by using ITO by a sputtering method, instead of being formed by using silver (Ag). At this time, the film-forming rate was set to 0.01 nm/sec in the production of Sample 312, and set to 0.1 nm/sec in the production of Sample 313. By performing the aforesaid processes, transparent conductive layers 1b-1 to 1b-3 each having a film-thickness of 120 nm and composed of ITO were formed. Incidentally, the nitrogen-containing layers 1a-1 to 1a-3 were formed by using Compound E.

<Production of Samples 314 and 315>

As shown in FIG. 14, single-layer structure dual emission type organic electroluminescence elements were produced as comparisons. At this time, similar to the process described above for forming the organic electroluminescence element of each of Samples 301 to 308, a light emitting unit 3 was formed on the first electrode 5 (an anode) composed of ITO, and further, a transparent conductive layer 1b was formed on the light emitting unit 3. The film-thickness of the transparent conductive layer 1b was 8.4 nm. The transparent conductive layer 1b was formed as the second electrode 7 (as an anode). Incidentally, as the material for constituting the nitrogen-containing layer 1a that configures the uppermost layer of the light emitting units 3, Compound A represented by the structural formula shown before was used for Sample 314, and Compound E represented by the structural formula shown before was used for Sample 315.

Thereafter, the organic electroluminescence element was sealed by performing the same process as that of Example 2.

<Evaluation of Each Sample of Example 3, Part 1>

The luminescent chromaticity of the organic electroluminescence element (the light-emitting panel) produced in each of Samples 301 to 315 was evaluated. As a result, it is confirmed that light is emitted for the elements of all Samples 301 to 315 other than Sample 313, and it is also confirmed that, when 2-degree viewing angle front brightness on the side of the substrate 11 is 500 cd/$m^2$, the chromaticity in a CIE color system falls in a range of X=0.45±0.02 and Y=0.41±0.02, which shows the luminescent color is white. Incidentally, the brightness was measured using a spectroradiometer CS-1000 (manufactured by Konica Minolta Sensing Inc.).

<Evaluation of Each Sample of Example 3, Parts 2 to 5>

The driving voltage, the brightness unevenness, the external quantum efficiency (EQE) and the lifetime of the organic electroluminescence element (the light-emitting panel) produced in each of Samples 301 to 315 were measured in the same manner as Example 2. However, the driving voltage, the external quantum efficiency (EQE) and the lifetime were measured when the front brightness on the side of the substrate 11 was 500 cd/$m^2$. Further, the relative value of the external quantum efficiency (EQE) and the relative value of the lifetime of each organic electroluminescence element are calculated in the case where both the value of external quantum efficiency (EQE) and the value of the lifetime of the organic electroluminescence element of Sample 315 are regarded as 100. The results are also shown in the following Table 3.

[Table 3]

Example 3 <<Three-layer Tandem: Dual Emission>>

| Sample | Configuration of element | First electrode 5 (anode) | Nitrogen-containing layer 1a-1 (light emitting unit 3-1) | | Transparent conductive layer 1b-1 | | Nitrogen-containing layer 1a-2 (light emitting unit 3-2) | | Transparent conductive layer 1b-2 | | Nitrogen-containing layer 1a-3 (light emitting unit 3-3) | | Transparent conductive layer 1b-3 (secondelectrode 7:cathode) | | Result of Evaluation | | | | Other | Remark |
|---|---|---|---|---|---|---|---|---|---|---|---|---|---|---|---|---|---|---|---|---|
| | | Material /film-thickness (nm) | Material | ⊿Eef | Material /production method | Film-thickness (nm) | Material | ⊿Eef | Material /production method | Film-thickness (nm) | Material | ⊿Eef | Material /production method | Film-thickness (nm) | Driving voltage (V) | Brightness unevenness | Relative value of EQE | Relative value of lifetime | | |
| 301 | Fig13 | ITO/180 | Compound A | -0.01 | Ag /deposition | 8.4nm | Compound A | -0.01 | Ag /deposition | 8.4nm | Compound A | -0.01 | Ag /deposition | 8.4nm | 15.9 | 0.59 | 209 | 195 | | Present invention |
| 302 | | | Compound B | -0.03 | | | Compound B | -0.03 | | | Compound B | -0.03 | | | 16.3 | 0.62 | 221 | 218 | | Present invention |
| 303 | | | Compound C | -0.08 | | | Compound C | -0.08 | | | Compound C | -0.08 | | | 12.9 | 0.64 | 264 | 258 | | Present invention |
| 304 | | | Compound D | -0.23 | | | Compound D | -0.23 | | | Compound D | -0.23 | | | 11.5 | 0.89 | 302 | 305 | | Present invention |
| 305 | | | Compound E | -0.33 | | | Compound E | -0.33 | | | Compound E | -0.33 | | | 11.8 | 0.90 | 297 | 293 | | Present invention |
| 306 | | | Compound F | -0.34 | | | Compound F | -0.34 | | | Compound F | -0.34 | | | 10.9 | 0.90 | 304 | 307 | | Present invention |
| 307 | | | | | | 5.5nm | | | | 5.5nm | | | | | 11.2 | 0.82 | 317 | 320 | | Present invention |
| 308 | | | Compound G | -0.41 | | 8.4nm | Compound G | -0.41 | | 8.4nm | Compound G | -0.41 | | | 13.2 | 0.86 | 269 | 267 | | Present invention |
| 309 | Fig13 | ITO/180 | Compound E | -0.33 | MgAg (55%) /co-deposition | 8.4nm | Compound E | -0.33 | MgAg (55%) /co-deposition | 8.4nm | Compound E | -0.33 | MgAg (55%) /co-deposition | 8.4nm | 11.0 | 0.56 | 296 | 237 | | Present invention |
| 310 | | | | | MgAg (45%) /co-deposition | | | | MgAg (45%) /co-deposition | | | | MgAg (45%) /co-deposition | | 11.4 | 0.49 | 296 | 143 | Short lifetime | comparison |
| 311 | | | | | MgAg (10%) /co-deposition | | | | MgAg (10%) /co-deposition | | | | MgAg (10%) /co-deposition | | 11.6 | 0.48 | 289 | 63 | Short lifetime | comparison |
| 312 | Fig13 | ITO/180 | Compound E | -0.33 | ITO/sputtering (0.01nm/sec) | 120nm | Compound E | -0.33 | ITO/sputtering (0.01nm/sec) | 120nm | Compound E | -0.33 | ITO/sputtering (0.01nm/sec) | 120nm | 15.1 | 0.54 | 203 | 162 | leak current occur | comparison |
| 313 | | | | | ITO/sputtering (0.1nm/sec) | | | | ITO/sputtering (0.1nm/sec) | | | | ITO/sputtering (0.1nm/sec) | | - | - | - | - | leak current occur | comparison |
| 314 | Fig14 | ITO/180 | - | - | - | - | - | - | - | - | Compound A | -0.01 | Ag deposition | 8.4nm | 5.2 | 0.67 | 84 | 82 | | comparison |
| 315 | | | - | - | - | - | - | - | - | - | Compound E | -0.33 | | | 4.4 | 0.94 | 100 | 100 | | comparison |

<Evaluation Results of Each Sample of Example 3>

As is clear from Table 3, it has been confirmed that, compared to the organic electroluminescence element of each of Samples 314 and 315 (in which the light emitting unit 3 has a single-layer structure), the organic electroluminescence element of each of Samples 301 to 309 (in which the transparent conductive layers 1b-1, 1b-2, 1b-3 each having silver (Ag) as a main component (50% or more) are each arranged, adjacent to the nitrogen-containing layer 1a, between each two layers of the laminated light emitting units 3-1, 3-2, 3-3 and on the top of the light emitting unit 3-3) is improved in both the external quantum efficiency (EQE) and the lifetime.

Particularly, it has been confirmed that, compared to the organic electroluminescence element of each of Samples 314 and 315 (in which the light emitting unit 3 has a single-layer structure), the organic electroluminescence element of each of Samples 304 to 309 (in which a compound whose effective action energy $\Delta Eef$ satisfies the condition of $\Delta Eef \leq -0.1$ is used as the compound containing nitrogen atom used in the nitrogen-containing layer 1a) is improved by two times or more in both the external quantum efficiency (EQE) and the lifetime.

In contrast, the organic electroluminescence element of each of Samples 310 to 313 (in which a transparent conductive layer 1b not having silver (Ag) as a main component is arranged between each two layers of the laminated light emitting units 3-1, 3-2, 3-3 and on the top of the light emitting unit 3-3) has a lifetime reduced by about 1.5 times, although having high external quantum efficiency (EQE).

Particularly, it has been confirmed that in each of Samples 312 and 313 in which ITO is used as each of the transparent conductive layers 1b-1, 1b-2, 1b-3, when the organic electroluminescence element is driven by a voltage of $\pm 2.5V$, the rectification ratio is high, and leak current occurs. Further, Sample 313, in which the transparent conductive layers 1b-1, 1b-2, 1b-3 were each formed using ITO at a film-forming rate of 0.1 nm/sec, failed to emit light.

Based on the above results, it is confirmed that, with the organic electroluminescence element with a tandem structure using a transparent conductive layer of the configuration of the present invention, it is possible to improving both the luminous efficiency and the lifetime characteristic.

## Example 4

«Production of Two-layer Tandem: Top Emission Type Organic Electroluminescence Element»

As shown in FIG. 15, a two-layer tandem: top emission type organic electroluminescence element was produced as Samples 401 to 413. Further, as shown in FIG. 16, a single-layer structure top emission type organic electroluminescence element was produced as each of Samples 414 and 415. Incidentally, the configuration of main components of each of Samples 401 to 415 is shown in Table 4.

<Production of Samples 401 to 408>

As shown in FIG. 15, first, the substrate 11 made of transparent glass and having a size of 50 mm x 50 mm and a thickness of 0.7 mm was subjected to ultrasonic cleaning with isopropyl alcohol, then dried with dry nitrogen gas, and then subjected to UV ozone cleaning for 5 minutes. Next, the cleaned substrate 11 was transferred to a second vacuum chamber of a vacuum deposition device, and a heating boat having aluminum placed therein and mounted on the second vacuum chamber was electrically heated, and thereby the first electrode 5 composed of aluminum and having a film-thickness of 130 nm was formed at a deposition rate of 0.3 nm/sec. The first electrode 5 was used as an anode.

First, the first layer of light emitting unit 3-1 was formed on the top of the first electrode 5 in the same manner as that for forming the light emitting unit of each of Samples 201 to 208 of Example 2. However, in the production of each of Samples 401 to 408, when forming the electron transporting/injecting layer, which constitutes the uppermost layer of the light emitting unit 3-1, the nitrogen-containing layer 1a-1 was formed by using potassium fluoride and one of the nitrogen-containing compounds shown in Table 4. However, in the production of the Sample 401, instead of the nitrogen-containing layer 1a-1, a layer was formed by using the following Compound X containing no nitrogen.

[Chemical Formula 76]

Compound X

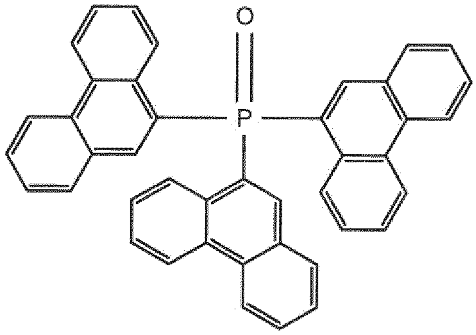

Next, the first layer of transparent conductive layer 1b-1 composed of silver (Ag) was formed in the same manner as that for forming the transparent conductive layer of each of Samples 201 to 208 of Example 2. The film-thickness of the transparent conductive layer 1b-1 was 6.5 nm.

Thereafter, in the production of each of Samples 401 to 408, the aforesaid processes from the process of forming the first layer of light emitting unit 3-1 to the process of forming the first layer of transparent conductive layer 1b-1 were repeatedly performed. By performing the above processes, the second layer of light emitting unit 3-2 and the second layer of transparent conductive layer 1b-2 were formed. However, respective nitrogen-containing compounds shown in Table 4 were used to form the nitrogen-containing layers 1a-2 of the second layer of light emitting units 3-2 of respective Samples 401 to 408. Further, the film-thickness of the second layer of transparent conductive layer 1b-2 was 8.4 nm, and the second layer of transparent conductive layer 1b-2 was formed as the second electrode 7 (as a cathode).

By performing the above processes, a two-layer tandem top emission type organic electroluminescence element was formed on the substrate 11. Thereafter, the organic electroluminescence element was sealed by performing the same process as that of Example 2.

<Production of Samples 409 to 411>

The organic electroluminescence element of each of Samples 409 to 411 was produced in the same manner as that of each of Samples 401 to 408 except that the two transparent conductive layers 1b-1, 1b-2 were each formed by using magnesium (Mg) and silver (Ag) by a co-deposition method, instead of being formed by using silver (Ag). At this time, the transparent conductive layers 1b-1 to 1b-2 were formed so that Mg : Ag = 45 : 55 (atom%) for Sample 409, Mg : Ag = 55 : 45 (atom%) for Sample 410, and Mg : Ag = 90 : 10 (atom%) for Sample 411. The film-thicknesses of two layers were each 8.4 nm. Incidentally, the nitrogen-containing layers 1a-1 to 1a-2 were formed by using Compound D.

<Production of Samples 412 and 413>

The organic electroluminescence element of each of Samples 412, 413 was produced in the same manner as that of each of Samples 401 to 408 except that the two transparent conductive layers 1b-1, 1b-2 were each formed by using ITO by a sputtering method, instead of being formed by using silver (Ag). At this time, the transparent conductive layers 1b-1 and 1b-2 composed of ITO and having a film-thickness of 120 nm were each formed at a film-forming rate of 0.01 nm/sec. Incidentally, in Sample 412, the nitrogen-containing layers 1a-1 and 1a-2 were formed by using Compound E, and in Sample 413, the nitrogen-containing layers 1a-1 and 1a-2 were formed by using Compound C.

<Production of Samples 414 and 415>

As shown in FIG. 16, single-layer structure top emission type organic electroluminescence elements were produced as comparisons. At this time, similar to the process described above for forming the organic electroluminescence element of each of Samples 401 to 408, a light emitting unit 3 was formed on the first electrode 5 (an anode) composed of aluminum, and further, a transparent conductive layer 1b was formed on the light emitting unit 3. Incidentally, in Sample 414, the nitrogen-containing layer 1a, which constituted the uppermost layer of the light emitting unit 3, was formed by using Compound A, and in Sample 415, the nitrogen-containing layer 1a was formed by using Compound F. The film-thickness of the transparent conductive layer 1b was 8.4 nm. The transparent conductive layer 1b was formed as the second electrode 7 (which is a cathode).

Thereafter, the organic electroluminescence element was sealed by performing the same process as that of Example 2.

<Evaluation of Each Sample of Example 4, Part 1>

The luminescent chromaticity of the organic electroluminescence element (the light-emitting panel) produced in each of Samples 401 to 415 was evaluated. As a result, it is confirmed that light is emitted by the organic electroluminescence elements of all Samples 401 to 415 other than Sample 413, and it is also confirmed that, when 2-degree viewing angle front brightness from the side the sealing substrate (i.e., the side of the second electrode 7) is 400 cd/$m^2$, the chromaticity in a CIE color system falls in a range of X=0.45$\pm$0.02 and Y=0.41$\pm$0.02, which shows the luminescent color is white. Incidentally, the brightness was measured using a spectroradiometer CS-1000 (manufactured by Konica Minolta Sensing Inc.).

<Evaluation of Each Sample of Example 4, Part 2>

The driving voltage, the brightness unevenness, the external quantum efficiency (EQE) and the lifetime of the organic electroluminescence element (the light-emitting panel) produced in each of Samples 401 to 415 were measured in the same manner as Example 2. However, the driving voltage, the external quantum efficiency (EQE) and the lifetime were measured when the front brightness on the side of the second electrode 7, arranged on the side opposite to the substrate 11, was 400 cd/$m^2$. Further, the relative value of the external quantum efficiency (EQE) and the relative value of the lifetime of each organic electroluminescence element are calculated in the case where both the value of external quantum efficiency (EQE) and the value of the lifetime of the organic electroluminescence element of Sample 414 are regarded as 100. The results are also shown in the following Table 4.

[Table 4]

| Example 4 <<Two-layer Tandem: Top Emission>> | | | | | | | | | | | | | | | | | |
|---|---|---|---|---|---|---|---|---|---|---|---|---|---|---|---|---|---|
| Sample | Configuration of element | First electrode 5 (anode) | Nitrogen-containing layer 1a-1 (light emitting unit 3-1) | | Transparent conductive layer 1b-1 | | Nitrogen-containing layer 1a-2 (light emitting unit 3-2) | | Transparent conductive layer 1b-2 (second electrode 7:cathode) | | Result of Evaluation | | | | Other | Remark |
| | | Material /film-thickness (nm) | Material | ⊿Eef | Material /production method | Film-thickness (nm) | Material | ⊿Eef | Material /production method | Film thickness (nm) | Driving voltage (V) | Brightness unevenness | Relative value of EQE | Relative value of lifetime | | |
| 401 | Fig15 | Al/130 | Compound X | Not measurable | Ag /deposition | 6.5nm | Compound X | Not measurable | Ag/deposition | 8.4nm | 12.2 | 0.27 | 145 | 122 | | comparison |
| 402 | | | Compound A | -0.01 | | | Compound B | -0.03 | | | 11.5 | 0.64 | 159 | 142 | | Present invention |
| 403 | | | Compound B | -0.03 | | | Compound A | -0.01 | | | 11.4 | 0.62 | 162 | 153 | | Present invention |
| 404 | | | Compound C | -0.08 | | | Compound E | -0.33 | | | 7.6 | 0.91 | 177 | 181 | | Present invention |
| 405 | | | Compound D | -0.23 | | | Compound F | -0.34 | | | 7.5 | 0.93 | 196 | 203 | | Present invention |
| 406 | | | Compound E | -0.33 | | | Compound C | -0.08 | | | 9.7 | 0.78 | 187 | 193 | | Present invention |
| 407 | | | Compound F | -0.34 | | | Compound D | -0.23 | | | 7.7 | 0.89 | 198 | 201 | | Present invention |
| 408 | | | Compound F | -0.34 | | | Compound E | -0.33 | | | 7.9 | 0.95 | 209 | 199 | | Present invention |

(continued)

Example 4 <<Two-layer Tandem: Top Emission>>

| Sample | Configuration of element | First electrode 5 (anode) | Nitrogen-containing layer 1a-1 (light emitting unit 3-1) | | Transparent conductive layer 1b-1 | | Nitrogen-containing layer 1a-2 (light emitting unit 3-2) | | Transparent conductive layer 1b-2 (second electrode 7:cathode) | | Result of Evaluation | | | | Other | Remark |
|---|---|---|---|---|---|---|---|---|---|---|---|---|---|---|---|---|
| | | Material /film-thickness (nm) | Material | ⊿Eef | Material /production method | Film-thickness (nm) | Material | ⊿Eef | Material /production method | Film thickness (nm) | Driving voltage (V) | Brightness unevenness | Relative value of EQE | Relative value of lifetime | | |
| 409 | Fig15 | Al/130 | Compound D | -0.23 | MgAg(55%)/co-deposition | 8.4nm | Compound D | -0.23 | MgAg(55%) /co-deposition | 8.4nm | 8.0 | 0.61 | 203 | 182 | | Present invention |
| 410 | | | | | MgAg(45%)/co-deposition | | | | MgAg(45%) /co-deposition | | 7.8 | 0.52 | 202 | 43 | Short lifetime | comparison |
| 411 | | | | | MgAg(10%) /co-deposition | | | | MgAg (10%) /co-deposition | | 7.8 | 0.49 | 202 | 39 | Short lifetime | comparison |
| 412 | Fig15 | Al/130 | Compound E | -0.33 | ITO/sputtering | 120nm | Compound E | -0.33 | ITO/sputtering | 120nm | 8.1 | 0.56 | 203 | 102 | leak current occu | comparison |
| 413 | | | Compound C | -0.08 | | | Compound C | -0.08 | | | - | - | - | - | leak current occur | comparison |
| 414 | Fig16 | Al/130 | - | - | - | - | Compound A | -0.01 | Ag Deposition | 8.4nm | - | 0.64 | 100 | 100 | | comparison |
| 415 | | | - | - | - | - | Compound F | -0.34 | | | - | 0.95 | 121 | 113 | | comparison |

<Evaluation Results of Each Sample of Example 4>

As is clear from Table 4, it has been confirmed that, compared to the organic electroluminescence element of each of Samples 414 and 415 (in which the light emitting unit 3 has a single-layer structure), the organic electroluminescence element of each of Samples 402 to 409 (in which the transparent conductive layers 1b-1, 1b-2 each having silver (Ag) as a main component (50% or more) are each arranged, adjacent to the nitrogen-containing layer 1a, between the laminated light emitting units 3-1, 3-2 and on the top of the light emitting unit 3-2) is improved by 1.4 times or more in both the external quantum efficiency (EQE) and the lifetime.

Particularly, it has been be confirmed that the organic electroluminescence element of each of Samples 405 to 409 (in which a compound whose effective action energy $\Delta Eef$ satisfies the condition of $\Delta Eef \leq -0.1$ is used as the compound containing nitrogen atom used in the nitrogen-containing layer 1a) has reduced brightness unevenness; and also, compared to the organic electroluminescence element of each of Samples 414 and 415 (in which the light emitting unit 3 has a single-layer structure), the organic electroluminescence element of each of Samples 405 to 409 is improved by nearly two times in both the external quantum efficiency (EQE) and the lifetime.

In contrast, compared to the organic electroluminescence element of each of Samples 414 and 415 (in which the light emitting unit 3 has a single-layer structure), the organic electroluminescence element of each of Samples 410 to 413 (in which the transparent conductive layers 1b-1, 1b-2 not having silver (Ag) as a main component are arranged between the laminated light emitting units 3-1, 3-2 and on the top of the light emitting unit 3-2) has about the same or shorter lifetime, although having high external quantum efficiency (EQE). Particularly, it has been confirmed that in each of Samples 412, 413, in which ITO is used as the transparent conductive layers 1b-1, 1b-2, when the organic electroluminescence element is driven by a voltage of $\pm$2.5V, the rectification ratio is high, and leak current occurs. Also, among these organic electroluminescence elements, the organic electroluminescence element of Sample 413 failed to emit light.

Further, the external quantum efficiency (EQE) and the lifetime of the organic electroluminescence element of Sample 401 (in which, instead of the nitrogen-containing layers 1a-1, 1a-2, layers composed of Compound X containing no nitrogen are formed adjacent to the transparent conductive layers 1b-1, 1b-2) are both improved, however the improvement is only about 1.2 times.

Based on the above results, it is confirmed that, with the organic electroluminescence element with a tandem structure using the transparent conductive layers 1b-1, 1b-2 of the configuration of the present invention, it is possible to improving both the luminous efficiency and the lifetime characteristic.

Example 5

<<Production of Three-color three-layer Tandem: Dual Emission Type Organic Electroluminescence Element>>

As shown in FIG. 17, a three-color three-layer tandem: top emission type organic electroluminescence element was produced as each of Samples 501 to 511. Incidentally, the configuration of main components of each of Samples 501 to 511 is shown in Table 5.

<Production of Samples 501 to 508>

As shown in FIG. 17, similar to Samples 201 to 208 of Example 2, a first electrode 5 composed of ITO and having a film-thickness of 180 nm was formed as an anode, and then the first electrode 5 was cleaned.

Next, similar to Samples 201 to 208 of Example 2, the first layer of light emitting unit 3-1 was formed on the top of the first electrode 5.

However, in the formation of the light emitting layer of each of Samples 501, 503, 505, 507, the host material H4 represented by the structural formula shown before and the phosphorescent compound Ir-9 (red: R) represented by the structural formula shown before were used at a deposition rate of H4 :Ir = 98.9 : 1.1 (by volume) so as to form a red light emitting layer having a film-thickness of 10 nm. Further, in the formation of the light emitting layer of each of Samples 502, 504, 506, 508, the host material H4 represented by the structural formula shown before and the phosphorescent compound Ir-4 (blue: B) represented by the structural formula shown before were used at a deposition rate of H4 : Ir = 92.6 : 7.4 (by volume) so as to form a blue light emitting layer having a film-thickness of 10 nm.

In each of Samples 501 to 508, potassium fluoride and one of the nitrogen-containing compounds shown in Table 5 were used to form the electron transporting/injecting layer (the nitrogen-containing layer 1a-1), which constitutes the uppermost layer of the light emitting unit 3-1.

Next, the first layer of transparent conductive layer 1b-1 composed of silver (Ag) was formed in the same manner as that for forming the transparent conductive layer of each of Samples 201 to 208 of Example 2. The film-thickness of the transparent conductive layer 1b-1 was 6.0 nm.

Thereafter, by repeatedly performing the aforesaid processes from the process of forming the light emitting

unit 3-1 to the process of forming the first layer of transparent conductive layer 1b-1, the second layer of light emitting unit 3-2 was formed on the transparent conductive layer 1b-1, and further, the second layer of transparent conductive layer 1b-2 was formed on the second layer of light emitting unit 3-2. However, in the formation of the light emitting layer of the light emitting unit 3-2, the host material H4 represented by the structural formula shown before and the phosphorescent compound Ir-1 (green: G) represented by the structural formula shown before were used at a deposition rate of H4 : Ir-1 = 95.5 : 4.5 (by volume) so as to form a green light emitting layer having a film-thickness of 10 nm.

Thereafter, by repeatedly performing the aforesaid processes from the process of forming the light emitting unit 3-1 to the process of forming the first layer of transparent conductive layer 1b-1, the third layer of light emitting unit 3-3 was formed on the transparent conductive layer 1b-2, and further, the third layer of transparent conductive layer 1b-3 was formed on the third layer of light emitting unit 3-3. The third layer of transparent conductive layer 1b-3 was formed as the second electrode 7 (which is a cathode). However, in the formation of the light emitting layer of the light emitting unit 3-3, a blue (B) light emitting layer having a film-thickness of 10 nm was formed for each of Samples 501, 503, 505, 507, and a red (R) light emitting layer having a film-thickness of 10 nm was formed for each of Samples 502, 504, 506, 508. The film-thickness of the transparent conductive layer 1b-3 was 8.4 nm.

By performing the above processes, a three-color three-layer tandem dual emission type organic electroluminescence element was formed on the substrate 11. Thereafter, the organic electroluminescence element was sealed by performing the same process as that of Example 2.

Incidentally, in the formation of the organic electroluminescence element, a deposition mask was used to form each of the layers; so that in the substrate 13 which had a size of (5 cm × 5 cm), the central area (4.5 cm × 4.5 cm) was formed as a light-emitting region A, and a light non-emitting region having a width of 0.25 cm was formed surrounding the light-emitting region A. The first electrode, which is an anode, the second electrode 7 (the third layer of transparent conductive layer 1b-3), which is a cathode, the first layer of transparent conductive layer 1b-1, and the second layer of transparent conductive layer 1b-2 were formed into a shape such that the terminal portions of these components were drawn out to the edge of the substrate 11 in a state where the these components were insulated from each other by the light emitting units 3-1 to 3-3.

<Production of Samples 509 to 511>

The organic electroluminescence element of each of Samples 509 to 511 was produced in the same manner as that of each of Samples 501 to 508 except that the three transparent conductive layers 1b-1 to 1b-3 were each formed by using magnesium (Mg) and silver (Ag) by a co-deposition method, instead of being formed by using silver (Ag). At this time, the transparent conductive layers 1b-1 to 1b-3 were formed so that Mg : Ag = 45 : 55 (atom%) for Sample 509, Mg : Ag = 55 : 45 (atom%) for Sample 510, and Mg : Ag = 90 : 10 (atom%) for Sample 511. The film-thickness of the first layer of transparent conductive layer 1b-1 and the film-thickness of the second layer of transparent conductive layer 1b-2 were 6.0 nm, and the film-thickness of the third layer of transparent conductive layer 1b-2 was 8.4 nm. Incidentally, the nitrogen-containing layers 1a-1 to 1a-3, which configured the uppermost layers of the light emitting units 3-1 to 3-3, were formed by using Compound E.

<Evaluation of Each Sample of Example 5, Part 1>

The luminescent chromaticity of the organic electroluminescence element (the light-emitting panel) produced in each of Samples 201 to 511 was evaluated. The results are also shown in Table 5.

[Table 5]

Example 5 <<Three-color three-layer Tandem: Dual Emission>>

| Sample | Configuration of element | First electrode 5 | Light emitting unit 3-1 | | | Transparent conductive layer 1b-1 | | Light emitting unit 3-2 | | | Transparent conductive layer 1b-2 | | Light emitting unit 3-3 | | | Transparent conductive layer 1b-3 (second electrode 7: Cathode) | | Luminescent color | Other | Remark |
|---|---|---|---|---|---|---|---|---|---|---|---|---|---|---|---|---|---|---|---|---|
| | | Material /film-thickness (nm) | Light emitting layer dopant | Nitrogen-containing layer 1a-1 Material | Nitrogen-containing layer 1a-1 ⊿Eef | Material /production method | Film-thickness (nm) | Light emitting layer dopant | Nitrogen-containing layer 1a-2 Material | Nitrogen-containing layer 1a-2 ⊿Eef | Material /production method | Film-thickness (nm) | Light emitting layer dopant | Nitrogen-containing layer 1a-3 Material | Nitrogen-containing layer 1a-3 ⊿Eef | Material /production method | Film-thickness (nm) | | | |
| 501 | Fig17 | ITO/180 | R | Compound B | -0.03 | Ag /deposition | 6.0nm | G | Compound B | -0.03 | Ag /deposition | 6.0nm | B | Compound B | -0.03 | Ag /deposition | 8.4nm | Red | | Present invention |
| 502 | Fig17 | ITO/180 | B | Compound B | -0.03 | Ag /deposition | 6.0nm | G | Compound B | -0.03 | Ag /deposition | 6.0nm | R | Compound B | -0.03 | Ag /deposition | 8.4nm | Yellow | | Present invention |
| 503 | Fig17 | ITO/180 | R | Compound D | -0.23 | Ag /deposition | 6.0nm | G | Compound D | -0.23 | Ag /deposition | 6.0nm | B | Compound D | -0.23 | Ag /deposition | 8.4nm | White | | Present invention |
| 504 | Fig17 | ITO/180 | B | Compound D | -0.23 | Ag /deposition | 6.0nm | G | Compound D | -0.23 | Ag /deposition | 6.0nm | R | Compound D | -0.23 | Ag /deposition | 8.4nm | White | | Present invention |
| 505 | Fig17 | ITO/180 | R | Compound E | -0.33 | Ag /deposition | 6.0nm | G | Compound E | -0.33 | Ag /deposition | 6.0nm | B | Compound E | -0.33 | Ag /deposition | 8.4nm | White | | Present invention |
| 506 | Fig17 | ITO/180 | B | Compound E | -0.33 | Ag /deposition | 6.0nm | G | Compound E | -0.33 | Ag /deposition | 6.0nm | R | Compound E | -0.33 | Ag /deposition | 8.4nm | White | | Present invention |
| 507 | Fig17 | ITO/180 | R | Compound G | -0.41 | Ag /deposition | 6.0nm | G | Compound G | -0.41 | Ag /deposition | 6.0nm | B | Compound G | -0.41 | Ag /deposition | 8.4nm | Faintly-orange | | Present invention |
| 508 | Fig17 | ITO/180 | B | Compound G | -0.41 | Ag /deposition | 6.0nm | G | Compound G | -0.41 | Ag /deposition | 6.0nm | R | Compound G | -0.41 | Ag /deposition | 8.4nm | Faintly-blue | | Present invention |
| 509 | Fig17 | ITO/180 | R | Compound E | -0.33 | MgAg(55%) /co-deposition | 6.0nm | G | Compound E | -0.33 | MgAg(55%) /co-deposition | 6.0nm | B | Compound E | -0.33 | MgAg(55%) /co-deposition | 8.4nm | White | | Present invention |
| 510 | Fig17 | ITO/180 | R | Compound E | -0.33 | MgAg(45%) /co-deposition | 6.0nm | G | Compound E | -0.33 | MgAg(45%) /co-deposition | 6.0nm | B | Compound E | -0.33 | MgAg(45%) /co-deposition | 8.4nm | White | Short lifetime | comparison |
| 511 | Fig17 | ITO/180 | R | Compound E | -0.33 | MgAg(10%) /co-deposition | 6.0nm | G | Compound E | -0.33 | MgAg(10%) /co-deposition | 6.0nm | B | Compound E | -0.33 | MgAg(10%) /co-deposition | 8.4nm | White | Short lifetime | comparison |

It is conformed that, at this time, for each of Samples 503 to 506 and 509 to 511, by applying a driving voltage V1 (=4V) between the first electrode 5 (as an anode) and the transparent conductive layer 1b-1 (as a cathode), applying a driving voltage V2 (=4V) between the transparent conductive layer 1b-1 (as an anode) and the transparent conductive layer 1b-2 (as a cathode), applying a driving voltage V3 (=4V) between the transparent conductive layer 1b-2 (as an anode) and the transparent conductive layer 1b-3 (i.e., the second electrode 7, as a cathode), and respectively fine-adjusting the driving voltages V1, V2, V3 starting from 4V, the luminescent color could be adjusted into a white range having a chromaticity of X=0.45±0.02 and Y=0.41±0.02 in a CIE color system.

On the other hand, for each of Samples 501, 502, 507 and 508, even if the driving voltages V1, V2, V3 were adjusted, white luminescent light having the aforesaid chromaticity range could not be obtained.

Further, it has been confirmed that, for each of Samples 503 to 506 and 509 to 511, various luminescent colors can be obtained by largely raising or dropping the driving voltages V1, V2, V3 respectively.

Example 6

<<Production of Inversely Laminated Two-layer Tandem: Dual Emission Type Organic Electroluminescence Element>>

As shown in FIG. 18, an inversely laminated two-layer tandem: dual emission type organic electroluminescence element was produced as each of Samples 601 to 606. Incidentally, the configuration of main components of each of Samples 601 to 606 is shown in Table 6.

<Production of Samples 601 to 606>

As shown in FIG. 18, similar to Samples 201 to 208 of Example 2, a first electrode 5 composed of ITO was formed as an anode, and then the first electrode 5 was cleaned.

Next, similar to Samples 201 to 208 of Example 2, the first layer of light emitting unit 3-1 was formed on the top of the first electrode 5. However, in the formation of the light emitting layer, the host material H4 represented by the structural formula shown before and the phosphorescent compound Ir-9 (red: R) represented by the structural formula shown before were used at a deposition rate of H4 : Ir = 98.9 : 1.1 (by volume) so as to form a red light emitting layer having a film-thickness of 10 nm. Further, in each of Samples 601 to 606, in the process for forming the electron transporting/injecting layer, which constitutes the uppermost layer of the light emitting unit 3-1, the nitrogen-containing layer 1a-1 was formed by using potassium fluoride and one of the nitrogen-containing compounds shown in Table 6.

Next, the first layer of transparent conductive layer 1b-1 composed of silver (Ag) was formed in the same manner as that for forming the transparent conductive layer of each of Samples 201 to 208 of Example 2. At this time, the film-thickness of the transparent conductive layer 1b-1 was controlled to 8.4 nm.

Thereafter, the second layer of light emitting unit 3-2 was formed on the transparent conductive layer 1b-1. The light emitting unit 3-2 was laminated in reverse order to the first layer of light emitting unit 3-1, i.e., the light emitting unit 3-2 was formed by laminating the electron transporting/injecting layer, the hole blocking layer, the light emitting layer, the hole transporting layer, and the hole injecting material, in this order, from the side of the transparent conductive layer 1b-1. However, in the formation of the light emitting layer, the host material H4 represented by the structural formula shown before and the phosphorescent compound Ir-4 (blue: B) represented by the structural formula shown before were used at a deposition rate of H4 : Ir = 92.6 : 7.4 (by volume) so as to form a blue light emitting layer having a film-thickness of 10 nm.

Next, the second layer of transparent conductive layer 1b-2 composed of silver (Ag) was formed as the second electrode 7 in the same manner as that for forming the transparent conductive layer of each of Samples 201 to 208 of Example 2. Here, the transparent conductive layer 1b-2 (the second electrode 7) was formed as an anode. At this time, the film-thickness of the transparent conductive layer 1b-2 (the second electrode 7) was 7.8 nm.

By performing the above processes, an inversely laminated two-layer tandem dual emission type organic electroluminescence element was formed on the substrate 11. Thereafter, the organic electroluminescence element was sealed by performing the same process as that of Example 2.

Incidentally, in the formation of the organic electroluminescence element, a deposition mask was used to form each of the layers; so that in the substrate 13 which had a size of (5 cm × 5 cm), the central area (4.5 cm × 4.5 cm) was formed as a light-emitting region A, and a light non-emitting region having a width of 0.25 cm was formed surrounding the light-emitting region A. The first electrode (which is an anode), the second electrode 7 (the second layer of transparent conductive layer 1b-2), and the first layer of transparent conductive layer 1b-1 (which is used as a cathode) were formed into a shape such that the terminal portions of these components were drawn out to the edge of the substrate 11 in a state where the these components were insulated from each other by the light emitting units 3-1 and 3-2.

<Evaluation of Each Sample of Example 6>

The brightness on the side of the first electrode 5, the brightness on the side of the second electrode, and the luminescent color of the organic electroluminescence element (the light-emitting panel) produced in each of Samples 601 to 606 were evaluated. At this time, as shown as Table 6, with respect to each organic electroluminescence element, a driving voltage V1 is applied between the first electrode 5 (as an anode) and the transparent conductive layer 1b-1 (as a cathode), and a driving voltage V2 is applied between the transparent conductive layer 1b-1 (as a cathode) and the transparent conductive layer 1b-2 (the second electrode 7: an anode), and thereby light emission was achieved. 2-degree viewing angle front brightnesses were measured as the brightness on the side of the first electrode 5 and the brightness on the side of the second electrode of each of Samples 601 to 606, and the relative values of the brightness on the side of the first electrode 5 and the brightness on the side of the second electrode of each sample were calculated when the front brightness on the side of the first electrode 5 of Sample 601 was regarded as 100. The luminescent color was judged based on color tone determined by visual observation. Incidentally, the brightness was measured using a spectroradiometer CS-1000 (manufactured by Konica Minolta Sensing Inc.). The above results are shown in Table 6.

[Table 6]

| Example 6 <<Inversely Laminated Two-layer Tandem: Dual Emission>> | | | | | | | | | | | | | | | | | |
|---|---|---|---|---|---|---|---|---|---|---|---|---|---|---|---|---|---|
| Sample | Configuration of element | First electrode 5 (anode) | Light emitting unit 3-1 | | | Transparent conductive layer 1b-1 (cathode) | | Light emitting unit 3-2 | Transparent conductive layer 1b-2 (second electrode 7:anode) | | driving voltage (V) | | Result of Evaluation | | | Remark |
| | | Material/film-thickness (nm) | Light emitting layer dopant | Nitrogen-containing layer 1a Material | Nitrogen-containing layer 1a $\Delta$Eef | Material/production method | Film-thickness (nm) | Light emitting layer dopant | Material/production method | Film-thickness (nm) | V1 | V2 | Relative brightness on side of first electrode 5 | Relative brightness on side of second electrode 7 | Luminescent color | |
| 601 | Fig18 | ITO/180 | R | Compound A | -0.01 | Ag/deposition | 8.4 | B | Ag/deposition | 7.8 | 5.0 | 5.0 | 100 | 45 | Red | Present invention |
| 602 | | | | | | | | | | | 7.0 | 3.0 | 134 | 4 | Red | Present invention |
| 603 | | | | | | | | | | | 3.0 | 7.0 | 39 | 78 | Blue-violet | Present invention |
| 604 | | | | Compound D | -0.23 | | | | | | 5.0 | 5.0 | 145 | 126 | White | Present invention |
| 605 | | | | | | | | | | | 7.0 | 3.0 | 203 | 86 | Red | Present invention |
| 606 | | | | | | | | | | | 3.0 | 7.0 | 102 | 159 | Blue-violet | Present invention |

<Evaluation Results of Each Sample of Example 6>

As is clear from Table 6, light emission of each tandem structure organic electroluminescence element having the transparent conductive layer 1b-1 arranged adjacent to the nitrogen-containing layer 1a-1 having the configuration of the present invention was achieved even if the layers constituting the light emitting units 3-1, 3-2 were inversely laminated.

Further, it has been confirmed that the luminescent color of the organic electroluminescence element of each Samples 601 to 606 can be controlled in a wide color range by adjusting the driving voltages V1, V2.

Explanation of Reference Numerals

1a, 1a-1, 1a-2, 1a-3 nitrogen-containing layer
1b, 1b-1, 1b-2, 1b-3 transparent conductive layer (conductive layer)
3, 3-1, 3-2, 3-3 light emitting unit
5 first electrode
7 second electrode
EL-1, EL-1', EL-1", EL-2, EL-3, EL-4, EL-5, EL-6 organic electroluminescence element

**Claims**

**1.** An organic electroluminescence element comprising:

a pair of electrodes;
a plurality of light emitting units stacked one on top of another between the pair of electrodes and each having a light emitting layer formed by using an organic material; and
a conductive layer arranged between the plurality of light emitting units,
wherein at least one of the electrodes and the conductive layer is configured as a transparent conductive layer formed by using silver or an alloy containing silver as a main component, and is arranged adjacent to a nitrogen-containing layer formed by using a compound containing nitrogen atom.

**2.** The organic electroluminescence element according to claim 1, wherein the compound is a compound whose effective action energy ΔEef between itself and silver represented by the following Formula (1) satisfies the following Formula (2):
[Mathematical Expression 1]

$$\Delta Eef = n \times \Delta E / s \quad \cdots\cdots (1)$$

n : Number of nitrogen atom(s) contained in compound and stably bonded with silver
ΔE : Interaction energy between nitrogen atom (N) and silver (Ag)
s : Surface area of compound

$$-0.5 \leqq \Delta Eef \leqq -0.10 \ [\mathrm{kcal/mol \cdot Å^2}] \quad \cdots\cdots (2)$$

**3.** The organic electroluminescence element according to claim 1 or 2, wherein the compound contains a compound represented by the following General Formula (1),

[Chemical Formula 1]

General Formula(1)

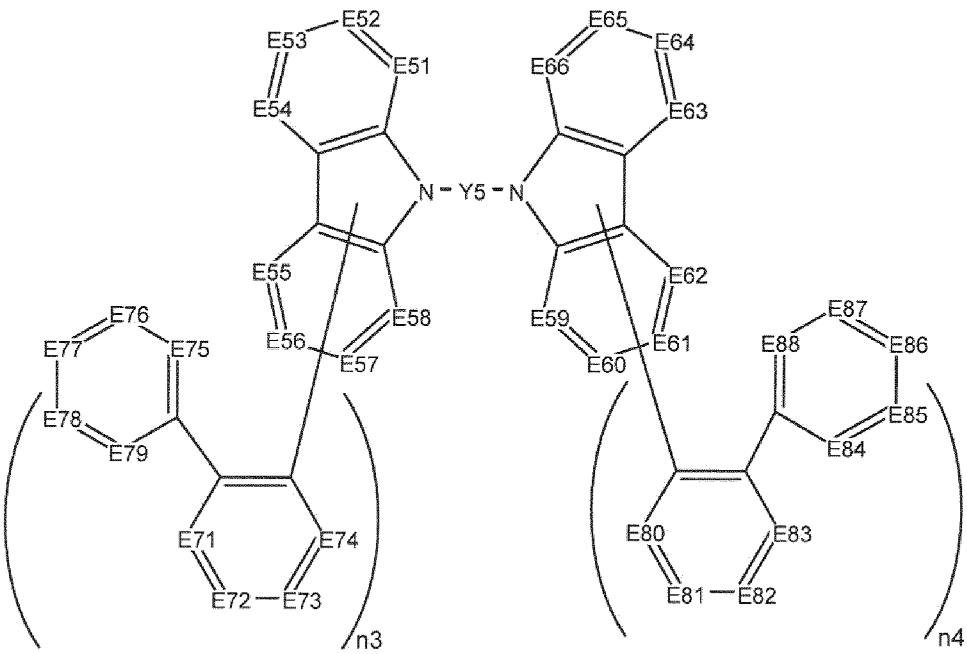

where

Y5 represents a divalent linking group which is an arylene group, a heteroarylene group or a combination of the arylene group and the heteroarylene group;
E51 to E66 and E71 to E88 each represent -C(R3)= or -N=, wherein R3 represents a hydrogen atom or a substituent, and wherein at least one of E71 to E79 and at least one of E80 to E88 each represent -N=; and
n3 and n4 each represent an integer of 0 to 4, wherein the sum of n3 and n4 is an integer of 2 or more.

**4.** The organic electroluminescence element according to claim 1 or 2, wherein the compound contains a compound represented by the following General Formula (2),

[Chemical Formula 2]

General Formula(2)

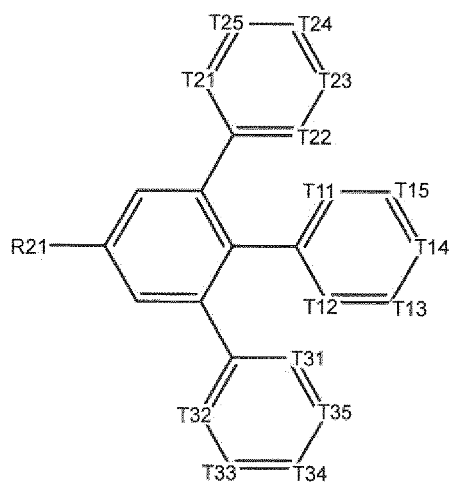

where

R21 represents a substituent;
T11, T12, T21 to T25, and T31 to T35 each represent - C(R22)= or -N=; and
T13 to T15 each represent -C(R22)=,
wherein R22 represents a hydrogen atom (H) or a substituent, at least one of T11 and T12 represents -N=, at least one of T21 to T25 represents -N=, and at least one of T31 to T35 represents -N=.

**5.** The organic electroluminescence element according to any one of claims 1 to 4, wherein the organic electroluminescence element is driven by applying a voltage to the pair of electrodes only.

**6.** The organic electroluminescence element according to any one of claims 1 to 4, wherein the organic electroluminescence element is driven by applying voltages to the pair of electrodes and the conductive layer.

FIG. 1

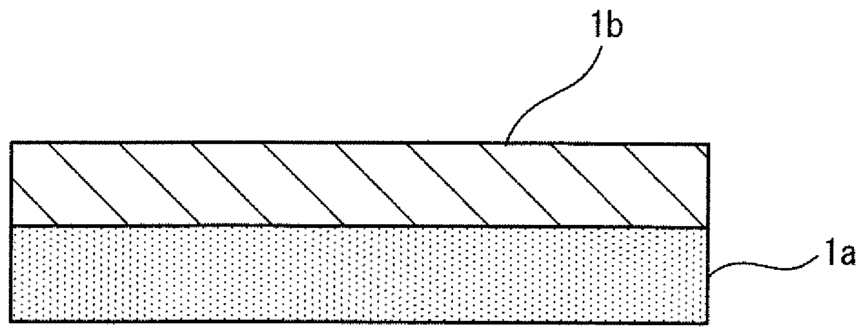


FIG. 2

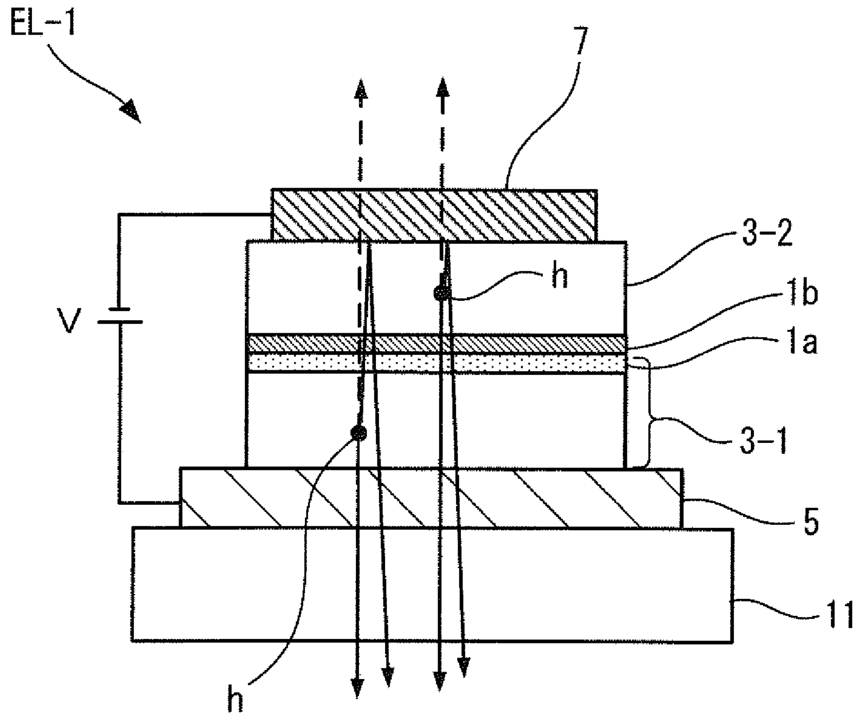

FIG. 3

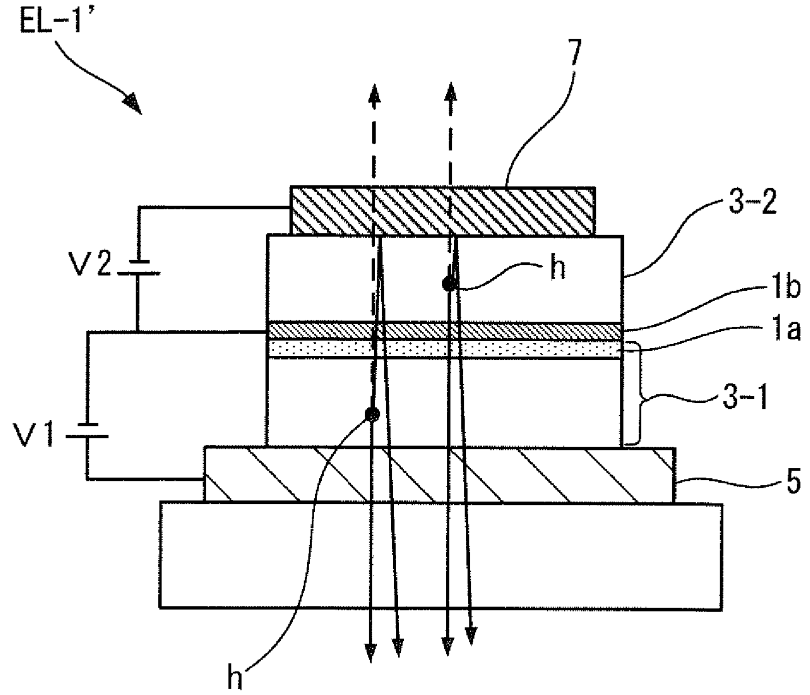


FIG. 4

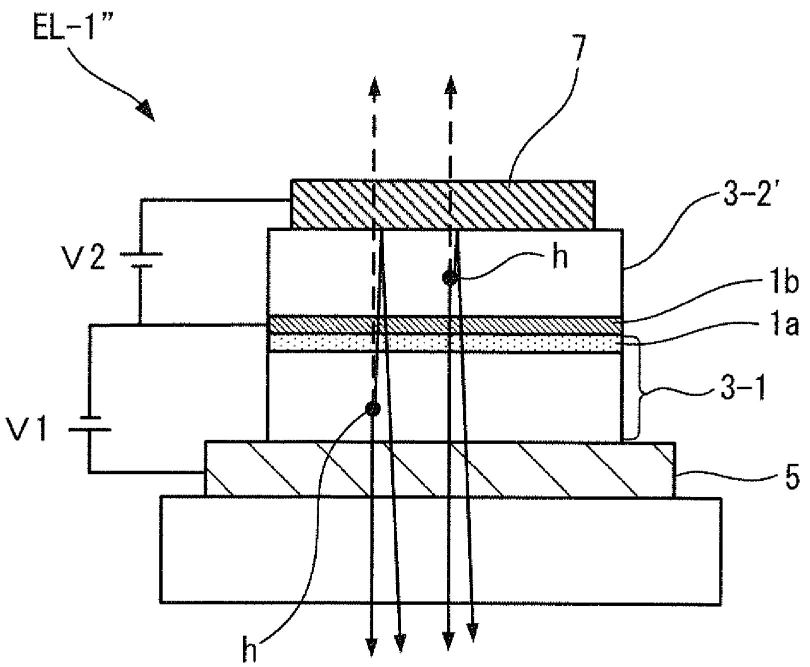

FIG. 5

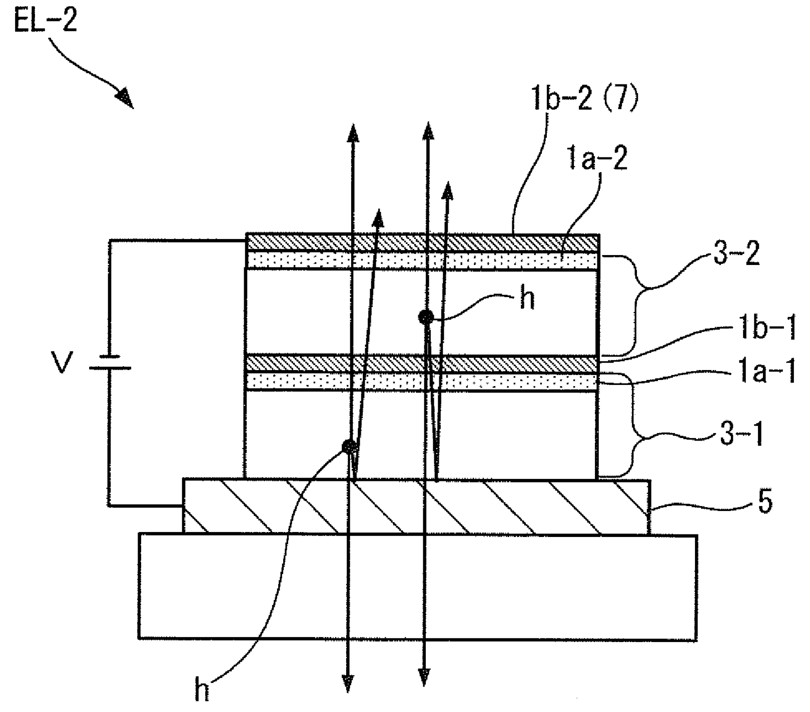
EL-2
1b-2 (7)
1a-2
3-2
h
1b-1
V
1a-1
3-1
5
h

FIG. 6

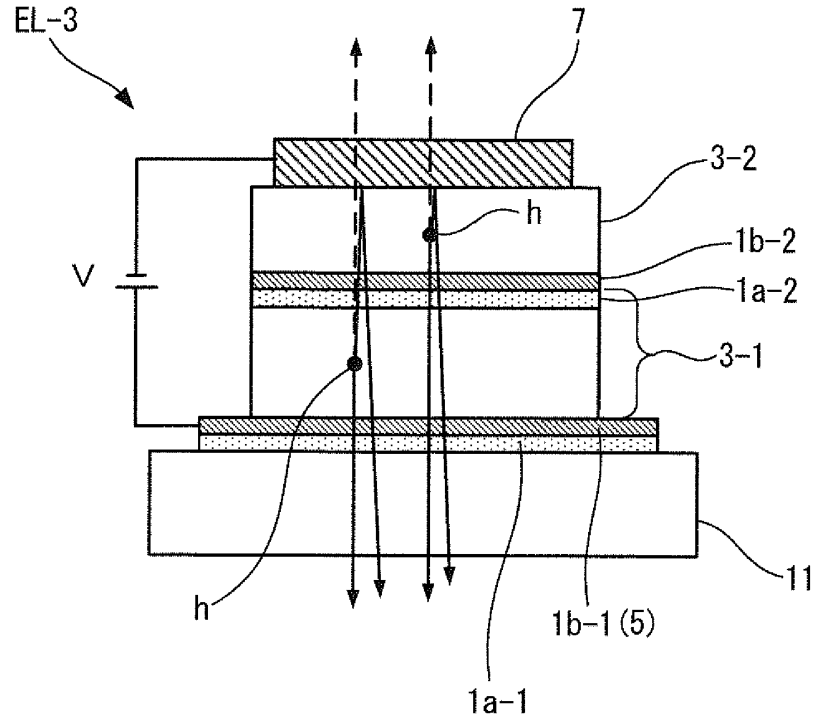
EL-3
7
3-2
h
1b-2
V
1a-2
3-1
11
h
1b-1 (5)
1a-1

FIG. 7

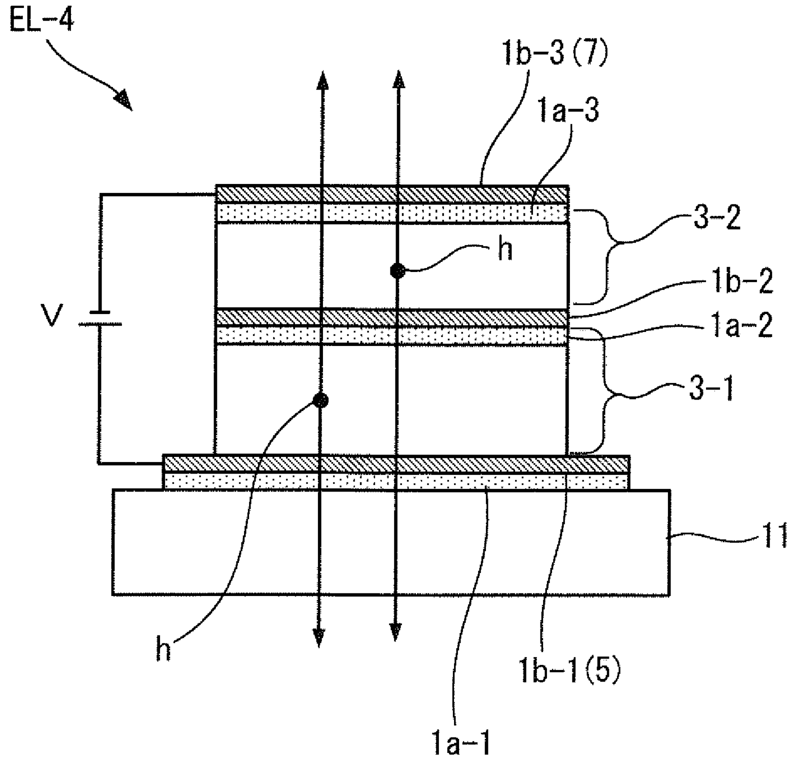
EL-4
1b-3 (7)
1a-3
3-2
h
1b-2
V
1a-2
3-1
11
h
1b-1 (5)
1a-1

FIG. 8

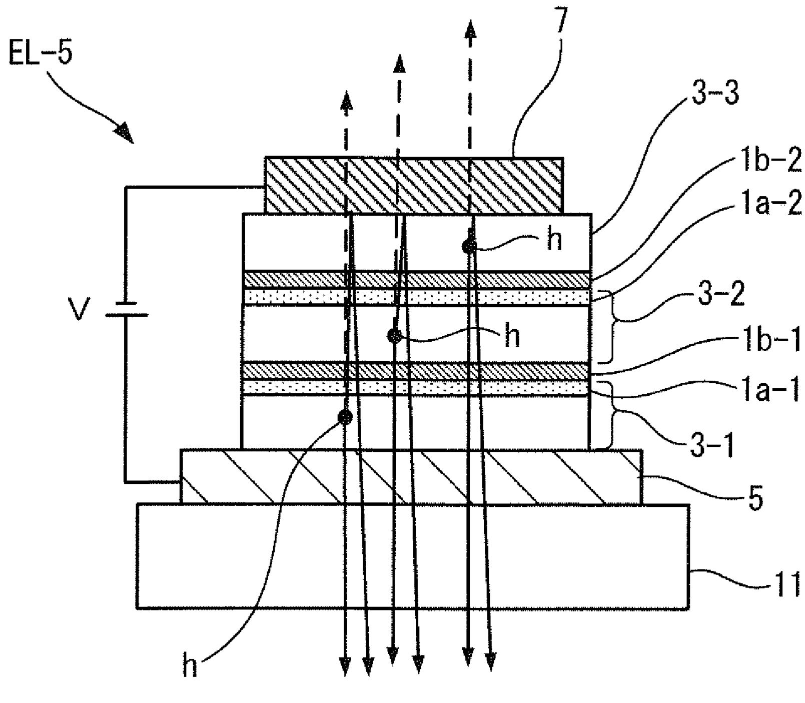
EL-5
7
3-3
1b-2
1a-2
h
V
3-2
h
1b-1
1a-1
3-1
5
11
h

FIG. 9

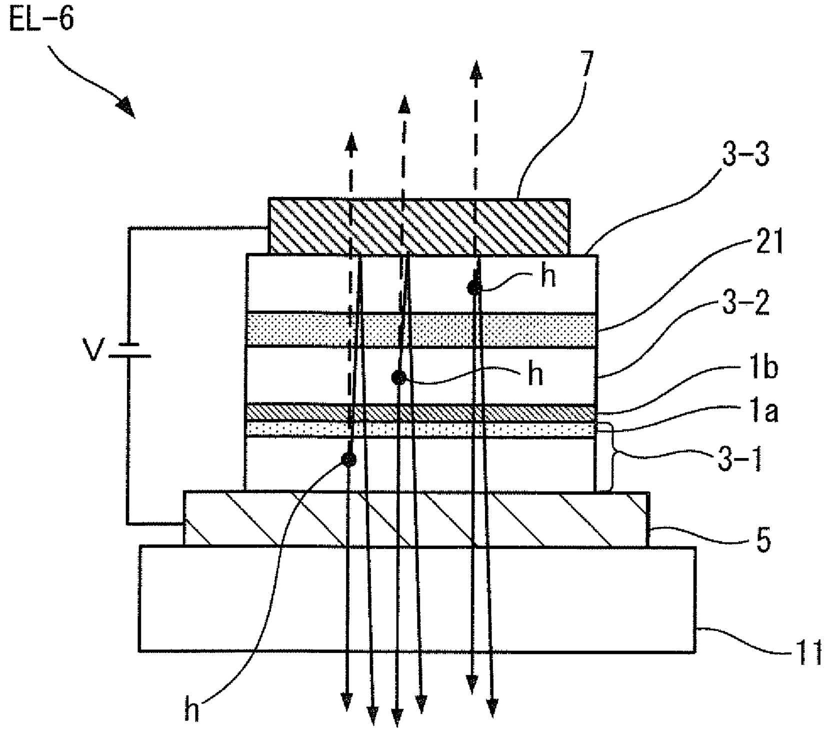
EL-6
7
3-3
21
3-2
1b
1a
3-1
5
11
h
h
h
V

FIG. 10

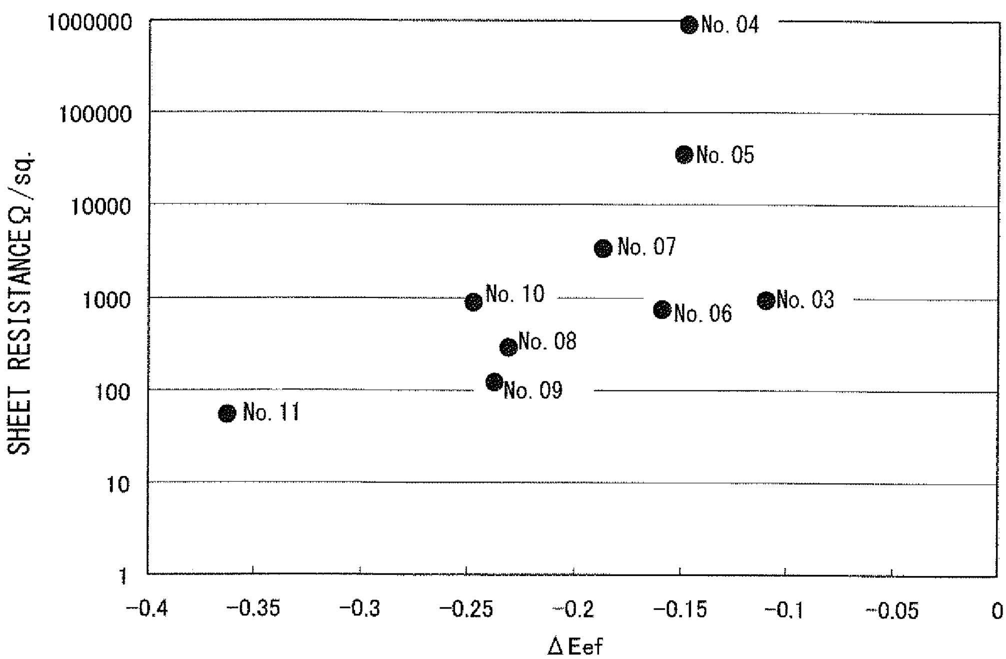
1000000
100000
10000
1000
100
10
1
SHEET RESISTANCE Ω/sq.
No. 04
No. 05
No. 07
No. 10
No. 06
No. 03
No. 08
No. 09
No. 11
-0.4
-0.35
-0.3
-0.25
-0.2
-0.15
-0.1
-0.05
0
ΔEef

FIG. 11

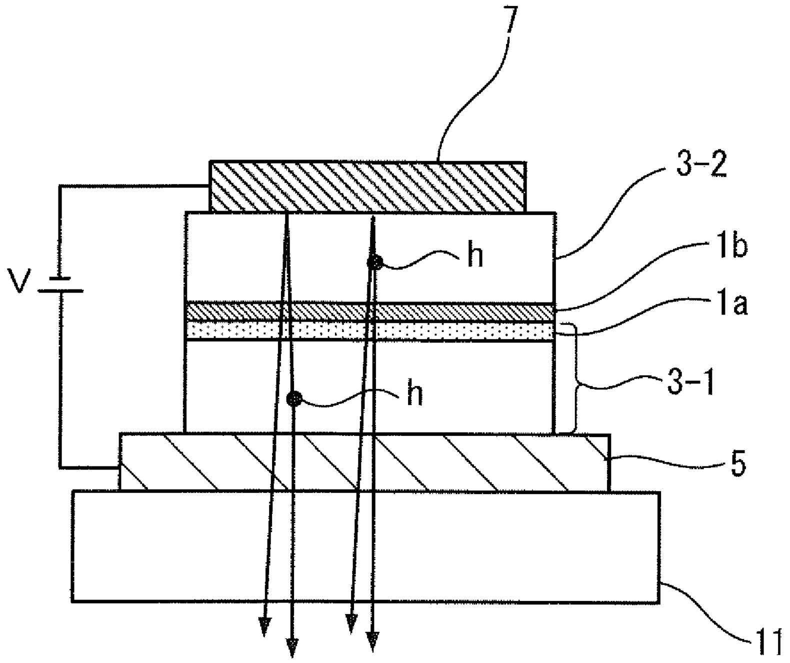


FIG. 12

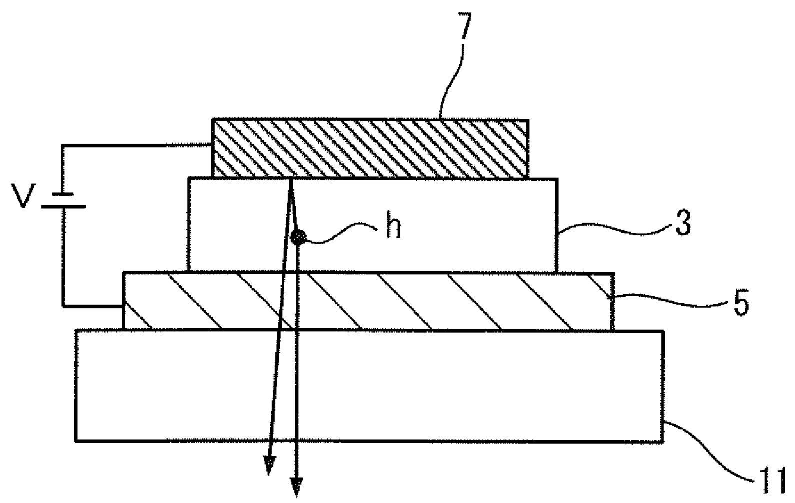

FIG. 13

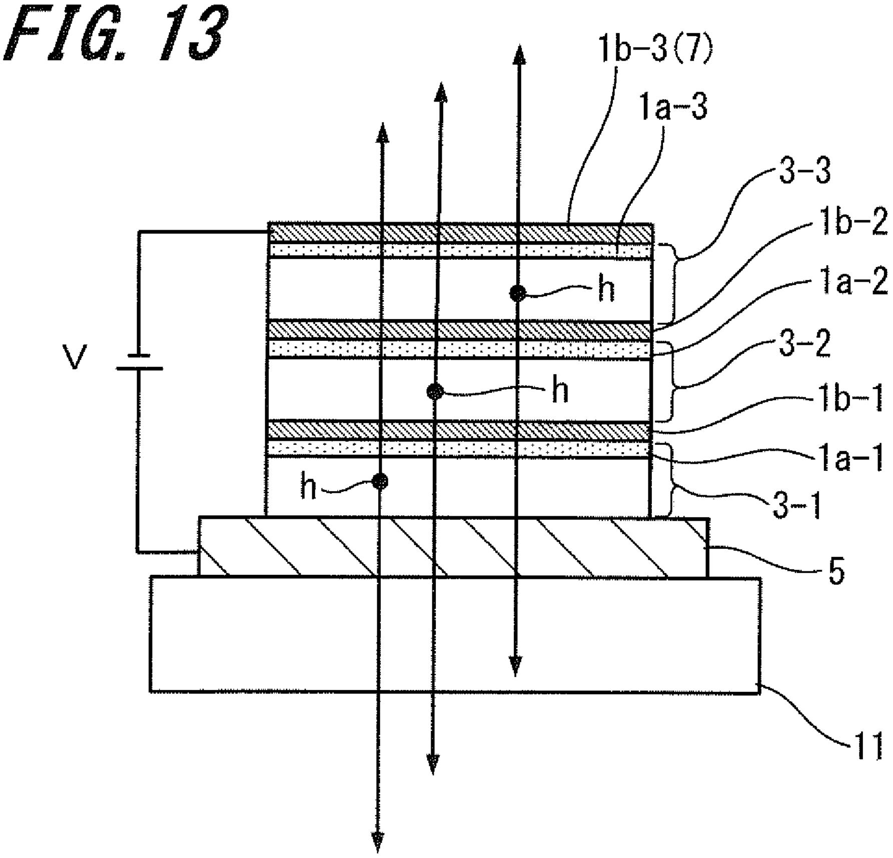


FIG. 14

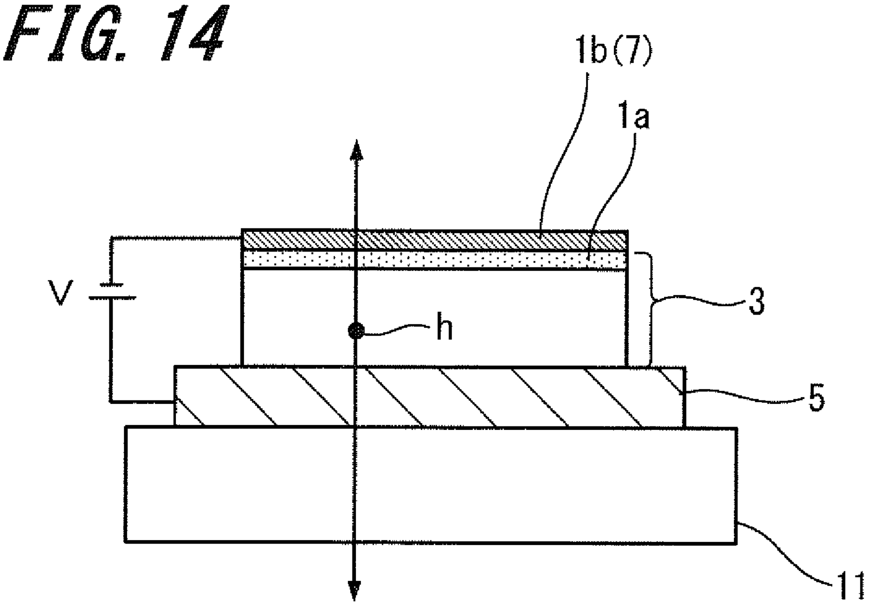

FIG. 15

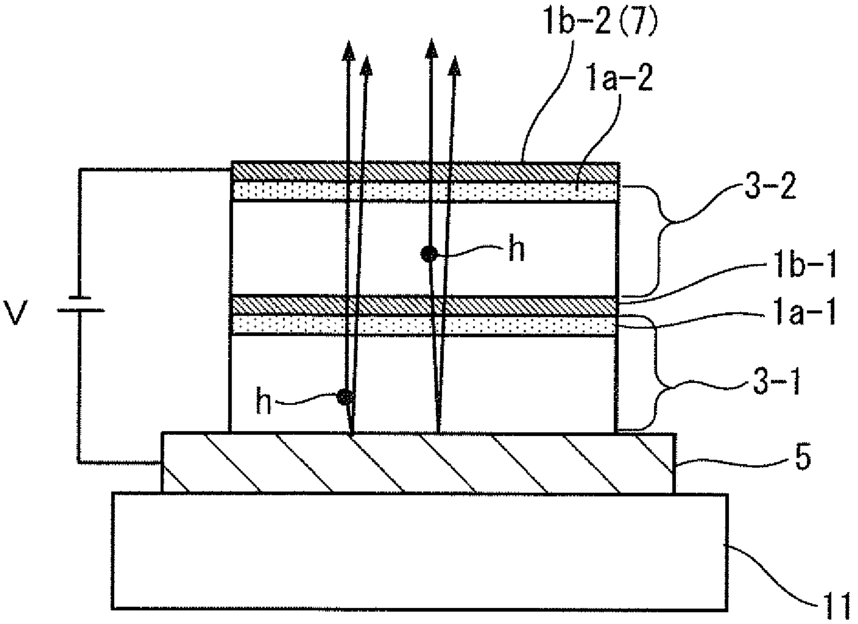


FIG. 16

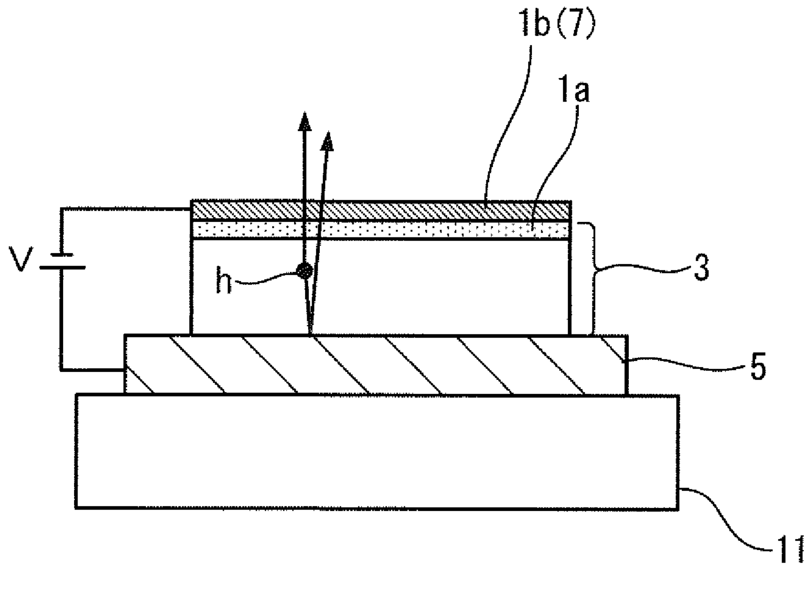

FIG. 17

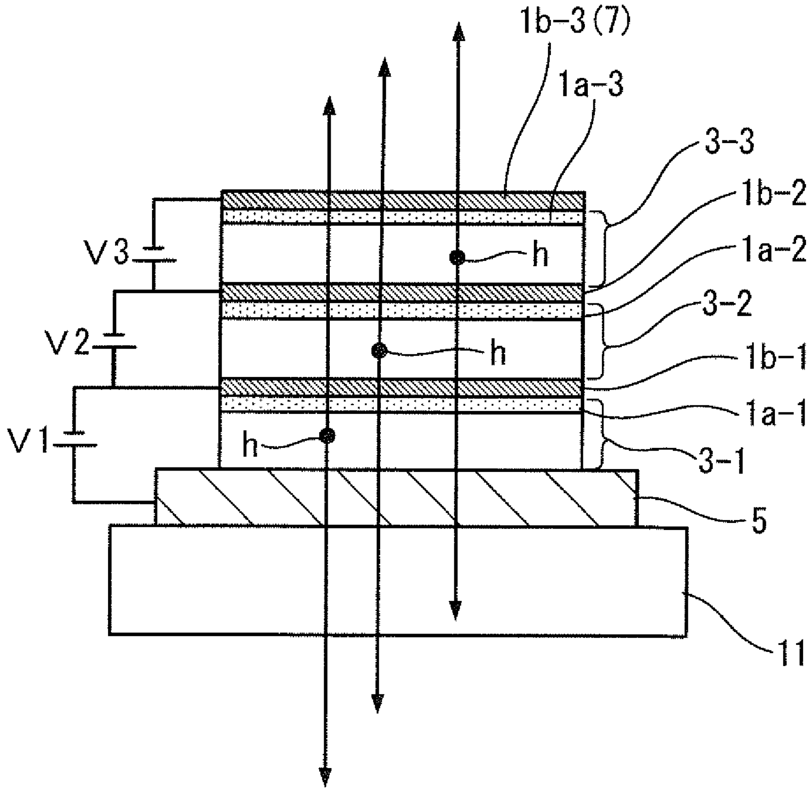
1b-3(7)
1a-3
3-3
1b-2
1a-2
3-2
1b-1
1a-1
3-1
5
11
V3
V2
V1
h
h
h

FIG. 18

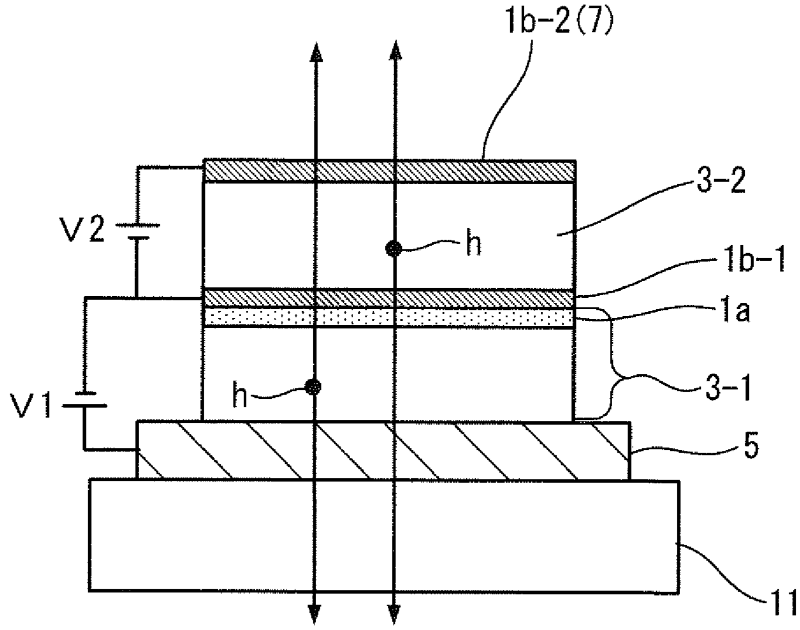
1b-2(7)
3-2
1b-1
1a
3-1
5
11
V2
V1
h
h

## INTERNATIONAL SEARCH REPORT

International application No. PCT/JP2013/056616

A. CLASSIFICATION OF SUBJECT MATTER
*H05B33/12*(2006.01)i, *C07D213/06*(2006.01)i, *C07D213/22*(2006.01)i, *C07D213/53*(2006.01)i, *C07D401/14*(2006.01)i, *C07D409/14*(2006.01)i, *C07D519/00*(2006.01)i, *H01L51/50*(2006.01)i, *H05B33/28*(2006.01)i

According to International Patent Classification (IPC) or to both national classification and IPC

B. FIELDS SEARCHED

Minimum documentation searched (classification system followed by classification symbols)
H05B33/12, C07D213/06, C07D213/22, C07D213/53, C07D401/14, C07D409/14, C07D519/00, H01L51/50, H05B33/28

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2011-77028 A (Hitachi Displays, Ltd.), 14 April 2011 (14.04.2011), paragraphs [0024], [0035], [0036], [0080], [0084], [0086]; fig. 1A & US 2011/0057920 A1 | 1-6 |
| Y | JP 2005-100921 A (Sony Corp.), 14 April 2005 (14.04.2005), paragraphs [0047] to [0049]; fig. 3 & US 2006/0227079 A1 & EP 1667494 A1 & WO 2005/027586 A1 & TW 290441 B & KR 10-1108215 B & KR 10-2006-0071399 A & CN 1871877 A | 1-5 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 04 June, 2013 (04.06.13) | 11 June, 2013 (11.06.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2013/056616

C (Continuation). DOCUMENTS CONSIDERED TO BE RELEVANT

| Category* | Citation of document, with indication, where appropriate, of the relevant passages | Relevant to claim No. |
|---|---|---|
| Y | JP 11-329749 A (TDK Corp.), 30 November 1999 (30.11.1999), paragraphs [0015] to [0018], [0045] to [0057] (Family: none) | 1-6 |
| X<br>Y | JP 2009-224274 A (Panasonic Electric Works Co., Ltd.), 01 October 2009 (01.10.2009), paragraphs [0052] to [0061]; fig. 1 (Family: none) | 1-2,5<br>3-4 |
| Y | JP 2010-251675 A (Konica Minolta Holdings, Inc.), 04 November 2010 (04.11.2010), paragraphs [0071], [0134] & US 2012/0261654 A1 & US 2012/0326601 A1 & US 2009/0284138 A1 & EP 2123733 A2 & EP 2460866 A2 & EP 2479234 A1 | 3 |
| Y | WO 2009/054253 A1 (Konica Minolta Holdings, Inc.), 30 April 2009 (30.04.2009), paragraphs [0127], [0366] (Family: none) | 4 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6337492 B1
- JP 3496681 B
- JP 2003272860 A
- JP 2005135600 A
- JP 2006332048 A
- JP 2005340187 A
- JP 2004068143 A
- JP 2001257076 A
- JP 2002308855 A
- JP 2001313179 A
- JP 2002319491 A
- JP 2001357977 A
- JP 2002334786 A
- JP 2002008860 A
- JP 2002334787 A
- JP 2002015871 A
- JP 2002334788 A
- JP 2002043056 A
- JP 2002334789 A
- JP 2002075645 A
- JP 2002338579 A
- JP 2002105445 A
- JP 2002343568 A
- JP 2002141173 A
- JP 2002352957 A
- JP 2002203683 A
- JP 2002363227 A
- JP 2002231453 A
- JP 2003003165 A
- JP 2002234888 A
- JP 2003027048 A
- JP 2002255934 A
- JP 2002260861 A
- JP 2002280183 A
- JP 2002299060 A
- JP 2002302516 A
- JP 2002305083 A
- JP 2002305084 A
- JP 2002308837 A
- JP 9045479 A
- JP 9260062 A
- JP 8288069 A
- JP 6325871 A
- JP 9017574 A
- JP 10074586 A
- US 5061569 A
- JP 4308688 A
- JP 11251067 A
- JP 4297076 A
- JP 2000196140 A
- JP 2001102175 A
- JP 10270172 A
- JP 11204258 A
- JP 11204359 A


### Non-patent literature cited in the description


- Spectroscopy II of Lecture of Experimental Chemistry. Maruzen Co., Ltd, 1992, vol. 7, 398
- *Organic Letters,* 2001, vol. 3 (16), 2579-2581
- *Inorganic Chemistry,* 1991, vol. 30 (8), 1685-1687
- *J. Am. Chem. Soc.,* 2001, vol. 123, 4304
- *Inorganic Chemistry,* 2001, vol. 40 (7), 1704-1711
- *Inorganic Chemistry,* 2002, vol. 41 (12), 3055-3066
- *New Journal of Chemistry,* 2002, vol. 26, 1171
- *European Journal of Organic Chemistry,* 2004, vol. 4, 695-709
- Electrode Material. Organic EL Element and Front of Industrialization thereof. N. T. S Co., Ltd, 30 November 1998, 123-166
- **J. HUANG.** *Applied Physics Letters,* 2002, vol. 80, 139
- *J. Appl. Phys.,* 2004, vol. 95, 5773
- Organic EL Element and Front of Industrialization thereof. N. T. S Co., Ltd, 30 November 1998, 273